(19) Europäisches Patentamt / European Patent Office / Office européen des brevets

(11) **EP 4 755 886 A1**

(12) **EUROPEAN PATENT APPLICATION**
published in accordance with Art. 153(4) EPC


(43) Date of publication:
**10.06.2026 Bulletin 2026/24**

(21) Application number: **24848336.4**

(22) Date of filing: **01.08.2024**

(51) International Patent Classification (IPC):
***C07D 471/04*** *(2006.01)* ***C07D 519/00*** *(2006.01)*
***C07D 405/12*** *(2006.01)* ***A61K 31/4709*** *(2006.01)*
***A61K 31/437*** *(2006.01)* ***A61P 35/00*** *(2006.01)*

(52) Cooperative Patent Classification (CPC):
**A61K 31/437; A61K 31/4709; A61P 35/00; C07D 405/12; C07D 471/04; C07D 519/00**

(86) International application number:
**PCT/CN2024/109119**

(87) International publication number:
**WO 2025/026381 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.08.2023 CN 202310967490**
**26.12.2023 CN 202311824824**
**02.02.2024 CN 202410153280**
**15.05.2024 CN 202410605782**



(71) Applicant: **Chengdu Chipscreen Pharmaceutical Ltd.**
**Chengdu, Sichuan 610000 (CN)**

(72) Inventors:
- **LIU, Gang**
  **Chengdu, Sichuan 610000 (CN)**
- **XIA, Shanghua**
  **Chengdu, Sichuan 610000 (CN)**
- **DONG, Wenbo**
  **Chengdu, Sichuan 610000 (CN)**
- **LI, Jian**
  **Chengdu, Sichuan 610000 (CN)**
- **WANG, Tingting**
  **Chengdu, Sichuan 610000 (CN)**
- **XIE, Honglei**
  **Chengdu, Sichuan 610000 (CN)**
- **ZHANG, Ping**
  **Chengdu, Sichuan 610000 (CN)**
- **MAO, Xuhua**
  **Chengdu, Sichuan 610000 (CN)**
- **SHAN, Song**
  **Chengdu, Sichuan 610000 (CN)**
- **PAN, Desi**
  **Chengdu, Sichuan 610000 (CN)**
- **LU, Xianping**
  **Chengdu, Sichuan 610000 (CN)**

(74) Representative: **Lavoix**
**Bayerstraße 83**
**80335 München (DE)**


(54) **HETEROCYCLYLALKYNYL SUBSTITUTED AMIDE DERIVATIVE, AND PREPARATION METHOD THEREFOR AND USE THEREOF**


(57) The present invention relates to a heterocyclylalkynyl substituted amide derivative as shown in a formula (I), and a preparation method therefor and the use thereof. The present invention also relates to a pharmaceutical composition comprising the compound as an active ingredient and the use of the compound or pharmaceutical composition for treating and/or preventing related diseases mediated by PRMT5.


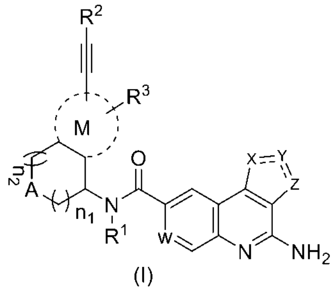

(I)

EP 4 755 886 A1

## Description

### TECHNICAL FIELD

The present disclosure relates to the field of pharmaceutical chemistry, and in particular, to a heterocycloalkynyl-substituted amide derivative, a preparation method therefor and use thereof.

### BACKGROUND

Epigenetic regulation of gene expression plays an important biological role in protein maturation and cell differentiation, and plays an important role in many human diseases. Arginine guanido methylation catalyzed by protein arginine methyltransferase (PRMT) is a common post-translational modification in eukaryotic cells. It affects various biological processes such as cell signaling, gene transcription, mRNA translation, and DNA recombination and repair (Cell Mol. Life Sci. 2015. 72(11): 2041-2059).

PRMT5 is one of the members of the PRMT family, and its mediated methylation plays a crucial role in maintaining normal intracellular environmental balance. However, an increasing number of studies have shown that the abnormal expression of PRMT5 is associated with the development of various tumors. PRMT5 is overexpressed in a variety of tumors, and the pathogenesis varies in different tumors (Cell Mol. Life Sci. 2015. 72(11): 2041-2059).

Homozygous deletion of cancer suppressor genes is a key driver of tumorigenesis. The deletion of the cancer suppressor gene CDKN2A located on human chromosome 9p21 is one of the genes with the highest mutation frequency in tumors, and the incidence rate is 15%. Due to its close proximity to CDKN2A, the methylthioadenosine phosphorylase gene (MTAP) is often concomitantly deleted in tumors, and it plays an important role in the methionine and adenine salvage pathways (Cell Reports, 2016, 15: 574-587). The deletion of MTAP may lead to the accumulation of its substrate methylthioadenosine (MTA). Since MTA is structurally similar to S-adenosylmethionine (SAM), it can selectively compete with SAM for binding to PRMT5, inhibit part of the activity of PRMT5, and sensitize further inhibition of PRMT5, i.e., synthetic lethality (Science, 2016, VOL 351 ISSUE 6278: 1214-1217).

However, PRMT5 is a known and necessary gene. The knockout or siRNA silencing of PRMT5 in normal tissues will lead to abnormal physiological functions, such as hematocytopenia, infertility, skeletal muscle loss, and cardiac hypertrophy (Journal of Clinical Investigation, 2015, 125(9): 3532-44). At present, none of the PRMT5 inhibitors in clinical stage can cause synthetic lethality in the context of MTAP deletion. Thus, new strategies are needed to exploit the metabolic fragility caused by MTAP deletion.

The development of small-molecule inhibitors targeting PRMT5 MTA enables preferential targeting of MTAP-deficient tumor cells. Because normal cells exhibit no deletion of MTAP and maintain a relatively low MTA concentration, these inhibitors do not show significant inhibitory effect on normal cells, thereby increasing the therapeutic index (AACR Annual Meeting, 2021, Abstract LB003) and providing a new strategy for tumor treatment.

### SUMMARY

#### Problems to be Solved by the Present Disclosure:

Although patent applications related to some PRMT5 inhibitors have been disclosed, new compounds still need to be further developed, considering the large market demand for MTAP-/-cancer patients and the insufficient clinical effects of existing PRMT5 inhibitors. Through continuous efforts, the inventors of the present application have designed compounds having a structure represented by general formula (I) and found that the compounds having such a structure exhibit an excellent inhibitory effect and action on PRMT5, demonstrating great application prospects.

#### Solutions to Problems:

To solve the above problems, the inventors of the present application have carried out intensive research and found that a class of heterocycloalkynyl-substituted amide derivatives can achieve the desired objective, thus resulting in the present disclosure.

To achieve the above objective, the present disclosure provides the following technical solutions:

A compound represented by formula I or a tautomer, a stereoisomer, a pharmaceutically acceptable salt, or a deuterated compound thereof:

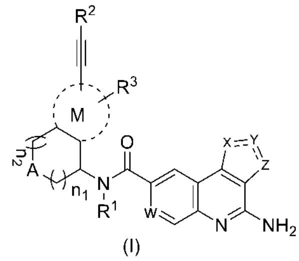

(I)

wherein

$\overline{---}$ is selected from a single bond and a double bond, or $\overline{---}$ is absent;
when $\overline{---}$ is a single bond or a double bond, X, Y, and Z are independently selected from $CR^4R^5$, $NR^6$, O, N, S, and $CR^4$;
when $\overline{---}$ is absent, Y is absent, X is hydrogen, and Z is $CR^4R^5$;
W is selected from $CR^7$ and N;
A is selected from $CR^8R^9$, $NR^8$, and O;
ring M is selected from 5-10 membered aryl and 5-10 membered heteroaryl, wherein the 5-10 membered heteroaryl contains 1-4 heteroatoms selected from N, O, and S;
$R^3$, $R^4$, $R^5$, $R^6$, $R^7$, $R^8$, and $R^9$ are independently selected from hydrogen, deuterium, halogen, cyano, $C_{1-6}$ alkyl, $C_{1-6}$ alkoxy, $C_{3-6}$ cycloalkyl, and $C_{3-6}$ cycloalkoxy, wherein the $C_{1-6}$ alkyl and $C_{1-6}$ alkoxy are unsubstituted or substituted with one or more $R_i$, and the $C_{3-6}$ cycloalkyl and $C_{3-6}$ cycloalkoxy are unsubstituted or substituted with one or more $R_j$;
$R^1$ is selected from $C_{1-6}$ alkyl, $C_{3-10}$ cycloalkyl-($C_{1-6}$ alkyl)$_m$-, 4-10 membered heterocyclyl-($C_{1-6}$ alkyl)$_n$-, 5-10 membered heteroaryl-($C_{1-6}$ alkyl)$_p$-, and 5-10 membered aryl-($C_{1-6}$ alkyl)$_q$-, wherein n = 0 or 1, m = 0 or 1, p = 0 or 1, and q = 0 or 1; the heterocyclyl and heteroaryl each independently contain 1-4 ring heteroatoms selected from O, N, and S, wherein the $C_{1-6}$ alkyl is unsubstituted or substituted with one or more $R_i$, the $C_{3-10}$ cycloalkyl is unsubstituted or substituted with one or more $R_j$, the 4-10 membered heterocyclyl is unsubstituted or substituted with one or more $R_k$, and the 5-10 membered aryl and 5-10 membered heteroaryl are unsubstituted or are each independently substituted with one or more $R_l$;
$R^2$ is selected from 5-10 membered heteroaryl and 4-10 membered heterocyclyl, wherein the 5-10 membered heteroaryl and 4-10 membered heterocyclyl each independently contain 1-4 heteroatoms selected from N, O, and S, and the 5-10 membered heteroaryl and 4-10 membered heterocyclyl are unsubstituted or substituted with one or more identical or different $R^b$;
$n_1$ and $n_2$ are each independently 0, 1, or 2;
each $R_i$, $R_j$, $R_k$, and $R_l$ is independently selected from hydroxy, deuterium, halogen, cyano, $C_{1-6}$ alkyl optionally substituted with one or more groups selected from halogen, cyano, and hydroxy, $C_{1-6}$ alkoxy optionally substituted with one or more groups selected from halogen, cyano, hydroxy, -NR'R", and $C_{1-6}$ alkoxy, $C_{3-10}$ cycloalkyl, and 5-10 membered heteroaryl optionally substituted with one or more $C_{1-6}$ alkyl, the 5-10 membered heteroaryl containing 1-4 heteroatoms selected from N, O, and S, wherein R' and R" are each independently selected from H, $C_{1-6}$ alkyl, and halogenated $C_{1-6}$ alkyl, or R' and R", together with the nitrogen atom linked thereto, form 4-8 membered heterocyclyl, the 4-8 membered heterocyclyl containing 1-4 heteroatoms selected from N, O, and S;
each $R^b$ is independently selected from hydroxy, deuterium, cyano, halogen, -NR'R", $C_1$-$C_6$ alkyl, $C_{1-6}$ alkoxy, and $C_3$-$C_6$ cycloalkyl, wherein the $C_1$-$C_6$ alkyl, $C_1$-$C_6$ alkoxy, and $C_3$-$C_6$ cycloalkyl are unsubstituted or are each independently substituted with one or more substituents selected from halogen, hydroxy, cyano, and $C_1$-$C_6$ alkoxy;
in -NR'R", R' and R" are each independently selected from hydrogen, $C_{1-6}$ alkyl, and halogenated $C_{1-6}$ alkyl, or R' and R", together with the nitrogen atom linked thereto, form 4-8 membered heterocyclyl, the 4-8 membered heterocyclyl containing 1-4 heteroatoms selected from N, O, and S.

In some specific embodiments, ring M is selected from 5-6 membered aryl and 5-6 membered heteroaryl.

In the present disclosure, unless otherwise specified, each occurrence of the 5-10 membered heteroaryl preferably means that each 5-10 membered heteroaryl contains 1-4 heteroatoms selected from N, O, and S; each occurrence of the 5-6 membered heteroaryl preferably means that each 5-6 membered heteroaryl contains 1-3 heteroatoms selected from N, O, and S.

In some specific embodiments, ring M is selected from phenyl, pyridyl, pyrazinyl, pyrimidinyl, pyridazinyl, pyrazolyl, imidazolyl, triazolyl, pyrrolyl, furanyl, oxazolyl, isoxazolyl, and thiazolyl.

In some specific embodiments, ring M is selected from phenyl and pyridyl.

In some specific embodiments, when $\overline{---}$ is a single bond or a double bond, X, Y, and Z are independently selected from $CR^4R^5$, $NR^6$, O, N, S, and $CR^4$.

In some specific embodiments, when $\overline{---}$ is absent, Y is absent, X is hydrogen, and Z is $CR^4R^5$.

In some specific embodiments, A is selected from O.

In some specific embodiments, $n_1$ and $n_2$ are each independently 0 or 1.

In some specific embodiments, the group

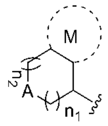

is selected from

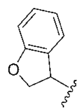 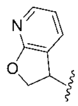 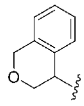 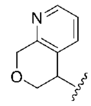.

In some specific embodiments, the group

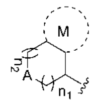

is selected from

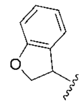 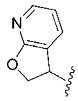 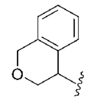 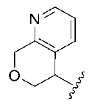.

In some specific embodiments, the group

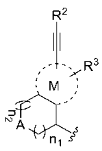

is selected from

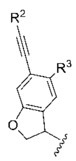 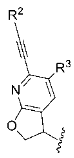 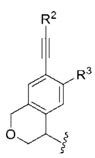 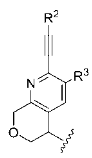.

In some specific embodiments, the group

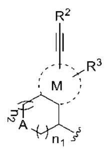

is selected from

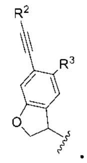.

In some specific embodiments, $R^2$ is selected from 5-6 membered heteroaryl and 4-6 membered heterocyclyl, wherein the 5-6 membered heteroaryl and 4-6 membered heterocyclyl each independently contain 1-3 heteroatoms selected from N, O, and S, and the 5-6 membered heteroaryl and 4-6 membered heterocyclyl are unsubstituted or are each independently substituted with one or more identical or different $R^b$.

In some specific embodiments, $R^2$ is selected from 5-6 membered heteroaryl, 4-6 membered heterocyclyl, and 5-6 membered heteroaryl-fused 5-6 membered heterocyclyl, wherein the 5-6 membered heteroaryl, 4-6 membered heterocyclyl, and 5-6 membered heterocyclyl each independently contain 1, 2, or 3 heteroatoms selected from N, O, and S, the 5-6 membered heteroaryl, 4-6 membered heterocyclyl, and 5-6 membered heteroaryl-fused 5-6 membered heterocyclyl are unsubstituted or are each independently substituted with one or more identical or different $R^b$.

In some specific embodiments, $R^2$ is selected from 5-6 membered heteroaryl, wherein the 5-6 membered heteroaryl contains 1-3 heteroatoms selected from N, O, and S, and the 5-6 membered heteroaryl is unsubstituted or substituted with one or more identical or different $R^b$.

In some specific embodiments, $R^2$ is selected from pyrazolyl, imidazolyl, thiazolyl, isothiazolyl, oxazolyl, isoxazolyl, pyridyl, pyrazinyl, pyrimidinyl, and pyridazinyl, which are unsubstituted or are each independently substituted with 1, 2, or 3 $R^b$.

In some specific embodiments, $R^2$ is selected from 5-6 membered heteroaryl, wherein the 5-6 membered heteroaryl contains 1, 2, or 3 N heteroatoms, and the 5-6 membered heteroaryl is unsubstituted or substituted with one or more identical or different $R^b$.

In some specific embodiments, $R^2$ is selected from pyrazolyl, imidazolyl, and pyridyl, which are unsubstituted or are each independently substituted with 1, 2, or 3 $R^b$.

In some specific embodiments, $R^2$ is selected from

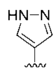 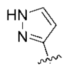 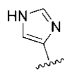 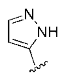 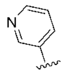,

which are unsubstituted or independently substituted with 1, 2, or 3 $R^b$.

In some specific embodiments, $R^2$ is selected from 5-membered heteroaryl, wherein the 5-membered heteroaryl contains 1-3 heteroatoms selected from N, O, and S, and the 5-membered heteroaryl is unsubstituted or substituted with 1, 2, or 3 $R^b$.

In some specific embodiments, $R^2$ is selected from pyrazolyl, imidazolyl, thiazolyl, isothiazolyl, oxazolyl, and isoxazolyl, which are unsubstituted or substituted with 1, 2, or 3 $R^b$.

In some specific embodiments, $R^2$ is selected from 5-membered heteroaryl, wherein the 5-membered heteroaryl contains 1-2 heteroatoms selected from N and S, and the 5-membered heteroaryl is unsubstituted or substituted with 1, 2, or 3 $R^b$.

In some specific embodiments, $R^2$ is selected from pyrazolyl, imidazolyl, thiazolyl, and isothiazolyl, which are unsubstituted or are each independently substituted with 1, 2, or 3 $R^b$.

In some specific embodiments, $R^2$ is selected from 5-membered heteroaryl, wherein the 5-membered heteroaryl contains 1, 2, or 3 N heteroatoms, and the 5-membered heteroaryl is unsubstituted or substituted with 1, 2, or 3 identical or different $R^b$.

In some specific embodiments, $R^2$ is selected from pyrazolyl and imidazolyl, which are unsubstituted or are each independently substituted with 1, 2, or 3 $R^b$.

In some specific embodiments, $R^2$ is selected from pyrazolyl, wherein the pyrazolyl is unsubstituted or independently substituted with 1, 2, or 3 $R^b$.

In some specific embodiments, $R^2$ is selected from

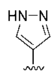,

wherein the

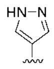

is unsubstituted or independently substituted with 1, 2, or 3 $R^b$.

In some specific embodiments, $R^2$ is selected from

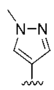 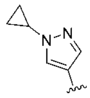 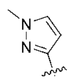 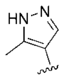 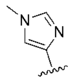 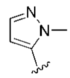 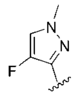 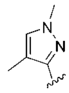

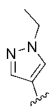 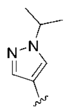 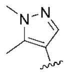 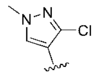 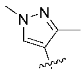 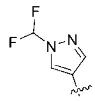 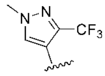 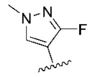 .

In some specific embodiments, $R^2$ is selected from 6-membered heteroaryl, wherein the 6-membered heteroaryl contains 1, 2, or 3 heteroatoms selected from N, O, and S, and the 6-membered heteroaryl is unsubstituted or substituted with one or more identical or different $R^b$.

In some specific embodiments, $R^2$ is selected from 6-membered heteroaryl, wherein the 6-membered heteroaryl contains 1, 2, or 3 N heteroatoms, and the 6-membered heteroaryl is unsubstituted or substituted with 1, 2, or 3 identical or different $R^b$.

In some specific embodiments, $R^2$ is selected from pyridyl, wherein the pyridyl is unsubstituted or substituted with 1, 2, or 3 identical or different $R^b$.

In some specific embodiments, $R^2$ is selected from

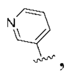,

wherein the

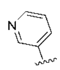

is unsubstituted or substituted with 1, 2, or 3 identical or different $R^b$.

In some specific embodiments, $R^2$ is selected from

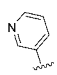 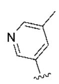 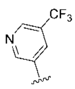 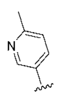 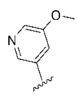 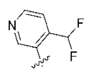 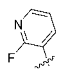 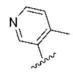 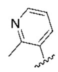 .

In some specific embodiments, $R^2$ is selected from 5-6 membered heteroaryl-fused 5-6 membered heterocyclyl, wherein the 5-6 membered heteroaryl and 5-6 membered heterocyclyl each independently contain 1, 2, or 3 heteroatoms selected from N, O, and S, and the 5-6 membered heteroaryl-fused 5-6 membered heterocyclyl is unsubstituted or substituted with one or more identical or different $R^b$.

In some specific embodiments, $R^2$ is selected from 5-6 membered heteroaryl-fused 5-6 membered heterocyclyl, wherein the 5-6 membered heteroaryl and 5-6 membered heterocyclyl each independently contain 1, 2, or 3 N heteroatoms, and the 5-6 membered heteroaryl-fused 5-6 membered heterocyclyl is unsubstituted or substituted with one or more identical or different $R^b$.

In some specific embodiments, $R^2$ is selected from 5-membered heteroaryl-fused 6-membered heterocyclyl, wherein the 5-membered heteroaryl and 6-membered heterocyclyl each independently contain 1, 2, or 3 N heteroatoms, and the 5-membered heteroaryl-fused 6-membered heterocyclyl is unsubstituted or substituted with one or more identical or different $R^b$.

In some specific embodiments, $R^2$ is selected from imidazolyl-fused piperazinyl, wherein the imidazolyl-fused piperazinyl is unsubstituted or substituted with 1, 2, or 3 identical or different $R^b$.

In some specific embodiments, $R^2$ is selected from

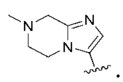.

In some specific embodiments, $R^2$ is selected from 5-6 membered heteroaryl-fused 5-6 membered heterocyclyl, 5-6 membered heteroaryl-fused 5-6 membered heteroaryl, and 5-6 membered heteroaryl-fused phenyl, wherein the 5-6 membered heteroaryl and 5-6 membered heterocyclyl each independently contain 1, 2, or 3 heteroatoms selected from N, O, and S, and the 5-6 membered heteroaryl-fused 5-6 membered heterocyclyl, 5-6 membered heteroaryl-fused 5-6 membered heteroaryl, and 5-6 membered heteroaryl-fused phenyl are unsubstituted or substituted with one or more identical or different $R^b$.

In some specific embodiments, $R^2$ is selected from 5-6 membered heteroaryl-fused 5-6 membered heterocyclyl, 5-6 membered heteroaryl-fused 5-6 membered heteroaryl, and 5-6 membered heteroaryl-fused phenyl, wherein the 5-6 membered heteroaryl is selected from imidazolyl, pyrazolyl, and pyridyl, the 5-6 membered heterocyclyl is selected from pyrrolidinyl, piperidinyl, and piperazinyl, and the 5-6 membered heteroaryl-fused 5-6 membered heterocyclyl, 5-6 membered heteroaryl-fused 5-6 membered heteroaryl, and 5-6 membered heteroaryl-fused phenyl are unsubstituted or substituted with one or more identical or different $R^b$.

In some specific embodiments, $R^2$ is selected from imidazopyrrolidinyl, imidazopiperidinyl, imidazopiperazinyl, pyrazolophenyl, and pyrazolopyridyl, wherein the imidazopyrrolidinyl, imidazopiperidinyl, imidazopiperazinyl, pyrazolophenyl, and pyrazolopyridyl are unsubstituted or substituted with one or more identical or different $R^b$.

In some specific embodiments, $R^2$ is selected from

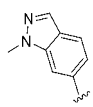 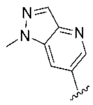 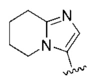 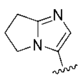 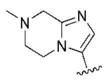.

In some specific embodiments, $R^2$ is selected from 4-6 membered heterocyclyl, wherein the 4-6 membered heterocyclyl contains 1-3 heteroatoms selected from N, O, and S, and the 4-6 membered heterocyclyl is unsubstituted or substituted with 1, 2, or 3 identical or different $R^b$.

In some specific embodiments, $R^2$ is selected from 4-6 membered heterocycloalkyl and 4-6 membered heterocycloalkenyl, wherein the 4-6 membered heterocycloalkyl and 4-6 membered heterocycloalkenyl each independently contain 1-3 heteroatoms selected from N, O, and S, and the 4-6 membered heterocycloalkyl and 4-6 membered heterocycloalkenyl are unsubstituted or are each independently substituted with 1, 2, or 3 identical or different $R^b$.

In some specific embodiments, $R^2$ is selected from 4-6 membered heterocycloalkyl and 4-6 membered heterocycloalkenyl, wherein the 4-6 membered heterocycloalkyl and 4-6 membered heterocycloalkenyl each independently contain 1-2 heteroatoms selected from N and O, and the 4-6 membered heterocycloalkyl and 4-6 membered heterocycloalkenyl are unsubstituted or are each independently substituted with 1, 2, or 3 identical or different $R^b$.

In some specific embodiments, $R^2$ is selected from tetrahydro-2H-pyranyl, oxetanyl, 1,2,3,6-tetrahydropyridyl, and 3,6-dihydro-2H-pyranyl, wherein the tetrahydro-2H-pyranyl, oxetanyl, 1,2,3,6-tetrahydropyridyl, and 3,6-dihydro-2H-pyranyl are unsubstituted or are each independently substituted with 1, 2, or 3 identical or different $R^b$.

In some specific embodiments, $R^2$ is selected from

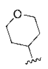 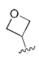 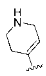 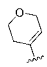,

wherein the

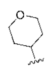 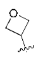 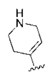 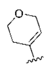

is unsubstituted or independently substituted with 1, 2, or 3 identical or different $R^b$.

In some specific embodiments, $R^2$ is selected from

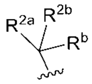,

wherein $R^{2a}$ and $R^{2b}$, together with the carbon atom linked thereto, form 4-6 membered heterocyclyl, wherein the 4-6

membered heterocyclyl contains 1-3 heteroatoms selected from N, O, and S, and the 4-6 membered heterocyclyl is unsubstituted or independently substituted with 1, 2, or 3 identical or different $R^b$.

In some specific embodiments, $R^2$ is selected from

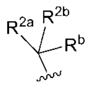,

wherein $R^{2a}$ and $R^{2b}$, together with the carbon atom linked thereto, form 4-6 membered heterocycloalkyl or 4-6 membered heterocycloalkenyl, wherein the 4-6 membered heterocycloalkyl and 4-6 membered heterocycloalkenyl each independently contain 1-3 heteroatoms selected from N, O, and S, and the 4-6 membered heterocycloalkyl and 4-6 membered heterocycloalkenyl are unsubstituted or are each independently substituted with 1, 2, or 3 identical or different $R^b$.

In some specific embodiments, $R^2$ is selected from

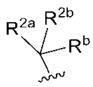,

wherein $R^{2a}$ and $R^{2b}$, together with the carbon atom linked thereto, form 4-6 membered heterocycloalkyl or 4-6 membered heterocycloalkenyl, wherein the 4-6 membered heterocycloalkyl and 4-6 membered heterocycloalkenyl each independently contain 1-2 heteroatoms selected from N and O, and the 4-6 membered heterocycloalkyl and 4-6 membered heterocycloalkenyl are unsubstituted or are each independently substituted with 1, 2, or 3 identical or different $R^b$.

In some specific embodiments, $R^2$ is selected from

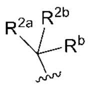,

wherein $R^{2a}$ and $R^{2b}$, together with the carbon atom linked thereto, form tetrahydro-2H-pyranyl, oxetanyl, 1,2,3,6-tetrahydropyridyl, and 3,6-dihydro-2H-pyranyl, wherein the tetrahydro-2H-pyranyl, oxetanyl, 1,2,3,6-tetrahydropyridyl, and 3,6-dihydro-2H-pyranyl are unsubstituted or are each independently substituted with 1, 2, or 3 identical or different $R^b$.

In some specific embodiments, $R^2$ is selected from

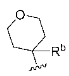 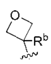 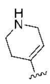 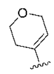,

wherein the group

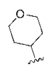 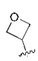 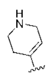 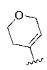

is unsubstituted or independently substituted with 1, 2, or 3 identical or different $R^b$.

In some specific embodiments, each $R^b$ is independently selected from H, $C_1$-$C_6$ alkyl, $C_1$-$C_6$ alkoxy, and $C_3$-$C_6$ cycloalkyl, wherein the $C_1$-$C_6$ alkyl, $C_1$-$C_6$ alkoxy, and $C_3$-$C_6$ cycloalkyl are unsubstituted or are each independently substituted with 1, 2, 3, or 4 halogens.

In some specific embodiments, each $R^b$ is independently selected from H, $C_1$-$C_3$ alkyl, $C_1$-$C_3$ alkoxy, and $C_3$-$C_5$ cycloalkyl, wherein the $C_1$-$C_3$ alkyl, $C_1$-$C_3$ alkoxy, and $C_3$-$C_5$ cycloalkyl are unsubstituted or are each independently substituted with 1, 2, 3, or 4 substituents selected from F, Cl, and Br.

In some specific embodiments, each $R^b$ is independently selected from H, methyl, ethyl, propyl, isopropyl, methoxy, ethoxy, propoxy, isopropoxy, cyclopropyl, cyclobutyl, cyclopentyl, and cyclohexyl.

In some specific embodiments, each $R^b$ is independently selected from methyl, methoxy, and cyclopropyl.

In some specific embodiments, each $R^b$ is independently selected from cyclopropyl.

In some specific embodiments, each $R^b$ is independently selected from methoxy.

In some specific embodiments, each $R^b$ is independently selected from methyl.

In some specific embodiments, $R^2$ is selected from

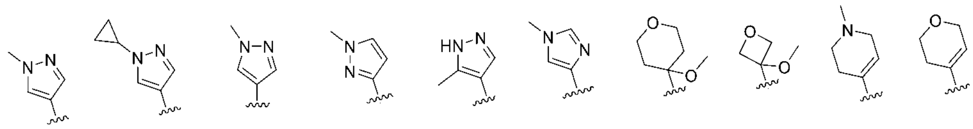.

In some specific embodiments, $R^2$ is selected from

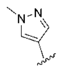.

In some specific embodiments, each $R^b$ is independently selected from H, deuterium, halogen, $C_1$-$C_6$ alkyl, $C_1$-$C_6$ alkoxy, and $C_3$-$C_6$ cycloalkyl, wherein the $C_1$-$C_6$ alkyl, $C_1$-$C_6$ alkoxy, and $C_3$-$C_6$ cycloalkyl are unsubstituted or are each independently substituted with 1, 2, 3, or 4 halogens.

In some specific embodiments, each $R^b$ is independently selected from H, deuterium, halogen, $C_1$-$C_3$ alkyl, $C_1$-$C_3$ alkoxy, and $C_3$-$C_5$ cycloalkyl, wherein the $C_1$-$C_3$ alkyl, $C_1$-$C_3$ alkoxy, and $C_3$-$C_5$ cycloalkyl are unsubstituted or are each independently substituted with 1, 2, 3, or 4 halogens.

In some specific embodiments, each $R^b$ is independently selected from H, deuterium, F, Cl, Br, methyl, ethyl, propyl, isopropyl, methoxy, ethoxy, propoxy, isopropoxy, cyclopropyl, cyclobutyl, cyclopentyl, and cyclohexyl, wherein the methyl, ethyl, propyl, isopropyl, methoxy, ethoxy, propoxy, isopropoxy, cyclopropyl, cyclobutyl, cyclopentyl, and cyclohexyl are unsubstituted or are each independently substituted with 1, 2, 3, or 4 substituents selected from F, Cl, and Br.

In some specific embodiments, each $R^b$ is independently selected from H, deuterium, F, Cl, Br, methyl, ethyl, propyl, isopropyl, methoxy, ethoxy, propoxy, isopropoxy, cyclopropyl, cyclobutyl, cyclopentyl, cyclohexyl, $-CHF_2$, and $-CF_3$.

In some specific embodiments, each $R^b$ is independently selected from H, deuterium, F, Cl, methyl, ethyl, isopropyl, methoxy, cyclopropyl, $-CHF_2$, and $-CF_3$.

In some specific embodiments, $R^b$ described herein may be further deuterated.

In some specific embodiments, each $R^b$ is independently selected from H, deuterium, F, Cl, methyl, ethyl, isopropyl, methoxy, cyclopropyl, $-CHF_2$, $-CF_3$, and $-CD_3$.

In some specific embodiments, $R^2$ is selected from

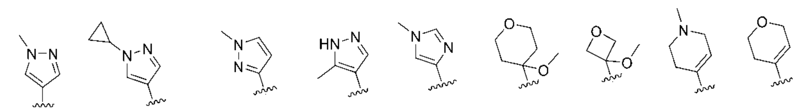

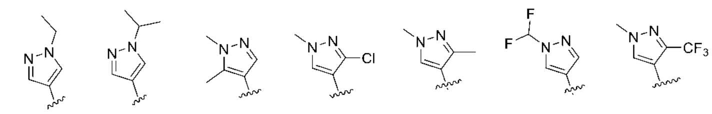

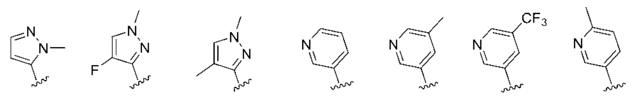

In some specific embodiments, $R^2$ is selected from

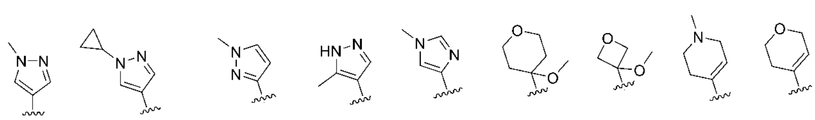

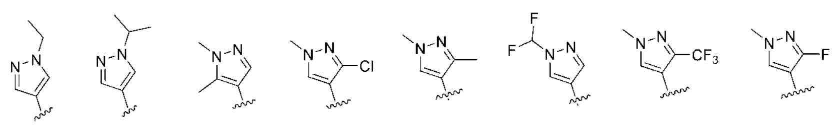

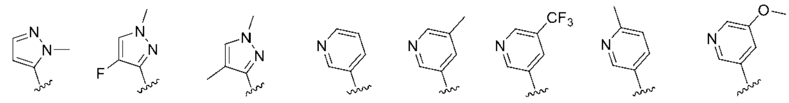

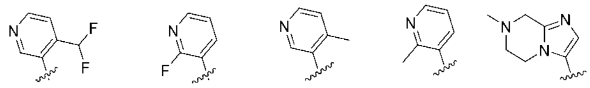

In some specific embodiments, $R^2$ is selected from

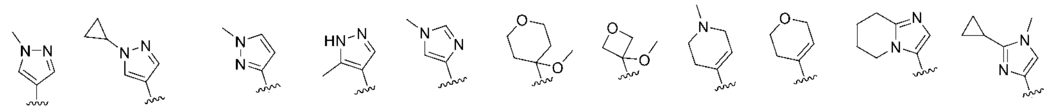

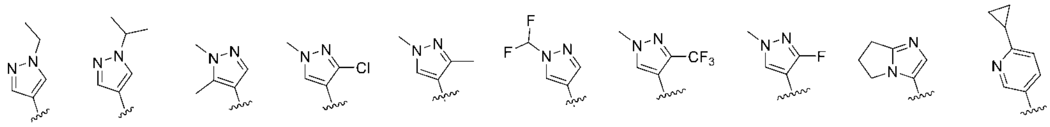

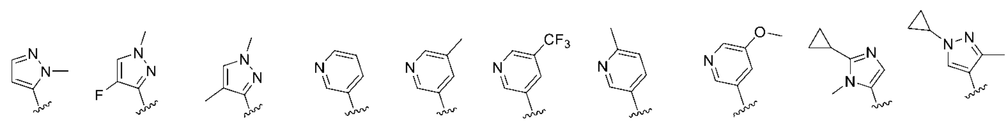

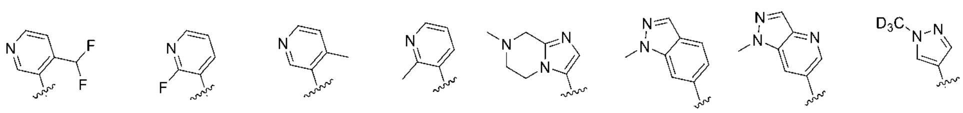

.

In some specific embodiments, $R^3$ is selected from hydrogen, halogen, $C_{1-6}$ alkyl, and $C_{3-6}$ cycloalkyl.

In some specific embodiments, $R^3$ is selected from hydrogen, F, Cl, Br, $C_{1-4}$ alkyl, $C_{5-6}$ alkyl, and $C_{3-5}$ cycloalkyl.

In some specific embodiments, $R^3$ is selected from hydrogen, F, Cl, Br, $C_{1-4}$ alkyl, and $C_{3-5}$ cycloalkyl.

In some specific embodiments, $R^3$ is selected from hydrogen, F, Cl, Br, methyl, ethyl, propyl, isopropyl, butyl, cyclopropyl, cyclobutyl, and cyclopentyl.

In some specific embodiments, $R^3$ is selected from hydrogen, F, Cl, methyl, ethyl, and cyclopropyl.

In some specific embodiments, $R^3$ is selected from hydrogen, F, Cl, Br, and $C_{1-4}$ alkyl.

In some specific embodiments, $R^3$ is selected from hydrogen, F, Cl, methyl, and ethyl.

In some specific embodiments, $R^3$ is selected from hydrogen, F, and methyl.

In some specific embodiments, the group

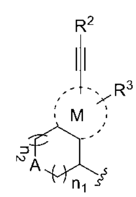

is selected from

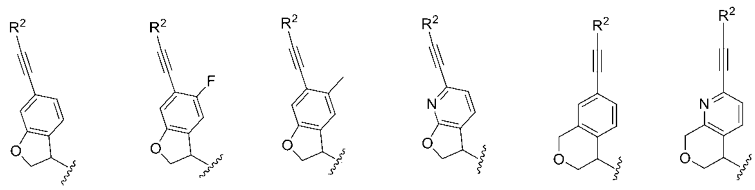

.

In some specific embodiments, the group

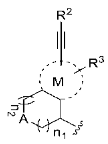

is selected from

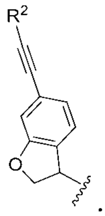

.

In some specific embodiments, the group

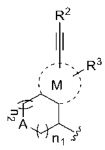

is selected from:

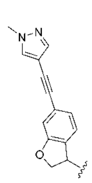 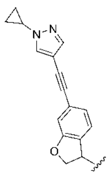 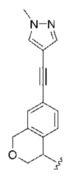 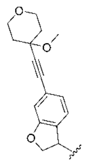 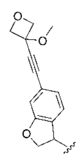 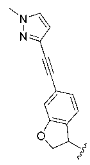 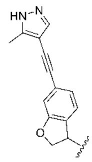 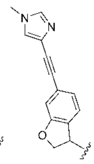 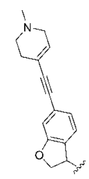 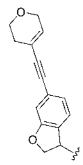

.

In some specific embodiments, the group

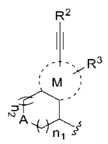

is selected from

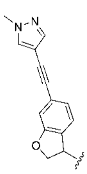 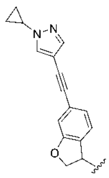 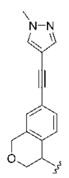 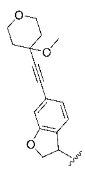 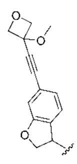 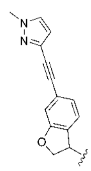 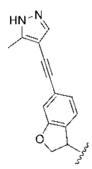 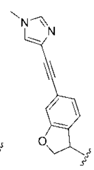 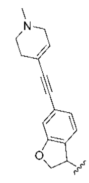 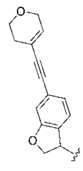

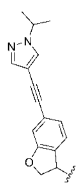 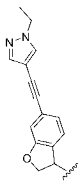 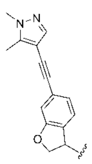 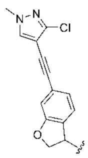 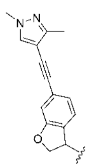 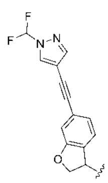 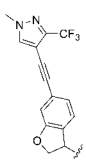 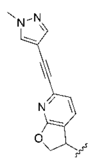

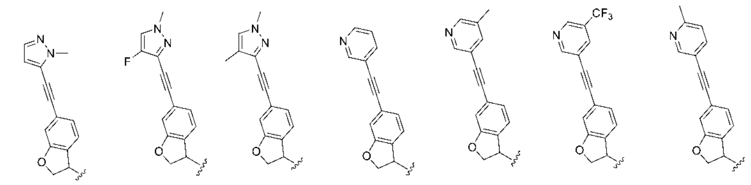

In some specific embodiments, the group

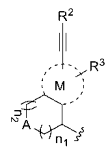

is selected from

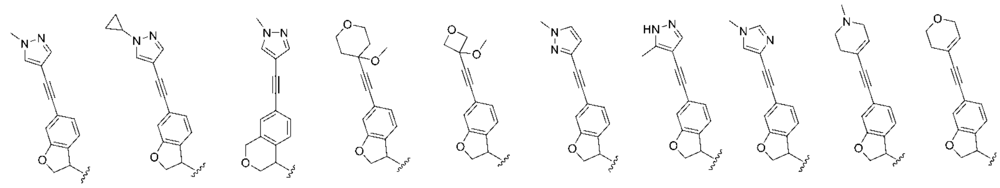

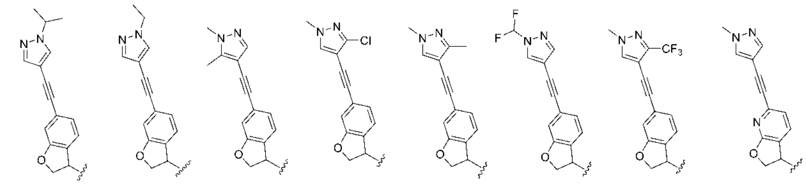

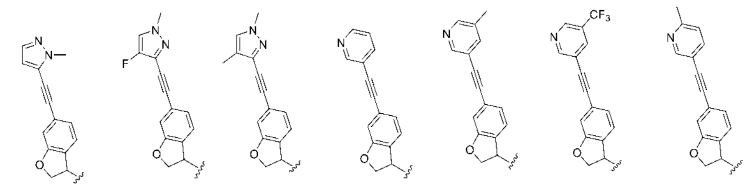

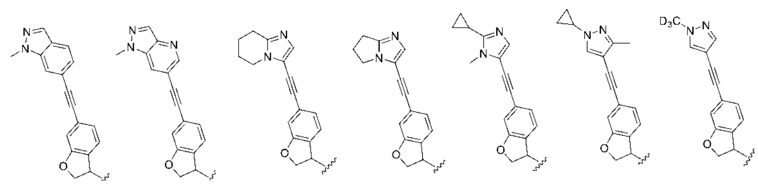

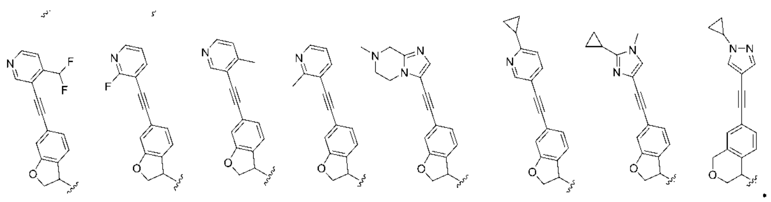

In some specific embodiments, the group

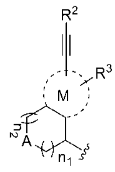

is selected from

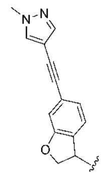

In some specific embodiments, the group

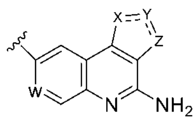

is selected from

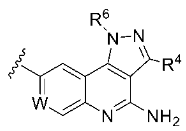 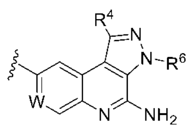 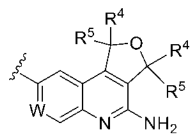 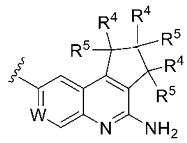 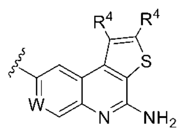 ,

wherein W is selected from N and $CR^7$.

In some specific embodiments, W is selected from $CR^7$.

In some specific embodiments, W is selected from N.

In some specific embodiments, $R^4$, $R^5$, $R^6$, and $R^7$ are each independently selected from hydrogen, halogen, and $C_{1-6}$ alkyl.

In some specific embodiments, $R^4$, $R^5$, $R^6$, and $R^7$ are each independently selected from hydrogen, halogen, $C_{1-4}$ alkyl, and $C_{5-6}$ alkyl.

In some specific embodiments, $R^4$, $R^5$, $R^6$, and $R^7$ are each independently selected from hydrogen, halogen, and $C_{1-4}$ alkyl.

In some specific embodiments, $R^4$, $R^5$, $R^6$, and $R^7$ are each independently selected from hydrogen, F, Cl, Br, methyl, ethyl, propyl, and isopropyl.

In some specific embodiments, $R^4$, $R^5$, $R^6$, and $R^7$ are each independently selected from hydrogen, F, Cl, and methyl.

In some specific embodiments, $R^4$, $R^5$, $R^6$, and $R^7$ described herein may be further deuterated.

In some specific embodiments, $R^4$, $R^5$, $R^6$, and $R^7$ are each independently selected from hydrogen, F, Cl, methyl, and $-CD_3$.

In some specific embodiments, the group

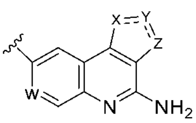

is selected from

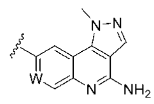 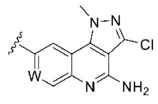 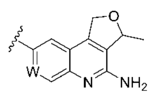 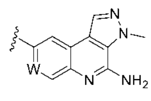

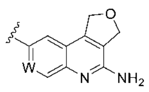 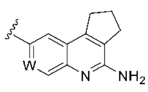 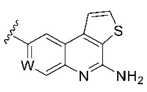 ,

wherein W is selected from N and $CR^7$.

In some specific embodiments, the group

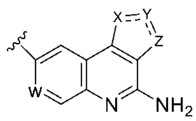

is selected from

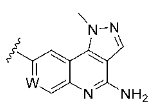 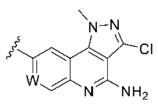 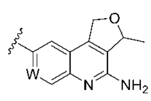 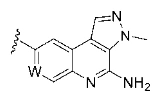

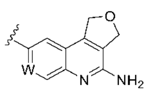 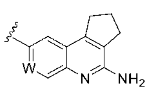 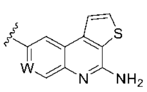 ,

wherein W is selected from N and $CR^7$, and $R^7$ is selected from H, F, Cl, and methyl.

In some specific embodiments, the group

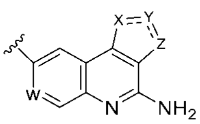

is selected from:

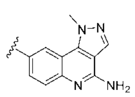 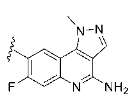 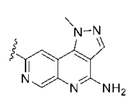 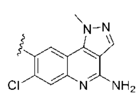 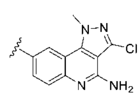 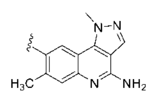

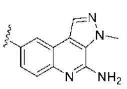 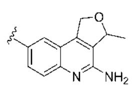 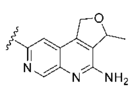 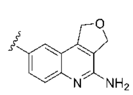 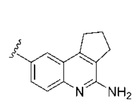 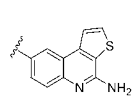 .

In some specific embodiments, the group

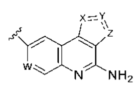

is selected from

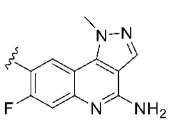 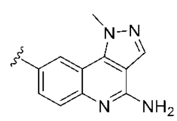 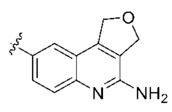 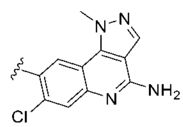 .

In some specific embodiments, the group

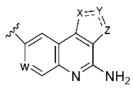

is selected from

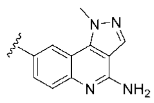
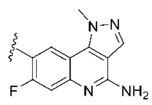
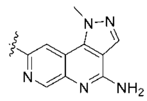
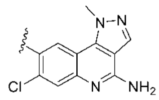
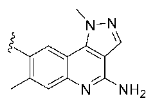
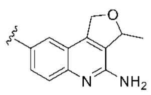
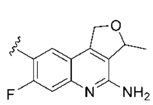
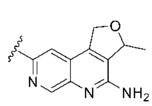
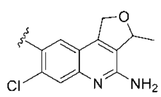
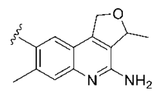
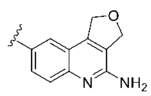
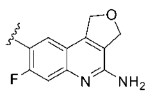
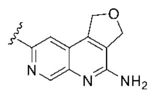
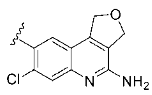
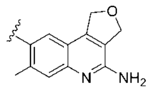
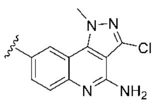
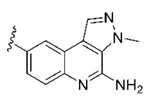
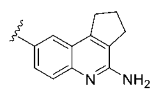
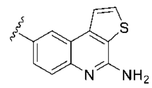

In some specific embodiments, the group

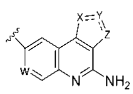

is selected from

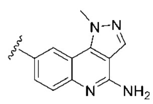
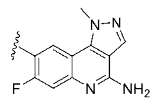
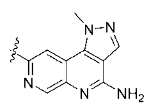
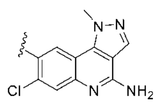
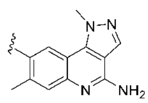
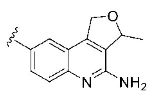
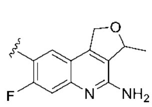
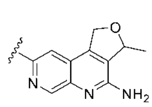
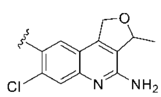
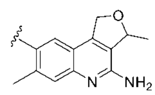
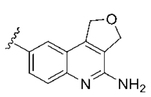
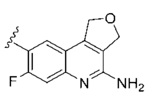
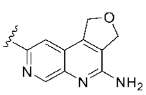
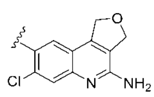
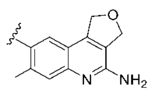.

In some specific embodiments, the group

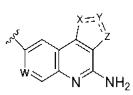

is selected from

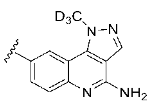 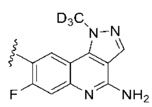 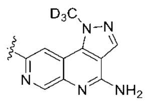 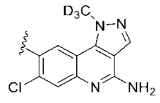 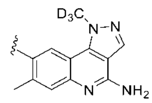

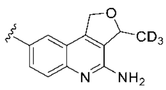 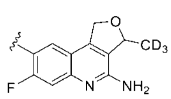 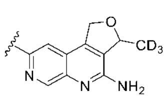 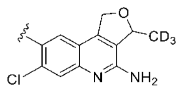 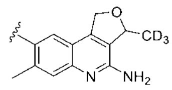.

In some specific embodiments, $R^1$ is selected from $C_{1-6}$ alkyl, $C_{3-10}$ cycloalkyl, $C_{3-10}$ cycloalkyl-$C_{1-6}$ alkyl-, and 5-10 membered heteroaryl, wherein the $C_1$-$C_6$ alkyl is unsubstituted or substituted with one or more $R_i$, the $C_3$-$C_{10}$ cycloalkyl is unsubstituted or substituted with one or more $R_j$, and the 5-10 membered heteroaryl is unsubstituted or substituted with one or more $R_l$.

In some specific embodiments, $R^1$ is selected from $C_{1-4}$ alkyl, $C_{3-6}$ monocycloalkyl, $C_{5-8}$ bridged cycloalkyl, $C_{5-8}$ spirocycloalkyl, $C_{3-6}$ monocycloalkyl-$C_{1-4}$ alkyl-, $C_{5-8}$ bridged cycloalkyl-$C_{1-4}$ alkyl-, $C_{5-8}$ spirocycloalkyl-$C_{1-4}$ alkyl-, and 5-6 membered heteroaryl, wherein the $C_{1-4}$ alkyl is unsubstituted or substituted with one or more $R_i$, the $C_{3-6}$ monocycloalkyl, $C_{5-8}$ bridged cycloalkyl, and $C_{5-8}$ spirocycloalkyl are unsubstituted or substituted with one or more $R_j$, and the 5-10 membered heteroaryl is unsubstituted or substituted with one or more $R_l$.

In some specific embodiments, $R^1$ is selected from methyl, ethyl, propyl, butyl, cyclopropyl, cyclobutyl, cyclopentyl, cyclohexyl,

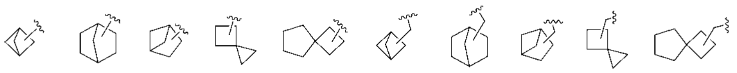, 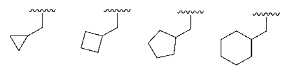,

pyridyl, pyrazinyl, pyrimidinyl, pyridazinyl, pyrazolyl, imidazolyl, triazolyl, pyrrolyl, furanyl, oxazolyl, isoxazolyl, and thiazolyl, wherein the methyl, ethyl, propyl, and butyl are unsubstituted or substituted with one or more $R_i$, the cyclopropyl, cyclobutyl, cyclopentyl, cyclohexyl,

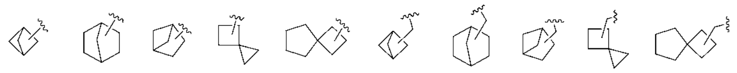, and 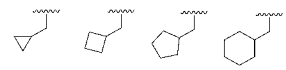

are unsubstituted or substituted with one or more $R_j$, and the pyridyl, pyrazinyl, pyrimidinyl, pyridazinyl, pyrazolyl, imidazolyl, triazolyl, pyrrolyl, furanyl, oxazolyl, isoxazolyl, and thiazolyl are unsubstituted or substituted with one or more $R_1$.

In some specific embodiments, $R^1$ is selected from methyl, ethyl, cyclopropyl, cyclobutyl, cyclopentyl,

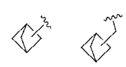, 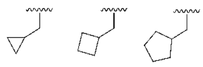,

pyrazolyl, imidazolyl, triazolyl, and pyrrolyl, wherein the methyl and ethyl are unsubstituted or substituted with 1, 2, or 3 $R_i$, the cyclopropyl, cyclobutyl, cyclopentyl,

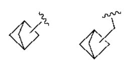, and 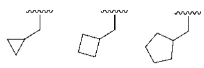

are unsubstituted or substituted with 1, 2, or 3 $R_j$, and the pyrazolyl, imidazolyl, triazolyl, and pyrrolyl are unsubstituted or substituted with 1, 2, or 3 $R_l$.

In some specific embodiments, $R^1$ is selected from methyl, ethyl, cyclopropyl,

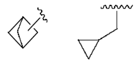,

and pyrazolyl, wherein the methyl and ethyl are unsubstituted or substituted with 1, 2, or 3 $R_i$, the cyclopropyl and

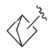 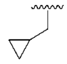

are unsubstituted or substituted with 1, 2, or 3 $R_j$, and the pyrazolyl is unsubstituted or substituted with 1, 2, or 3 $R_l$.

It should be understood that in the definition of substituent $R^1$ in the present disclosure, the substitution with $R_i$ is always performed on alkyl or alkylene, the substitution with $R_j$ is always performed on cycloalkyl, including monocycloalkyl, bridged cycloalkyl, and spirocycloalkyl, the substitution with $R_k$ is always performed on heterocyclyl, and the substitution with $R_l$ is always performed on aryl or heteroaryl.

For example, in

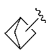 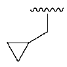,

if the substituent $R_j$ appears, the substitution position for $R_j$ is located on cyclopropyl or

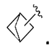.

In some specific embodiments, $R^1$ is selected from methyl, ethyl, cyclopropyl,

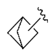 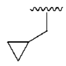,

and pyrazolyl, wherein the methyl, ethyl, cyclopropyl, and

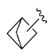 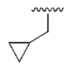

are unsubstituted, and the pyrazolyl is unsubstituted or substituted with 1, 2, or 3 $R_l$.

In some specific embodiments, each $R_i$, $R_j$, $R_k$, and $R_l$ is independently selected from halogen, halogenated $C_{1-6}$ alkyl, $C_{1-6}$ alkyl, and $C_{3-6}$ cycloalkyl.

In some specific embodiments, each $R_i$, $R_j$, $R_k$, and $R_l$ is independently selected from halogen, halogenated $C_{1-4}$ alkyl, $C_{1-4}$ alkyl, and $C_{3-6}$ cycloalkyl.

In some specific embodiments, each $R_i$, $R_j$, $R_k$, and $R_l$ is independently selected from halogen, $C_{1-4}$ alkyl, and $C_{3-6}$ cycloalkyl.

In some specific embodiments, each $R_i$, $R_j$, $R_k$, and $R_l$ is independently selected from F, Cl, Br, methyl, ethyl, propyl, isopropyl, cyclopropyl, and cyclobutyl.

In some specific embodiments, each $R_i$, $R_j$, $R_k$, and $R_l$ is independently selected from F, Cl, methyl, and ethyl.

In some specific embodiments, each $R_i$, $R_j$, $R_k$, and $R_l$ is independently selected from methyl.

In some specific embodiments, each $R_l$ is independently selected from halogen, halogenated $C_{1-6}$ alkyl, $C_{1-6}$ alkyl, and $C_{3-6}$ cycloalkyl.

In some specific embodiments, each $R_l$ is independently selected from halogen, halogenated $C_{1-4}$ alkyl, $C_{1-4}$ alkyl, and $C_{3-6}$ cycloalkyl.

In some specific embodiments, each $R_l$ is independently selected from halogen, $C_{1-4}$ alkyl, and $C_{3-6}$ cycloalkyl.

In some specific embodiments, each $R_l$ is independently selected from F, Cl, Br, methyl, ethyl, propyl, isopropyl, cyclopropyl, and cyclobutyl.

In some specific embodiments, each $R_l$ is independently selected from F, Cl, methyl, and ethyl.

In some specific embodiments, each $R_l$ is independently selected from methyl.

In some specific embodiments, each $R_l$ is independently selected from D, F, Cl, methyl, and ethyl.

In some specific embodiments, $R^1$ is selected from methyl, ethyl, cyclopropyl, and

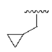 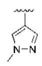 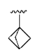.

In some specific embodiments, $R^1$ is selected from methyl, ethyl, and cyclopropyl.

In some specific embodiments, $R^1$ is selected from methyl.

In some specific embodiments, $R^1$ is selected from $-CD_3$.

In some specific embodiments, formula (I) has a structure shown as formula IA:

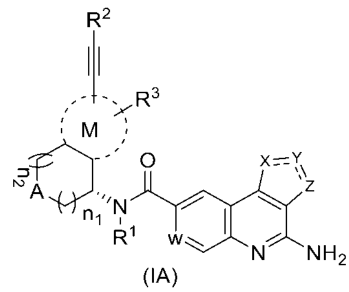

(IA) ,

wherein $R^1$, $R^2$, $R^3$, A, $n_1$, $n_2$, ring M, W, X, Y, Z and $\overline{- - -}$ are each as defined above.
formula (I) has a structure shown as formula IB:

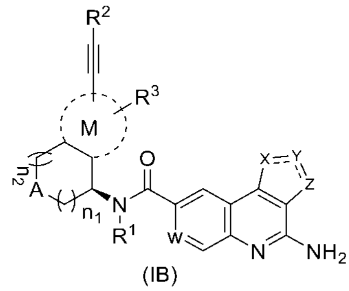

(IB) ,

wherein $R^1$, $R^2$, $R^3$, A, $n_1$, $n_2$, ring M, W, X, Y, Z and $\overline{- - -}$ are each as defined above.

As an exemplary compound of the present disclosure, the compound represented by formula (I) is selected from any of the following specific compounds:

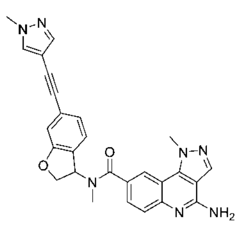

**Compound 1**

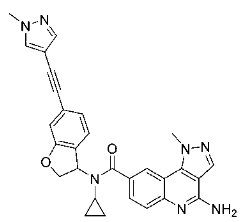

**Compound 2**

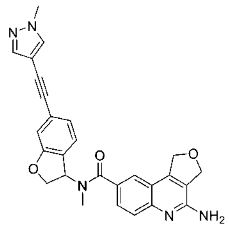

**Compound 3**

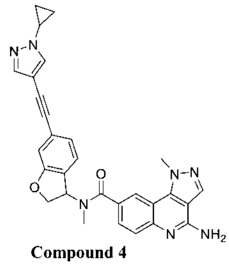

**Compound 4**

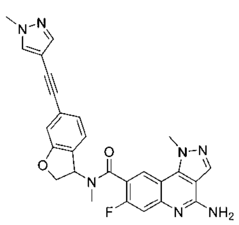

**Compound 5**

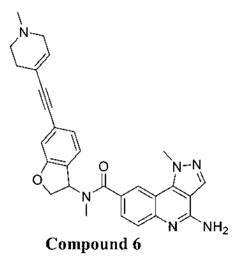

**Compound 6**

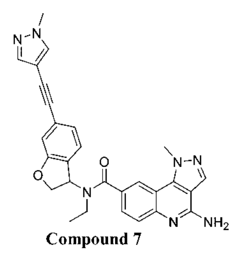

**Compound 7**

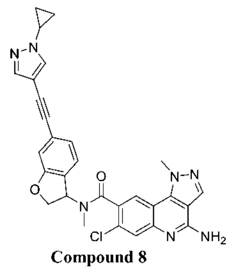

**Compound 8**

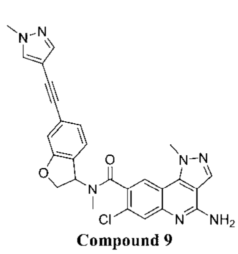
Compound 9

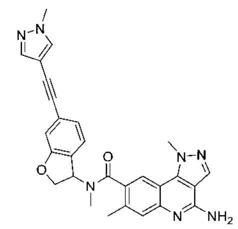
Compound 10

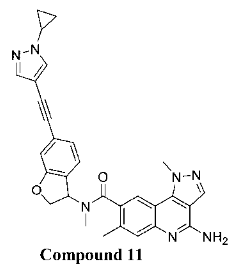
Compound 11

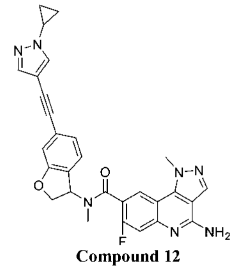
Compound 12

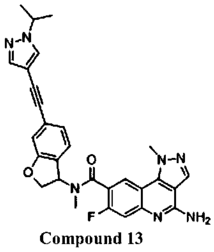
Compound 13

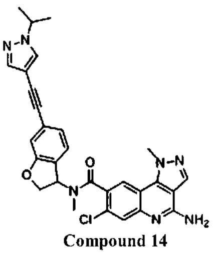
Compound 14

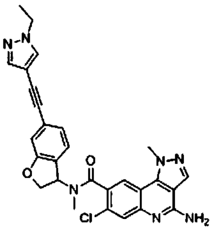
Compound 15

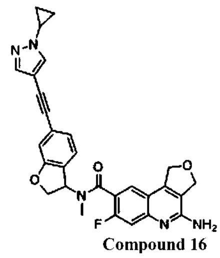
Compound 16

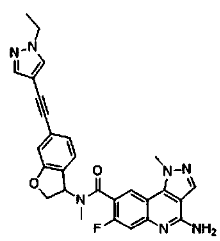
Compound 17

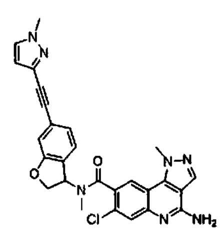
Compound 18

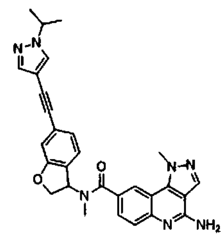
Compound 19

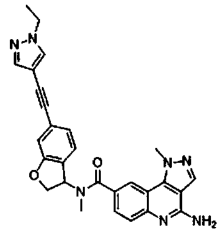
Compound 20

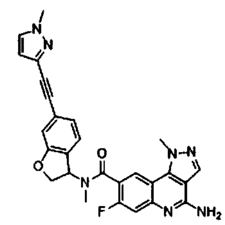
Compound 21

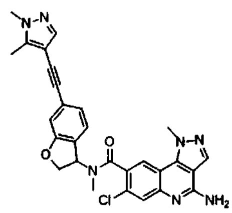
Compound 22

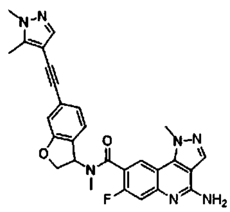
Compound 23

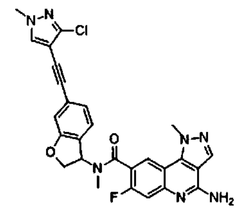
Compound 24

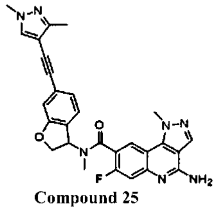
Compound 25

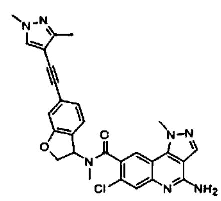
Compound 26

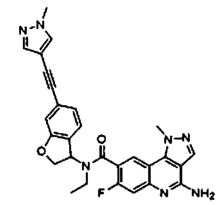
Compound 27

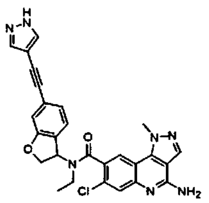
Compound 28

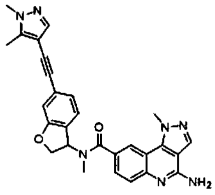
Compound 29

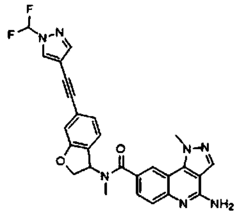
Compound 30

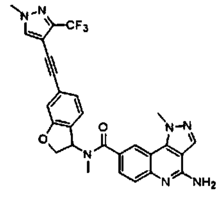
Compound 31

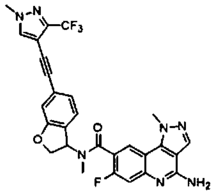
Compound 32

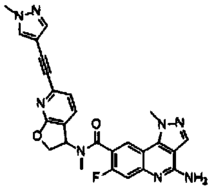
Compound 33

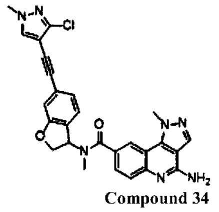
Compound 34

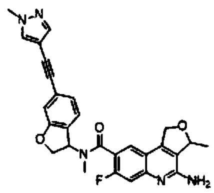
Compound 35

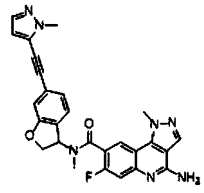
Compound 36

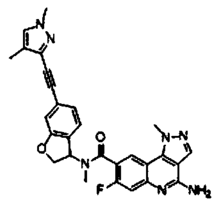
Compound 37

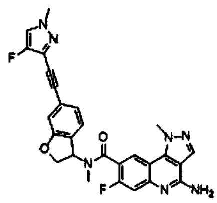
Compound 38

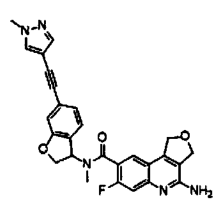
Compound 39

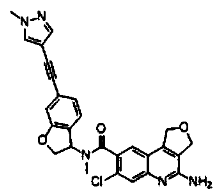
Compound 40

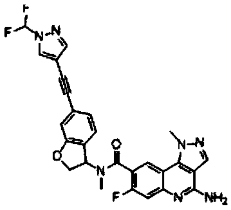
Compound 41

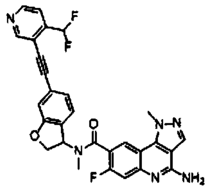
Compound 42

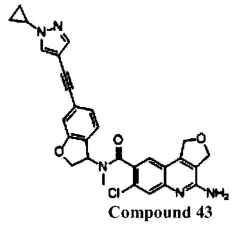
Compound 43

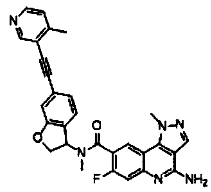
Compound 44

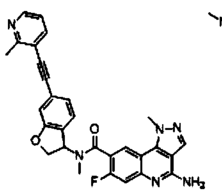
Compound 45

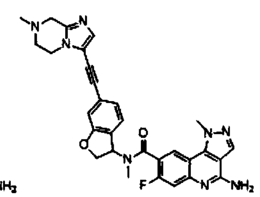
Compound 46

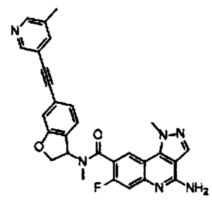
Compound 47

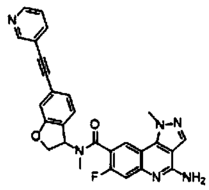
Compound 48

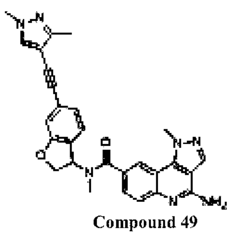
Compound 49

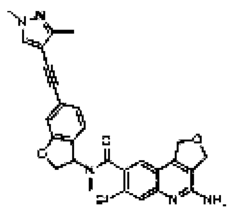
Compound 50

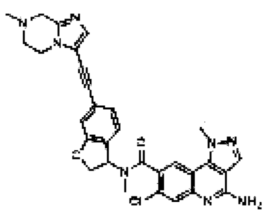
Compound 51

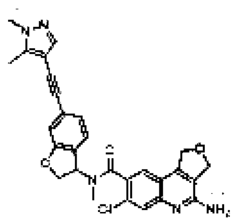
Compound 52

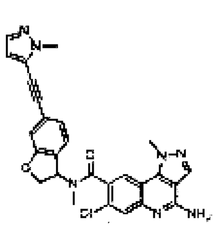
Compound 53

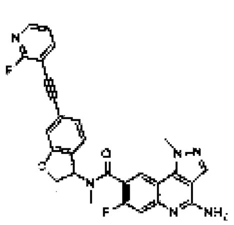
Compound 54

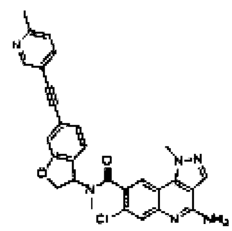
Compound 55

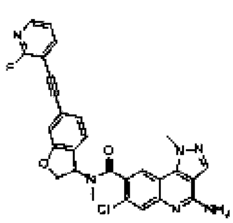
Compound 56

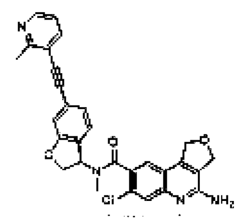

Compound 57

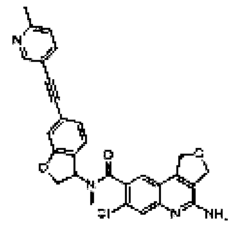

Compound 58

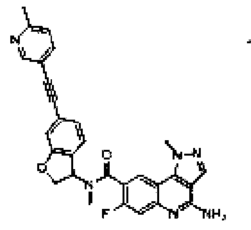

Compound 59

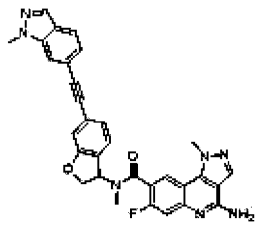

Compound 60

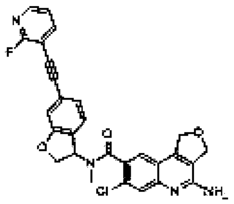

Compound 61

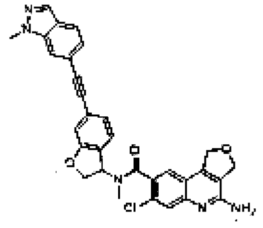

Compound 62

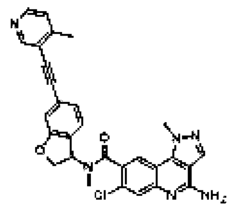

Compound 63

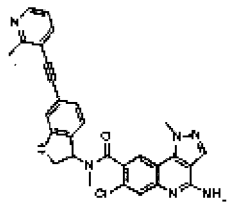

Compound 64

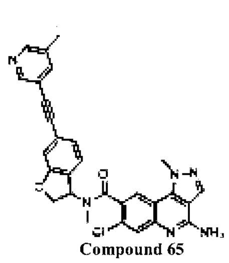

Compound 65

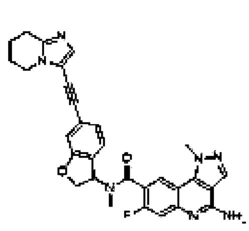

Compound 66

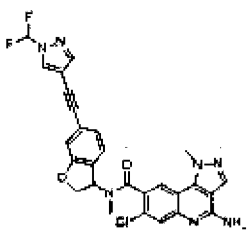

Compound 67

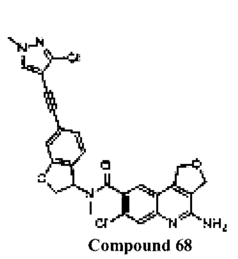

Compound 68

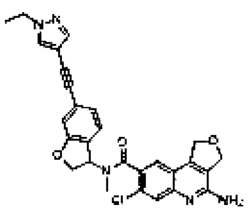

Compound 69

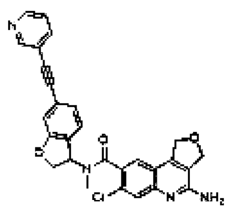

Compound 70

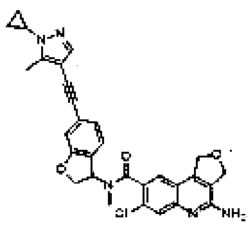

Compound 71

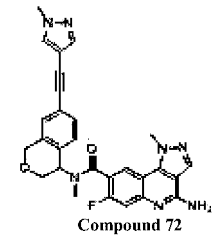

Compound 72

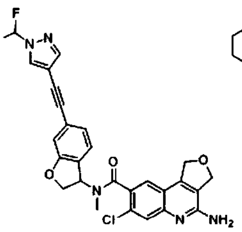

Compound 73

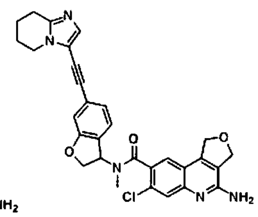

Compound 74

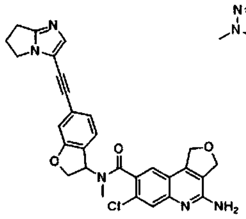

Compound 75

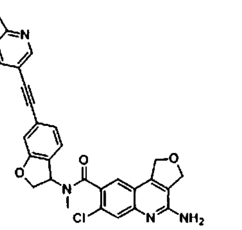

Compound 76

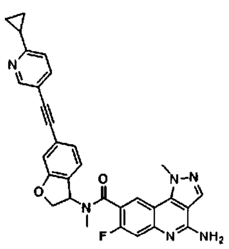

Compound 77

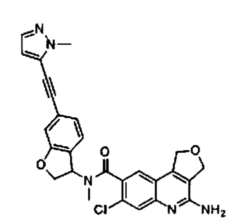

Compound 78

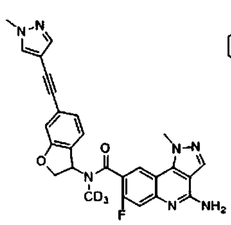

Compound 79

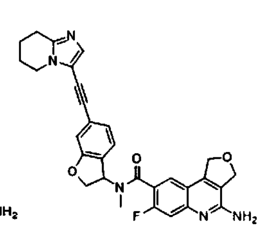

Compound 80

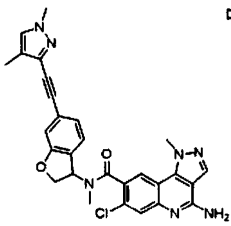
Compound 81

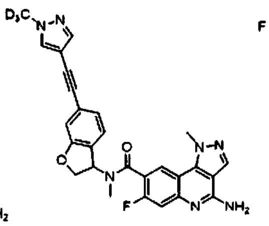
Compound 82

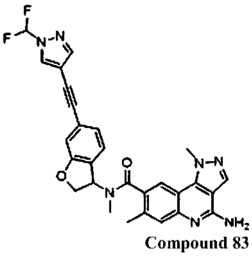
Compound 83

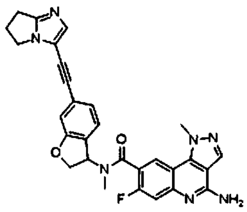
Compound 84

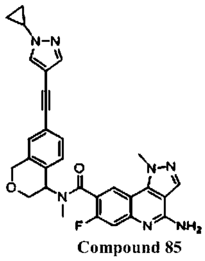
Compound 85

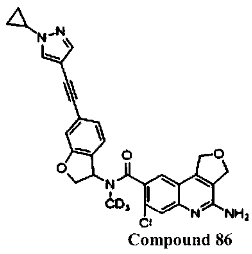
Compound 86

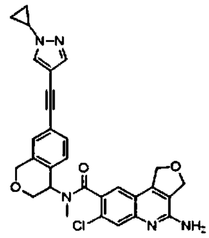
Compound 87

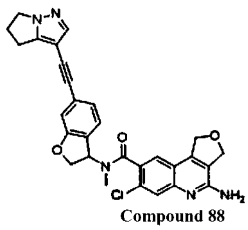
Compound 88

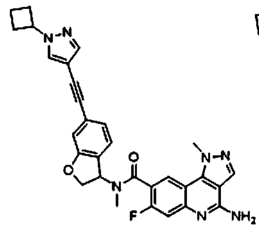
Compound 89

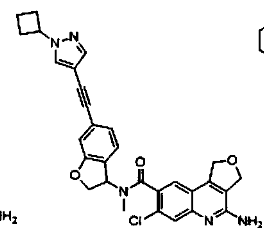
Compound 90

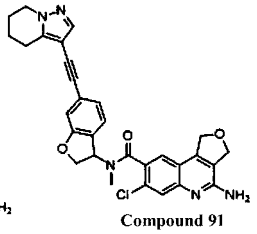
Compound 91

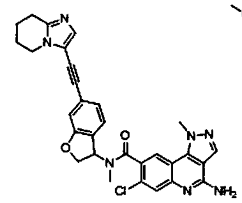
Compound 93

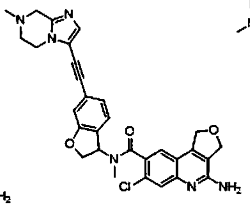
Compound 94

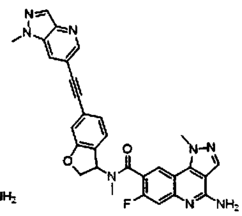
Compound 95

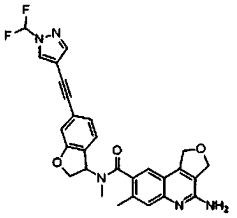
Compound 98

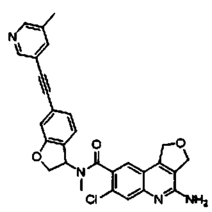
Compound 100

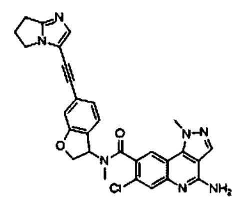
Compound 101

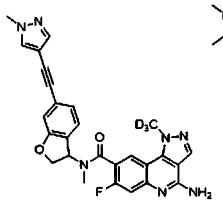
Compound 103

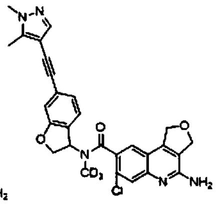
Compound 104

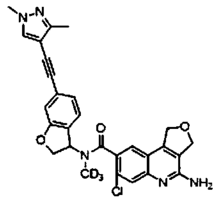
Compound 105

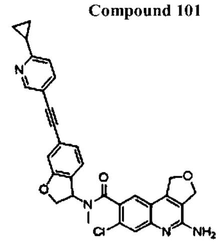
Compound 106

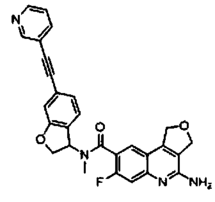
Compound 107

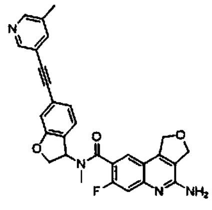
Compound 108

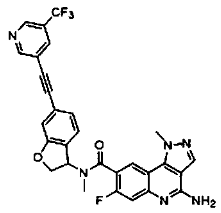
Compound 113

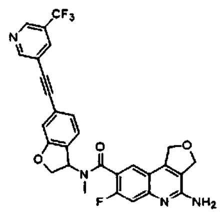
Compound 114

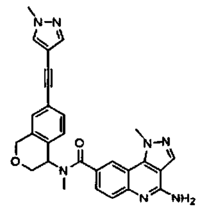
Compound 115

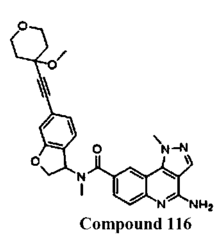
Compound 116

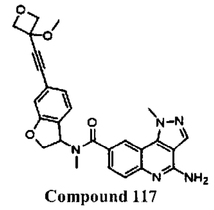
Compound 117

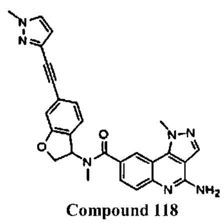
Compound 118

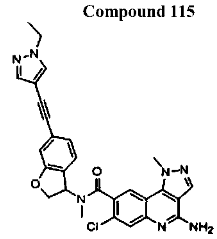
Compound 119

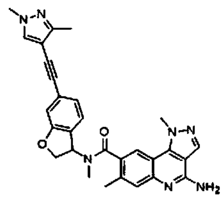
Compound 120

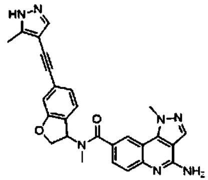
Compound 121

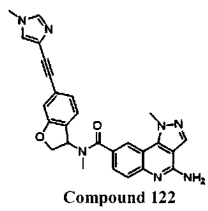
Compound 122

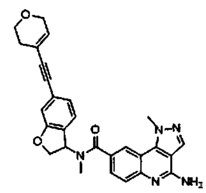
Compound 123

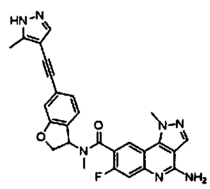
Compound 124

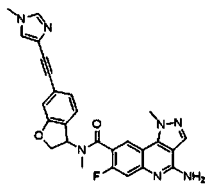
Compound 125

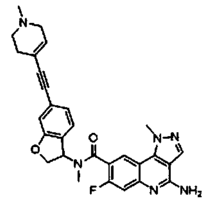
Compound 126

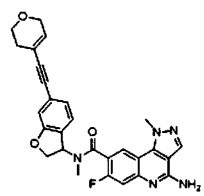
Compound 127

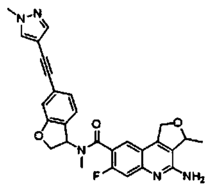
Compound 128

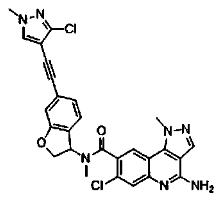
Compound 129

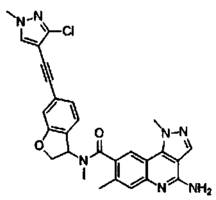
Compound 130

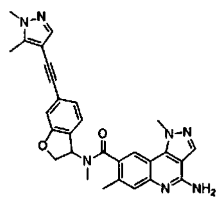
Compound 131

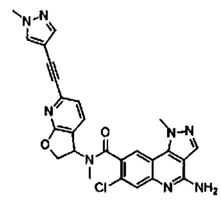
Compound 132

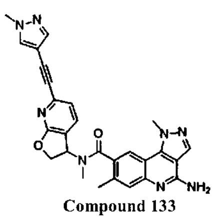
Compound 133

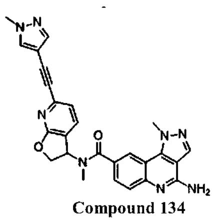
Compound 134

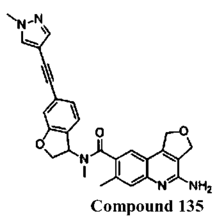
Compound 135

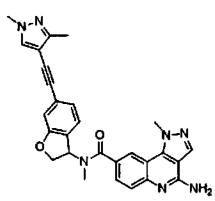
Compound 136

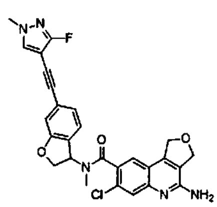
Compound 137

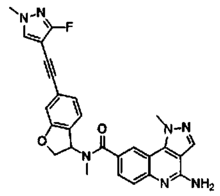
Compound 138

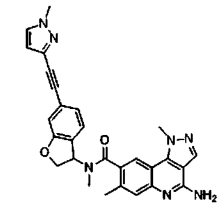
Compound 139

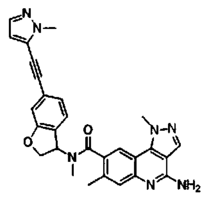
Compound 140

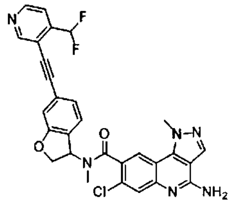
Compound 141

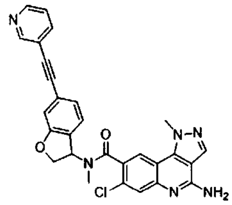
Compound 142

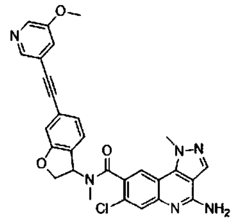
Compound 143

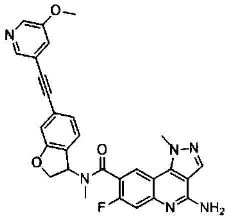
Compound 144

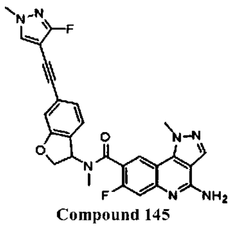
Compound 145

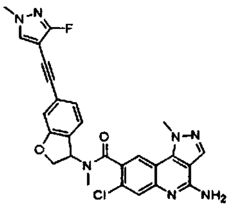
Compound 146

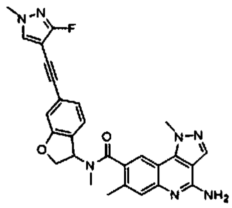
Compound 147

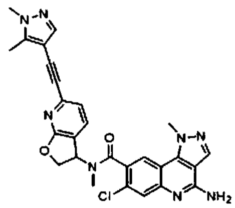
Compound 148

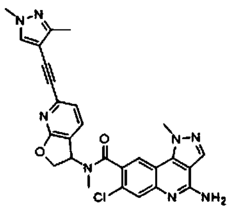
Compound 149

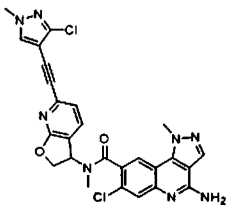
Compound 150

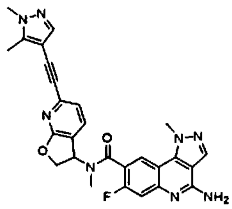
Compound 151

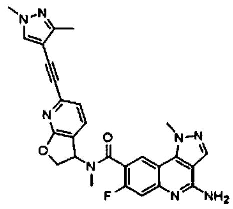
Compound 152

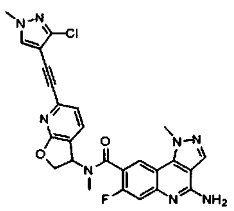
Compound 153

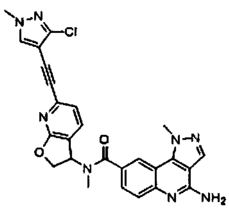
Compound 154

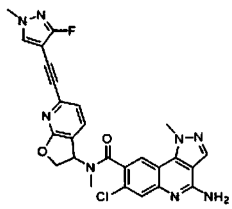
Compound 155

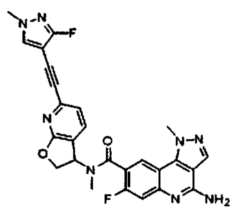
Compound 156

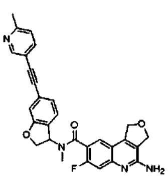
Compound 157

Further, the present disclosure also provides the S configurations of the exemplary compounds described above:

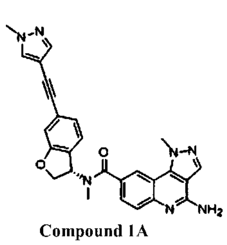
Compound 1A

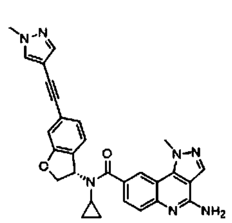
Compound 2A

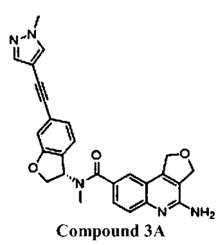
Compound 3A

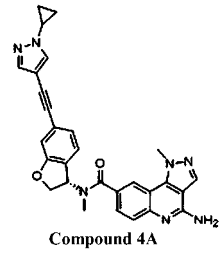
Compound 4A

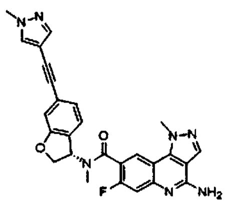
Compound 5A

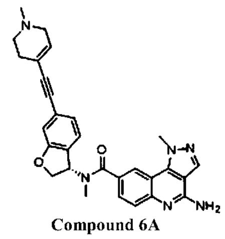
Compound 6A

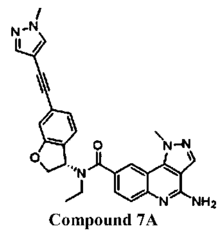
Compound 7A

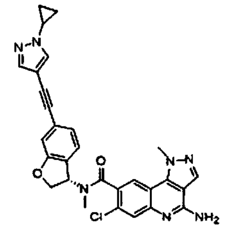
Compound 8A

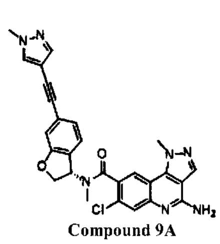
Compound 9A

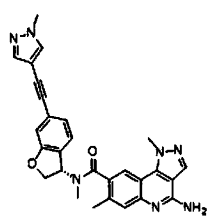
Compound 10A

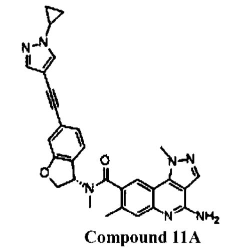
Compound 11A

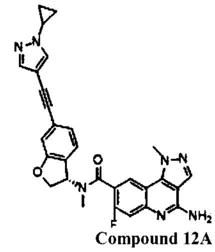
Compound 12A

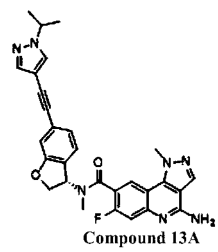
Compound 13A

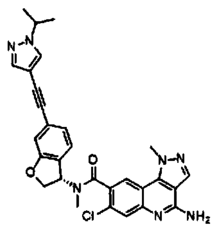
Compound 14A

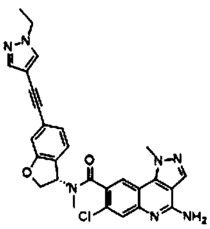
Compound 15A

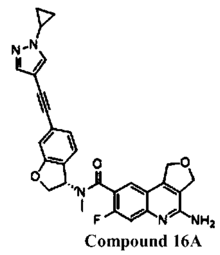
Compound 16A

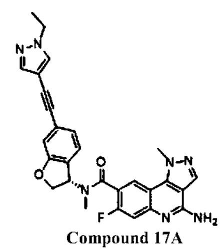
Compound 17A

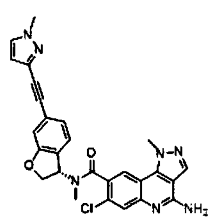
Compound 18A

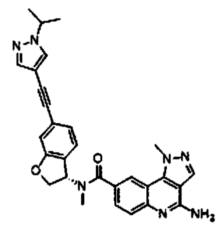
Compound 19A

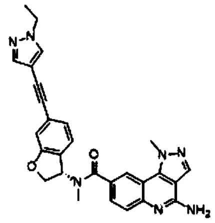
Compound 20A

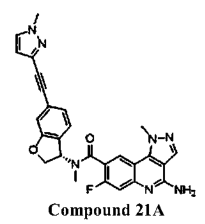
Compound 21A

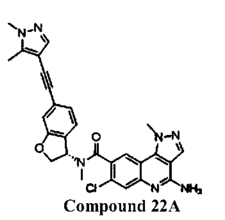
Compound 22A

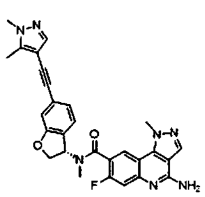
Compound 23A

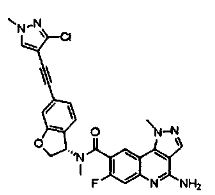
Compound 24A

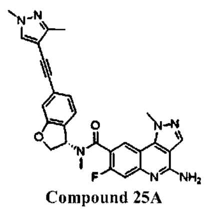
Compound 25A

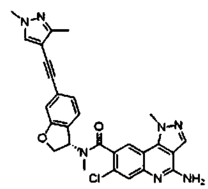
Compound 26A

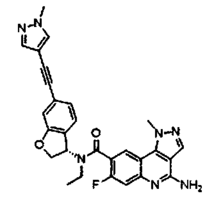
Compound 27A

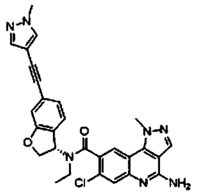
Compound 28A

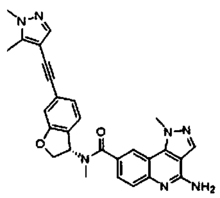
Compound 29A

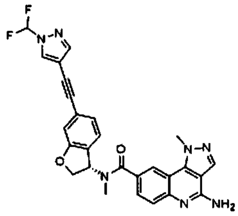
Compound 30A

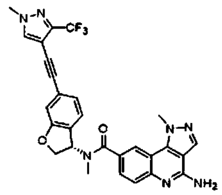
Compound 31A

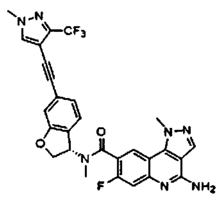
Compound 32A

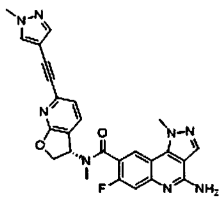
Compound 33A

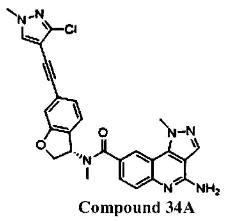
Compound 34A

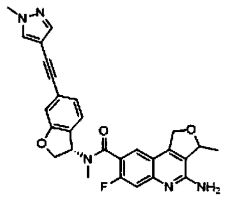
Compound 35A

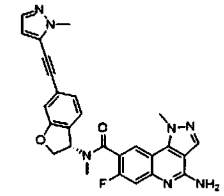
Compound 36A

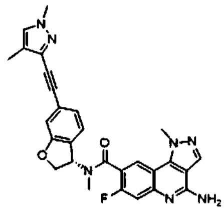
Compound 37A

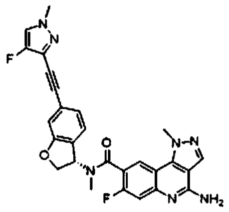
Compound 38A

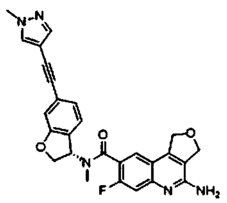
Compound 39A

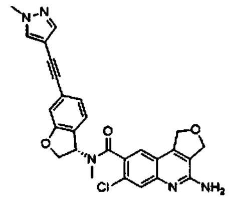
Compound 40A

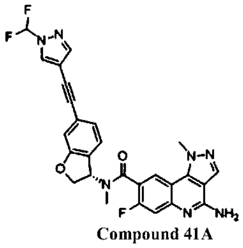
Compound 41A

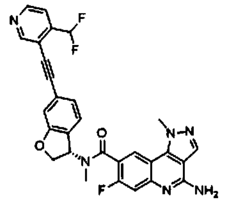
Compound 42A

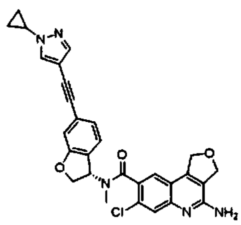
Compound 43A

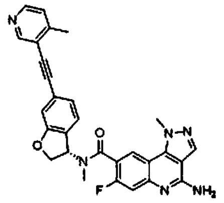
Compound 44A

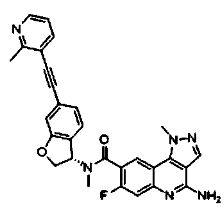
Compound 45A

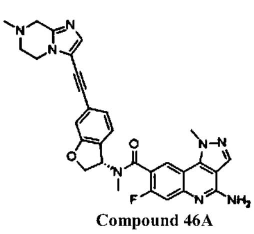
Compound 46A

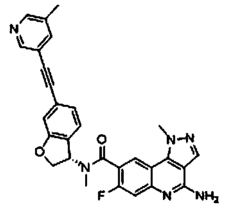
Compound 47A

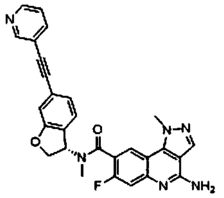
Compound 48A

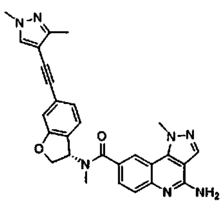
Compound 49A

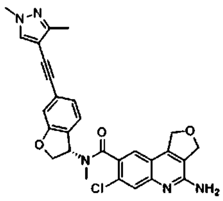
Compound 50A

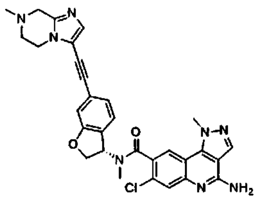
Compound 51A

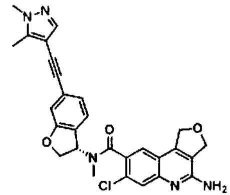
Compound 52A

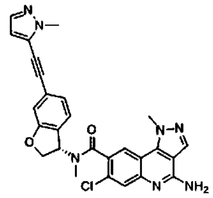
Compound 53A

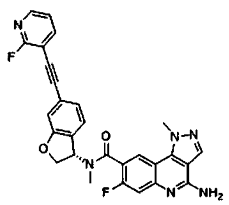
Compound 54A

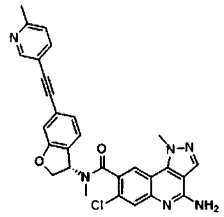
Compound 55A

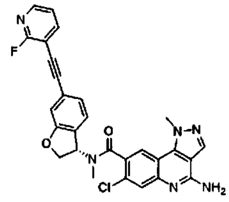
Compound 56A

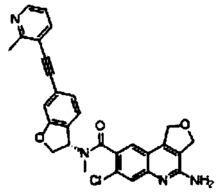
Compound 57A

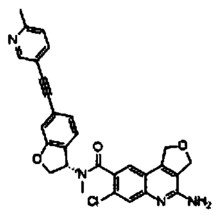
Compound 58A

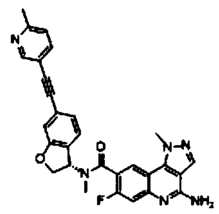
Compound 59A

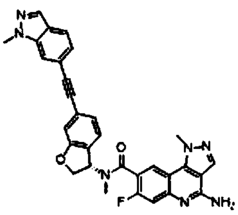
Compound 60A

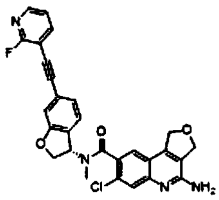
Compound 61A

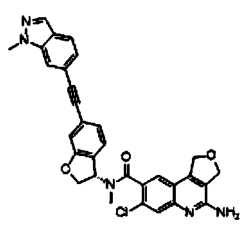
Compound 62A

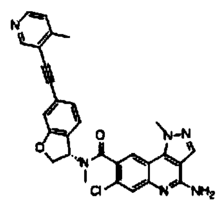
Compound 63A

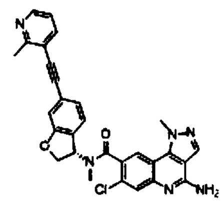
Compound 64A

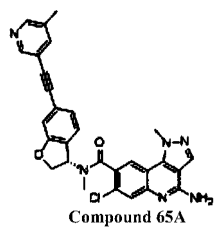
Compound 65A

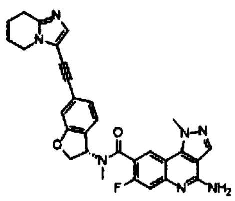
Compound 66A

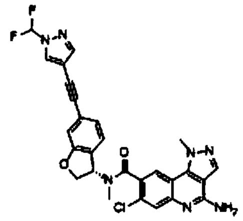
Compound 67A

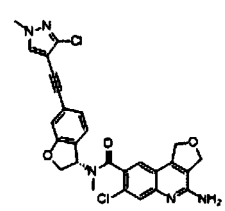
Compound 68A

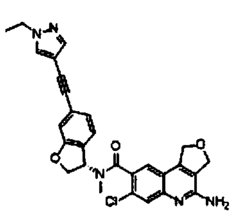
Compound 69A

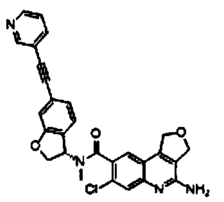
Compound 70A

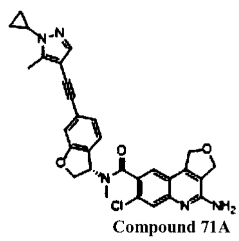
Compound 71A

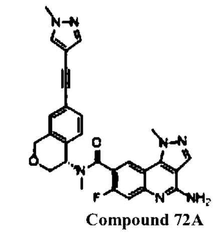
Compound 72A

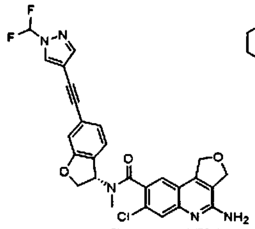
Compound 73A

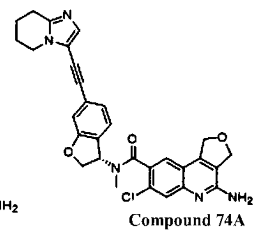
Compound 74A

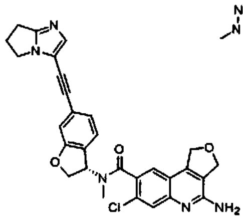
Compound 75A

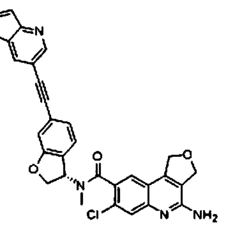
Compound 76A

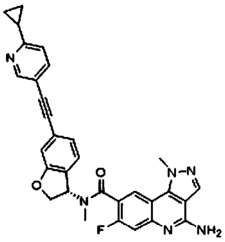
Compound 77A

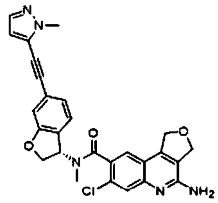
Compound 78A

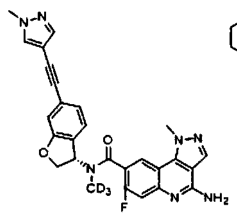
Compound 79A

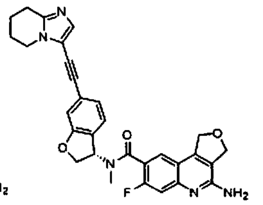
Compound 80A

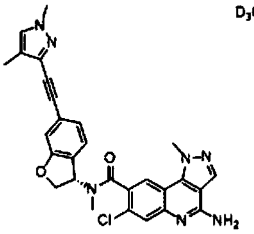
Compound 81A

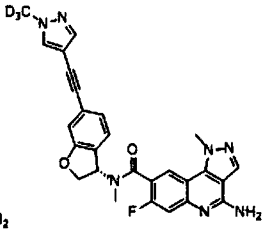
Compound 82A

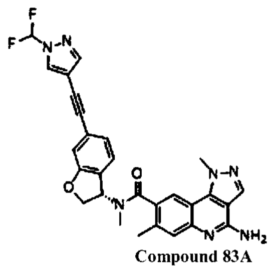
Compound 83A

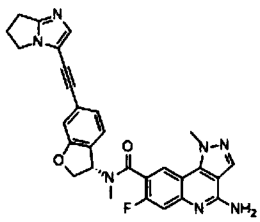
Compound 84A

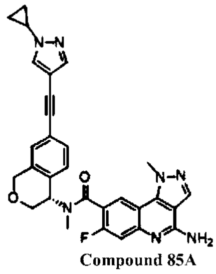
Compound 85A

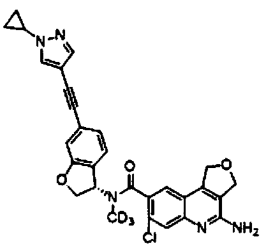
Compound 86A

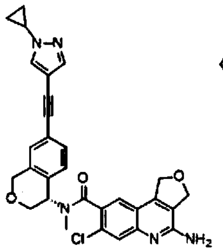
Compound 87A

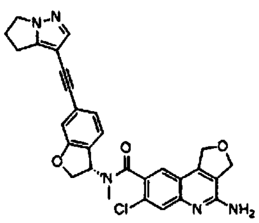
Compound 88A

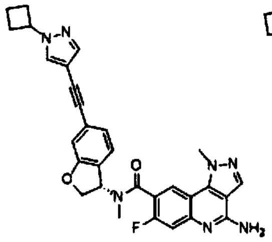
Compound 89A

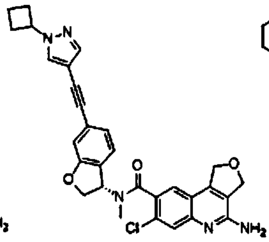
Compound 90A

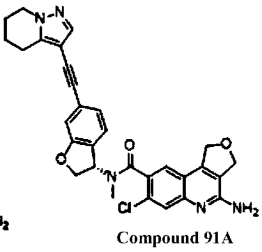
Compound 91A

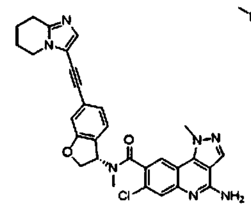

Compound 93A

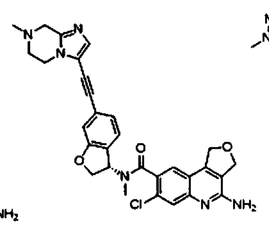

Compound 94A

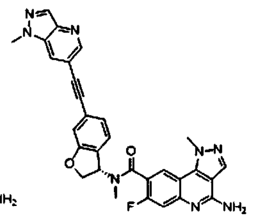

Compound 95A

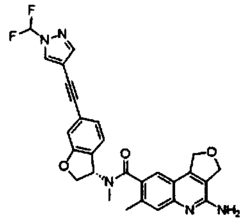

Compound 98A

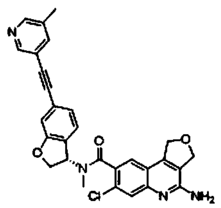

Compound 100A

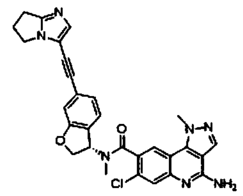

Compound 101A

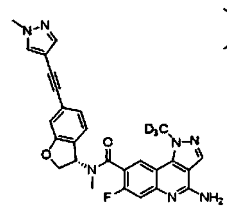

Compound 103A

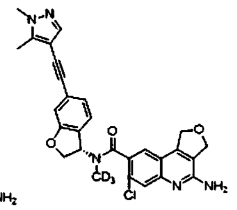

Compound 104A

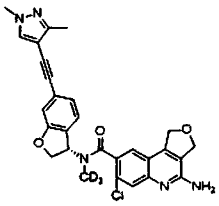

Compound 105A

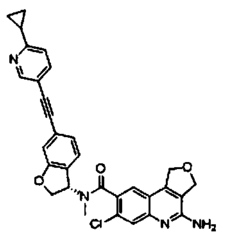

Compound 106A

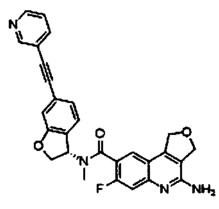

Compound 107A

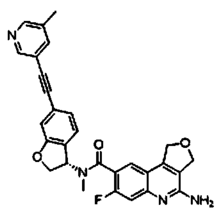

Compound 108A

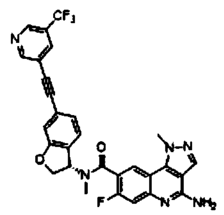

Compound 113A

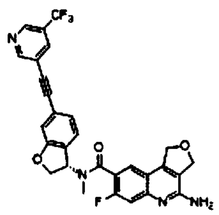

Compound 114A

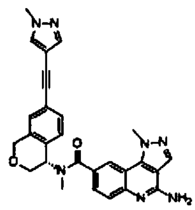

Compound 115A

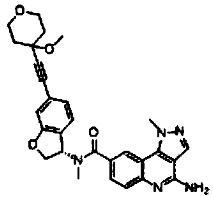

Compound 116A

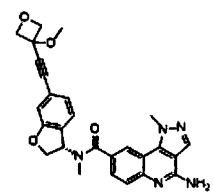

Compound 117A

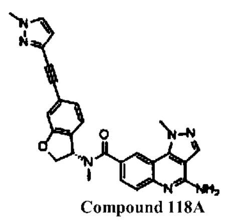

Compound 118A

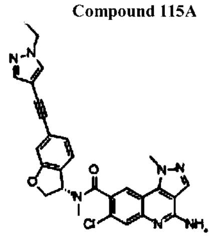

Compound 119A

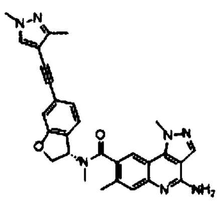

Compound 120A

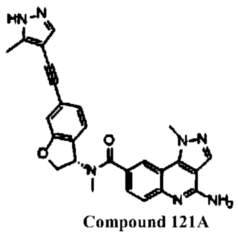

Compound 121A

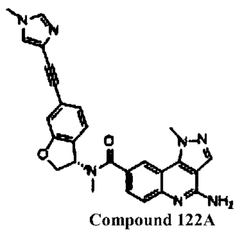

Compound 122A

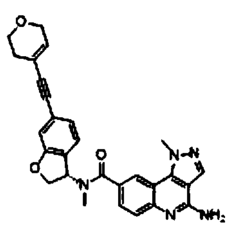

Compound 123A

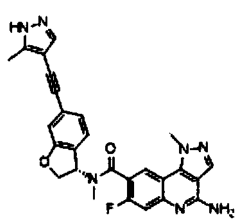

Compound 124A

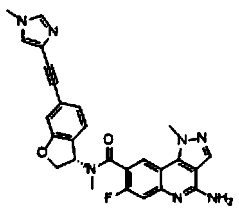
Compound 125A

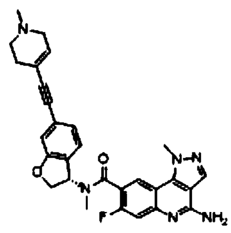
Compound 126A

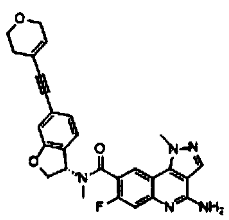
Compound 127A

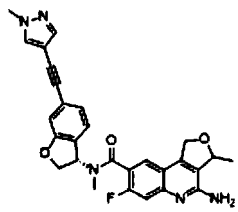
Compound 128A

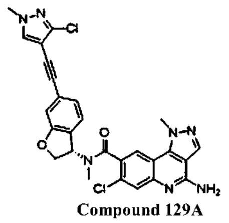
Compound 129A

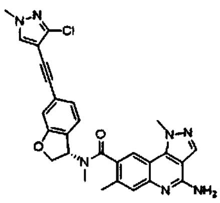
Compound 130A

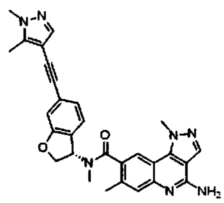
Compound 131A

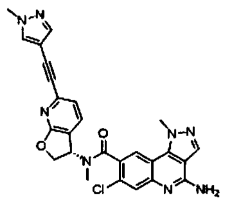
Compound 132A

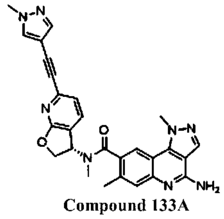
Compound 133A

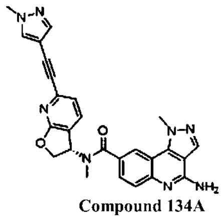
Compound 134A

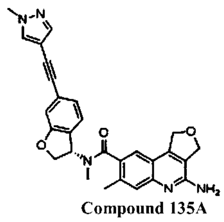
Compound 135A

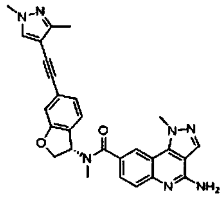
Compound 136A

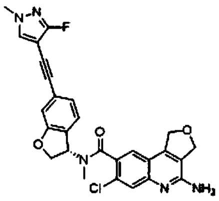
Compound 137A

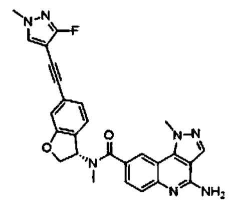
Compound 138A

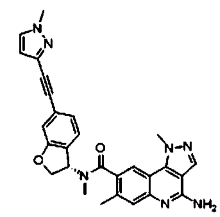
Compound 139A

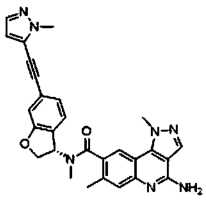
Compound 140A

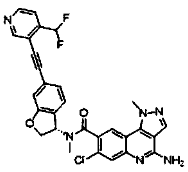
Compound 141A

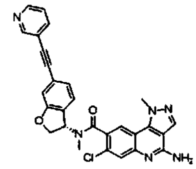
Compound 142A

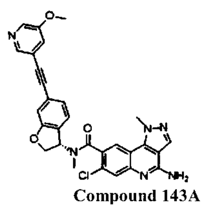
Compound 143A

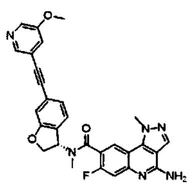
Compound 144A

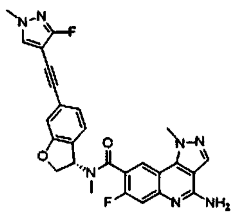
Compound 145A

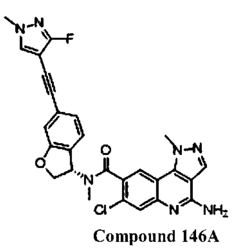
Compound 146A

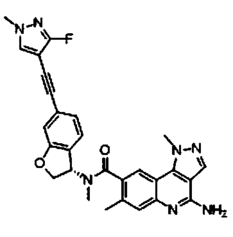
Compound 147A

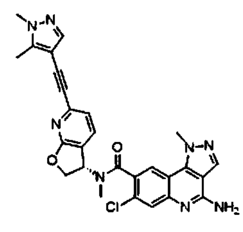
Compound 148A

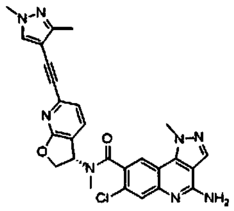
Compound 149A

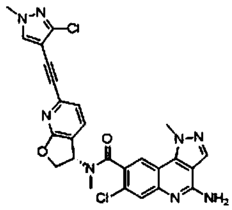
Compound 150A

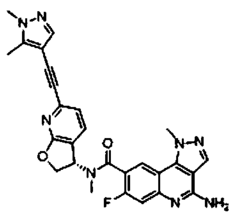
Compound 151A

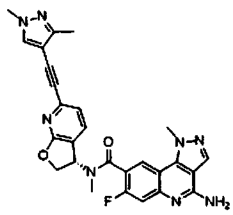
Compound 152A

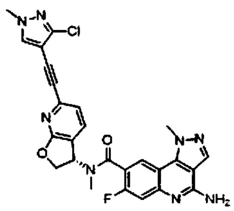
Compound 153A

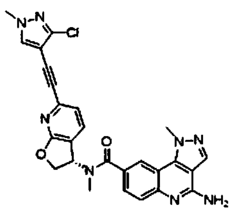
Compound 154A

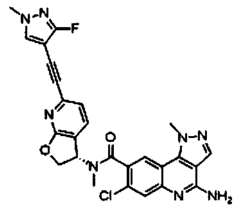
Compound 155A

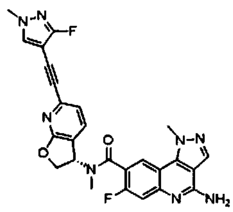
Compound 156A

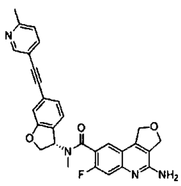
Compound 157A

.

The term "pharmaceutically acceptable salt" refers to a salt of the compound of the present disclosure prepared from the compound with specific substituents discovered in the present disclosure and a relatively non-toxic acid or base. When the compound of the present disclosure comprises a relatively acidic functional group, a base addition salt may be obtained by contacting a sufficient amount of base with the compound in a pure solution or a suitable inert solvent. When the compound of the present disclosure comprises a relatively basic functional group, an acid addition salt may be obtained by contacting a sufficient amount of acid with the compound in a pure solution or a suitable inert solvent.

The term "deuterated compound" means that the compound of the present disclosure comprises at least one deuterium atom, and specifically means that one or more hydrogen atoms in the compound of the present disclosure can be replaced or substituted by deuterium atoms. In some embodiments, the compound comprises two or more deuterium atoms. In some embodiments, the compound comprises 1, 2, 3, 4, 5, 6, 7, 8, 9, 10, 11, or 12 deuterium atoms. Synthetic methods for including an isotope in an organic compound are known in the art.

**Preparation Method:**

The present disclosure further provides a method for preparing the compound. The compound of formula (I) of the present disclosure may be prepared by the following exemplary methods and examples; however, these methods and examples are not construed in any way as limiting the scope of the present disclosure. The compound of the present disclosure may also be synthesized by synthetic techniques known to those skilled in the art, or comprehensively using synthetic methods known in the art and the method of the present disclosure. The product from each reaction step is obtained by separation techniques known in the art, including but not limited to extraction, filtration, distillation, crystallization, chromatography separation, etc. The starting materials and chemical reagents required for the synthesis may be conventionally synthesized according to literature (as provided by Scifinder) or purchased.

Synthetic process route:

## Route I:

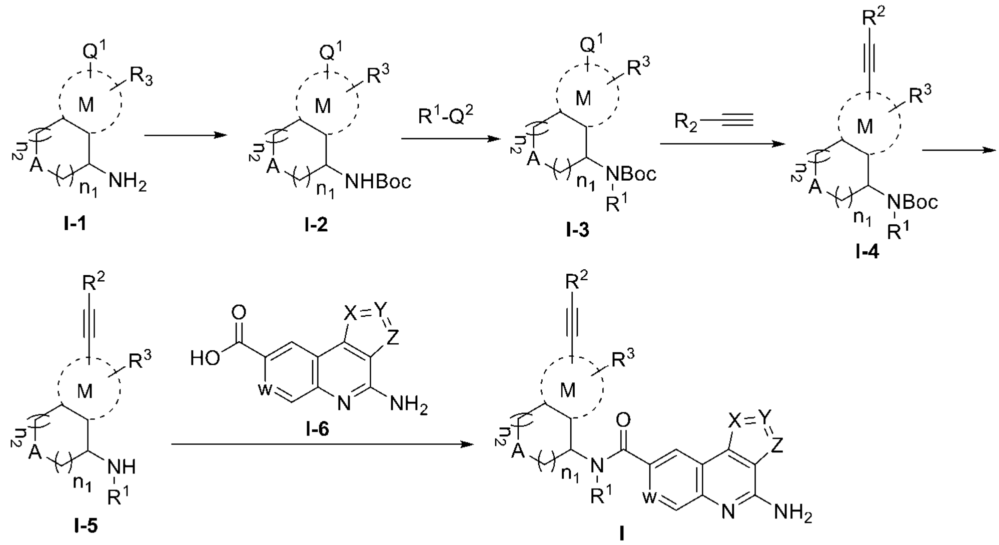


Step 1: Reacting a compound represented by formula I-1 with di-tert-butyl dicarbonate to yield a compound represented by formula I-2;

Step 2: Reacting the compound represented by formula I-2 with a compound represented by $R^1$-$Q^2$ under alkaline conditions well known in the art to yield a compound represented by formula I-3;

Step 3: Reacting the compound represented by formula I-3 with a compound represented by $R_2$-≡ under coupling conditions well known in the art to yield a compound represented by formula I-4;

Step 4: removing a Boc protecting group of the compound represented by formula I-4 under acidic conditions to yield a compound represented by formula I-5;

Step 5: Reacting the compound represented by formula I-5 with a compound represented by formula I-6 under condensation conditions well known in the art to yield the compound represented by formula I;

wherein, $Q^1$ and $Q^2$ are halogens, and X, Y, Z, W, A, $R^1$, $R^2$, $R^3$, $n_1$, $n_2$, and ring M are as defined in the formula (I) herein.

## Route II:

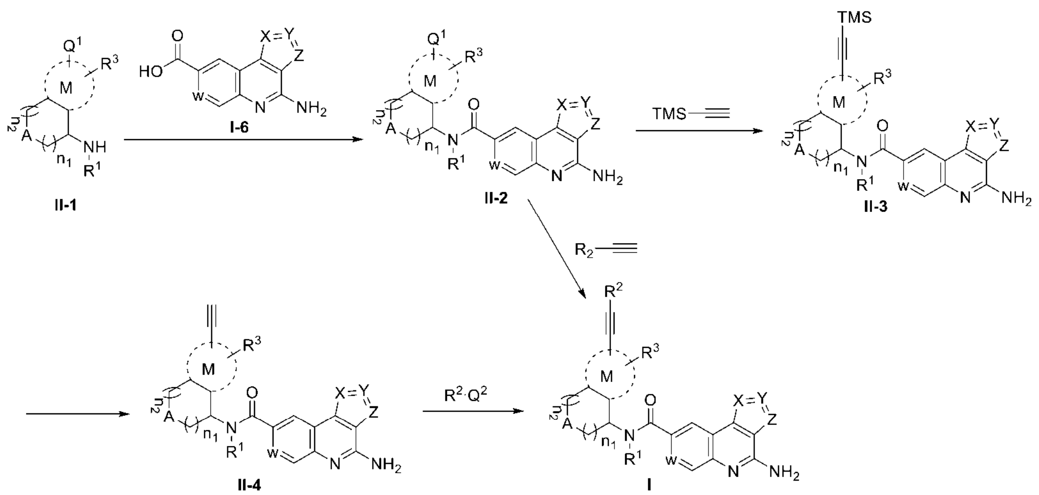


Step 1: Reacting a compound represented by formula II-1 with a compound represented by formula I-6 under condensation conditions well known in the art to yield a compound represented by formula II-2;

Step 2: Reacting the compound represented by formula II-2 with a compound represented by TMS-≡ under coupling conditions well known in the art to yield a compound represented by formula II-3;

Step 3: Reacting the compound represented by formula II-3 with tetrabutylammonium fluoride to yield a compound

represented by formula II-4;

Step 4: Reacting the compound represented by formula II-4 with a compound represented by $R_2$-$Q_2$ under coupling conditions well known in the art to yield the compound represented by formula I;

or,

Step 2: Reacting the compound represented by formula II-2 with a compound represented by $R_2$-≡ under coupling conditions well known in the art to yield the compound represented by formula I;

wherein, $Q^1$ and $Q^2$ are halogens, and X, Y, Z, W, A, $R^1$, $R^2$, $R^3$, $n_1$, $n_2$, and ring M are as defined in the formula (I) herein.

Route III:

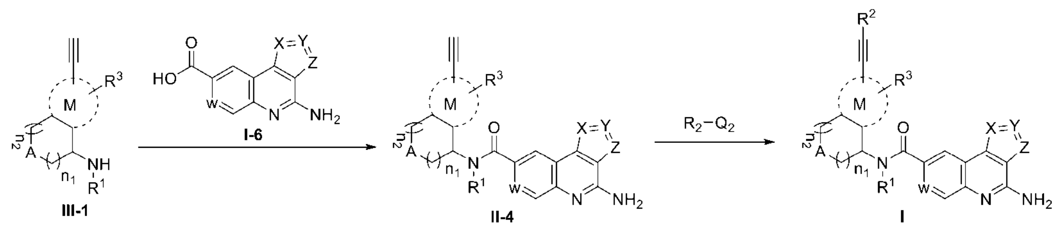


Step 1: Reacting a compound represented by formula III-1 with a compound represented by formula I-6 under condensation conditions well known in the art to yield a compound represented by formula II-4;

Step 2: Reacting the compound represented by formula II-4 with a compound represented by $R_2$-$Q_2$ under coupling conditions well known in the art to yield the compound represented by formula I;

wherein, $Q^2$ is halogen, and X, Y, Z, W, A, $R^1$, $R^2$, $R^3$, $n_1$, $n_2$, and ring M are as defined in the formula (I) herein.

**Pharmaceutical Composition:**

The present disclosure further provides a pharmaceutical composition for treating and/or preventing a disease associated with abnormal PRMT5 expression, which comprises a therapeutically and/or prophylactically effective amount of the compound or the tautomer, the stereoisomer, the pharmaceutically acceptable salt, or the deuterated compound thereof as described above, and optionally a pharmaceutical auxiliary material.

In some embodiments, the disease associated with abnormal PRMT5 expression is a tumor or cancer.

In some embodiments, the disease associated with abnormal PRMTS expression is a disease associated with abnormal PRMT5 expression and characterized by MTAP deletion.

In some embodiments, the disease associated with abnormal PRMT5 expression is a tumor or cancer characterized by MTAP deletion.

Methods for preparing various pharmaceutical compositions comprising certain amounts of active ingredients are known or, according to the content of the present disclosure, apparent to those skilled in the art. Methods for preparing the pharmaceutical composition comprise incorporating a suitable pharmaceutical excipient, carrier, diluent, etc., as described in REMINGTON'S PHARMACEUTICAL SCIENCES, Martin, E.W., ed., Mack Publishing Company, 19th ed. (1995).

The present disclosure further provides a PRMT5 inhibitor, which comprises a therapeutically and/or prophylactically effective amount of the compound or the tautomer, the stereoisomer, the pharmaceutically acceptable salt, or the deuterated compound thereof as described above.

**Pharmaceutical Use:**

The present disclosure further provides use of the compound or the tautomer, the stereoisomer, the pharmaceutically acceptable salt, or the deuterated compound thereof as described above, or the pharmaceutical composition as described above in preparing a PRMT5 inhibitor.

The present disclosure further provides use of the compound or the tautomer, the stereoisomer, the pharmaceutically acceptable salt, or the deuterated compound thereof as described above, or the pharmaceutical composition as described above in preparing a medicament for treating and/or preventing a disease associated with abnormal PRMT5 expression.

The present disclosure further provides use of the compound or the tautomer, the stereoisomer, the pharmaceutically acceptable salt, or the deuterated compound thereof as described above, or the pharmaceutical composition as described above in treating and/or preventing a disease associated with abnormal PRMT5 expression. The present disclosure further provides the compound or the tautomer, the stereoisomer, the pharmaceutically acceptable salt, or the

deuterated compound thereof as described above, or the pharmaceutical composition as described above for use in treating and/or preventing a disease associated with abnormal PRMT5 expression.

In some embodiments, the disease associated with abnormal PRMT5 expression is a tumor or cancer.

In some embodiments, the disease associated with abnormal PRMT5 expression is a disease associated with abnormal PRMT5 expression and characterized by MTAP deletion.

In some embodiments, the disease associated with abnormal PRMT5 expression is a tumor or cancer characterized by MTAP deletion.

The present disclosure further provides a method for treating and/or preventing a disease associated with abnormal PRMT5 expression, which comprises administering to an individual in need thereof a therapeutically and/or prophylactically effective amount of the compound or the tautomer, the stereoisomer, the pharmaceutically acceptable salt, or the deuterated compound thereof as described above, or the pharmaceutical composition as described above.

In some embodiments, the disease associated with abnormal PRMT5 expression is a tumor or cancer.

In some embodiments, the disease associated with abnormal PRMT5 expression is a disease associated with abnormal PRMT5 expression and characterized by MTAP deletion.

In some embodiments, the disease associated with abnormal PRMT5 expression is a tumor or cancer characterized by MTAP deletion.

In the present disclosure, "treating" or "treatment" usually refers to acquiring desired pharmacological and/or physiological effects. The effect may be prophylactic in terms of completely or partially preventing the disease or symptoms thereof, and/or therapeutic in terms of partially or completely stabilizing or curing the disease and/or side effects caused by the disease. As used herein, "treating" or "treatment" encompasses any treatment of a disease in a patient, including: (a) preventing a disease or symptom in a patient who is susceptible to the disease or symptom but has not yet been diagnosed as having the disease; (b) inhibiting symptoms of the disease, that is, preventing its progression; or (c) alleviating symptoms of the disease, that is, causing regression of the disease or symptom.

In the present disclosure, "subject" or "individual in need" refers to a vertebrate. In certain embodiments, the vertebrate refers to a mammal. The mammal includes, but is not limited to, livestock (such as cattle), pets (such as cats, dogs, and horses), primates, mice, and rats. In certain embodiments, the mammal refers to a human.

In the present disclosure, "effective amount" refers to an amount effective, at doses and for periods of time necessary, in achieving a desired therapeutic or prophylactic effect. The "therapeutically effective amount" of a substance/molecule of the present disclosure may vary depending on factors such as the disease state, age, sex, and body weight of the individual, as well as the ability of the substance/molecule to elicit a desired response in the individual. A therapeutically effective amount also encompasses an amount in which any toxic or detrimental consequence of the substance/molecule is outweighed by its therapeutically beneficial effects. "Prophylactically effective amount" refers to an amount effective, at doses and for periods of time necessary, in achieving a desired prophylactic effect. Typically but not necessarily, the prophylactically effective amount will be less than the therapeutically effective amount as the prophylactic dose is used in the subject prior to the onset of the disease or at an early stage of the disease. In the case of cancer, the therapeutically effective amount of the drug can reduce the number of cancer cells, reduce the tumor volume, inhibit (that is, slow to some extent, preferably stop) the infiltration of cancer cells into peripheral organs, inhibit (that is, slow to some extent, preferably stop) tumor metastasis, inhibit tumor growth to some extent, and/or palliate one or more cancer-associated symptoms to some extent.

**Definitions of Terms:**

According to a convention in the art,

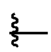

is used in the structural formulas herein to depict the bond that is the point of attachment of the moiety or substituent to the core or main structure.

A dash "-" that is not between two letters or symbols is used to indicate a point of attachment for a substituent. For example, $C_{3-6}$ cycloalkyl-($C_{1-6}$ alkyl)$_r$- means being linked to the remainder of the molecule via ($C_{1-6}$ alkyl)$_r$-.

The term "substituted" as used herein means that any one or more hydrogen atoms on a specified atom or group are replaced with a selection from a specified group, provided that the normal valence of the specified atom is not exceeded.

In each part of the specification, the substituents of the compound of the present disclosure are disclosed according to the group types or ranges. It is specifically noted that the present disclosure includes every individual subcombination of the members of these group types and ranges. For example, the term "$C_{1-6}$ alkyl" specifically refers to individually disclosed methyl, ethyl, $C_3$ alkyl, $C_4$ alkyl, $C_5$ alkyl, and $C_6$ alkyl, or individually disclosed "$C_{1-4}$ alkyl", or

individually disclosed "$C_{1-3}$ alkyl".

The term "alkyl" is meant to include branched and linear saturated aliphatic hydrocarbon groups with a specified number of carbon atoms. For example, "$C_{1-6}$ alkyl" refers to $C_1$, $C_2$, $C_3$, $C_4$, $C_5$, and $C_6$. In addition, for example, "$C_{1-6}$ alkyl" refers to alkyl having 1 to 6 carbon atoms. Alkyl may be unsubstituted or substituted such that one or more hydrogen atoms thereof are replaced with another chemical group. Examples of alkyl include, but are not limited to, methyl, ethyl, propyl (e.g., n-propyl and isopropyl), butyl (e.g., n-butyl, isobutyl, and tert-butyl), pentyl (e.g., n-pentyl, isopentyl, and neopentyl), etc. It should be understood by those skilled in the art that $C_{1-6}$ alkyl herein includes monovalent $C_{1-6}$ alkyl and divalent $C_{1-6}$ alkylene. For example, $C_1$-$C_6$ alkyl in $C_3$-$C_6$ cycloalkyl-($C_1$-$C_6$ alkyl)$_r$- refers to $C_1$ -$C_6$ alkylene.

The term "alkoxy" refers to any of the above alkyl groups (e.g., $C_{1-6}$ alkyl, $C_{1-4}$ alkyl, $C_{1-3}$ alkyl, etc.) attached to the remainder of the molecule via an oxygen atom (-O-).

The term "halogenated $C_{1-6}$ alkyl" or "halogenated $C_{1-6}$ alkoxy" means that one or more (e.g., 2 or 3) hydrogen atoms in the alkyl or alkoxy are substituted with halogen atoms, such as fluorine, chlorine, or bromine. The alkyl or alkoxy is as defined above. In some embodiments, the term "halogenated $C_{1-6}$ alkyl" preferably refers to fluoro-substituted $C_{1-6}$ alkyl, and may be, for example, $-CF_3$, $-CHF_2$, $-CH_2F$, $-CH_2CH_2F$, $-CH_2CHF_2$, $-CH_2CF_3$, or the like. In some embodiments, the term "halogenated $C_{1-6}$ alkoxy" preferably refers to fluoro-substituted $C_{1-6}$ alkoxy, and may be, for example, $-OCF_3$, $-OCHF_2$, $-OCH_2F$, $-OCH_2CH_2F$, $-OCH_2CHF_2$, $-OCH_2CF_3$, or the like.

The term "hydroxy-substituted $C_{1-6}$ alkyl" means that one hydrogen atom in the alkyl is substituted with hydroxy, and the alkyl is as defined above. As an example, the "hydroxy-substituted $C_{1-6}$ alkyl" may be hydroxymethyl.

The term "cycloalkyl" refers to a cyclized alkyl group, including monocyclic, bicyclic, or polycyclic ring systems. When a cycloalkyl group is bicyclic or polycyclic, each ring thereof should be a saturated carbocycle or carbocyclic residue. Every two rings of a bicyclic or polycyclic cycloalkyl group may be connected in manners including bridging, fusion, or spiro-linkage. For example, $C_{3-10}$ cycloalkyl is meant to include $C_3$, $C_4$, $C_5$, $C_6$, $C_7$, $C_8$, $C_9$, and $C_{10}$ cycloalkyl. Examples of cycloalkyl include, but are not limited to, cyclopropyl, cyclobutyl, cyclopentyl, cyclohexyl,

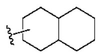 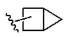 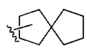 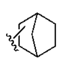 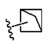,

etc.

The term "cycloalkenyl" refers to cycloalkyl having at least one carbon-carbon double bond in the cycloalkyl as defined above, e.g.,

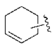.

The term "cycloalkoxy" refers to any of the cycloalkyl groups described above (e.g., $C_{3-6}$ cycloalkyl) attached to the remainder of the molecule via an oxygen atom (-O-).

The term "carbocycle" or "carbocyclic residue" refers to any stable 3-, 4-, 5-, 6-, or 7-membered monocyclic ring or 5-, 6-, 7-, 8-, 9-, 10-, 11-, 12-, 13-, or 14-membered bicyclic or polycyclic ring, wherein any one ring may be saturated, partially saturated, unsaturated, or aromatic. Every two rings of a bicyclic or polycyclic carbocycle may be connected in manners including bridging, fusion, or spiro-linkage. Examples of these carbocycles include, but are not limited to, cyclopropyl, cyclobutyl, cyclobutenyl, cyclopentyl, cyclopentenyl, cyclohexyl, cyclohexenyl, cycloheptenyl, cycloheptyl, adamantyl, cyclooctyl, phenyl, naphthyl, [2,2,2]bicyclooctane,

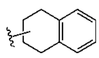 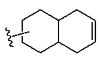 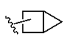 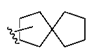 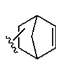 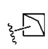,

etc.

The term "aryl" refers to a monocyclic, bicyclic, or tricyclic aromatic hydrocarbon group having 5-14 carbon atoms in the ring moiety. When the "aryl" is bicyclic or tricyclic, each ring thereof is an aromatic ring. Every two rings of a bicyclic or tricyclic aryl group may be connected in manners including bridging, fusion, or spiro-linkage. Examples include phenyl and naphthyl, each of which may be optionally substituted.

The terms "heterocycle", "heterocyclic", and "heterocyclyl" are used interchangeably and refer to substituted and unsubstituted 4-8 membered monocyclic or bicyclic groups, 8-10 membered bicyclic or tricyclic groups, and 10-14 membered tricyclic or polycyclic groups, wherein at least one ring has at least one heteroatom (O, S, or N), and the ring containing the heteroatom preferably has 1, 2, or 3 heteroatoms selected from O, S, and N. In these groups, each heteroatom-containing ring may contain 1 to 2 oxygen or sulfur atoms and/or 1-4 nitrogen atoms, provided that the total number of heteroatoms in each ring is 4 or less, and further provided that the ring contains at least one carbon atom. In some preferred embodiments, the heteroatom refers only to N or O, and the total number thereof does not exceed 3;

preferably, only 1-2 heteroatoms are contained. Carbon and sulfur atoms are optionally oxidized, and nitrogen atoms are optionally quaternized. Where the valence permits, a ring atom on a heterocycle is optionally substituted with =O (oxo) (e.g.,

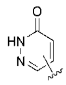 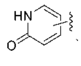

). The rings completing bicyclic and tricyclic groups may contain only carbon atoms and may be saturated, partially saturated, fully unsaturated, aromatic, or non-aromatic. A heterocyclyl group may be attached to any available nitrogen or carbon atom. As described above, heterocyclyl includes "spiroheterocyclyl", "heterobridged ring group", "heterocycloalkenyl", and the like as described below. Exemplary heterocyclyl includes, but is not limited to, azetidinyl, oxetanyl, pyrrolidinyl, imidazolinyl, oxazolidinyl, isoxazolinyl, thiazolidinyl, tetrahydrofuranyl, piperidinyl, piperazinyl, 2-oxopiperazinyl, 2-oxopiperidinyl, 2-oxopyrrolidinyl, 2-oxoazepinyl, 1-pyridonyl, 4-piperidonyl, tetrahydropyranyl, morpholinyl, 1,3-dioxolanyl, quinuclidinyl, and the like.

The term "saturated heterocyclyl" refers to the aforementioned "heterocyclyl" in which the monocyclic, bicyclic, or tricyclic group is in a fully saturated state. The "heterocyclyl" is as described above. As an example, the saturated heterocyclyl may be morpholinyl (e.g.,

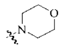

), piperidinyl (e.g.,

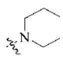

), piperazinyl (

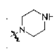

), or the like.

The term "heterocycloalkenyl" refers to heterocyclyl having at least one carbon-carbon double bond in the heterocycle as defined above, e.g.,

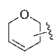 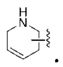

The term "heteroaryl" refers to substituted and unsubstituted aryl described above having at least one heteroatom (O, N, or S) in at least one ring, including aromatic 5-8 membered monocyclic groups, 8-10 membered bicyclic groups, and 10-14 membered tricyclic groups; the heteroatom-containing ring preferably has 1, 2, or 3 ring heteroatoms selected from O, N, and S. Each heteroatom-containing ring of heteroaryl may contain 1 or 2 oxygen or sulfur atoms and/or 1-4 nitrogen atoms, provided that the total number of heteroatoms in each ring is 4 or less and each ring has at least one carbon atom. Each ring of a bicyclic or tricyclic heteroaryl group is an aromatic ring. The term "spiro ring group" refers to a bicyclic ring structure having one common ring atom, and each monocyclic ring is a saturated or unsaturated, aromatic or non-aromatic carbocycle having 3-7 carbon atoms. Exemplary spirocycloalkyl includes, but is not limited to: spiro[4.5]decane, spiro[3.4]octane, spiro[2.3]hexane,

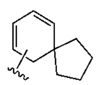,

and the like. Herein, the spiro ring group does not include aryl as defined above.

The terms "saturated spiro ring group" and "spirocycloalkyl" refer to "spiro ring group" as defined above in which each monocyclic ring is in a fully saturated state. Exemplary spirocycloalkyl includes, but is not limited to, spiro[4.5]decane, spiro[3.4]octane, and spiro[2.3]hexane.

The term "spiroheterocyclyl" refers to a bicyclic ring structure having one common ring atom or a tricyclic ring

structure having two independent common ring atoms, and each monocyclic ring is a saturated or unsaturated monocyclic group having 3-8 ring atoms, wherein at least one ring has 1 or 2 ring atoms selected from N, O, and $S(O)_n$, wherein n is an integer from 0 to 2, and the remaining ring atoms are C. In addition, 1 or 2 ring carbon atoms in the heterocyclyl ring are optionally replaced by a -CO- group. Exemplary spiroheterocyclyl includes, but is not limited to: 5-azaspiro[2.3]hexane and 6-oxaspiro[3.4]-7-octanone. Herein, the spiroheterocyclyl does not include the heteroaryl as defined above.

The terms "saturated spiroheterocyclyl" and "spiroheterocycloalkyl" refer to the "spiroheterocyclyl" as defined above in which each monocyclic ring is in a fully saturated state. Exemplary spirocycloalkyl includes, but is not limited to,

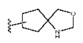 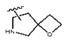 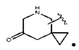.

The term "bridged ring group" refers to a saturated or unsaturated, aromatic or non-aromatic 3-8 membered monocyclic carbocyclic group, wherein two non-adjacent ring atoms are linked by a $(CRR)_n$ group, $C_{2-6}$ alkenyl, $C_{2-6}$ alkynyl, or a bond, wherein n is an integer from 1 to 3, and each R is independently H or methyl (wherein the $(CRR)_n$ group, the $C_{2-6}$ alkenyl, the $C_{2-6}$ alkynyl, or the bond is also referred to herein as a bridging group). The bridged ring group is optionally substituted with one or two substituents independently selected from alkyl, halogen, alkoxy, hydroxy, and cyano, wherein the alkyl, alkoxy, alkenyl, and alkynyl are each as defined above. Examples of the bridged ring group include, but are not limited to, bicyclo[2.2.1]heptane, bicyclo[2.2.2]octane, and the like. It should be understood herein that when two non-adjacent ring atoms of a monocyclic 4-7 membered hydrocarbon group are linked by a bond, the "bridged ring group" may be referred to as a "fused ring group" or a "condensed ring group". Herein, the bridged ring group does not include aryl as defined above.

The terms "saturated bridged ring group" and "bridged cycloalkyl" refer to "bridged ring group" as defined above in which each ring is in a saturated state. Herein, the saturated should be interpreted as meaning that each ring carbon atom in the bridged ring group is in a saturated state (including the carbon atoms in the bridging group).

The term "heterobridged ring group" refers to the "bridged ring group" as defined above in which 1, 2, 3, or 4 ring carbon atoms (including carbon atoms in the bridging group) are replaced with heteroatoms selected from N, O, and S(O) n, wherein n is an integer from 0 to 2. Examples of "heterobridged ring group" include, but are not limited to, 2-azabicyclo [2.2.2]octane, quinuclidine, 7-oxabicyclo[2.2.1]heptane, and the like. Herein, the heterobridged ring group does not include the heteroaryl as defined above.

The terms "saturated heterobridged ring group" and "heterobridged cycloalkyl" refer to the "heterobridged ring group" as defined above in which each ring is in a saturated state, wherein the saturated should be interpreted as meaning that each ring atom in the bridged ring group is in a saturated state (including carbon atoms/heteroatoms in the bridging group).

Unless indicated otherwise, when an explicitly named aryl (e.g., phenyl), cycloalkyl (e.g., cyclohexyl), heterocycle (e.g., pyrrolidinyl, piperidinyl, or morpholinyl), or heteroaryl (e.g., imidazolyl, pyrazolyl, or triazolyl) is mentioned, it means that a ring having 0-3, preferably 0-2, substituents is mentioned, and the substituents are selected from the substituents described above for aryl, cycloalkyl, heterocycle, and/or heteroaryl as appropriate.

The term "heteroatom" shall include oxygen, sulfur, and nitrogen.

The term "halogen" shall include "F, Cl, Br, and I".

When the term "unsaturated" is used herein to described a ring or a group, the ring or the group may be fully unsaturated or partially unsaturated.

When the term "saturated" is used herein to described a ring or a group, the ring or the group should be fully saturated, unless otherwise specified.

From all the above description, it will be apparent to those skilled in the art that any group whose name is a composite name, e.g., "$C_{3-10}$ cycloalkyl-C(O)-", shall refer to a moiety conventionally derived therefrom, e.g., constructed from carbonyl substituted with $C_{3-10}$ cycloalkyl, wherein the cycloalkyl is as defined above. Other similar composite names may be understood with reference to the foregoing content.

The term "optionally" means that it may or may not occur. For example, "$C_{1-6}$ alkyl optionally substituted with 1-3 $R^d$" means that the $C_{1-6}$ alkyl may or may not be substituted with 1-3 $R^d$. Other similar definitions may be understood with reference to the foregoing content.

The term "the ring atom of heterocyclyl is optionally oxo-substituted" means that the ring atom on the heterocycle is optionally substituted with =O (oxo) (for example, the ring atom $CH_2$ of

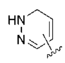 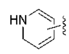,

after oxo-substitution, results in the group

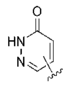 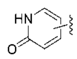

).

Throughout the specification, groups and substituents thereof may be chosen by one skilled in the art to provide stable moieties and compounds and compounds useful as pharmaceutically acceptable compounds and/or intermediate compounds useful in preparing pharmaceutically acceptable compounds.

Herein, unless otherwise explicitly indicated, the descriptive phrase "... be each independently selected from" used throughout the present disclosure may mean that specific items expressed by the same or different symbols in different groups do not affect each other, or that specific items expressed by the same or different symbols in the same group do not affect each other.

The term "XXX is substituted at any substitutable position with one or more substituents selected from YYY" means that XXX may be substituted at any substitutable position with one or more substituents and the substituents are selected from YYY. When XXX is substituted with a plurality of substituents selected from YYY at any substitutable positions, the plurality of substituents may be identical or different. "Plurality of" means 2 or more, preferably 2, 3, or 4, and more preferably 2 or 3. For example, $C_{1-6}$ alkyl substituted at any substitutable positions with one or more substituents selected from cyano and hydroxy means that $C_{1-6}$ alkyl may be substituted with one or more cyano at any substitutable positions, may also be substituted with one or more hydroxy at any substitutable positions, or may further be substituted with one or more cyano and hydroxy (e.g., one cyano and one hydroxy, or two cyano and one hydroxy, or two cyano and two hydroxy) at any substitutable positions.

Herein, when the structure does not accord with the chemical name, the structure prevails.

**Effects of the Present Disclosure:**

The compounds of the present disclosure exhibit excellent inhibitory activity against PRMT5. The compounds of the present disclosure demonstrate excellent selective inhibition against the human MTAP-deficient HCT-116 cell line over the MTAP wild-type (WT) HCT-116 cell line. The compounds can be used in the development of small-molecule drugs targeting PRMT5 MTA and preferentially act on MTAP-deficient tumor cells.

Further, it can be found from experiments that the compounds of the present disclosure exhibit different degrees of advantages in terms of human liver microsome stability, mouse pK, rat pK, and *in vivo* anti-tumor efficacy in an established mouse tumor model.

Further, compared with existing compounds, the compounds of the present disclosure possess a higher safety window, and are significantly superior to the prior art in terms of safety (e.g., toxicity from inhibition of hERG, CYP, etc.).

## DETAILED DESCRIPTION

It should also be understood that the terms used herein are for the purpose of describing specific embodiments only, and are not intended to be limiting. In addition, although any methods, apparatus, and materials similar or equivalent to those described herein may be used for practicing or testing the present disclosure, the preferred methods, apparatus, and materials are now described.

The structures of the compounds were determined by nuclear magnetic resonance (NMR) or mass spectrometry (MS). The NMR analysis was performed using a Bruker ASCENA-400 nuclear magnetic resonance instrument, with deuterated dimethyl sulfoxide (DMSO-d6), deuterated chloroform (CDCl3), and deuterated methanol ($CD_3OD$) as solvents and tetramethylsilane (TMS) as an internal standard. The chemical shifts were given in $10^{-6}$ (ppm).

The reaction monitoring and MS analysis were performed using a Thermofisher ESQ (ESI) mass spectrometer.

The HPLC analysis was performed using a Thermo Fisher U3000 DAD high pressure liquid chromatograph (with a GL Sciences ODS-HL HP 3 μm 3.0 × 100 mm chromatography column).

The thin-layer chromatography silica gel plates used were Qingdao Haiyang GF254 silica gel plates. The silica gel plates used in the thin-layer chromatography (TLC) analysis were 0.15-0.2 mm silica gel plates, and the plates used in the thin-layer chromatography separation and purification of the products were 0.9-1.0 mm preparative high-performance thin-layer chromatography plates. A Qingdao Haiyang 200-300 mesh silica gel was used as a carrier in the column chromatography. The developing solvent systems used included A: a dichloromethane and methanol system; B: a petroleum ether and ethyl acetate system. The volume ratio of the solvents was adjusted depending on the polarity of the compound. The preparative intermediate-pressure liquid chromatography purification was performed using a biotage isera one preparative liquid chromatography system.

In the following examples, unless otherwise specified, all reaction starting materials were commercially available from suppliers in the scifinder database. For example, some of the reagents in the examples of the present disclosure were

commercially available from manufacturers such as Sun Chemical Technology (Shanghai) Co., Ltd., Shanghai Accela ChemBio Co., Ltd., Nanjing PharmaBlock Sciences Inc., Jiangsu Aikon Biopharmaceutical R&D Co., Ltd., and Shanghai Bide Pharmatech Co., Ltd. Further, unless otherwise specified, the starting materials used in the examples of the present disclosure were all analytically pure. Unless otherwise specified, the ratios of two liquid substances involved herein were all volume ratios, and the percentages of the substances involved were mass percentages.

**Example 1: 4-amino-*N*,1-dimethyl-*N*-(6-((1-methyl-1*H*-pyrazol-4-yl)ethynyl)-2,3-dihydroben zofuran-3-yl)-1*H*-pyrazolo[4,3-*c*]quinoline-8-carboxamide (Compound 1)**

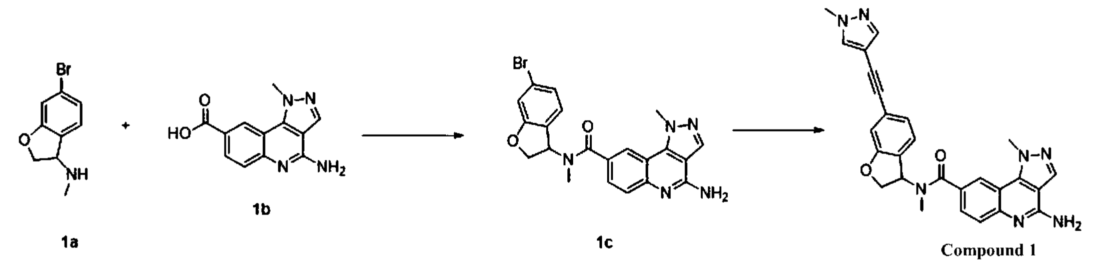

1a 1b 1c Compound 1

**Preparation of compound 1c**

Compound **1a** (86 mg, 0.38 mmol) was dissolved in *N,N*-dimethylacetamide (3 mL), and compound **1b** (94.2 mg, 0.39 mmol, see WO2022169948A1 for the preparation method), *N,N*-diisopropylethylamine (205.17 μL, 1.24 mmol), and O-(7-azabenzotriazol-1-yl)-*N,N,N',N'*-tetramethyluronium hexafluorophosphate (235.5 mg, 0.62 mmol) were added. After completion of the addition, the mixture was stirred at 25 °C for 3 h, water was added to quench the reaction, and the reaction solution was extracted with ethyl acetate (15 mL × 3). The organic phases were combined, washed with saturated brine, dried over anhydrous sodium sulfate, filtered, and concentrated, and the residue was purified by normal phase silica gel column chromatography (dichloromethane:methanol = 10:1) to give compound **1c** (60 mg, 0.13 mmol), ESI-MS(m/z): 452.3 $[M+H]^+$.

**Preparation of compound 1**

To a solution of compound **1c** (66 mg, 0.15 mmol) in *N*-methylpyrrolidone (1 mL) were sequentially added 4-ethynyl-1-methyl-1*H*-pyrazole (32 mg, 0.3 mmol), cuprous iodide (6 mg, 0.03 mmol), *N,N*-diisopropylethylamine (39 mg, 0.3 mmol), and tetrakis(triphenylphosphine)palladium (23 mg, 0.02 mmol) under nitrogen atmosphere. After completion of the addition, the mixture was reacted in a microwave reactor at 70 °C for 3 h. After the reaction was completed, water (10 mL) was added, and the reaction solution was extracted with ethyl acetate (10 mL × 3). The organic phases were combined, washed with saturated brine, dried over anhydrous sodium sulfate, and filtered. The solvent was removed by rotary evaporation, and the residue was purified by reversed phase column chromatography (acetonitrile:0.05% aqueous ammonium bicarbonate solution = 0:100% - 50%:50%) to give compound **1** (19 mg). ESI-MS (m/z): 478.1 $[M+H]^+$. $^1H$ NMR (400 MHz, DMSO-$d_6$) δ 8.33 (s, 1H), 8.28 (s, 1H), 8.08 (s, 1H), 7.69 (s, 1H), 7.65 - 7.63 (m 2H), 7.45 (d, *J* = 7.6 Hz, 1H), 7.16 (s, 2H), 7.10 (d, *J* = 8.0 Hz, 1H), 6.99 (s, 1H), 4.81 - 4.68 (m, 2H), 4.44 (s, 3H), 3.87 (s, 3H), 2.70 (s, 3H).

**Example 2: 4-amino-*N*-cyclopropyl-1-methyl-*N*-{6-[(1-methylpyrazol-4-yl)ethynyl]-2,3-dihyd ro-1-benzofuran-3-yl}pyrazolo[4,3-*c*]quinoline-8-carboxamide (Compound 2)**

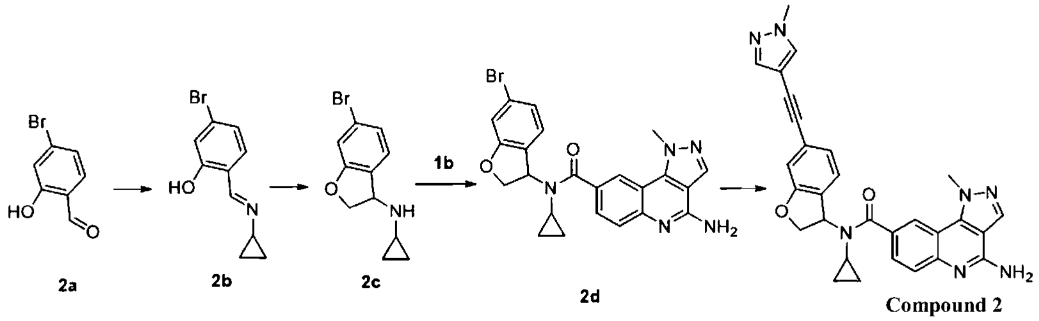

**Preparation of compound 2b**

To a solution of compound **2a** (1.0 g, 4.98 mmol) in dichloromethane (10 mL) were added cyclopropylamine (0.38 mL, 5.48 mmol) and acetic acid (0.09 mL, 1.49 mmol), and the mixture was allowed to react at 25 °C overnight. After the reaction was completed, the reaction solution was concentrated to give compound **2b** (1.0 g). ESI-MS (m/z): 240.0 $[M+H]^+$.

**Preparation of compound 2c**

To a solution of trimethylsulfoxonium iodide (2.3 g, 10.41 mmol) in tetrahydrofuran (10 mL) was added potassium tert-butoxide (1.16 g, 10.41 mmol), and the mixture was stirred at 25 °C for 30 min. Compound **2b** (1.0 g, 4.16 mmol) was dissolved in tetrahydrofuran (5 mL), and the resulting solution was then added dropwise to the system. The mixture was allowed to react at room temperature for 1 h, then heated to 50 °C, and then allowed to react for 3 h. Potassium tert-butoxide (4.16 mmol, 467 mg) was then added, and the mixture was allowed to react at 25 °C overnight. After the reaction was completed, water (15 mL) was added to quench the reaction, and the reaction solution was extracted with ethyl acetate (20 mL × 3). The organic phase was dried over anhydrous sodium sulfate and filtered. The filtrate was concentrated, and the residue was subjected to normal phase column chromatography (ethyl acetate/petroleum ether = 0:100% - 50%:50%) to give compound **2c** (200.0 mg). ESI-MS (m/z): 254.2 $[M+H]^+$.

**Preparation of compound 2d**

To a solution of compound **2c** (100 mg, 0.39 mmol) in *N*,*N*-dimethylacetamide (5 mL) were added compound **1b** (114.4 mg, 0.47 mmol), O-(I*H*-benzotriazol-1-yl)-*N*,*N*,*N'*,*N'*-tetramethylisouronium tetrafluoride (189.6 mg, 0.59 mmol), and *N*,*N*-diisopropylethylamine (0.20 mL, 1.18 mmol), and the mixture was allowed to react at 25 °C for 3 h. Water (20 mL) was added, and the reaction solution was extracted with ethyl acetate (20 mL × 3). The organic phase was washed with saturated brine, dried over anhydrous sodium sulfate, and filtered. The filtrate was concentrated, and the residue was subjected to normal phase column chromatography (methanol/dichloromethane = 0:100% - 10%:90%) to give compound **2d** (130.0 mg). ESI-MS (m/z): 478.2 $[M+H]^+$.

**Preparation of compound 2**

To a solution of compound **2d** (48 mg, 0.1 mmol) in N-methylpyrrolidone (1 mL) were sequentially added 4-ethynyl-1-methyl-1*H*-pyrazole (21 mg, 0.2 mmol), cuprous iodide (4 mg, 0.02 mmol), *N,N*-diisopropylethylamine (26 mg, 0.2 mmol), and tetrakis(triphenylphosphine)palladium (12 mg, 0.01 mmol) under nitrogen atmosphere. After completion of the addition, the mixture was reacted in a microwave reactor at 70 °C for 3 h. After the reaction was completed, water (10 mL) was added, and the reaction solution was extracted with ethyl acetate (10 mL × 3). The organic phases were combined, washed with saturated brine, dried over anhydrous sodium sulfate, and filtered. The solvent was removed by rotary evaporation, and the residue was purified by reversed phase column chromatography (acetonitrile:0.05% aqueous ammonium bicarbonate solution = 0:100% - 40%:60%) to give compound 2 (23 mg). ESI-MS (m/z): 503.7 $[M+H]^+$. $^1H$ NMR (400 MHz, DMSO-$d_6$) δ 8.46 (d, *J* = 2.0 Hz, 1H), 8.28 (s, 1H), 8.08 (s, 1H), 7.77 (dd, *J* = 8.0, 2.0 Hz, 1H), 7.70 (d, *J* = 0.8 Hz, 1H), 7.60 - 7.57 (m, 2H), 7.16 (s, 2H), 7.09 (dd, *J* = 7.6, 1.2 Hz, 1H), 6.98 (d, *J* = 1.2 Hz, 1H), 5.96 (dd, *J* = 9.2, 4.0 Hz, 1H), 4.85 - 4.67 (m, 2H), 4.42 (s, 3H), 3.87 (s, 3H), 2.88 - 2.79 (m, 1H), 0.46 - 0.06 (m, 4H).

**Example 3: 4-amino-*N*-methyl-*N*-(6-((1-methyl-1*H*-pyrazol-4-yl)ethynyl)-2,3-dihydrobenzofu ran-3-yl)-1,3-dihydrofuran[3,4-*c*]quinoline-8-carboxamide (Compound 3)**

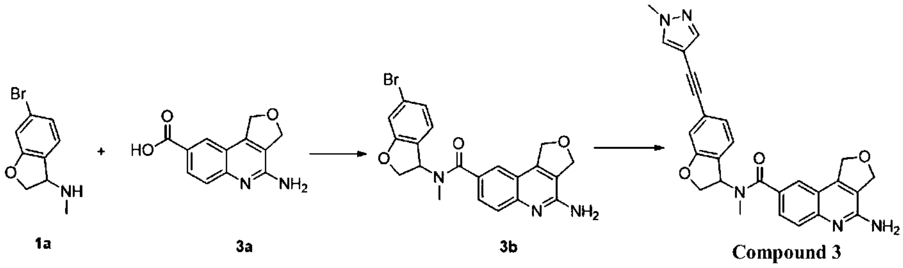

1a 3a 3b Compound 3

**Preparation of compound 3b**

To a solution of compound **1a** (50 mg, 0.22 mmol) in N,Ndimethylformamide (2 mL) were added compound **3a** (50.6 mg, 0.22 mmol, see WO2022169948 A1 for the preparation method), *N,N*-diisopropylethylamine (108.93 μL, 0.66 mmol), and O-(7-azabenzotriazol-1-yl)-*N,N,N',N'*-tetramethyluronium hexafluorophosphate(208.4 mg, 0.55 mmol). The mixture was allowed to react at 25 °C overnight. Water (50 mL) was added to the reaction solution, and the reaction solution was extracted with ethyl acetate (10 mL × 3). The organic phases were washed three times with saturated brine, combined, dried over anhydrous sodium sulfate, and filtered. The filtrate was concentrated, and the residue was subjected to normal phase column chromatography (dichloromethane/methanol = 10:1) to give compound **3b** (50 mg). ESI-MS (m/z): 439.92 $[M+H]^+$.

**Preparation of compound 3**

To a solution of **3b** (50 mg, 0.11 mmol) in N-methylpyrrolidone (2 mL) were added cuprous iodide (4.3 mg, 0.02 mmol), tetrakis(triphenylphosphine)palladium (13.1 mg, 0.01 mmol), *N,N*-diisopropylethylamine (56.43 μL, 0.34 mmol), and 4-ethynyl-1-methylpyrazole (155.8 mg, 1.59 mmol). After completion of the addition, the mixture was reacted in a microwave reactor at 70 °C for 3 h under nitrogen atmosphere. The reaction solution was filtered through celite. Water (50 mL) was added, and the reaction solution was extracted with ethyl acetate (10 mL × 3). The organic phases were combined, washed with saturated brine, dried over anhydrous sodium sulfate, and filtered. The filtrate was concentrated, and the residue was subjected to normal phase column chromatography (dichloromethane/methanol = 10:1) to give a crude compound. The crude compound was then subjected to reversed phase column chromatography (acetonitrile/0.05% ammonium bicarbonate solution = 0:100% - 40%:60%) to give compound **3** (16 mg). ESI-MS (m/z): 466.1 $[M+H]^+$. $^1H$ NMR (400 MHz, DMSO-$d_6$) δ 8.06 (s, 1H), 7.68 - 7.57 (m, 2H), 7.39 (d, *J* = 7.6 Hz, 1H), 7.08 (d, *J* = 7.6 Hz, 1H), 6.96 (s, 1H), 6.69 (s, 2H), 6.30 - 5.64 (m, 1H), 5.40 - 5.33 (m, 2H), 5.06 - 4.97 (m, 2H), 4.72 - 4.60 (m, 2H), 3.85 (s, 3H), 2.62 (s, 3H).

**Example 4: 4-amino-*N*-(6-((1-cyclopropyl-1*H*-pyrazol-4-yl)ethynyl)-2,3-dihydrobenzofuran-3 -yl)-*N*,1-dimethyl-1*H*-pyrazolo[4,3-c]quinoline-8-carboxamide (Compound 4)**

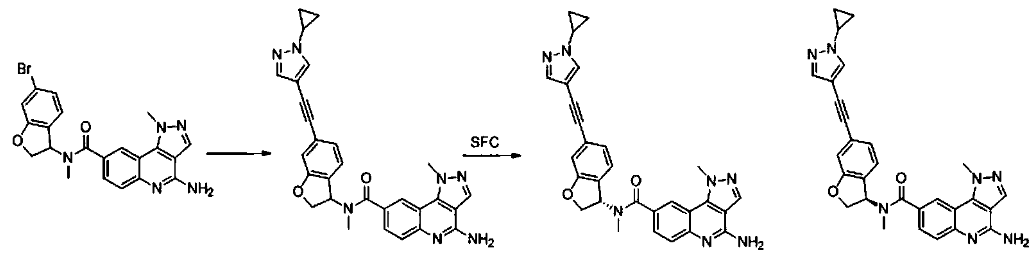


1c Compound 4 Compound 4A Compound 4B

To a solution of **1c** (63 mg, 0.14 mmol) in *N*-methylpyrrolidone (2 mL) were added cuprous iodide (4 mg, 0.02 mmol), tetrakis(triphenylphosphine)palladium (32 mg, 0.03 mmol), *N,N*-diisopropylethylamine (90 mg, 0.7 mmol), and 1-cyclopropyl-4-ethynyl-1*H*-pyrazole (55 mg, 0.42 mmol). After completion of the addition, the mixture was reacted in a microwave reactor at 80 °C for 3 h under nitrogen atmosphere. The reaction solution was filtered through celite. Water (15 mL) was added, and the reaction solution was extracted with ethyl acetate (10 mL × 3).

The organic phases were combined, washed with saturated brine, dried over anhydrous sodium sulfate, and filtered. The filtrate was concentrated, and the residue was subjected to reversed phase column chromatography

(acetonitrile/0.05% ammonium bicarbonate solution = 0:100% - 50%:50%) to give compound **4** (15 mg). ESI-MS (m/z): 504.1 [M+H]$^+$. $^1$H NMR (400 MHz, DMSO-$d_6$) δ 8.33 (s, 1H), 8.28 (s, 1H), 8.18 (s, 1H), 7.71 - 7.62 (m, 3H), 7.45 (d, *J* = 7.6 Hz, 1H), 7.16 - 7.06 (m, 3H), 6.97 (s, 1H), 6.39 - 5.84 (m, 1H), 4.79 - 4.69 (m, 2H), 4.44 (s, 3H), 3.79 - 3.74 (m, 1H), 2.70 (s, 3H), 1.11 - 0.97 (m, 4H).

Compound **4** was separated by SFC (instrument: Waters 150 Prep-SFC; chromatographic column: Chiral AS column; mobile phase A: $CO_2$, mobile phase B: methanol (containing 0.1% $NH_3$•$H_2O$); B%: 35%) to give compounds **4A** and **4B.** Compound **4A:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 2.176 min, ee = 100%.

Compound **4B:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 2.565 min, ee = 100%.

### Example 5: 4-amino-7-fluoro-*N*,1-dimethyl-*N*-(6-((1-methyl-1*H*-pyrazol-4-yl)ethynyl)-2,3-dih ydrobenzofuran-3-yl)-1*H*-pyrazolo[4,3-*c*]quinoline-8-carboxamide (Compound 5)

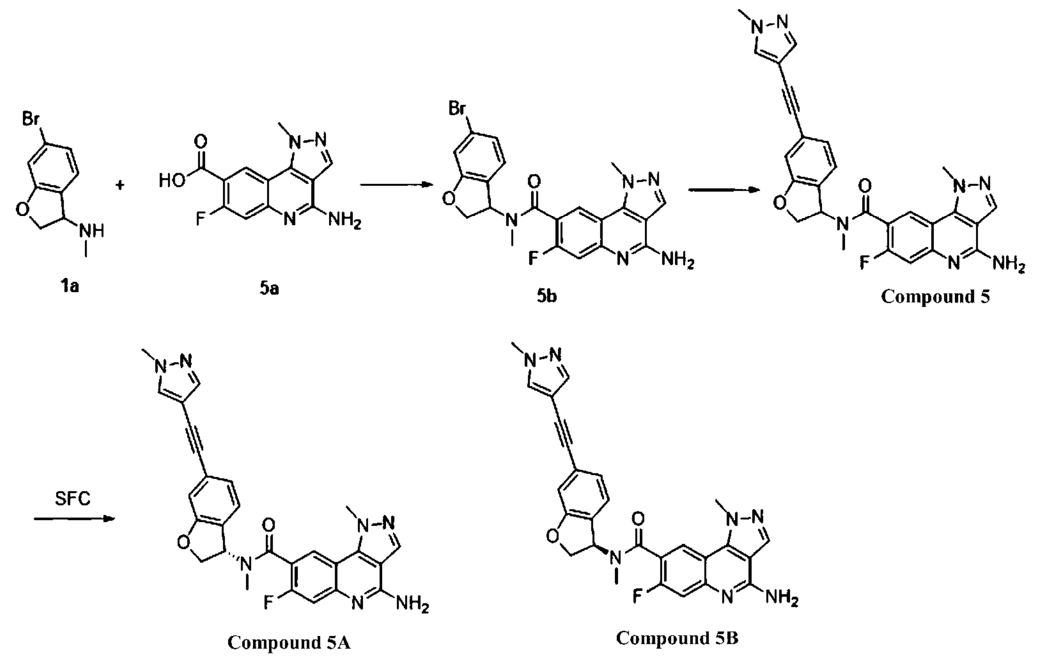


1a 5a 5b Compound 5

Compound 5A Compound 5B

### Preparation of compound 5b

To a solution of compound **1a** (50 mg, 0.22 mmol) in *N,N*-dimethylformamide (2 mL) were added compound **5a** (57.2 mg, 0.22 mmol, see WO2022169948 A1 for the preparation method), *N,N*-diisopropylethylamine (108.93 μL, 0.66 mmol), and O-(7-azabenzotriazol-1-yl)-*N,N,N',N'*-tetramethyluronium hexafluorophosphate(208.4 mg, 0.55 mmol). After completion of the addition, the mixture was allowed to react at 25 °C overnight. Water (50 mL) was added to the reaction solution, and the reaction solution was extracted with ethyl acetate (10 mL × 3). The organic phases were washed three times with saturated brine, combined, dried over anhydrous sodium sulfate, and filtered. The filtrate was concentrated, and the residue was subjected to normal phase column chromatography (dichloromethane/methanol = 10:1) to give compound **5b** (55 mg). ESI-MS (m/z): 469.95 [M+H]$^+$.

### Preparation of compound 5

To a solution of compound **5b** (55 mg, 0.12 mmol) in N-methylpyrrolidone (2 mL) were added cuprous iodide (4.5 mg, 0.02 mmol), tetrakis(triphenylphosphine)palladium (13.5 mg, 0.01 mmol), *N,N*-diisopropylethylamine (58.11 μL, 0.35 mmol), and 4-ethynyl-1-methylpyrazole (62.0 mg, 0.58 mmol). After completion of the addition, the mixture was reacted in a microwave reactor at 70 °C for 3 h under nitrogen atmosphere. The reaction solution was filtered through celite. Water (50 mL) was added, and the reaction solution was extracted with ethyl acetate (10 mL × 3). The organic phases were

combined, washed with saturated brine, dried over anhydrous sodium sulfate, and filtered. The filtrate was concentrated, and the residue was subjected to normal phase column chromatography (methanol/dichloromethane = 10:1) to give a crude compound. The crude compound was then subjected to reversed phase column chromatography (acetonitrile/0.05% ammonium bicarbonate solution = 0:100% - 45%:55%) to give compound **5** (18 mg). ESI-MS (m/z): 496.0 $[M+H]^+$. $^1H$ NMR (400 MHz, DMSO-$d_6$) δ 8.29 - 8.22 (m, 2H), 8.05 (s, 1H), 7.67 (s, 1H), 7.39 - 7.27 (m, 4H), 7.12 - 7.06 (m, 1H), 6.99 - 6.92 (m, 1H), 6.48 - 5.56 (m, 1H), 4.83 - 4.64 (m, 2H), 4.41 - 4.39 (m, 3H), 3.85 (s, 3H), 2.69 - 2.57 (m, 3H).

Compound **5** was separated by SFC (instrument: Waters 150 Prep-SFC; chromatographic column: Chiral AS column; mobile phase A: $CO_2$, mobile phase B: methanol (containing 0.1% $NH_3$•$H_2O$); B%: 35%) to give compounds **5A** and **5B.** Compound **5A:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 1.997 min, ee = 99.7%. $^1H$ NMR (400 MHz, DMSO-$d_6$) δ 8.29 - 8.24 (m, 2H), 8.07 (s, 1H), 7.67 (s, 1H), 7.40 - 7.29 (m, 4H), 7.12 - 7.07 (m, 1H), 6.99 - 6.94 (m, 1H), 6.48 - 5.56 (m, 1H), 4.83 - 4.64 (m, 2H), 4.41 - 4.39 (m, 3H), 3.85 (s, 3H), 2.69 - 2.58 (m, 3H). $^1H$ NMR (400 MHz, DMSO-$d_6$, 90 °C) δ 8.25 - 8.23 (m, 2H), 7.96 (s, 1H), 7.60 (s, 1H), 7.34 - 7.30 (m, 2H), 7.07 (dd, *J* = 7.6, 1.4 Hz, 1H), 6.98 (s, 2H), 6.92 (s, 1H), 4.69 - 4.58 (m, 2H), 4.38 (s, 3H), 3.84 (s, 3H), 2.65 (s, 3H). $[\alpha]^{D}_{25}$ +201.4 (c 1mg/mL, DMF).

Compound **5B:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 2.237 min, ee = 100%. $^1H$ NMR (400 MHz, DMSO-$d_6$) δ 8.33 - 8.22 (m, 2H), 8.05 (s, 1H), 7.67 (s, 1H), 7.40 - 7.27 (m, 4H), 7.12 - 7.06 (m, 1H), 6.99 - 6.95 (m, 1H), 6.46 - 5.56 (m, 1H), 4.83 - 4.63 (m, 2H), 4.41 - 4.39 (m, 3H), 3.85 (s, 3H), 2.69 - 2.57 (m, 3H). $^1H$ NMR (400 MHz, DMSO-$d_6$, 90 °C) δ 8.25 - 8.23 (m, 2H), 7.96 (s, 1H), 7.60 (s, 1H), 7.34 - 7.31 (m, 2H), 7.07 (d, *J* = 7.6 Hz, 1H), 6.98 (s, 2H), 6.92 (s, 1H), 4.70 - 4.58 (m, 2H), 4.38 (s, 3H), 3.84 (s, 3H), 2.65 (s, 3H). $[\alpha]^{D}_{25}$ -215.6 (c 1mg/mL, DMF).

### Example 5A: *(S)*-4-amino-7-fluoro-*N*,1-dimethyl-*N*-(6-((1-methyl-1*H*-pyrazol-4-yl)ethynyl)-2,3-dihydrobenzofuran-3-yl)-1*H*-pyrazolo[4,3-*c*]quinoline-8-carboxamide (Compound 5A)

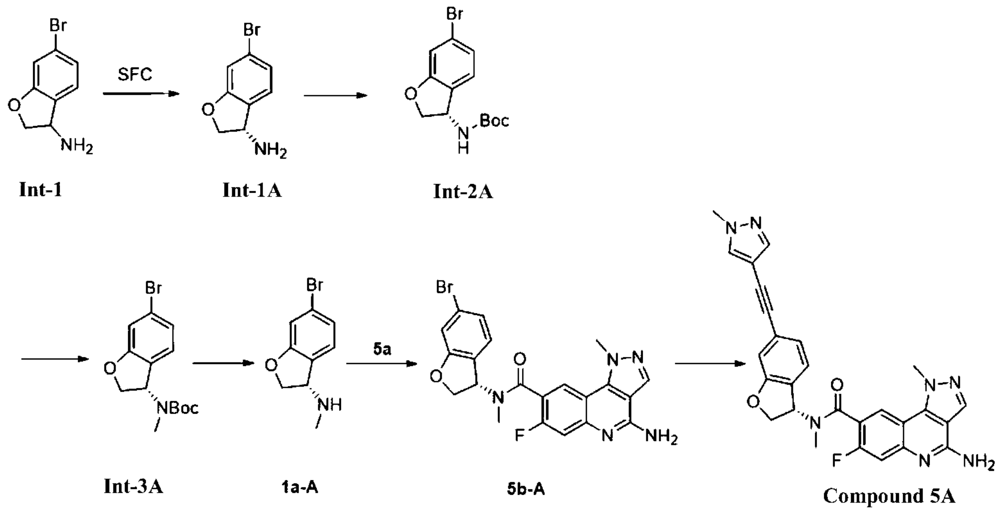


Int-1 Int-1A Int-2A

Int-3A 1a-A 5b-A Compound 5A

**Compound Int-1 was separated by SFC** (instrument: Shimadzu Prep-SFC; chromatographic column: OptiChiral A1 column (20 × 250 mm, 5 μm); mobile phase A: $CO_2$, mobile phase B: ethanol (containing 0.1% $NH_3$•$H_2O$); B%: 35%) to give two fractions.

Compound **Int-1A:** SFC analytical method: instrument: SHIMADZU LC-20AD; chromatographic column: OptiChiral A1-3 column (100 × 4.6 mm, 3 μm); mobile phase A: $CO_2$, mobile phase B: ethanol (containing 0.05% diethylamine); B%: 30%; retention time: 1.924 min, ee = 100%.

**Reference was made to** WO2024131901 A1 **for the preparation process of compound Int-3A.**

Confirmation of absolute configuration of compound **Int-2A:** the crystal structure of **Int-2A** was determined by MicroED. The crystal structure belongs to a triclinic crystal system with a P1 (No. 1) space group. The unit cell constants measured by MicroED are that a = 5.160(18) Å, b = 5.599(12) Å, c = 11.582(9) Å, α = 97.51(12)°, β = 89.49(15)°, γ = 99.60(18)°, and the unit cell volume V = 327.1(14) $Å^3$. Z' of the system is 1, and the asymmetric unit of the crystal consists of one API molecule.

### Preparation of compound 1a-A

Compound **Int-3A** (310 mg, 0.95 mmol) was dissolved in tetrahydrofuran (2 m L), and a 4 M dioxane solution of hydrochloric acid (2 mL) was added. The r eaction solution was stirred at 25 °C for 1 h. The reaction solution was then concentrated to dryness by rotary evaporation, slurried with methyl tert-butyl et her (5 mL), and filtered to give **1a-A** (180 mg). ESI-MS (m/z): 227.96 $[M+H]^+$.

**Preparation of compound 5A:** For the synthesis process of preparing compound **5A** from compound **1a-A,** reference was made to the synthesis process of preparing compound **5** from compound **1a**. ESI-MS (m/z): 496.0 $[M+H]^+$. $[\alpha]^{D}_{25}$ +213.4 (c 1mg/mL, DMF).

### Example 6: 4-amino-*N*,1-dimethyl-*N*-(6-((1-methyl-1,2,3,6-tetrahydropyridin-4-yl)ethynyl)-2, 3-dihydrobenzofuran-3-yl)-1*H*-pyrazolo[4,3-*c*]quinoline-8-carboxamide (Compound 6)

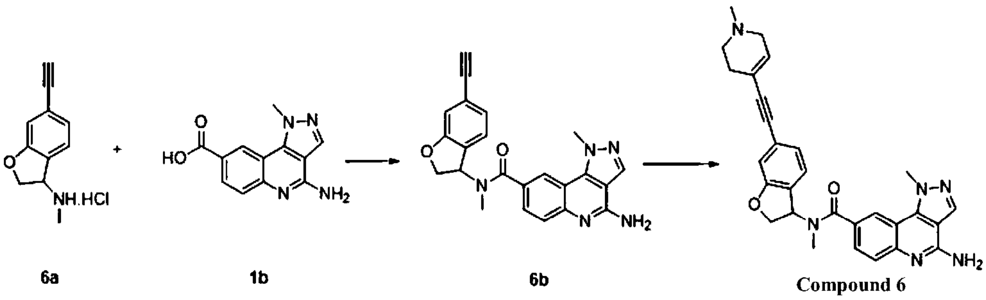

6a 1b 6b Compound 6

### Preparation of compound 6b

To a solution of compound **6a** (41.9 mg, 0.20 mmol, see WO2024131901 A1 for the preparation method) in *N,N*-dimethylacetamide (3 mL) were added compound **1b** (48.4 mg, 0.20 mmol), triethylamine (0.07 mL, 0.50 mmol), and O-(7-azabenzotriazol-1-yl)-*N,N,N',N'*-tetramethyluronium hexafluorophosphate (156.0 mg, 0.30 mmol). After completion of the addition, the mixture was allowed to react at 25 °C for 18 h. The reaction solution was diluted with ethyl acetate (10 mL) and water (10 mL), followed by liquid separation. The organic phase was concentrated and purified by high-pressure liquid chromatography to give compound **6b** (5 mg). ESI-MS (m/z): 398.17 $[M+H]^+$.

### Preparation of compound 6

To a solution of compound **6b** (50 mg, 0.13 mmol) in *N,N*-dimethylformamide (2 mL) were sequentially added 1-methyl-1,2,3,6-tetrahydropyridin-4-yl trifluoromethanesulfonate (64 mg, 0.26 mmol), cuprous iodide (9.90 mg, 0.05 mmol), *N,N*-diisopropylethylamine (134.16 mg, 1.04 mmol), and tetrakis(triphenylphosphine)palladium (30.1 mg, 0.03 mmol) under nitrogen atmosphere. After completion of the addition, the mixture was allowed to react at 25 °C for 3 h. Water (10 mL) was added to the reaction solution, and the reaction solution was extracted with ethyl acetate (10 mL $\times$ 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give a crude product. The crude product was subjected to reversed phase chromatography (acetonitrile/0.05% aqueous ammonia bicarbonate solution = 0:100% - 40%:60%) to give compound **6** (15 mg). ESI-MS (m/z): 493.38 $[M+H]^+$. $^1H$ NMR (400 MHz, DMSO-$d_6$) $\delta$ 8.33 (s, 1H), 8.28 (s, 1H), 7.67 - 7.62 (m, 2H), 7.43 (d, *J* = 7.6 Hz, 1H), 7.16 (s, 2H), 7.05 (d, *J* = 7.6 Hz, 1H), 6.94 (s, 1H), 6.43 - 5.80 (m, 2H), 4.75 - 4.70 (m, 2H), 4.44 (s, 3H), 2.99 - 2.96 (m, 2H), 2.69 (s, 3H), 2.50 - 2.47 (m, 2H), 2.31 - 2.26 (m, 3H).

### Example 7: 4-amino-*N*-ethyl-1-methyl-N-(6-((1-methyl-1H-pyrazol-4-yl)ethynyl)-2,3-dihydro benzofuran-3-yl)-1*H*-pyrazolo[4,3-*c*]quinoline-8-carboxamide (Compound 7)

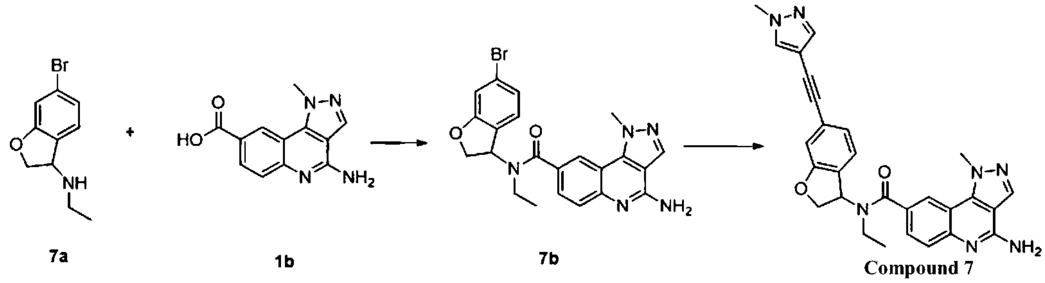

7a 1b 7b Compound 7

## Preparation of compound 7b

Compound **1b** (100 mg, 0.41 mol) was dissolved in *N,N*-dimethylformamide (10 mL), and *N,N*-diisopropylethylamine (0.36 mL, 2.1 mol) was added. The mixture was stirred for 5 min under an ice bath, and then *N,N,N,N'*-tetramethyl-*O*-(7-azabenzotriazol-1-yl)uronium hexafluorophosphate (236 mg, 0.62 mol) was added. The mixture was allowed to react for another 5 min, and finally, **7a** (110 mg, 0.45 mol) was added. The mixture was allowed to react for another hour, water (30 mL) was then added to the reaction solution, and the reaction solution was extracted with ethyl acetate (20 mL × 3). The organic phases were washed three times with saturated brine, combined, dried over anhydrous sodium sulfate, and filtered. The filtrate was concentrated, and the residue was subjected to column chromatography (methanol:dichloromethane = 1:20) to give compound **7b** (120 mg). ESI-MS (m/z): 465.91 $[M+H]^+$.

## Preparation of compound 7

To a solution of compound **7b** (120 mg, 0.26 mmol) in N-methylpyrrolidone (3 mL) were sequentially added 4-ethynyl-1-methyl-1*H*-pyrazole (54.7 mg, 0.52 mmol), cuprous iodide (9.8 mg, 0.05 mmol), *N,N*-diisopropylethylamine (0.18 mL, 1.03 mmol), and tetrakis(triphenylphosphine)palladium (29.8 mg, 0.026 mmol) under nitrogen atmosphere. After completion of the addition, the mixture was reacted in a microwave reactor at 70 °C for 3 h. Water (50 mL) was added to the reaction solution, and the reaction solution was extracted with ethyl acetate (20 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give compound 7 (60 mg). ESI-MS (m/z): 492.22 $[M+H]^+$. $^1$H NMR (400 MHz, DMSO) δ 8.28 (s, 2H), 8.08 (s, 1H), 7.69 - 7.59 (m, 3H), 7.48 (d, *J* = 7.6 Hz, 1H), 7.16 (s, 2H), 7.08 (dd, *J* = 7.6 1.2 Hz, 1H), 6.99 (s, 1H), 5.92 (s, 1H), 4.80 - 4.63 (m, 2H), 4.44 (s, 3H), 3.87 (s, 3H), 3.30 - 3.22 (m, 2H), 0.99 - 0.89 (m, 3H).

## Example 8: 4-amino-7-chloro-*N*-(6-((1-cyclopropyl-1*H*-pyrazol-4-yl)ethynyl)-2,3-dihydroben zofuran-3-yl)-*N*,1-dimethyl-1*H*-pyrazolo[4,3-*c*]quinoline-8-carboxamide (Compound 8)

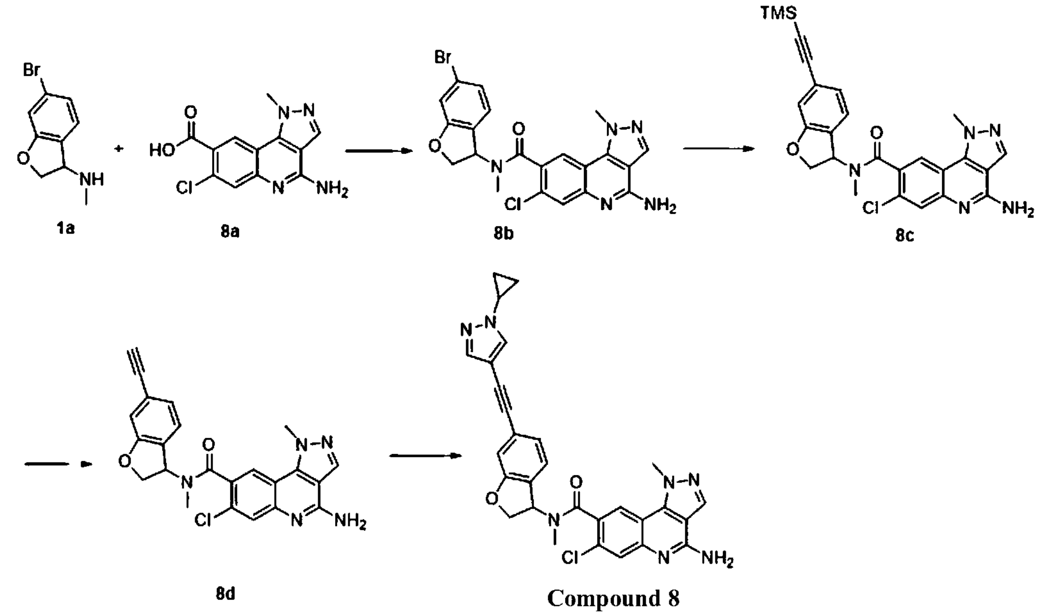

1a 8a 8b 8c

8d Compound 8

**Preparation of compound 8b**

To a solution of compound **1a** (230 mg, 1.01 mmol) in *N,N*-dimethylformamide (10 mL) were added compound **8a** (279 mg, 1.01 mmol, see WO2022169948 A1 for the preparation method), triethylamine (255.1 mg, 2.52 mmol), and O-(7-azabenzotriazol-1-yl)-*N*,*N*,*N'*,*N'*-tetramethyluronium hexafluorophosphate (576.3 mg, 1.51 mmol), and the mixture was allowed to react at 25 °C overnight. Water (50 mL) was added to the reaction solution, and the reaction solution was extracted with ethyl acetate (25 mL × 2). The organic phases were washed three times with saturated brine, combined, dried over anhydrous sodium sulfate, and filtered. The filtrate was concentrated, and the residue was subjected to normal phase column chromatography (dichloromethane/methanol = 50:1) to give compound **8b** (280 mg). ESI-MS (m/z): 487.89 $[M+H]^+$.

**Preparation of compound 8c**

To a solution of compound **8b** (280 mg, 0.58 mmol) in *N*-methylpyrrolidone (3 mL) were added cuprous iodide (21.9 mg, 0.12 mmol), tetrakis(triphenylphosphine)palladium (66.5 mg, 0.06 mmol), *N,N*-diisopropylethylamine (371.52 mg, 2.88 mmol), and trimethylsilylacetylene (113 mg, 1.15 mmol). After completion of the addition, the mixture was reacted in a microwave reactor at 70 °C for 3 h under nitrogen atmosphere. The reaction solution was filtered through celite. Water (50 mL) was added, and the reaction solution was extracted with ethyl acetate (25 mL × 2). The organic phases were combined, washed with saturated brine, dried over anhydrous sodium sulfate, and filtered. The filtrate was concentrated, and the residue was subjected to normal phase column chromatography (dichloromethane/methanol = 20:1) to give compound **8c** (220 mg). ESI-MS (m/z): 504.3 $[M+H]^+$.

**Preparation of compound 8d**

Tetrabutylammonium fluoride (870 μL, 0.87 mmol) was added to a solution of compound **8c** (220 mg, 0.44 mmol) in tetrahydrofuran (5 mL), and the mixture was allowed to react at 25 °C for 1 h. Water (15 mL) was added to the reaction solution, and the reaction solution was extracted with ethyl acetate (25 mL × 2). The organic phase was dried over anhydrous sodium sulfate and filtered. The filtrate was concentrated, and the residue was separated and purified by a preparative plate (dichloromethane/methanol = 10:1) to give compound **8d** (160 mg). ESI-MS (m/z): 432.03 $[M+H]^+$.

**Preparation of compound 8**

To a solution of compound **8d** (43 mg, 0.10 mmol) in *N,N*-dimethylformamide (2 mL) were sequentially added 1-cyclopropyl-4-iodopyrazole (46.6 mg, 0.20 mmol), cuprous iodide (7.60 mg, 0.04 mmol), *N,N*-diisopropylethylamine (51.6 mg, 0.4 mmol), and tetrakis(triphenylphosphine)palladium (23 mg, 0.02 mmol) under nitrogen atmosphere. After completion of the addition, the mixture was allowed to react at 25 °C for 3 h. Water (10 mL) was added to the reaction solution, and the reaction solution was extracted with ethyl acetate (10 mL ×3). The organic phases were combined,

washed with saturated brine, dried over anhydrous sodium sulfate, and filtered. The solvent was removed by rotary evaporation, and the residue was subjected to column chromatography (methanol:dichloromethane = 1:20) to give a crude product. The crude product was then subjected to reversed phase chromatography (acetonitrile/0.05% aqueous ammonia bicarbonate solution = 0:100% - 40%:60%) to give compound **8** (28 mg). ESI-MS (m/z): 538.15 [M+H]$^+$. $^1$H NMR (400 MHz, DMSO-$d_6$) δ 8.41 - 8.21 (m, 2H), 8.19 - 8.15 (m, 1H), 7.72 - 7.61 (m, 2H), 7.54 - 7.30 (m, 3H), 7.15 - 6.94 (m, 2H), 6.53 - 6.48 and 5.46 - 5.42 (m, 1H), 4.91 - 4.53 (m, 2H), 4.48 - 4.38 (m, 3H), 3.79 - 3.74 (m, 1H), 2.73 - 2.50 (m, 3H), 1.11 - 1.04 (m, 2H), 1.03 - 0.97 (m, 2H).

**Example 8A: (*S*)-4-amino-7-chloro-*N*-(6-((1-cyclopropyl-1*H*-pyrazol-4-yl)ethynyl)-2,3-dihydro benzofuran-3-yl)-*N*,1-dimethyl-1*H*-pyrazolo[4,3-*c*]quinoline-8-carboxamide (Compound 8A)**

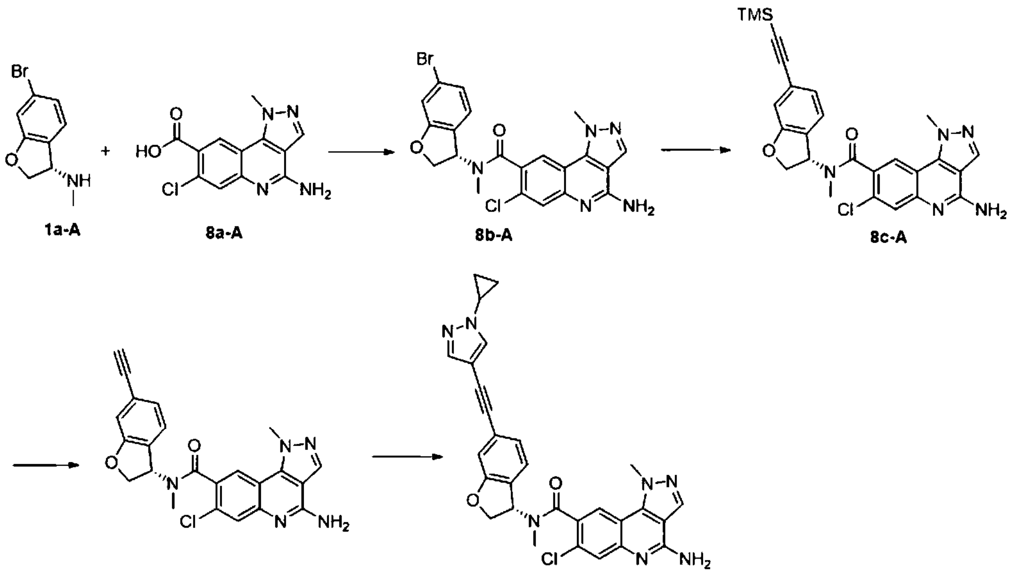

1a-A 8a-A 8b-A 8c-A

8d-A Compound 8A

For the synthesis process of preparing compound **8A** from compound **1a-A**, reference was made to the synthesis process of preparing compound **8** from compound **1a**. ESI-MS (m/z): 538.0 [M+H]$^+$. $^1$H NMR (400 MHz, DMSO-$d_6$) δ 8.41 - 8.21 (m, 2H), 8.19 - 8.15 (m, 1H), 7.72 - 7.61 (m, 2H), 7.54 - 7.38 (m, 1H), 7.32 - 7.30 (m, 2H), 7.15 - 6.94 (m, 2H), 6.53 - 5.42 (m, 1H), 4.91 - 4.53 (m, 2H), 4.48 - 4.38 (m, 3H), 3.79 - 3.74 (m, 1H), 2.73 - 2.50 (m, 3H), 1.11 - 0.97 (m, 4H).

**Example 9: 4-amino-7-chloro-*N*,1-dimethyl-*N*-(6-((1-methyl-1*H*-pyrazol-4-yl)ethynyl)-2,3-dih ydrobenzofuran-3-yl)-1*H*-pyrazolo[4,3-*c*]quinoline-8-carboxamide (Compound 9)**

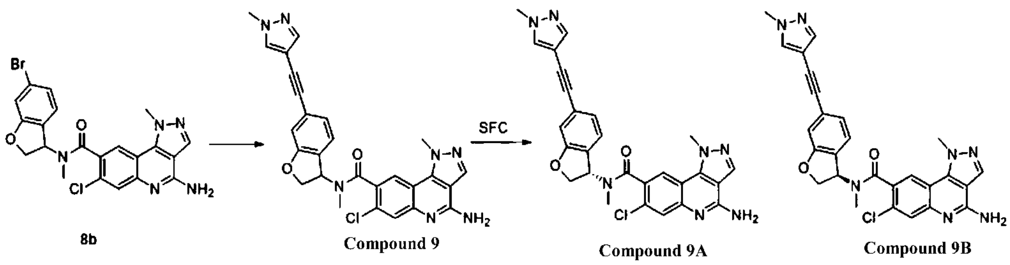


8b Compound 9 Compound 9A Compound 9B

To a 10 mL microwave tube were added reactant **8b** (48.6 mg, 0.10 mmol), cuprous iodide (3.8 mg, 0.02 mmol), tetrakis(triphenylphosphine)palladium (11.5 mg, 0.01 mmol), and *N*-methylpyrrolidone (2 mL). After nitrogen bubbling for 3 min, *N,N*-diisopropylethylamine (52.94 μL, 0.32 mmol) and *N*-methyl-4-alkynylpyrazole (21.2 mg, 0.20 mmol) were added. The reaction solution was reacted in a microwave reactor at 70 °C and stirred for 3 h. After the reaction was completed, water was added to quench the reaction, and the reaction solution was extracted three times with ethyl acetate

(30 mL). The organic phases were combined, washed with saturated brine, dried over anhydrous sodium sulfate, and filtered. The filtrate was concentrated, and the residue was subjected to column chromatography (methanol:dichloromethane = 1:20) to give a crude product. The crude product was separated by preparative reversed-phase column chromatography (acetonitrile:0.05% aqueous ammonium bicarbonate solution = 0:100% - 35%:65%) to give compound **9** (15 mg). ESI-MS (m/z): 511.90 $[M+H]^+$. $^1H$ NMR (400 MHz, DMSO-$d_6$) δ 8.41 - 8.21 (m, 2H), 8.10 - 8.05 (m, 1H), 7.72 - 7.61 (m, 2H), 7.54 - 7.30 (m, 3H), 7.15 - 6.91 (m, 2H), 6.54 - 6.48 and 5.47 - 5.42 (m, 1H), 4.87 - 4.56 (m, 2H), 4.47 - 4.41 (m, 3H), 3.87 - 3.86 (m, 3H), 2.73 - 2.50 (m, 3H).

Compound **9** was separated by SFC (instrument: Waters 150 Prep-SFC; chromatographic column: Chiral AS column; mobile phase A: $CO_2$, mobile phase B: methanol (containing 0.1% $NH_3$•$H_2O$); B%: 45%) to give compounds **9A** and **9B.** Compound **9A:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 2.187 min, ee = 100%. ESI-MS (m/z): 512.2 $[M+H]^+$.

Compound **9B:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 2.376 min, ee = 100%. ESI-MS (m/z): 512.3 $[M+H]^+$.

**Example 10: 4-amino-*N*,1,7-trimethyl-*N*-(6-((1-methyl-1*H*-pyrazol-4-yl)ethynyl)-2,3-dihydrob enzofuran-3-yl)-1*H*-pyrazolo[4,3-*c*]quinoline-8-carboxamide (Compound 10)**

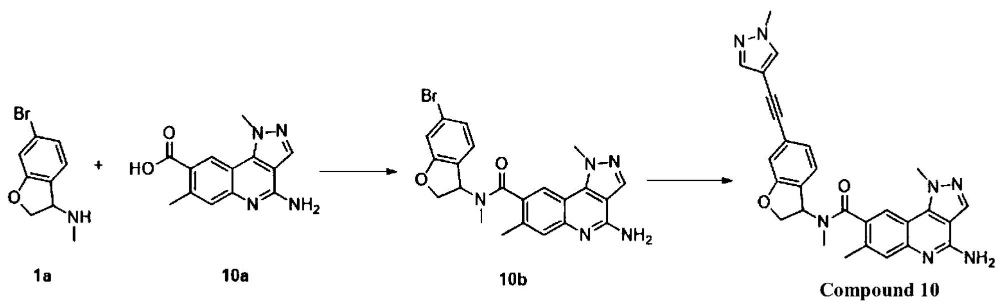

1a 10a 10b Compound 10

**Preparation of compound 10b**

To a solution of compound **1a** (210 mg, 0.92 mmol) in *N,N*-dimethylformamide (10 mL) were added compound **10a** (256.3 mg, 0.92 mmol, see WO2022169948 A1 for the preparation method), triethylamine (232.9 mg, 2.30 mmol), and O-(7-azabenzotriazol-1-yl)-*N,N,N',N'*-tetramethyluronium hexafluorophosphate (526.2 mg, 1.38 mmol), and the mixture was allowed to react at 25 °C overnight. Water (50 mL) was added to the reaction solution, and the reaction solution was extracted with ethyl acetate (25 mL × 2). The organic phases were washed three times with saturated brine, combined, dried over anhydrous sodium sulfate, and filtered. The filtrate was concentrated, and the residue was subjected to normal phase column chromatography (dichloromethane/methanol = 50:1) to give compound **10b** (360 mg). ESI-MS (m/z): 468.07 $[M+H]^+$.

**Preparation of compound 10**

To a 10 mL microwave tube were added reactant **10b** (46.6 mg, 0.10 mmol), cuprous iodide (3.8 mg, 0.02 mmol), tetrakis(triphenylphosphine)palladium (11.5 mg, 0.01 mmol), and a solvent *N*-methylpyrrolidone (2 mL). After nitrogen bubbling for 3 min, *N,N*-diisopropylethylamine (52.94 μL, 0.32 mmol) and *N*-methyl-4-alkynylpyrazole (21.2 mg, 0.20 mmol) were added. The reaction solution was reacted in a microwave reactor at 70 °C and stirred for 3 h. After the reaction was completed, water was added to quench the reaction, and the reaction solution was extracted three times with ethyl acetate (30 mL). The organic phases were combined, washed with saturated brine, dried over anhydrous sodium sulfate, and filtered. The filtrate was concentrated, and the residue was subjected to column chromatography (methanol:dichloromethane = 1:20) to give a crude product. The crude product was separated by preparative reversed-phase column chromatography (acetonitrile:0.05% aqueous ammonium bicarbonate solution = 0:100% - 35%:65%) to give compound **10** (15 mg). ESI-MS (m/z): 491.96 $[M+H]^+$. $^1H$ NMR (400 MHz, DMSO-$d_6$) δ 8.24 (s, 1H), 8.07 - 8.04 (m, 2H), 7.69 (s, 1H), 7.52 - 7.43 (m, 2H), 7.17 - 6.95 (m, 4H), 6.55 - 6.48 and 5.54 - 5.45 (m, 1H), 4.91 - 4.54 (m, 2H), 4.39 (s, 3H), 3.87 (s, 3H), 3.30 (s, 3H), 2.72 - 2.37 (m, 3H).

**Example 10A: (*S*)-4-amino-*N*,1,7-trimethyl-*N*-(6-((1-methyl-1*H*-pyrazol-4-yl)ethynyl)-2,3-dihyd robenzofuran-3-yl)-1*H*-pyrazolo[4,3-*c*]quinoline-8-carboxamide (Compound 10A)**

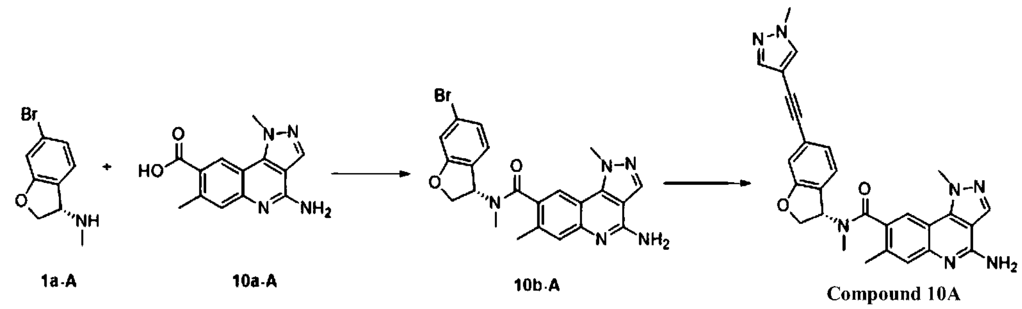

1a-A 10a-A 10b-A Compound 10A

For the synthesis process of preparing compound **10A** from compound **1a-A**, reference was made to the synthesis process of preparing compound **10** from compound **1a**.

Compound **10A:** ESI-MS (m/z): 492.01 [M+H]$^+$. $^1$H NMR (400 MHz, DMSO-d6) δ 8.24 (s, 1H), 8.07 - 8.04 (s, 1H), 7.69 (s, 1H), 7.52 - 7.44 (m, 2H), 7.14 - 6.95 (m, 4H), 6.55 - 5.45 (m, 1H),4.91 - 4.54 (m, 2H), 4.39 (s, 3H), 3.87 (s, 3H), 3.30 (s, 3H), 2.72 - 2.37 (s, 3H).

**Example 11: 4-amino-*N*-(6-((1-cyclopropyl-1*H*-pyrazol-4-yl)ethynyl)-2,3-dihydrobenzofuran-3 -yl)-*N*,1,7-trimethyl-1*H*-pyrazolo[4,3-*c*]quinoline-8-carboxamide (Compound 11)**

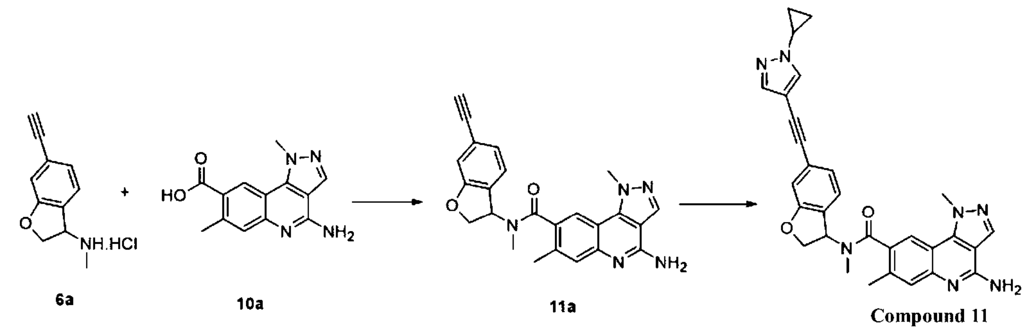

6a 10a 11a Compound 11

## Preparation of compound 11b

To a solution of compound **10a** (30.8 mg, 0.12 mmol) in *N,N*-dimethylacetamide (5 mL) were added compound **6a** (25 mg, 0.12 mmol), triethylamine (0.05 mL, 0.36 mmol), and O-(1*H*-benzotriazol-1-yl)-*N,N,N',N'*-tetramethylisouronium tetrafluoroborate (57.8 mg, 0.18 mmol), and the mixture was allowed to react at 25 °C overnight. Water (10 mL) was added to the reaction solution, and the reaction solution was extracted with ethyl acetate (3 mL × 10). The organic phases were washed three times with saturated brine, combined, dried over anhydrous sodium sulfate, and filtered. The filtrate was concentrated, and the residue was subjected to reversed phase column chromatography (acetonitrile/0.05% aqueous ammonium bicarbonate solution = 0:100% - 50%:50%) to give compound **11a** (7.0 mg). ESI-MS (m/z): 412.15.

## Preparation of compound 11

To a solution of compound **11a** (50 mg, 0.12 mmol) in N,N-dimethylformamide (2 mL) were sequentially added 1-cyclopropyl-4-iodopyrazole (56.9 mg, 0.24 mmol), cuprous iodide (9.90 mg, 0.05 mmol), *N,N*-diisopropylethylamine (134.16 mg, 1.04 mmol), and tetrakis(triphenylphosphine)palladium (30.1 mg, 0.03 mmol) under nitrogen atmosphere. After completion of the addition, the mixture was allowed to react at 25 °C for 3 h. Water (10 mL) was added to the reaction solution, and the reaction solution was extracted with ethyl acetate (10 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give a

crude product. The crude product was then subjected to reversed phase chromatography (acetonitrile/0.05% aqueous ammonia bicarbonate solution = 0:100% - 40%:60%) to give compound **11** (20 mg). ESI-MS (m/z): 518.22 $[M+H]^+$. $^1H$ NMR (400 MHz, DMSO-$d_6$) δ 8.39 (s, 1H), 8.30 - 8.10 (m, 2H), 7.95 - 7.38 (m, 4H), 7.26 - 6.94 (m, 3H), 6.52 - 6.50 and 5.50 - 5.42 (m, 1H), 4.89 - 4.42 (m, 5H), 3.80 - 3.74 (m, 1H), 2.73 and 2.49 (s, 3H), 2.56 and 2.39 (s, 3H), 1.14 - 1.05 (m, 2H), 1.05 - 0.95 (m, 2H).

**Example 12: 4-amino-*N*-(6-((1-cyclopropyl-1*H*-pyrazol-4-yl)ethynyl)-2,3-dihydrobenzofuran-3 -yl)-7-fluoro-*N*,1-dimethyl-1*H*-pyrazolo[4,3-*c*]quinoline-8-carboxamide (Compound 12)**

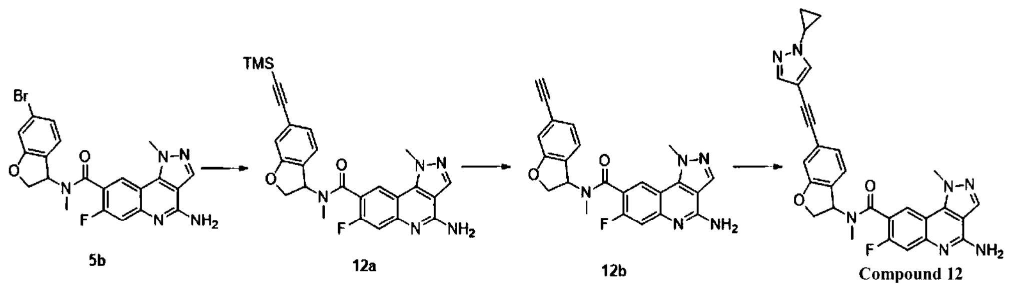

5b 12a 12b Compound 12

**Preparation of compound 12a**

To a solution of compound **5b** (808 mg, 1.72 mmol) in *N*-methylpyrrolidone (10 mL) were sequentially added trimethylsilylacetylene (338.5 mg, 3.45 mmol), cuprous iodide (65.6 mg, 0.34 mmol), *N,N*-diisopropylethylamine (890.9 mg, 6.89 mmol), and tetrakis(triphenylphosphine)palladium (199.1 mg, 0.17 mmol) under nitrogen atmosphere. After completion of the addition, the mixture was reacted in a microwave reactor at 100 °C for 3 h. Water (200 mL) was added to the reaction solution, and the reaction solution was extracted with ethyl acetate (30 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give compound **12a** (595 mg). ESI-MS (m/z): 488.18 $[M+H]^+$.

**Preparation of compound 12b**

To a solution of compound **12a** (595 mg, 1.22 mmol) in tetrahydrofuran (10 mL) was added a solution of tetrabutylammonium fluoride (2.44 mL, 1 M). After completion of the addition, the mixture was allowed to react at room temperature for 1 h. Water was added, and the reaction solution was extracted with ethyl acetate (30 mL × 3). The organic phases were combined, washed with saturated brine, dried over anhydrous sodium sulfate, and filtered. The filtrate was concentrated, and the residue was subjected to column chromatography (methanol:dichloromethane = 1:20) to give compound **12b** (470 mg). ESI-MS (m/z): 416.12 $[M+H]^+$.

**Preparation of compound 12**

To a solution of compound **12b** (62.3 mg, 0.15 mmol) in *N*-methylpyrrolidone (1.5 mL) were sequentially added 1-cyclopropyl-4-iodo-1*H*-pyrazole (70.2 mg, 0.3 mmol), cuprous iodide (5.7 mg, 0.03 mmol), *N,N*-diisopropylethylamine (77.5 mg, 0.6 mmol), and tetrakis(triphenylphosphine)palladium (17.3 mg, 0.015 mmol) under nitrogen atmosphere. After completion of the addition, the mixture was allowed to react under an oil bath at 50 °C for 3 h. Water (100 mL) was added to the reaction solution, and the reaction solution was extracted with ethyl acetate (20 mL ×3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give compound **12** (25 mg). ESI-MS (m/z): 521.97 $[M+H]^+$. $^1H$ NMR (400 MHz, DMSO) δ 8.32 - 8.24 (m, 2H), 8.18 (s, 1H), 7.68 (s, 1H), 7.42 - 7.28 (m, 4H), 7.14 - 7.05 (m, 1H), 7.02 - 6.92 (m, 1H), 6.51 - 5.58 (m, 1H), 4.88 - 4.66 (m, 2H), 4.44 - 4.40 (m, 3H), 3.81 - 3.73 (m, 1H), 2.71 - 2.59 (m, 3H), 1.09 - 0.95 (m, 4H).

**Example 12A: (*S*)-4-amino-*N*-(6-((1-cyclopropyl-1*H*-pyrazol-4-yl)ethynyl)-2,3-dihydrobenzofur an-3-yl)-7-fluoro-*N*,1-dimethyl-1*H*-pyrazolo[4,3-c]quinoline-8-carboxamide (Compound 12A)**

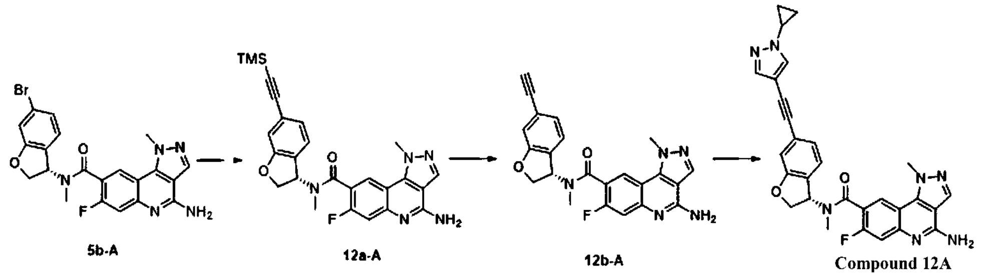

5b-A 12a-A 12b-A Compound 12A

For the synthesis process of preparing compound **12A** from compound 5b-A, reference was made to the synthesis process of preparing compound **12** from compound **5b.**

Compound **12A:** ESI-MS (m/z): 521.94 [M+H]$^+$. $^1$H NMR (400 MHz, DMSO) δ 8.34 - 8.23 (m, 2H), 8.18 (s, 1H), 7.69 (s, 1H), 7.45 - 7.29 (m, 4H), 7.16 - 7.05 (m, 1H), 7.02 - 6.92 (m, 1H), 6.50 - 5.56 (m, 1H), 4.87 - 4.65 (m, 1H), 4.44 - 4.40 (m, 3H), 3.84 - 3.70 (m, 1H), 2.71 - 2.60 (m, 1H), 1.11 - 0.96 (m, 4H).

**Example 13: 4-amino-7-fluoro-*N*-(6-((1-isopropyl-1*H*-pyrazol-4-yl)ethynyl)-2,3-dihydrobenzof uran-3-yl)-*N*,1-dimethyl-1*H*-pyrazolo[4,3-c]quinoline-8-carboxamide (Compound 13)**

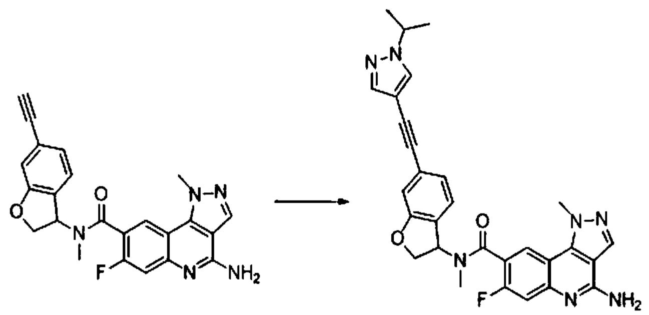

12b Compound 13

Referring to the preparation process of compound **12,** compound 1-cyclopropyl-4-iodo-1*H*-pyrazole was replaced with compound 4-iodo-1-isopropyl-1*H*-pyrazole (70.8 mg, 0.3 mmol), and the mixture was reacted at 50 °C for 3 h under nitrogen atmosphere to give compound **13** (28 mg). ESI-MS (m/z): 524.05 [M+H]$^+$. $^1$H NMR (400 MHz, DMSO) δ 8.39 - 8.22 (m, 2H), 8.17 (s, 1H), 7.70 (s, 1H), 7.44 - 7.28 (m, 4H), 7.17 - 7.05 (m, 1H), 7.02 - 6.93 (m, 1H), 6.51 - 5.52 (m, 1H), 4.87 - 4.66 (m, 2H), 4.57 - 4.48 (m, 1H), 4.44 - 4.40 (m, 3H), 2.71 - 2.60 (m, 3H), 1.45 - 1.42 (m, 6H).

**Example 14: 4-amino-7-chloro-*N*-(6-((1-isopropyl-1*H*-pyrazol-4-yl)ethynyl)-2,3-dihydrobenzof uran-3-yl)-*N*,1-dimethyl-1*H*-pyrazolo[4,3-*c*]quinoline-8-carboxamide (Compound 14)**

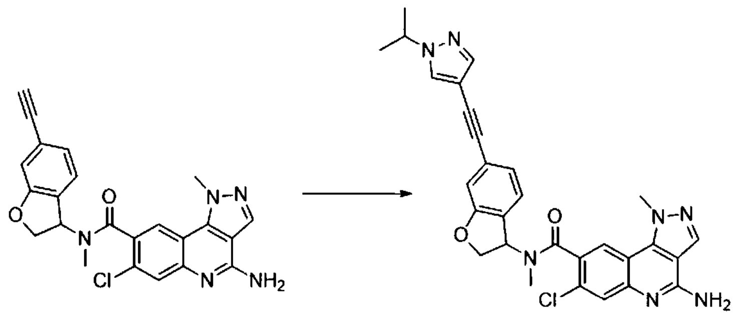

8d　Compound 14

Referring to the preparation process of compound **8,** compound 1-cyclopropyl-4-iodopyrazole was replaced with compound 4-iodo-1-(propan-2-yl)pyrazole (21.9 mg, 0.09 mmol), and the mixture was reacted at 50 °C for 3 h under nitrogen atmosphere to give compound **14** (12 mg). ESI-MS (m/z): 540.17 $[M+H]^+$. $^1H$ NMR (400 MHz, DMSO+$D_2O$) δ 8.41 - 8.19 (m, 2H), 8.14 - 8.08 (m, 1H), 7.77 - 7.64 (m, 2H), 7.49 - 7.25 (m, 1H), 7.12 - 6.88 (m, 2H), 6.51 - 6.42 and 5.42 - 5.34 (m, 1H), 4.84 - 4.46 (m, 3H), 4.44 and 4.38 (s, 3H), 2.7 - 2.47 (m, 3H), 1.45 - 1.34 (m, 6H).

**Example 15: 4-amino-7-chloro-*N*-(6-((1-ethyl-1*H*-pyrazol-4-yl)ethynyl)-2,3-dihydrobenzofura n-3-yl)-*N*,1-dimethyl-1*H*-pyrazolo[4,3-*c*]quinoline-8-carboxamide (Compound 15)**

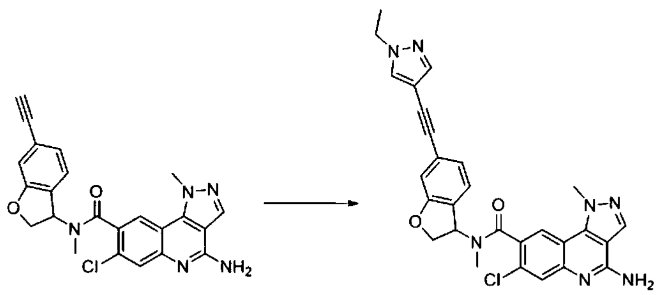

8d　Compound 15

Referring to the preparation process of compound **8,** compound 1-cyclopropyl-4-iodopyrazole was replaced with compound 1-ethyl-4-iodopyrazole (30.8 mg, 0.14 mmol), and the mixture was reacted at 50 °C for 3 h under nitrogen atmosphere to give compound **15** (16.4 mg). ESI-MS (m/z): 526.0 $[M+H]^+$. $^1H$ NMR (400 MHz, DMSO) δ 8.43 - 8.11 (m, 3H), 7.72 - 7.61 (m, 2H), 7.54 - 7.29 (m, 3H), 7.17 - 7.04 (m, 1H), 7.04 - 6.91 (m, 1H), 6.55 - 6.48 and 5.48 - 5.38 (m, 1H), 4.86 - 4.55 (m, 2H), 4.47 and 4.41 (s, 3H), 4.21 - 4.09 (m, 2H), 2.73 - 2.50 (m, 3H), 1.44 - 1.34 (m, 3H).

**Example 16: 4-amino-*N*-(6-((1-cyclopropyl-1*H*-pyrazol-4-yl)ethynyl)-2,3-dihydrobenzofuran-3 -yl)-7-fluoro-*N*-methyl-1,3-dihydrofuro[3,4-*c*]quinoline-8-carboxamide (Compound 16)**

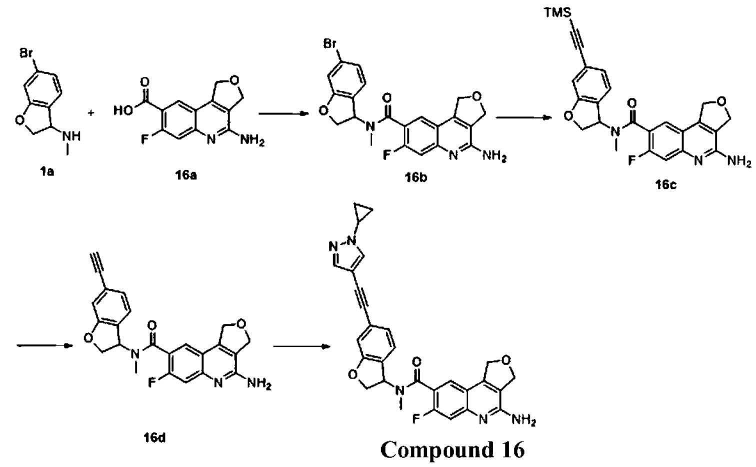

1a 16a 16b 16c

16d Compound 16

**Preparation of compound 16b**

To a solution of compound **1a** (100 mg, 0.44 mmol) in N,N-dimethylformamide (10 mL) were added compound **16a** (109 mg, 0.44 mmol, see WO2022169948 A1 for the preparation method), triethylamine (110.9 mg, 1.10 mmol), and O-(7-azabenzotriazol-1-yl)-*N*,*N*,*N'*,*N'*-tetramethyluronium hexafluorophosphate (250.5 mg, 0.66 mmol), and the mixture was allowed to react at 25 °C overnight. Water (50 mL) was added to the reaction solution, and the reaction solution was extracted with ethyl acetate (25 mL × 2). The organic phases were washed three times with saturated brine, combined, dried over anhydrous sodium sulfate, and filtered. The filtrate was concentrated, and the residue was subjected to normal phase column chromatography (dichloromethane/methanol = 50:1) to give compound **16b** (118 mg). ESI-MS (m/z): 459.85 $[M+H]^+$.

**Preparation of compound 16c**

To a solution **of 16b** (118 mg, 0.26 mmol) in N-methylpyrrolidone (3 mL) were added cuprous iodide (9.8 mg, 0.05 mmol), tetrakis(triphenylphosphine)palladium (29.8 mg, 0.03 mmol), triethylamine (104.2 mg, 2.88 mmol), and trimethylsilylacetylene (50.6 mg, 0.51 mmol). After completion of the addition, the mixture was reacted in a microwave reactor at 70 °C for 3 h under nitrogen atmosphere. The reaction solution was filtered through celite. Water (50 mL) was added, and the reaction solution was extracted with ethyl acetate (25 mL × 2). The organic phases were combined, washed with saturated brine, dried over anhydrous sodium sulfate, and filtered. The filtrate was concentrated, and the residue was subjected to normal phase column chromatography (dichloromethane/methanol = 20:1) to give compound **16c** (105 mg). ESI-MS (m/z): 476.2 $[M+H]^+$.

**Preparation of compound 16d**

Tetrabutylammonium fluoride (440 μL, 0.44 mmol) was added to a solution of compound **16c** (105 mg, 0.22 mmol) in tetrahydrofuran (5 mL), and the mixture was allowed to react at 25 °C for 1 h. Water (15 mL) was added to the reaction solution, and the reaction solution was extracted with ethyl acetate (25 mL × 2). The organic phase was dried over anhydrous sodium sulfate and filtered. The filtrate was concentrated, and the residue was separated and purified by a preparative plate (dichloromethane/methanol = 15:1) to give compound **16d** (89.1 mg). ESI-MS (m/z): 404.09 $[M+H]^+$.

**Preparation of compound 16**

To a solution of compound **16d** (43 mg, 0.11 mmol) in N,N-dimethylformamide (2 mL) were sequentially added 1-cyclopropyl-4-iodopyrazole (49.9 mg, 0.22 mmol), cuprous iodide (8.10 mg, 0.04 mmol), triethylamine (43.1 mg, 0.43 mmol), and tetrakis(triphenylphosphine)palladium (24.6 mg, 0.02 mmol) under nitrogen atmosphere. After completion of the addition, the mixture was allowed to react at 25 °C for 3 h. Water (10 mL) was added to the reaction solution, and the reaction solution was extracted with ethyl acetate (10 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give a crude product. The crude product was then subjected to preparative reversed-phase column chromatography (acetonitrile/0.05% aqueous ammonia bicarbonate solution = 0:100% - 40%:60%) to give compound **16** (18 mg). ESI-MS (m/z): 510.18 $[M+H]^+$. $^1H$ NMR (400 MHz, DMSO-$d_6$) δ 8.19 - 8.18 (m, 1H), 7.69 - 7.65 (m, 2H), 7.38 - 7.31 (m, 2H), 7.13 - 7.07 (m, 1H), 7.00 - 6.95 (m, 1H),

6.85 (s, 2H), 6.45 - 6.42 and 5.52 - 5.49 (m, 1H), 5.38 - 5.32 (m, 2H), 5.04 - 5.00 (m, 2H), 4.82 - 4.59 (m, 2H), 3.80 - 3.74 (m, 1H), 2.68 and 2.55 (s, 3H), 1.11 - 0.97 (m, 4H).

**Example 16A: (*S*)-4-amino-*N*-(6-((1-cyclopropyl-1*H*-pyrazol-4-yl)ethynyl)-2,3-dihydrobenzofur an-3-yl)-7-fluoro-*N*-methyl-1,3-dihydrofuro[3,4-c]quinoline-8-carboxamide (Compound 16A)**

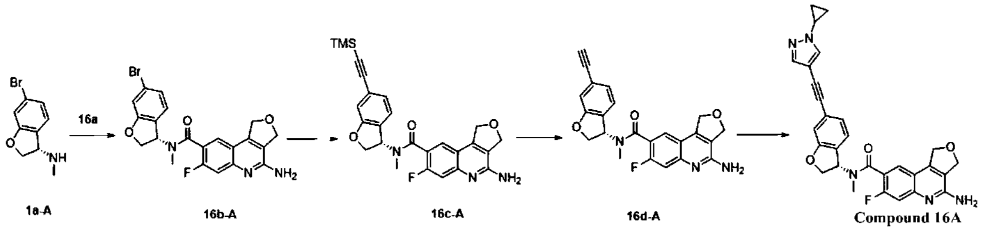


1a-A 16b-A 16c-A 16d-A Compound 16A

For the synthesis process of preparing compound **16A** from compound **1a-A**, reference was made to the synthesis process of preparing compound **16** from compound **1a.**

Compound **16A:** ESI-MS (m/z): 510.08 [M+H]+. 1H NMR (400 MHz, DMSO) δ 8.20 - 8.17 (m, 1H), 7.71 - 7.62 (m, 2H), 7.40 - 7.26 (m, 2H), 7.14 - 7.05 (m, 1H), 7.01 - 6.94 (m, 1H), 6.85 (s, 2H), 6.47 - 6.38 and 5.53 - 5.46 (m, 1H), 5.40 - 5.32 (m, 2H), 5.04 - 5.00 (m, 2H), 4.83 - 4.56 (m, 2H), 3.82 - 3.72 (m, 1H), 2.68 and 2.55 (s, 3H), 1.10 - 1.05 (m, 2H), 1.02 - 0.97 (m, 2H).

**Example 17: 4-amino-*N*-(6-((1-ethyl-1*H*-pyrazol-4-yl)ethynyl)-2,3-dihydrobenzofuran-3-yl)-7-f luoro-*N*,1-dimethyl-1*H*-pyrazolo[4,3-*c*]quinoline-8-carboxamide (Compound 17)**

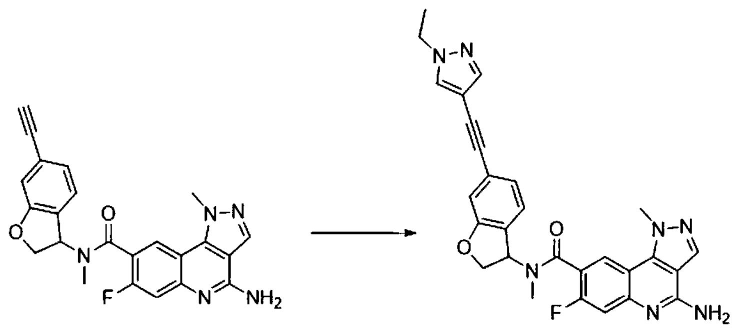

12b Compound 17

Referring to the preparation process of compound **12,** compound 1-cyclopropyl-4-iodo-1*H*-pyrazole was replaced with compound 1-ethyl-4-iodo-1*H*-pyrazole (66.6 mg, 0.3 mmol) to give compound **17** (30 mg). ESI-MS (m/z): 510.05 [M+H]+. 1H NMR (400 MHz, DMSO) δ 8.36 - 8.21 (m, 2H), 8.13 (s, 1H), 7.70 (s, 1H), 7.43 - 7.28 (m, 4H), 7.16 - 7.05 (m, 1H), 7.03 - 6.94 (m, 1H), 6.50 - 5.54 (m, 1H), 4.87 - 4.61 (m, 2H), 4.45 - 4.40 (m, 3H), 4.20 - 4.13 (m, 2H), 2.71 - 2.60 (m, 3H), 1.42 - 1.37 (m, 3H).

**Example 18: 4-amino-7-chloro-*N*,1-dimethyl-*N*-(6-((1-methyl-1*H*-pyrazol-3-yl)ethynyl)-2,3-dih ydrobenzofuran-3-yl)-1*H*-pyrazolo[4,3-c]quinoline-8-carboxamide (Compound 18)**

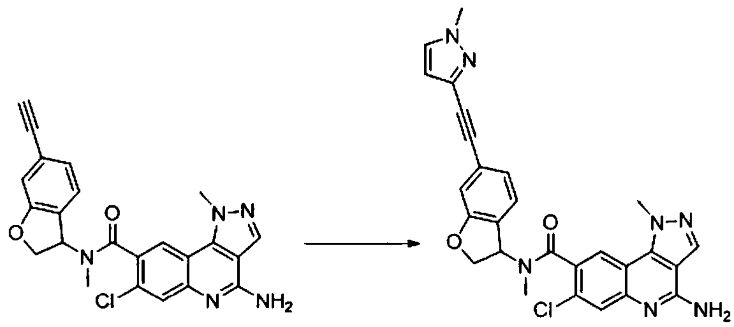

8d

Compound 18

Referring to the preparation process of compound **8,** compound 1-cyclopropyl-4-iodopyrazole was replaced with compound 3-iodo-1-methylpyrazole (41.6 mg, 0.20 mmol) to give compound **18** (21 mg). ESI-MS (m/z): 511.97 [M+H]$^+$. $^1$H NMR (400 MHz, DMSO-$d_6$) δ 8.41 - 8.21 (m, 2H), 7.79 - 7.78 (m, 1H), 7.71 - 7.61 (m, 1H), 7.57 - 7.28 (m, 3H), 7.20 - 6.96 (m, 2H), 6.53 - 5.43 (m, 2H), 4.87 - 4.55 (m, 2H), 4.46 and 4.41 (s, 3H), 3.88 - 3.87 (m, 3H), 2.74 - 2.53 (m, 3H).

**Example 19: 4-amino-*N*-(6-((1-isopropyl-1*H*-pyrazol-4-yl)ethynyl)-2,3-dihydrobenzofuran-3-yl )-*N*,1-dimethyl-1*H*-pyrazolo[4,3-*c*]quinoline-8-carboxamide (Compound 19)**

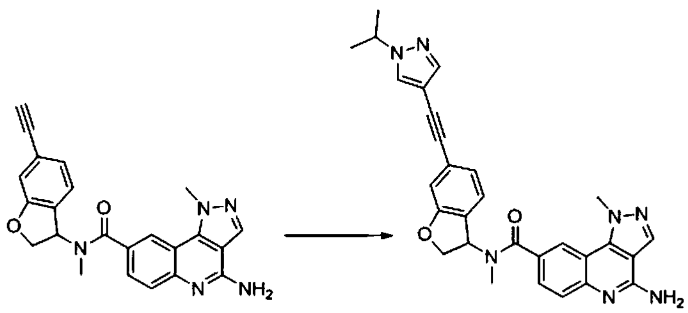

6b

Compound 19

To a solution of compound **6b** (49.6 mg, 0.125 mmol) in *N*-methylpyrrolidone (1.5 mL) were sequentially added 4-iodo-1-isopropyl-1*H*-pyrazole (59.0 mg, 0.25 mmol), cuprous iodide (4.8 mg, 0.025 mmol), N,N-diisopropylethylamine (64.6 mg, 0.5 mmol), and tetrakis(triphenylphosphine)palladium (14.5 mg, 0.0125 mmol) under nitrogen atmosphere. After completion of the addition, the mixture was allowed to react under an oil bath at 50 °C for 3 h. Water (100 mL) was added to the reaction solution, and the reaction solution was extracted with ethyl acetate (20 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give compound **19** (30 mg). ESI-MS (m/z): 506.05 [M+H]$^+$. $^1$H NMR (400 MHz, DMSO) δ 8.34 (s, 1H), 8.28 (s, 1H), 8.17 (s, 1H), 7.70 (s, 1H), 7.68 - 7.62 (m, 2H), 7.48 - 7.42 (m, 1H), 7.18 (s, 2H), 7.12 - 7.07 (m, 1H), 6.98 (s, 1H), 6.30 - 5.87 (m, 1H), 4.79 - 4.70 (m, 2H), 4.57 - 4.49 (m, 1H), 4.44 (s, 3H), 2.70 (s, 3H), 1.46 - 1.43 (m, 6H).

**Example 19A: (*S*)-4-amino-*N*-(6-((1-isopropyl-1*H*-pyrazol-4-yl)ethynyl)-2,3-dihydrobenzofuran-3-yl)-*N*,1-dimethyl-1*H*-pyrazolo[4,3-*c*]quinoline-8-carboxamide (Compound 19A)**

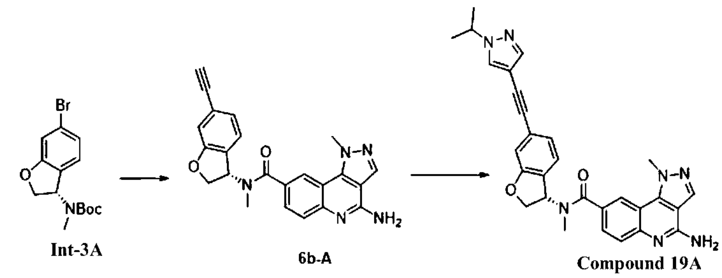

Int-3A 6b-A Compound 19A

Reference was made to WO2024131901 A1 for the preparation process of compound **6b-A.** For the synthesis process of preparing compound **19A** from compound **6b-A,** reference was made to the synthesis process of preparing compound **19** from compound **6b.**

Compound **19A:** ESI-MS (m/z): 506.13 $[M+H]^+$. $^1H$ NMR (400 MHz, DMSO) δ 8.33 (s, 1H), 8.28 (s, 1H), 8.17 (s, 1H), 7.70 (s, 1H), 7.67 - 7.62 (m, 2H), 7.48 - 7.42 (m, 1H), 7.16 (s, 2H), 7.11 - 7.06 (m, 1H), 6.97 (s, 1H), 4.80 - 4.67 (m, 2H), 4.57 - 4.48 (m, 1H), 4.44 (s, 3H), 2.69 (s, 3H), 1.45 - 1.42 (m, 6H).

**Example 20: 4-amino-*N*-(6-((1-ethyl-1*H*-pyrazol-4-yl)ethynyl)-2,3-dihydrobenzofuran-3-yl)-*N*, 1-dimethyl-1*H*-pyrazolo[4,3-*c*]quinoline-8-carboxamide (Compound 20)**

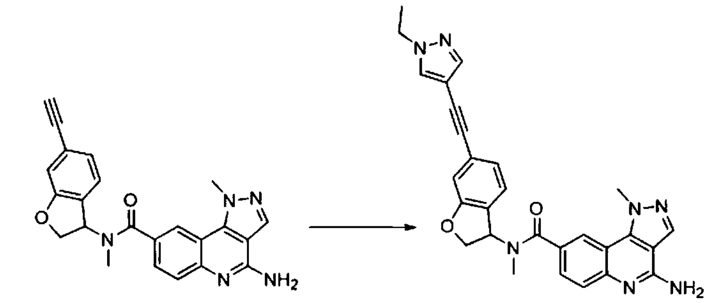

6b Compound 20

Referring to the preparation process of compound **19,** compound 4-iodo-1-isopropyl-1*H*-pyrazole was replaced with compound 1-ethyl-4-iodo-1*H*-pyrazole (55.5 mg, 0.25 mmol) to give compound **20** (15 mg). ESI-MS (m/z): 492.02 $[M+H]^+$. $^1H$ NMR (400 MHz, DMSO) δ 8.36 (s, 2H), 8.14 (s, 1H), 7.70 - 7.39 (m, 6H), 7.14 - 7.07 (m, 1H), 6.99 (s, 1H), 6.36 - 5.82 (m, 1H), 4.82 - 4.68 (m, 2H), 4.45 (s, 3H), 4.20 - 4.13 (m, 2H), 2.70 (s, 3H), 1.43 - 1.36 (m, 3H).

**Example 20A: (*S*)-4-amino-*N*-(6-((1-ethyl-1*H*-pyrazol-4-yl)ethynyl)-2,3-dihydrobenzofuran-3-yl )-*N*,1-dimethyl-1*H*-pyrazolo[4,3-*c*]quinoline-8-carboxamide (Compound 20A)**

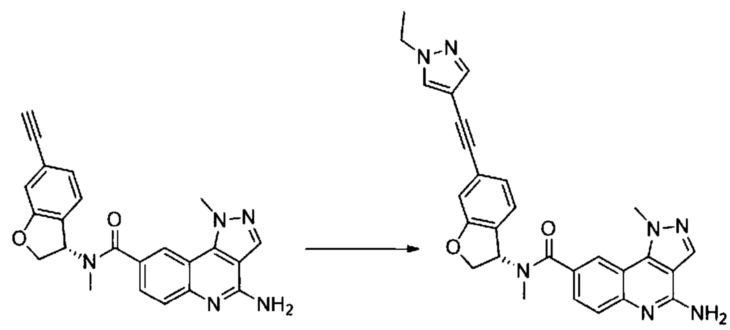

6b-A Compound 20A

For the synthesis process of preparing compound **20A** from compound **6b-A,** reference was made to the

synthesis process of preparing compound **20** from compound **6b.**

Compound **20A:** ESI-MS (m/z): 492.02 [M+H]$^+$. $^1$H NMR (400 MHz, DMSO) δ 8.34 (s, 1H), 8.28 (s, 1H), 8.13 (s, 1H), 7.70 (s, 1H), 7.68 - 7.61 (m, 2H), 7.48 - 7.42 (m, 1H), 7.19 (s, 2H), 7.12 - 7.07 (m, 1H), 6.98 (s, 1H), 4.80 - 4.68 (m, 2H), 4.44 (s, 3H), 4.22 - 4.11 (m, 2H), 2.69 (s, 3H), 1.41 - 1.37 (m, 3H).

**Example 21: 4-amino-7-fluoro-*N*,1-dimethyl-*N*-(6-((1-methyl-1*H*-pyrazol-3-yl)ethynyl)-2,3-dih ydrobenzofuran-3-yl)-1*H*-pyrazolo[4,3-*c*]quinoline-8-carboxamide (Compound 21)**

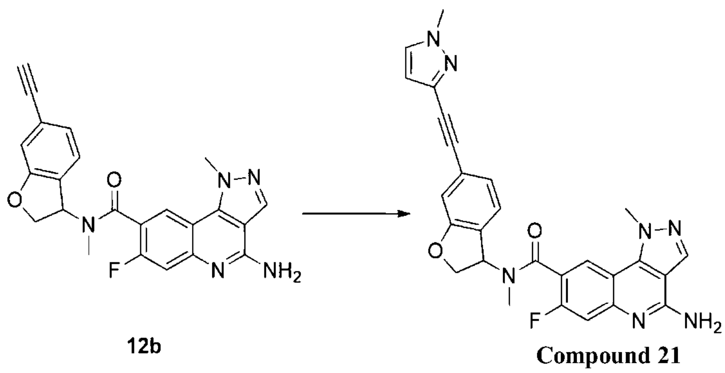

12b

Compound 21

Referring to the preparation process of compound **12,** compound 1-cyclopropyl-4-iodo-1*H*-pyrazole was replaced with compound 3-iodo-1-methylpyrazole (21 mg, 0.10 mmol), and the mixture was reacted at 25 °C for 3 h to give compound **21** (20 mg). ESI-MS (m/z): 495.74 [M+H]$^+$. $^1$H NMR (400 MHz, DMSO-$d_6$) δ 8.40 - 8.24 (m, 2H), 7.81 - 7.78 (m, 1H), 7.51 - 7.26 (m, 4H), 7.21 - 7.12 (m, 1H), 7.09 - 7.01 (m, 1H), 6.55 - 6.51 (m, 1H), 6.50 - 6.45 and 5.64 - 5.57 (m, 1H), 4.86 - 4.65 (m, 2H), 4.44 - 4.41 (m, 3H), 3.89 (s, 3H), 2.72 and 2.61 (s, 3H).

**Example 21A: (*S*)-4-amino-7-fluoro-*N*,1-dimethyl-*N*-(6-((1-methyl-1*H*-pyrazol-3-yl)ethynyl)-2,3-dihydrobenzofuran-3-yl)-1*H*-pyrazolo[4,3-*c*]quinoline-8-carboxamide (Compound 21A)**

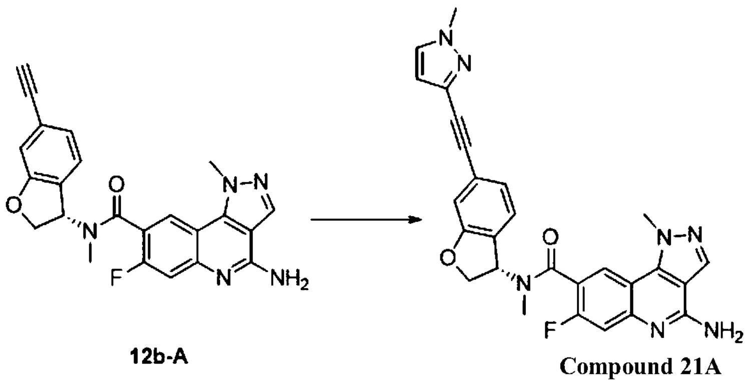

12b-A

Compound 21A

For the synthesis process of preparing compound **21A** from compound **12b-A,** reference was made to the synthesis process of preparing compound **21** from compound **12b.**

Compound **21A:** ESI-MS (m/z): 496.08 [M+H]$^+$. $^1$H NMR (400 MHz, DMSO-d6) δ 8.40 - 8.24 (m, 2H), 7.81 - 7.78 (m, 1H), 7.51 - 7.26 (m, 4H), 7.21 - 7.12 (m, 1H), 7.09 - 7.01 (m, 1H), 6.55 - 6.51 (m, 1H), 6.51 - 5.57 (m, 1H), 4.90 - 4.62 (m, 2H), 4.44 - 4.41 (s, 3H), 3.89 (s, 3H), 2.72 and 2.61 (s, 3H).

**Example 22: 4-amino-7-chloro-*N*-(6-((1,5-dimethyl-1*H*-pyrazol-4-yl)ethynyl)-2,3-dihydrobenz ofuran-3-yl)-*N*,1-dimethyl-1*H*-pyrazolo[4,3-*c*]quinoline-8-carboxamide (Compound 22)**

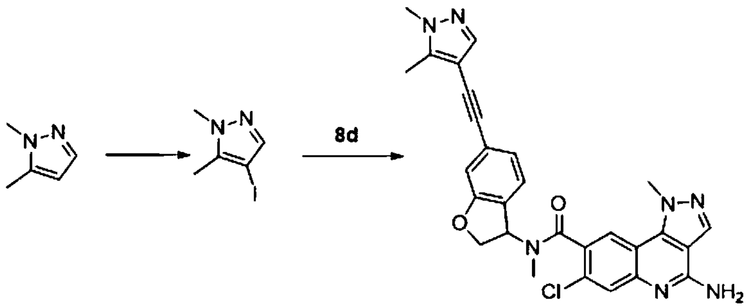


22a 22b Compound 22

**Preparation of compound 22b**

To a solution of compound **22a** (1.0 g, 10.41 mmol) in *N*,*N*-dimethylformamide (10 mL) was added *N*-iodosuccinimide (2.5 g, 10.93 mmol). After completion of the addition, the mixture was allowed to react at 25 °C for 1 h. Water (10 mL) was added to the reaction solution, and the reaction solution was extracted with ethyl acetate (10 mL × 3). The organic phases were combined, washed with saturated brine, dried over anhydrous sodium sulfate, and filtered. The solvent was removed by rotary evaporation, and the residue was subjected to column chromatography (petroleum ether:ethyl acetate = 6:1) to give **22b** (1.75 g).

**Preparation of compound 22**

Referring to the preparation process of compound **8,** compound 1-cyclopropyl-4-iodopyrazole was replaced with compound **22b** (41.1 mg, 0.19 mmol) to give compound **22** (15 mg). ESI-MS (m/z): 526.07 $[M+H]^+$. $^1H$ NMR (400 MHz, DMSO-$d_6$) δ 8.41 - 8.21 (m, 2H), 7.72 - 7.26 (m, 5H), 7.15 - 6.93 (m, 2H), 6.55 - 6.48 and 5.48 - 5.40 (m, 1H), 4.86 - 4.57 (m, 2H), 4.47 and 4.41 (s, 3H), 3.78 - 3.77 (m, 3H), 2.76 - 2.41 (m, 3H), 2.38 - 2.36 (m, 3H).

**Example 22A: (*S*)-4-amino-7-chloro-*N*-(6-((1,5-dimethyl-1*H*-pyrazol-4-yl)ethynyl)-2,3-dihydrob enzofuran-3-yl)-*N*,1-dimethyl-1*H*-pyrazolo[4,3-*c*]quinoline-8-carboxamide (Compound 22A)**

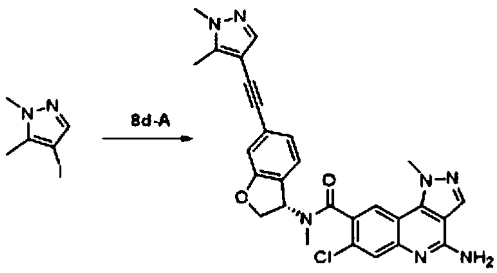


22b Compound 22A

For the synthesis process of preparing compound **22A** from compound 22b and compound **8d-A,** reference was made to the synthesis process of preparing compound **22** from compound 22b and compound **8d.**

Compound **22A:** ESI-MS (m/z): 525.95 $[M+H]^+$.

**Example 23: 4-amino-*N*-(6-((1,5-dimethyl-1*H*-pyrazol-4-yl)ethynyl)-2,3-dihydrobenzofuran-3-yl)-7-fluoro-*N*,1-dimethyl-1*H*-pyrazolo[4,3-*c*]quinoline-8-carboxamide (Compound 23)**

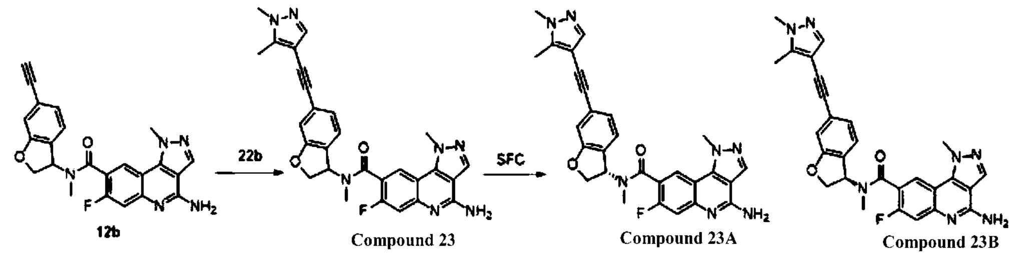


12b Compound 23 Compound 23A Compound 23B

Referring to the preparation process of compound **12,** compound 1-cyclopropyl-4-iodo-1*H*-pyrazole was replaced with compound **22b** (44.4 mg, 0.20 mmol), and the mixture was reacted at room temperature for 3 h to give compound **23** (15 mg). ESI-MS (m/z): 510.38 [M+H]$^+$. $^1$H NMR (400 MHz, DMSO-d6) δ 8.41 - 8.21 (m, 2H), 7.59 (s, 1H), 7.47 - 7.20 (m, 4H), 7.17 - 7.05 (m, 1H), 7.04 - 6.96 (m, 1H), 6.49 - 6.43 and 5.62 - 5.54 (m, 1H), 4.87 - 4.65 (m, 2H), 4.43 - 4.41 (m, 3H), 3.78 (s, 3H), 2.72 and 2.60 (s, 3H), 2.37 (s, 3H).

Compound **23** was separated by SFC (instrument: Waters 150 Prep-SFC; chromatographic column: Chiral AS column; mobile phase A: $CO_2$, mobile phase B: methanol (containing 0.1% $NH_3$•$H_2O$); B%: 40%) to give compounds **23A** and **23B.**

Compound **23A:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 1.948 min, ee = 100%. $[\alpha]^D_{25}$ +247 (c 1mg/mL, DMF).

Compound **23B:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 2.155 min, ee = 100%. $[\alpha]^D_{25}$ -271 (c 1mg/mL, DMF).

**Example 24: 4-amino-*N*-(6-((3-chloro-1-methyl-1*H*-pyrazol-4-yl)ethynyl)-2,3-dihydrobenzofur an-3-yl)-7-fluoro-*N*,1-dimethyl-1*H*-pyrazolo[4,3-*c*]quinoline-8-carboxamide (Compound 24)**

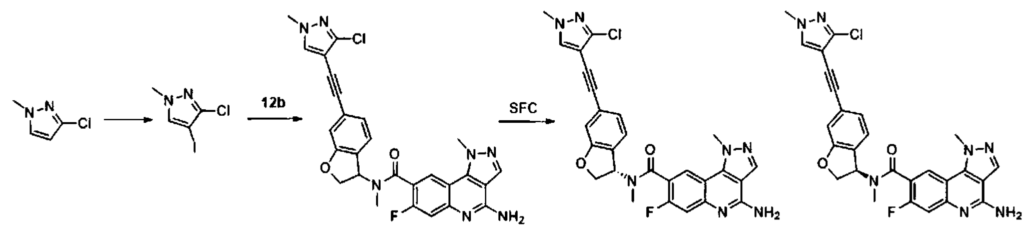


24a 24b Compound 24 Compound 24A Compound 24B

**Preparation of compound 24b**

To a solution of 3-chloro-1-methylpyrazole (116 mg, 1.0 mmol) in acetonitrile (3 mL) was added *N*-iodosuccinimide (235.2 mg, 1.05 mmol). After completion of the addition, the mixture was reacted at 25 °C for 1 h. Water (10 mL) was added to the reaction solution, and the reaction solution was extracted with ethyl acetate (10 mL × 3). The organic phases were combined, washed with saturated brine, dried over anhydrous sodium sulfate, and filtered. The solvent was removed by rotary evaporation, and the residue was subjected to column chromatography (petroleum ether:ethyl acetate = 6:1) to give compound **24b** (210 mg). ESI-MS (m/z): 242.89 [M+H]$^+$.

**Preparation of compound 24**

Referring to the preparation process of compound **12,** compound 1-cyclopropyl-4-iodo-1*H*-pyrazole was replaced with compound **24b** (24.2 mg, 0.10 mmol), and the mixture was reacted at 25 °C for 3 h to give compound **24** (20 mg). ESI-MS (m/z): 530.17 [M+H]$^+$. $^1$H NMR (400 MHz, DMSO-$d_6$) δ 8.42 - 8.15 (m, 2H), 7.97 - 7.73 (m, 1H), 7.49 - 7.28 (m, 4H), 7.20 - 7.12 (m, 1H), 7.04 - 6.97 (m, 1H), 6.51 - 6.39 and 5.63 - 5.52 (m, 1H), 4.86 - 4.62 (m, 2H), 4.43 - 4.41 (s, 3H), 3.85 (s, 3H), 2.71 and 2.60 (s, 3H).

Compound **24** was separated by SFC (instrument: Waters 150 Prep-SFC; chromatographic column: Chiral AS column; mobile phase A: $CO_2$, mobile phase B: methanol (containing 0.1% $NH_3$•$H_2O$); B%: 40%) to give compounds **24A** and **24B.** Compound **24A:** SFC analytical method: instrument: SHIMADZU LC-20AD; chromatographic column: Chiral AS

column; mobile phase A: $CO_2$, mobile phase B: methanol (containing 0.05% diethylamine); B%: 5-40%; retention time: 0.789 min, ee = 99.71%.

Compound **24B:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 1.213 min, ee = 99.83%.

**Example 25: 4-amino-*N*-(6-((1,3-dimethyl-1*H*-pyrazol-4-yl)ethynyl)-2,3-dihydrobenzofuran-3-yl)-7-fluoro-*N*,1-dimethyl-1*H*-pyrazolo[4,3-*c*]quinoline-8-carboxamide (Compound 25)**

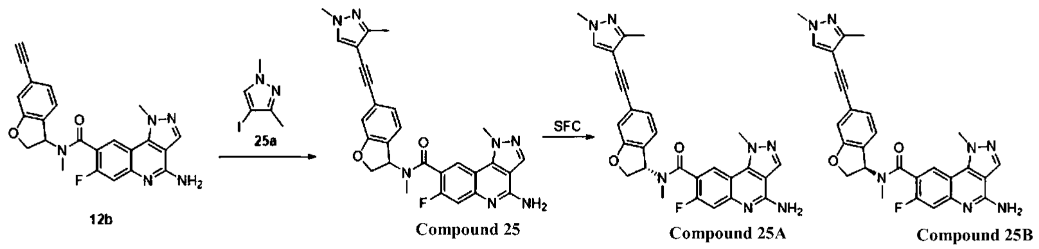


12b Compound 25 Compound 25A Compound 25B

Referring to the preparation process of compound **12,** compound 1-cyclopropyl-4-iodo-1*H*-pyrazole was replaced with compound **25a** (22.2 mg, 0.10 mmol), and the mixture was reacted at 25 °C for 3 h to give compound **25** (20 mg). ESI-MS (m/z): 510.16 $[M+H]^+$. $^1H$ NMR (400 MHz, DMSO-d6) δ 8.40 - 8.25 (m, 2H), 7.94 (s, 1H), 7.41 - 7.30 (m, 2H), 7.15 - 7.06 (m, 1H), 7.03 - 6.95 (m, 1H), 6.49 - 6.43 and 5.62 - 5.55 (m, 1H), 4.87 - 4.60 (m, 2H), 4.43 - 4.41 (m, 3H), 3.78 (s, 3H), 2.71 and 2.60 (s, 3H), 2.24 (s, 3H).

Compound **25** was separated by SFC (instrument: Waters 150 Prep-SFC; chromatographic column: Chiral AS column; mobile phase A: $CO_2$, mobile phase B: methanol (containing 0.1% $NH_3 \bullet H_2O$); B%: 40%) to give compounds **25A** and **25B.** Compound **25A:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 1.832 min, ee = 99.94%. $[\alpha]^D_{25}$ +231.4 (c 1mg/mL, DMF).

Compound **25B:** SFC analytical method: instrument: SHIMADZU LC-20AD; chromatographic column: Chiral AS column; mobile phase A: $CO_2$, mobile phase B: methanol (containing 0.05% diethylamine); B%: 5-40%; retention time: 2.019 min, ee = 100%. $[\alpha]^D_{25}$ -253 (c 1mg/mL, DMF).

**Example 26: 4-amino-7-chloro-*N*-(6-((1,3-dimethyl-1*H*-pyrazol-4-yl)ethynyl)-2,3-dihydrobenz ofuran-3-yl)-*N*,1-dimethyl-1*H*-pyrazolo[4,3-*c*]quinoline-8-carboxamide (Compound 26)**

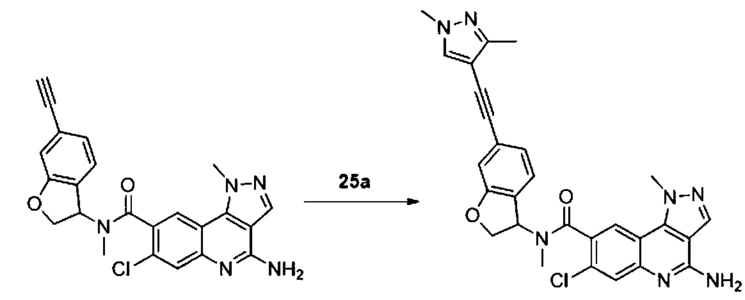


8d Compound 26

Referring to the preparation process of compound **8,** compound 1-cyclopropyl-4-iodopyrazole was replaced with compound **25a** (22.2 mg, 0.10 mmol), and the mixture was reacted at 25 °C for 3 h to give compound **26** (20 mg). ESI-MS (m/z): 525.98 $[M+H]^+$. $^1H$ NMR (400 MHz, DMSO-$d_6$) δ 8.44 - 8.21 (m, 2H), 7.95 - 7.93 (m, 1H), 7.70 - 7.63 (m, 1H), 7.52 - 7.27 (m, 3H), 7.14 - 6.95 (m, 2H), 6.52 - 6.50 and 5.47 - 5.42 (m, 1H), 4.89 - 4.55 (m, 2H), 4.47 - 4.41 (m, 3H), 3.79 and 3.78 (s, 3H), 2.74 - 2.51 (m, 3H), 2.25 - 2.23 (m, 3H).

**Example 26A: (*S*)-4-amino-7-chloro-*N*-(6-((1,3-dimethyl-1*H*-pyrazol-4-yl)ethynyl)-2,3-dihydrob enzofuran-3-yl)-*N*,1-dimethyl-1*H*-pyrazolo[4,3-*c*]quinoline-8-carboxamide (Compound 26A)**

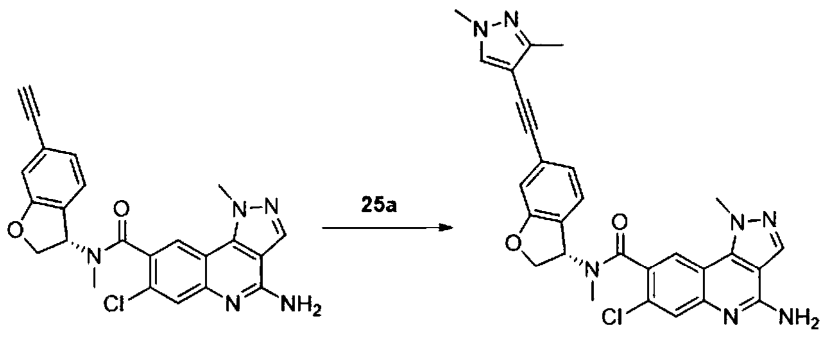


8d-A Compound 26A

For the synthesis process of preparing compound **26A** from compound **8d-A,** reference was made to the synthesis process of preparing compound **26** from compound **8d.**

Compound **26A:** ESI-MS (m/z): 525.97 [M+H]$^+$. $^1$H NMR (400 MHz, DMSO-$d_6$) δ 8.44 - 8.21 (m, 2H), 7.95 - 7.93 (m, 1H), 7.70 - 7.63 (m, 1H), 7.52 - 7.27 (m, 3H), 7.14 - 6.95 (m, 2H), 6.52 - 5.42 (m, 1H), 4.89 - 4.55 (m, 2H), 4.47 - 4.41 (m, 3H), 3.79 - 3.78 (m, 3H), 2.74 - 2.51 (m, 3H), 2.25 - 2.23 (m, 3H).

**Example 27: 4-amino-*N*-ethyl-7-fluoro-1-methyl-*N*-(6-((1-methyl-1*H*-pyrazol-4-yl)ethynyl)-2,3 -dihydrobenzofuran-3-yl)-1*H*-pyrazolo[4,3-*c*]quinoline-8-carboxamide (Compound 27)**

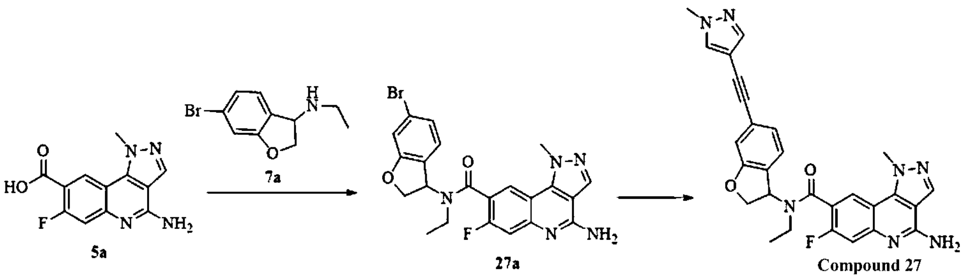

5a 7a 27a Compound 27

**Preparation of compound 27a**

Compound **5a** (106 mg, 0.41 mol) was dissolved in *N,N*-dimethylformamide (10 mL), and *N,N*-diisopropylethylamine (0.36 mL, 2.04 mol) was added. The mixture was stirred for 5 min under an ice bath, and then *N,N,N',N'*-tetramethyl-*O*-(7-azabenzotriazol-1-yl)uronium hexafluorophosphate (232.53 mg, 0.61 mol) was added. The mixture was allowed to react for another 5 min, and finally, **7a** (113.54 mg, 0.41 mol) was added. The mixture was allowed to react for another hour. Water (100 mL) was added to the reaction solution, and the reaction solution was extracted with ethyl acetate (20 mL × 3). The organic phases were washed three times with saturated brine, combined, dried over anhydrous sodium sulfate, and filtered. The filtrate was concentrated, and the residue was subjected to column chromatography (methanol:dichloromethane = 1:20) to give compound **27a** (120 mg). ESI-MS (m/z): 484.11 [M+H]$^+$.

**Preparation of compound 27**

To a solution of compound **27a** (120 mg, 0.25 mmol) in *N*-methylpyrrolidone (3 mL) were sequentially added 4-ethynyl-1-methyl-1*H*-pyrazole (53.0 mg, 0.50 mmol), cuprous iodide (9.5 mg, 0.05 mmol), *N,N*-diisopropylethylamine (0.17 mL, 0.99 mmol), and tetrakis(triphenylphosphine)palladium (28.7 mg, 0.025 mmol) under nitrogen atmosphere. After completion of the addition, the mixture was reacted in a microwave reactor at 100 °C for 3 h. Water (50 mL) was added to the reaction solution, and the reaction solution was extracted with ethyl acetate (20 mL × 3). The organic phases were

combined, washed with saturated brine, dried over anhydrous sodium sulfate, and filtered. The solvent was removed by rotary evaporation, and the residue was subjected to column chromatography (methanol:dichloromethane = 1:20) to give compound **27** (15 mg). ESI-MS (m/z): 510.1 [M+H]$^+$. $^1$H NMR (400 MHz, DMSO) δ 8.38 - 8.17 (m, 2H), 8.07 (s, 1H), 7.69 (s, 1H), 7.50 - 7.26 (m, 4H), 7.14 - 6.95 (m, 2H), 6.20 - 5.53 (m, 1H), 4.91 - 4.53 (m, 2H), 4.42 (s, 3H), 3.87 (s, 3H), 3.25 - 3.05 (m, 2H), 1.10 - 0.72 (m, 3H).

**Example 28: 4-amino-7-chloro-*N*-ethyl-1-methyl-*N*-(6-((1-methyl-1*H*-pyrazol-4-yl)ethynyl)-2,3 -dihydrobenzofuran-3-yl)-1*H*-pyrazolo[4,3-*c*]quinoline-8-carboxamide (Compound 28)**

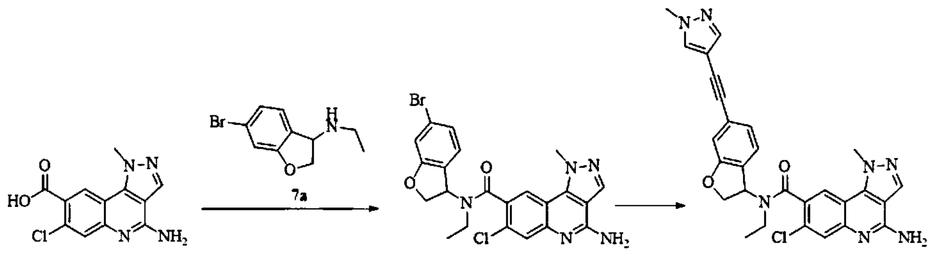


8a 28a Compound 28

**Preparation of compound 28a**

Referring to the preparation process of compound **27a,** compound **5a** was replaced with compound **8a** (120 mg, 0.43 mol) to give compound **28a** (160 mg). ESI-MS (m/z): 499.94 [M+H]$^+$.

**Preparation of compound 28**

Referring to the preparation process of compound **27,** compound **27a** was replaced with compound **28a** (100 mg, 0.2 mmol) to give compound **28** (20 mg). ESI-MS (m/z): 526.2 [M+H]$^+$. $^1$H NMR (400 MHz, DMSO) δ 8.40 - 8.15 (m, 2H), 8.11 - 8.04 (m, 1H), 7.72 - 7.62 (m, 2H), 7.55 - 7.36 (m, 1H), 7.30 (s, 2H), 7.16 - 6.93 (m, 2H), 6.22 - 5.31 (m, 1H), 4.91 - 4.57 (m, 2H), 4.51 - 4.37 (m, 3H), 3.87 (s, 3H), 3.32 - 2.91 (m, 2H), 1.14 - 0.75 (m, 3H).

**Example 29: 4-amino-*N*-(6-((1,5-dimethyl-1*H*-pyrazol-4-yl)ethynyl)-2,3-dihydrobenzofuran-3-yl)-*N*,1-dimethyl-1*H*-pyrazolo[4,3-*c*]quinoline-8-carboxamide (Compound 29)**

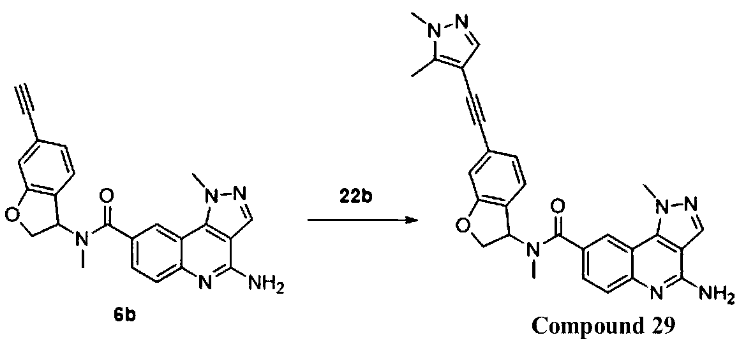


6b Compound 29

Referring to the preparation process of compound **19,** compound 4-iodo-1-isopropyl-1*H*-pyrazole was replaced with compound **22b** (22.2 mg, 0.10 mmol), and the mixture was reacted at 25 °C for 3 h to give compound **29** (15 mg). ESI-MS (m/z): 492.14 [M+H]$^+$. $^1$H NMR (400 MHz, DMSO-$d_6$) δ 8.33 (s, 1H), 8.28 (s, 1H), 7.68 - 7.61 (m, 2H), 7.58 (s, 1H), 7.44 (d, *J* = 8.0 Hz, 1H), 7.17 (s, 2H), 7.11 (d, *J* = 8.0 Hz, 1H), 7.00 (s, 1H), 6.64 - 5.80 (m, 1H), 4.82 - 4.66 (m, 2H), 4.44 (s, 3H), 3.77 (s, 3H), 2.70 (s, 3H), 2.37 (s, 3H).

**Example 29A: (*S*)-4-amino-*N*-(6-((1,5-dimethyl-1*H*-pyrazol-4-yl)ethynyl)-2,3-dihydrobenzofura n-3-yl)-*N*,1-dimethyl-1*H*-pyrazolo[4,3-*c*]quinoline-8-carboxamide (Compound 29A)**

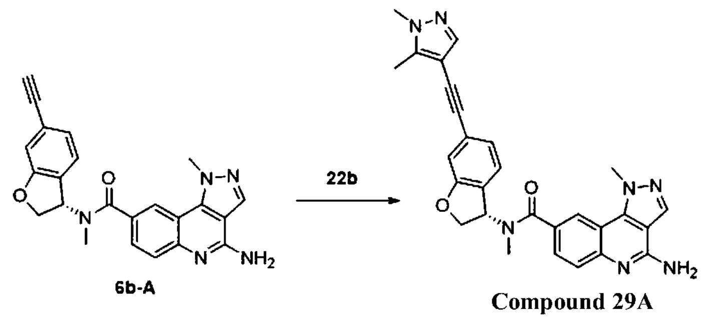


6b-A

Compound 29A

For the synthesis process of preparing compound **29A** from compound **6b-A,** reference was made to the synthesis process of preparing compound **29** from compound **6b.**

Compound **29A:** ESI-MS (m/z): 492.16 $[M+H]^+$. $^1H$ NMR (400 MHz, DMSO-$d_6$) δ 8.33 (s, 1H), 8.28 (s, 1H), 7.68 - 7.61 (m, 2H), 7.58 (s, 1H), 7.44 (d, *J* = 8.0 Hz, 1H), 7.18 (s, 2H), 7.11 (d, *J* = 8.0 Hz, 1H), 7.00 (s, 1H), 6.64 - 5.80 (m, 1H), 4.80 - 4.69 (m, 2H), 4.44 (s, 3H), 3.77 (s, 3H), 2.70 (s, 3H), 2.37 (s, 3H).

**Example 30: 4-amino-*N*-(6-((1-(difluoromethyl)-1*H*-pyrazol-4-yl)ethynyl)-2,3-dihydrobenzofu ran-3-yl)-*N*,1-dimethyl-1*H*-pyrazolo[4,3-*c*]quinoline-8-carboxamide (Compound 30)**

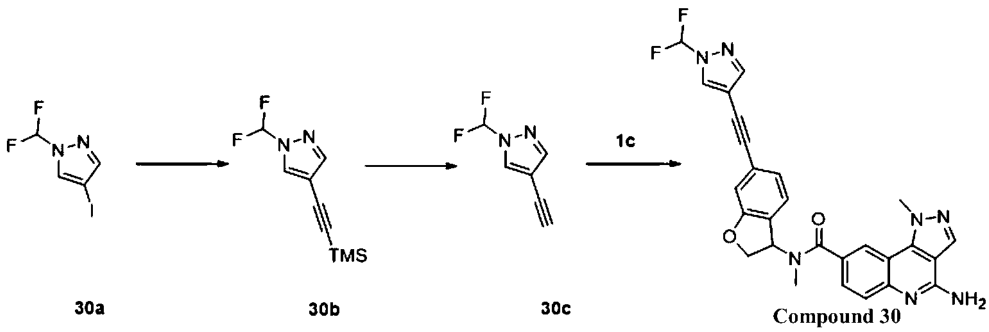


30a 30b 30c Compound 30

**Preparation of compound 30b**

To a solution of compound **30a** (300 mg, 1.23 mmol) in N-methylpyrrolidone (5 mL) were added cuprous iodide (36 mg, 0.19 mmol), tetrakis(triphenylphosphine)palladium (288 mg, 0.25 mmol), N,N-diisopropylethylamine (476 mg, 3.69 mmol), and trimethylsilylacetylene (242 mg, 2.46 mmol). After completion of the addition, the mixture was reacted in a microwave reactor at 80 °C for 3 h under nitrogen atmosphere. The reaction solution was filtered through celite. Water (50 mL) was added, and the reaction solution was extracted with ethyl acetate (50 mL ×3). The organic phases were combined, washed with saturated brine, dried over anhydrous sodium sulfate, and filtered. The filtrate was concentrated, and the residue was subjected to normal phase column chromatography (methanol/dichloromethane = 1:20) to give compound **30b** (178 mg). ESI-MS (m/z): 215.1 $[M+H]^+$.

**Preparation of compound 30c**

Tetrabutylammonium fluoride (434 mg, 1.66 mmol) was added to a solution of compound **30b** (178 mg, 0.83 mmol) in tetrahydrofuran (5 mL), and the mixture was allowed to react at 25 °C for 0.5 h. Water (30 mL) was added to the reaction solution, and the reaction solution was extracted with ethyl acetate (30 mL × 3). The organic phase was dried over anhydrous sodium sulfate and filtered. The filtrate was concentrated, and the residue was subjected to normal phase column chromatography (methanol/dichloromethane = 1:15) to give compound **30c** (89 mg). ESI-MS (m/z): 143.1 $[M+H]^+$.

**Preparation of compound 30**

To a solution of **1c** (45 mg, 0.1 mmol) in N-methylpyrrolidone (2 mL) were added cuprous iodide (4 mg, 0.02 mmol), tetrakis(triphenylphosphine)palladium (23 mg, 0.02 mmol), *N,N*-diisopropylethylamine (39 mg, 0.3 mmol), and **30c** (29 mg, 0.2 mmol). After completion of the addition, the mixture was reacted in a microwave reactor at 80 °C for 3 h under nitrogen atmosphere. The reaction solution was filtered through celite. Water (20 mL) was added, and the reaction solution was extracted with ethyl acetate (20 mL × 3). The organic phases were combined, washed with saturated brine, dried over anhydrous sodium sulfate, and filtered. The filtrate was concentrated, and the residue was subjected to reversed phase column chromatography (acetonitrile/0.05% ammonium bicarbonate solution = 0:100% - 50%:50%) to give compound **30** (15 mg). ESI-MS (m/z): 513.9 $[M+H]^+$. $^1$HNMR (400 MHz, DMSO-$d_6$) δ 8.67 (s, 1H), 8.34 (s, 1H), 8.28 (s, 1H), 8.08 (s, 1H), 7.86 (t, *J* = 58.8 Hz, 1H), 7.65 (s, 2H), 7.53 - 7.42 (m, 1H), 7.17 (s, 3H), 7.04 (s, 1H), 6.57 - 5.50 (m, 1H), 4.83 - 4.69 (m, 2H), 4.44 (s, 3H), 2.71 (s, 3H).

**Example 30A: (*S*)-4-amino-*N*-(6-((1-(difluoromethyl)-1*H*-pyrazol-4-yl)ethynyl)-2,3-dihydrobenz ofuran-3-yl)-*N*,1-dimethyl-1*H*-pyrazolo[4,3-*c*]quinoline-8-carboxamide (Compound 30A)**

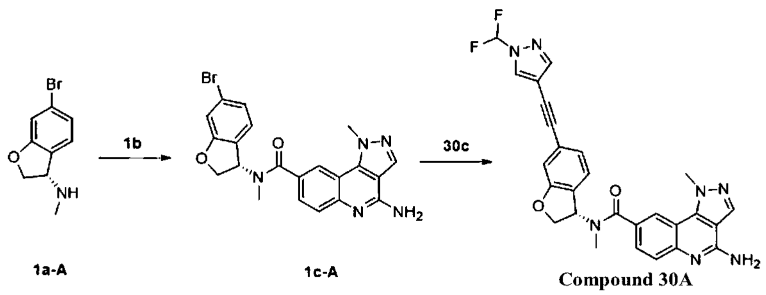


1a-A 1c-A Compound 30A

For the preparation process of compound **1c-A,** reference was made to the preparation process of compound **1c**. For the synthesis process of preparing compound **30A** from compound **1c-A**, reference was made to the synthesis process of preparing compound **30** from compound **1c.**

Compound **30A:** ESI-MS (m/z): 513.98 $[M+H]^+$. $^1$H NMR (400 MHz, DMSO) δ 8.65 (s, 1H), 8.34 - 8.24 (m, 2H), 8.05 (s, 1H), 7.83 (t, *J* = 59 Hz, 1H), 7.66 - 7.59 (m, 2H), 7.51 - 7.41 (m, 1H), 7.14 - 7.12 (m, 3H), 7.02 (s, 1H), 6.63 - 5.60 (m, 1H), 4.84 - 4.61 (m, 2H), 4.42 (s, 3H), 2.68 (s, 3H).

**Example 31: 4-amino-*N*,1-dimethyl-*N*-(6-((1-methyl-3-(trifluoromethyl)-1*H*-pyrazol-4-yl)ethy nyl)-2,3-dihydrobenzofuran-3-yl)-1*H*-pyrazolo[4,3-*c*]quinoline-8-carboxamide (Compound 31)**

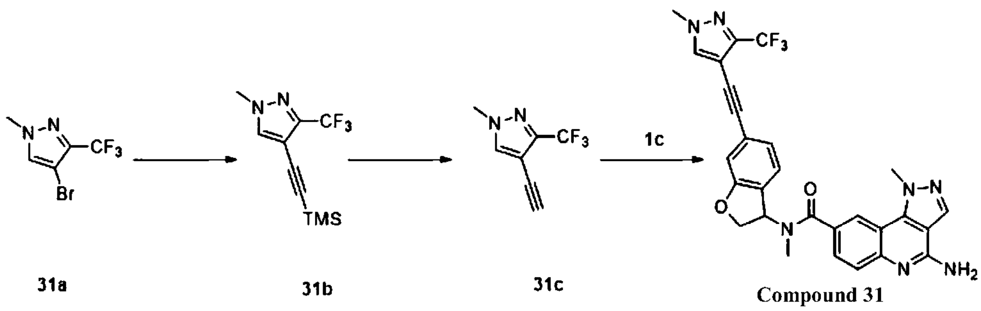


31a 31b 31c Compound 31

**Preparation of compound 31b**

To a solution of compound **31a** (500 mg, 2.19 mmol) in N-methylpyrrolidone (10 mL) were added cuprous iodide (63 mg, 0.33 mmol), tetrakis(triphenylphosphine)palladium (508 mg, 0.44 mmol), *N,N*-diisopropylethylamine (848 mg, 6.57 mmol), and trimethylsilylacetylene (430 mg, 4.38 mmol). After completion of the addition, the mixture was reacted in a microwave reactor at 80 °C for 3 h under nitrogen atmosphere. The reaction solution was filtered through celite. Water (50

mL) was added, and the reaction solution was extracted with ethyl acetate (50 mL ×3). The organic phases were combined, washed with saturated brine, dried over anhydrous sodium sulfate, and filtered. The filtrate was concentrated, and the residue was subjected to normal phase column chromatography (methanol/dichloromethane = 1:20) to give compound **31b** (350 mg). ESI-MS (m/z): 247.1 $[M+H]^+$.

**Preparation of compound 31c**

Tetrabutylammonium fluoride (741 mg, 2.84 mmol) was added to a solution of compound **31b** (350 mg, 1.42 mmol) in tetrahydrofuran (10 mL), and the mixture was allowed to react at 25 °C for 0.5 h. Water (50 mL) was added to the reaction solution, and the reaction solution was extracted with ethyl acetate (50 mL × 3). The organic phase was dried over anhydrous sodium sulfate and filtered. The filtrate was concentrated, and the residue was subjected to normal phase column chromatography (methanol/dichloromethane = 1:15) to give compound **31c** (165 mg). ESI-MS (m/z): 175.1 $[M+H]^+$.

**Preparation of compound 31**

Referring to the preparation process of compound **30,** compound **30c** was replaced with compound **31c** (35 mg, 0.2 mmol) to give compound **31** (15 mg). ESI-MS (m/z): 546.2 $[M+H]^+$. $^1$HNMR (400 MHz, DMSO-$d_6$) δ 8.33 (s, 2H), 8.28 (s, 1H), 7.64 (s, 2H), 7.48 (d, *J* = 7.6 Hz, 1H), 7.16 (s, 2H), 7.11 (d, *J* = 7.6 Hz, 1H), 6.98 (s, 1H), 6.47 - 5.70 (m, 1H), 4.82 - 4.68 (m, 2H), 4.43 (s, 3H), 3.96 (s, 3H), 2.70 (s, 3H).

**Example 31A: (*S*)-4-amino-*N*,1-dimethyl-*N*-(6-((1-methyl-3-(trifluoromethyl)-1*H*-pyrazol-4-yl)e thynyl)-2,3-dihydrobenzofuran-3-yl)-1*H*-pyrazolo[4,3-*c*]quinoline-8-carboxamide (Compound 31A)**

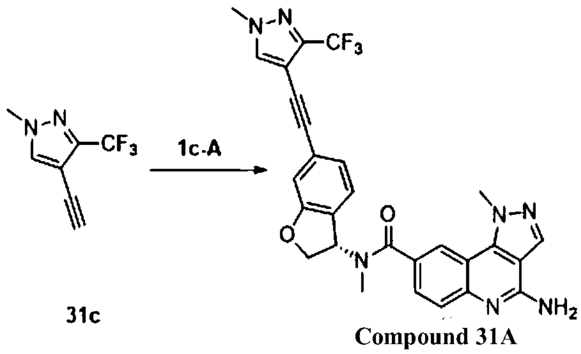


31c

Compound 31A

For the synthesis process of preparing compound **31A** from compound 31c and compound **1c-A**, reference was made to the synthesis process of preparing compound **31** from compound **1c**.

Compound **31A:** ESI-MS (m/z): 546.17 $[M+H]^+$. $^1$H NMR (400 MHz, DMSO) δ 8.31 (s, 2H), 8.26 (s, 1H), 7.67 - 7.60 (m, 2H), 7.50 - 7.43 (m, 1H), 7.14 (s, 2H), 7.11 - 7.06 (m, 1H), 6.97 (s, 1H), 6.44 - 5.74 (m, 1H), 4.82 - 4.70 (m, 2H), 4.42 (s, 3H), 3.94 (s, 3H), 2.68 (s, 3H).

**Example 32: 4-amino-7-fluoro-*N*,1-dimethyl-*N*-(6-((1-methyl-3-(trifluoromethyl)-1*H*-pyrazol-4 -yl)ethynyl)-2,3-dihydrobenzofuran-3-yl)-1*H*-pyrazolo[4,3-*c*]quinoline-8-carboxa mide (Compound 32)**

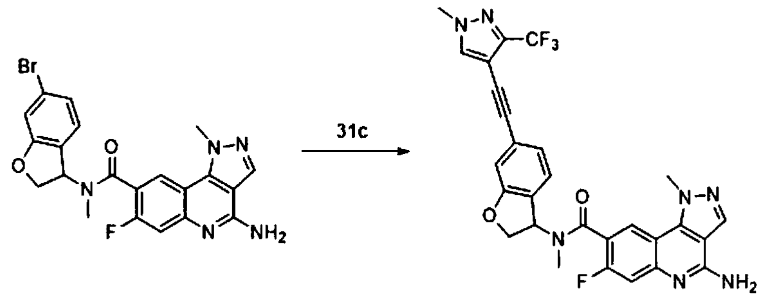


5b    Compound 32

Referring to the preparation process of compound **5,** compound 4-ethynyl-1-methylpyrazole was replaced with compound **31c** (35 mg, 0.2 mmol), and the mixture was reacted in a microwave reactor at 80 °C for 3 h under nitrogen atmosphere to give compound **32** (15 mg). ESI-MS (m/z): 563.6 [M+H]+. $^{1}$HNMR (400 MHz, DMSO-$d_6$) δ 8.48 - 8.16 (m, 3H), 7.55 - 7.19 (m, 4H), 7.18 - 6.96 (m, 2H), 6.48 - 5.60 (m, 1H), 4.87 - 4.61 (m, 2H), 4.43 - 4.40 (m, 3H), 3.96 (s, 3H), 2.72 - 2.60 (m, 3H).

**Example 33: 4-amino-7-fluoro-*N*,1-dimethyl-*N*-(6-((1-methyl-1*H*-pyrazol-4-yl)ethynyl)-2,3-dih ydrofuro[2,3-b] pyridin-3-yl)-1*H*-pyrazolo[4,3-*c*]quinoline-8-carboxamide (Compound 33)**

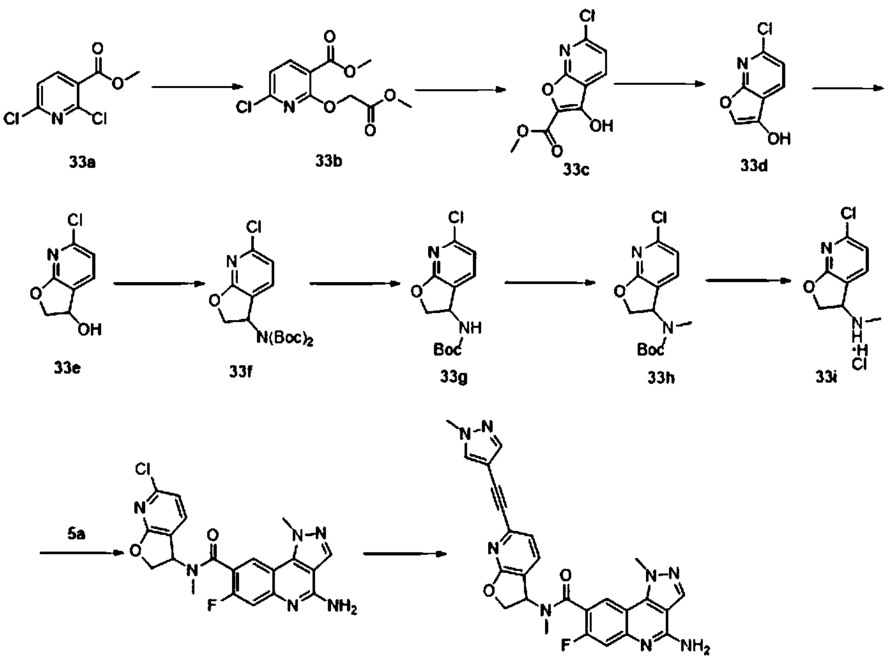


33a 33b 33c 33d 33e 33f 33g 33h 33i 33j

Compound 33

**Preparation of compound 33b**

Compound methyl glycolate (450 mg, 5.00 mmol) was dissolved in ethylene glycol dimethyl ether (10 mL). The mixture was cooled to 0 °C, and sodium hydride (210 mg, 5.25 mmol, 60% purity) was added. After the reaction solution was reacted at 0 °C for 30 min, compound **33a** (1.03 g, 5.00 mmol) was added. Then the reaction solution was stirred at 25 °C for 2 h. Water (5 mL) was added to the reaction solution to quench the reaction, and the reaction solution was extracted with ethyl acetate (30 mL ×3). The organic phases were combined and concentrated to give a crude product **33b** (1.15 g). MS (m/z): 259.95 [M+H]+.

**Preparation of compound 33c**

Compound **33b** (416 mg, 1.60 mmol) was dissolved in tetrahydrofuran (20 mL), and potassium tert-butoxide (215

mg, 1.92 mmol) was added. After the reaction solution was reacted at 25 °C for 1 h, water (20 mL) was added to the reaction solution to quench the reaction, and the reaction solution was extracted with ethyl acetate (5 mL × 3). The organic phases were combined and concentrated to give a crude product.

The crude product was then subjected to column chromatography (petroleum ether:ethyl acetate = 2:1) to give **33c** (240 mg). ESI-MS (m/z): 228.08 $[M+H]^+$.

**Preparation of compound 33d**

Compound **33c** (228 mg, 1.00 mmol) was dissolved in methanol (20 mL), and a 6 M hydrochloric acid solution (5 mL) was added. The reaction solution was heated to 100 °C and stirred for 16 h, then diluted with ethyl acetate (50 mL) and water (50 mL), and extracted with ethyl acetate (20 mL × 3). The organic phases were combined and concentrated to give a crude product. The crude product was subjected to column chromatography (petroleum ether:ethyl acetate = 5:1) to give **33d** (140 mg). ESI-MS (m/z): 170.01 $[M+H]^+$.

**Preparation of compound 33e**

Compound **33d** (138 mg, 0.81 mmol) was dissolved in methanol (5 mL), and sodium borohydride (46 mg, 1.22 mmol) was added. The reaction solution was stirred at 25 °C for 1 h, then diluted with ethyl acetate (20 mL) and water (20 mL), and extracted with ethyl acetate (10 mL × 3). The organic phases were combined and concentrated to give a crude product. The crude product was subjected to column chromatography (petroleum ether:ethyl acetate = 2:1) to give **33e** (98 mg). ESI-MS (m/z): 172.04 $[M+H]^+$.

**Preparation of compound 33f**

To a solution of compound **33e** (700 mg, 4.08 mmol) in tetrahydrofuran (20 mL) were added bis(tert-butoxycarbonyl)amine (1.06 g, 4.90 mmol), triphenylphosphine (1.60 g, 6.12 mmol), and diisopropyl azodicarboxylate (1.24 g, 6.12 mmol), and the mixture was reacted at 25 °C for 2 h under nitrogen atmosphere. The reaction solution was directly concentrated to give a crude product. The crude product was subjected to normal phase column chromatography (petroleum ether/ethyl acetate = 10:1) to give compound **33f** (1.10 g). ESI-MS (m/z): 371.15 $[M+H]^+$.

**Preparation of compound 33g**

Compound **33f** (1.10 g, 2.97 mmol) was dissolved in acetonitrile (20 mL), and lithium bromide (0.80 g, 8.90 mmol, 60% purity) was added. The reaction solution was reacted at 65 °C for 16 h. Water (5 mL) was added to the reaction solution to quench the reaction, and the reaction solution was extracted with ethyl acetate (5 mL × 3). The organic phases were combined and concentrated to give a crude product. The crude product was subjected to column chromatography (petroleum ether:ethyl acetate = 3:1) to give **33g** (610 mg). ESI-MS (m/z): 271.05 $[M+H]^+$.

**Preparation of compound 33h**

Compound **33g** (271 mg, 1.00 mmol) was dissolved in tetrahydrofuran (5 mL), and the mixture was cooled to 0 °C. Sodium hydride (80.0 mg, 2.00 mmol, 60% purity) was added. After the reaction solution was reacted at 0 °C for 1 h, iodomethane (0.10 mg, 1.20 mmol) was added. Then the reaction solution was stirred at 25 °C for 12 h. Water (5 mL) was added to the reaction solution to quench the reaction, and the reaction solution was extracted with ethyl acetate (5 mL × 3). The organic phases were combined and concentrated to give a crude product. The crude product was then purified by preparative thin-layer chromatography (petroleum ether:ethyl acetate = 20:1) to give **33h** (110 mg).

**Preparation of compound 33i**

Compound **33h** (70 mg, 0.25 mmol) was dissolved in tetrahydrofuran (2 mL), and a 4 M dioxane solution of hydrochloric acid (0.5 mL) was added. The reaction solution was stirred at 25 °C for 1 h and then concentrated to dryness by rotary evaporation to give compound **33i** (40 mg). ESI-MS (m/z): 184.96 $[M+H]^+$.

**Preparation of compound 33j**

To a solution of compound **5a** (51.9 mg, 0.20 mmol) in *N,N*-dimethylformamide (3 mL) were added compound **33i** (44.22 mg, 0.20 mmol), triethylamine (80.7 mg, 0.80 mmol), and O-(7-azabenzotriazol-1-yl)-*N*,*N*,*N*',*N*'-tetramethyluronium hexafluorophosphate (113.7 mg, 0.30 mmol), and the mixture was allowed to react at 25 °C overnight. Water (50

mL) was added to the reaction solution, and the reaction solution was extracted with ethyl acetate (25 mL × 2). The organic phases were washed three times with saturated brine, combined, dried over anhydrous sodium sulfate, and filtered. The filtrate was concentrated, and the residue was subjected to normal phase column chromatography (dichloromethane/-methanol = 10:1) to give compound **33j** (40 mg). ESI-MS (m/z): 426.95 [M+H]$^+$.

**Preparation of compound 33**

To a solution of compound **33j** (40 mg, 0.09 mmol) in *N,N*-dimethylformamide (2 mL) were sequentially added 4-ethynyl-1-methylpyrazole (9.9 mg, 0.09 mmol), XPhos Pd G3 95% (15.9 mg, 0.02 mmol), and triethylamine (37.9 mg, 0.37 mmol) under nitrogen atmosphere. After completion of the addition, the mixture was heated to 80 °C in a microwave reactor and reacted for 3 h. Water (10 mL) was added to the reaction solution, and the reaction solution was extracted with ethyl acetate (10 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give a crude product. The crude product was subjected to reversed phase column chromatography (acetonitrile/0.05% aqueous ammonia bicarbonate solution = 0:100% - 40%:60%) to give compound **33** (5 mg). ESI-MS (m/z): 496.96 [M+H]$^+$. $^1$H NMR (400 MHz, DMSO-$d_6$) δ 8.39 - 8.23 (m, 2H), 8.16 (s, 1H), 7.93 - 7.81 (m, 1H), 7.76 (s, 1H), 7.40 - 7.17 (m, 4H), 6.46 - 5.60 (m, 1H), 4.97 - 4.59 (m, 2H), 4.46 - 4.40 (m, 3H), 3.89 (s, 3H), 2.77 - 2.65 (m, 3H).

**Example 34: 4-amino-*N*-(6-((3-chloro-1-methyl-1*H*-pyrazol-4-yl)ethynyl)-2,3-dihydrobenzofur an-3-yl)-*N*,1-dimethyl-1*H*-pyrazolo[4,3-*c*]quinoline-8-carboxamide (Compound 34)**

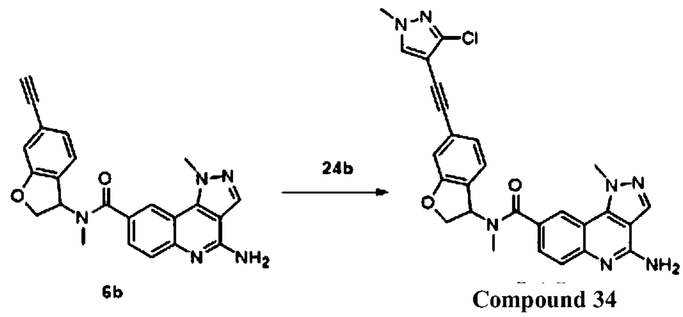


6b Compound 34

Referring to the preparation process of compound **19,** compound 4-iodo-1-isopropyl-1*H*-pyrazole was replaced with compound 3-chloro-4-iodo-1-methylpyrazole **(24b,** 24.2 mg, 0.10 mmol), and the mixture was reacted at 25 °C for 3 h to give compound **34** (22 mg). ESI-MS (m/z): 511.92 [M+H]$^+$. $^1$H NMR (400 MHz, DMSO-$d_6$) δ 8.34 (s, 1H), 8.28 (s, 1H), 8.18 (s, 1H), 7.68 - 7.62 (m, 2H), 7.48 (d, *J* = 8.0 Hz, 1H), 7.18 (s, 2H), 7.13 (d, *J* = 8.0 Hz, 1H), 7.01 (s, 1H), 6.57 - 5.70 (m, 1H), 4.83 - 4.70 (m, 2H), 4.44 (s, 3H), 3.85 (s, 3H), 2.70 (s, 3H).

**Example 34A: (*S*)-4-amino-*N*-(6-((3-chloro-1-methyl-1*H*-pyrazol-4-yl)ethynyl)-2,3-dihydrobenz ofuran-3-yl)-*N*,1-dimethyl-1*H*-pyrazolo[4,3-*c*]quinoline-8-carboxamide (Compound 34A)**

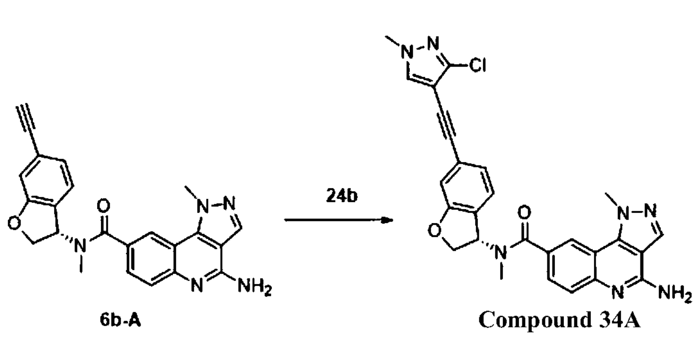


6b-A Compound 34A

For the synthesis process of preparing compound **34A** from compound **6b-A,** reference was made to the

synthesis process of preparing compound **34** from compound **6b.**

Compound **34A:** ESI-MS (m/z): 512.02 [M+H]+. $^1$H NMR (400 MHz, DMSO-$d_6$) δ 8.34 (s, 1H), 8.28 (s, 1H), 8.18 (s, 1H), 7.68 - 7.62 (m, 2H), 7.48 (d, *J* = 8.0 Hz, 1H), 7.18 (s, 2H), 7.13 (d, *J* = 8.0 Hz, 1H), 7.01 (s, 1H), 6.57 - 5.70 (m, 1H), 4.83 - 4.70 (m, 2H), 4.44 (s, 3H), 3.85 (s, 3H), 2.70 (s, 3H).

**Example 35: 4-amino-7-fluoro-*N*,3-dimethyl-*N*-(6-((1-methyl-1*H*-pyrazol-4-yl)ethynyl)-2,3-dih ydrobenzofuran-3-yl)-1,3-dihydrofuro[3,4-*c*]quinoline-8-carboxamide (Compound 35)**

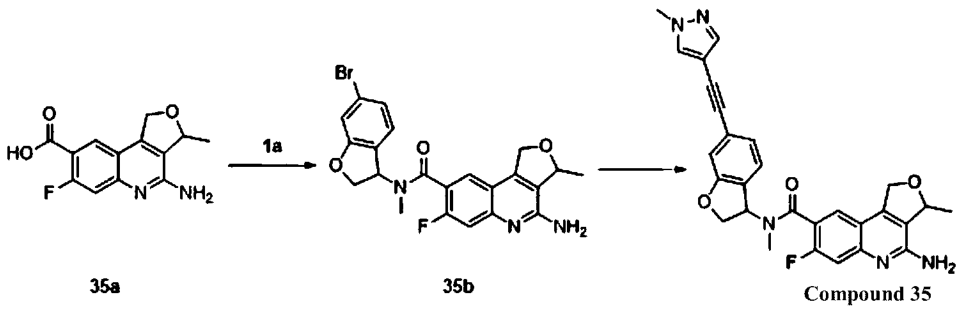


35a 35b Compound 35

**Preparation of compound 35b**

To a solution of compound **35a** (321.9 mg, 1.23 mmol) in *N,N*-dimethylformamide (10 mL) were added compound **1a** (325.33 mg, 1.23 mmol), triethylamine (310.6 mg, 3.07 mmol), and O-(7-azabenzotriazol-1-yl)-*N*,*N*,*N'*,*N'*-tetramethyluronium hexafluorophosphate (701.5 mg, 1.84 mmol), and the mixture was allowed to react at 25 °C overnight. Water (50 mL) was added to the reaction solution, and the reaction solution was extracted with ethyl acetate (25 mL × 2). The organic phases were washed three times with saturated brine, combined, dried over anhydrous sodium sulfate, and filtered. The filtrate was concentrated, and the residue was subjected to normal phase column chromatography (dichloromethane/-methanol = 10:1) to give compound 35b (510 mg). ESI-MS (m/z): 472.02 [M+H]+.

**Preparation of compound 35**

To a solution of compound **35b** (47.2 mg, 0.10 mmol) in N,N-dimethylformamide (2 mL) were sequentially added 4-ethynyl-1-methylpyrazole (21.2 mg, 0.20 mmol), cuprous iodide (7.7 mg, 0.04 mmol), triethylamine (40.9 mg, 0.40 mmol), and tetrakis(triphenylphosphine)palladium (23.4 mg, 0.02 mmol) under nitrogen atmosphere. After completion of the addition, the mixture was heated to 100 °C in a microwave reactor and reacted for 3 h. Water (10 mL) was added to the reaction solution, and the reaction solution was extracted with ethyl acetate (10 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give a crude product. The crude product was then subjected to reversed phase column chromatography (acetonitrile/0.05% aqueous ammonia bicarbonate solution = 0:100% - 40%:60%) to give compound 35 (21 mg). ESI-MS (m/z): 497.95 [M+H]+. $^1$H NMR (400 MHz, DMSO-$d_6$) δ 8.08 - 8.06 (m, 1H), 7.90 - 7.60 (m, 2H), 7.40 - 7.27 (m, 2H), 7.15 - 7.06 (m, 1H), 6.99 (d, *J* = 8.0 Hz, 1H), 6.76 (s, 2H),6.45 - 6.41 and 5.54 - 5.48 (m, 1H), 5.48 - 5.23 (m, 3H), 4.83 - 4.53 (m, 2H), 3.87 (s, 3H), 2.68 - 2.55 (m, 3H), 1.43 - 1.41 (m, 3H).

**Example 36: 4-amino-7-fluoro-*N*,1-dimethyl-*N*-(6-((1-methyl-1*H*-pyrazol-5-yl)ethynyl)-2,3-dih ydrobenzofuran-3-yl)-1*H*-pyrazolo[4,3-*c*]quinoline-8-carboxamide (Compound 36)**

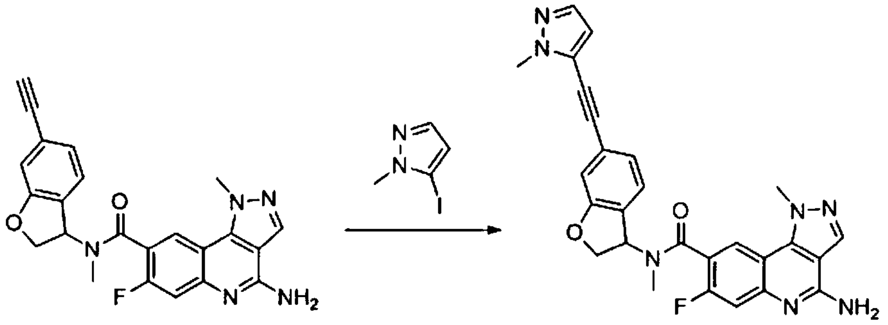

12b

Compound 36

Referring to the preparation process of compound **12,** compound 1-cyclopropyl-4-iodo-1*H*-pyrazole was replaced with compound 5-iodo-1-methylpyrazole (49.9 mg, 0.24 mmol), and the mixture was reacted at 50 C for 3 h under nitrogen atmosphere to give compound **36** (18 mg). ESI-MS (m/z): 496.0 $[M+H]^+$. $^1$H NMR (400 MHz, DMSO) δ 8.44 - 8.18 (m, 2H), 7.51 (s, 1H), 7.48 - 7.08 (m, 6H), 6.61 (s, 1H), 6.51 - 6.40 and 5.69 - 5.54 (m, 1H), 4.87 - 4.61 (m, 2H), 4.41 and 4.39 (s, 3H), 3.93 (s, 3H), 2.70 - 2.59 (m, 3H).

**Example 37: 4-amino-*N*-(6-((1,4-dimethyl-1*H*-pyrazol-3-yl)ethynyl)-2,3-dihydrobenzofuran-3-yl)-7-fluoro-*N*,1-dimethyl-1*H*-pyrazolo[4,3-*c*]quinoline-8-carboxamide (Compound 37)**

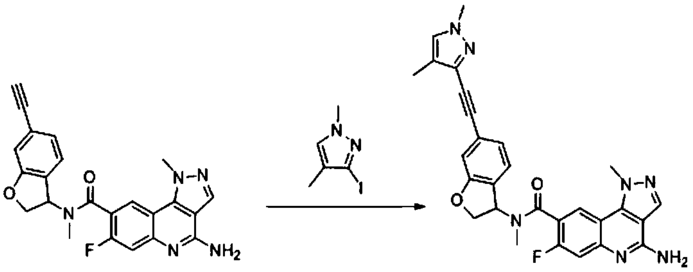

12b

Compound 37

Referring to the preparation process of compound **12,** compound 1-cyclopropyl-4-iodo-1*H*-pyrazole was replaced with compound 3-iodo-1,4-dimethylpyrazole (22.2 mg, 0.10 mmol), and the mixture was reacted at 25 °C for 3 h to give compound **37** (5 mg). ESI-MS (m/z): 510.14 $[M+H]^+$. $^1$H NMR (400 MHz, DMSO-$d_6$) δ 8.39 - 8.23 (m, 2H), 7.58 (s, 1H), 7.49 - 7.30 (m, 4H), 7.22 - 7.11 (m, 1H), 7.09 - 7.00 (m, 1H), 6.50 - 6.44 and 5.62 - 5.57(m, 1H), 4.88 - 4.60 (m, 2H), 4.43 - 4.41 (m, 3H), 3.81 (s, 3H), 2.72 - 2.61 (m, 3H), 2.09 (s, 3H).

**Example 38: 4-amino-7-fluoro-*N*-(6-((4-fluoro-1-methyl-1*H*-pyrazol-3-yl)ethynyl)-2,3-dihydro benzofuran-3-yl)-*N*,1-dimethyl-1*H*-pyrazolo[4,3-*c*]quinoline-8-carboxamide (Compound 38)**

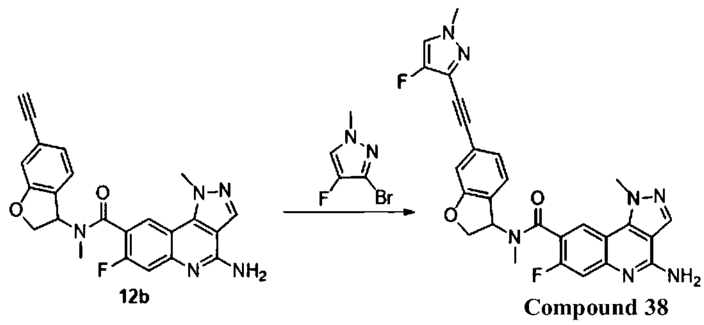

12b

Compound 38

Referring to the preparation process of compound **12,** compound 1-cyclopropyl-4-iodo-1*H*-pyrazole was

replaced with compound 3-bromo-4-fluoro-1-methylpyrazole (17.9 mg, 0.10 mmol), and the mixture was reacted in a microwave reactor at 80 °C for 3 h to give compound **38** (5 mg). ESI-MS (m/z): 514.21 [M+H]$^+$. $^1$H NMR (400 MHz, DMSO-d6) δ 8.39 - 8.22 (m, 2H), 8.00 (d, *J* = 4.8 Hz, 1H), 7.54 - 7.27 (m, 4H), 7.23 - 7.14 (m, 1H), 7.12 - 7.03 (m, 1H), 6.51 - 6.44 and 5.63 - 5.58 (m, 1H), 4.90 - 4.61 (m, 2H), 4.43 - 4.41 (m, 3H), 3.83 (s, 3H), 2.72 - 2.61 (m, 3H).

**Example 39: 4-amino-7-fluoro-*N*-methyl-*N*-(6-((1-methyl-1*H*-pyrazol-4-yl)ethynyl)-2,3-dihydr obenzofuran-3-yl)-1,3-dihydrofuro[3,4-*c*]quinoline-8-carboxamide (Compound 39)**

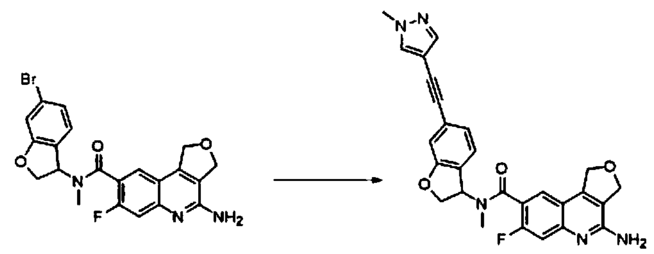

16b

Compound 39

To a solution of 16b (90 mg, 0.20 mmol) in N-methylpyrrolidone (3 mL) were added cuprous iodide (7.6 mg, 0.04 mmol), tetrakis(triphenylphosphine)palladium (23.1 mg, 0.02 mmol), *N,N*-diisopropylethylamine (99.38 μL, 0.60 mmol), and 4-ethynyl-1-methylpyrazole (63.7 mg, 0.60 mmol). After completion of the addition, the mixture was reacted in a microwave reactor at 100 °C for 4 h under nitrogen atmosphere. The reaction solution was filtered through celite. Water (50 mL) was added, and the reaction solution was extracted with ethyl acetate (25 mL × 2). The organic phases were combined, washed with saturated brine, dried over anhydrous sodium sulfate, and filtered. The filtrate was concentrated, and the residue was purified by a preparative plate (dichloromethane/methanol = 10:1) to give a crude compound. The crude compound was then subjected to reversed phase column chromatography (acetonitrile/0.05% ammonium bicarbonate solution = 0:100% - 45%:55%) to give compound **39** (22 mg). ESI-MS (m/z): 483.9 [M+H]$^+$. $^1$H NMR (400 MHz, DMSO) δ 8.08 - 8.05 (m, 1H), 7.75 - 7.60 (m, 2H), 7.39 - 7.27 (m, 2H), 7.14 - 7.04 (m, 1H), 6.96 (d, *J* = 17.2 Hz, 1H), 6.82 (s, 2H), 6.45 - 6.36 and 5.54 - 5.44 (m, 1H), 5.37 - 5.31 (m, 2H), 5.00 (s, 2H), 4.83 - 4.51 (m, 2H), 3.85 (s, 3H), 2.66 - 2.53 (m, 3H).

**Example 39A: (*S*)-4-amino-7-fluoro-*N*-methyl-*N*-(6-((1-methyl-1*H*-pyrazol-4-yl)ethynyl)-2,3-dih ydrobenzofuran-3-yl)-1,3-dihydrofuro[3,4-*c*]quinoline-8-carboxamide (Compound 39A)**

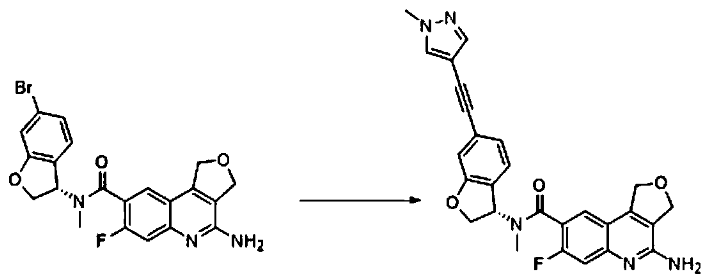

16b-A

Compound 39A

For the synthesis process of preparing compound **39A** from compound **16b-A,** reference was made to the synthesis process of preparing compound **39** from compound **16b.**

Compound **39A:** ESI-MS (m/z): 484.00 [M+H]$^+$. $^1$H NMR (400 MHz, DMSO) δ 8.10 - 8.07 (m, 1H), 7.74 - 7.62 (m, 2H), 7.42 - 7.28 (m, 2H), 7.15 - 7.06 (m, 1H), 7.05 - 6.95 (m, 1H), 6.85 (s, 2H), 6.48 - 6.39 and 5.55 - 5.46 (m, 1H), 5.42 - 5.33 (m, 2H), 5.03 (s, 2H), 4.85 - 4.57 (m, 2H), 3.88 (s, 3H), 2.69 - 2.56 (m, 3H).

**Example 40: 4-amino-7-chloro-*N*-methyl-*N*-(6-((1-methyl-1*H*-pyrazol-4-yl)ethynyl)-2,3-dihydr obenzofuran-3-yl)-1,3-dihydrofuro[3,4-*c*]quinoline-8-carboxamide (Compound 40)**

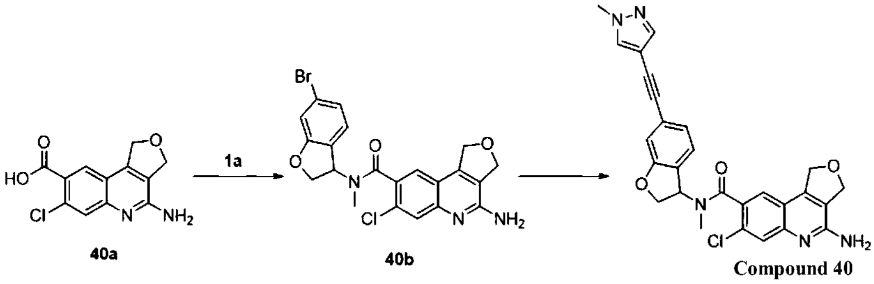


40a 40b Compound 40

**Preparation of compound 40b**

To a solution of compound **40a** (150 mg, 0.57 mmol, see WO2022169948 A1 for the preparation method) in *N,N*-dimethylformamide (5 mL) were added compound **1a** (149.9 mg, 0.57 mmol), *N,N*-diisopropylethylamine (366.1 mg, 2.83 mmol), and O-(7-azabenzotriazol-1-yl)-*N,N,N',N'*-tetramethyluronium hexafluorophosphate (323.2 mg, 0.85 mmol), and the mixture was allowed to react at 25 °C for 16 h. After the reaction was completed, the reaction solution was cooled to room temperature. Water (50 mL) was added, and the reaction solution was extracted with ethyl acetate (25 mL × 2). The organic phases were washed three times with saturated brine, combined, dried over anhydrous sodium sulfate, and filtered. The filtrate was concentrated, and the residue was subjected to normal phase column chromatography (dichloromethane/methanol = 20:1) to give compound **40b** (200 mg). ESI-MS (m/z): 473.96 $[M+H]^+$.

**Preparation of compound 40**

Referring to the preparation method for compound **39,** compound **16b** was replaced with compound **40b** (100 mg, 0.21 mmol), and the mixture was reacted in a microwave reactor at 100 °C for 4 h under nitrogen atmosphere to give compound **40** (40 mg). ESI-MS (m/z): 500.1 $[M+H]^+$. $^1$H NMR (400 MHz, DMSO) δ 8.07 - 8.03 (m, 1H), 7.86 - 7.56 (m, 3H), 7.44 - 7.28 (m, 1H), 7.13 - 7.02 (m, 1H), 7.00 - 6.91 (m, 1H), 6.83 (s, 2H), 6.50 - 5.27 (m, 3H), 5.07 - 4.94 (m, 2H), 4.83 - 4.44 (m, 2H), 3.88 - 3.81 (m, 3H), 2.67 and 2.45 (s, 3H).

**Example 40A: (*S*)-4-amino-7-chloro-*N*-methyl-*N*-(6-((1-methyl-1*H*-pyrazol-4-yl)ethynyl)-2,3-dih ydrobenzofuran-3-yl)-1,3-dihydrofuro[3,4-*c*]quinoline-8-carboxamide (Compound 40A)**

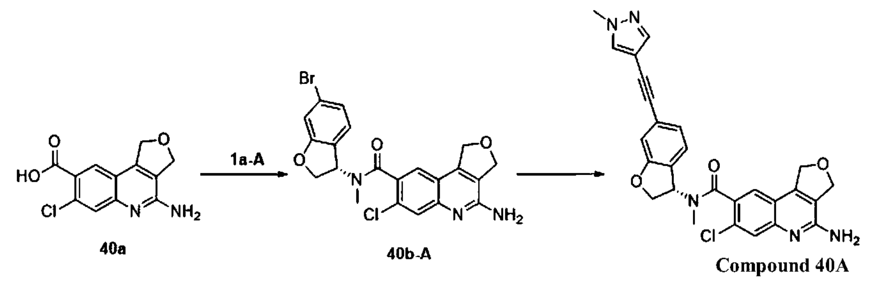


40a 40b-A Compound 40A

For the synthesis process of preparing compound **40A** from compound **1a-A**, reference was made to the synthesis process of preparing compound **40** from compound **1a**.

Compound **40A:** ESI-MS (m/z): 499.95 $[M+H]^+$. $^1$H NMR (400 MHz, DMSO) δ 8.08 - 8.03 (m, 1H), 7.85 - 7.56 (m, 3H), 7.44 - 7.26 (m, 1H), 7.12 - 7.02 (m, 1H), 7.00 - 6.91 (m, 1H), 6.83 (s, 2H), 6.52 - 5.28 (m, 3H), 5.07 - 4.94 (m, 2H), 4.84 - 4.45 (m, 2H), 3.85 - 3.84 (s, 3H), 2.67 and 2.45 (s, 3H).

**Example 41: 4-amino-*N*-(6-((1-(difluoromethyl)-1*H*-pyrazol-4-yl)ethynyl)-2,3-dihydrobenzofu ran-3-yl)-7-fluoro-*N*,1-dimethyl-1*H*-pyrazolo[4,3-*c*]quinoline-8-carboxamide (Compound 41)**

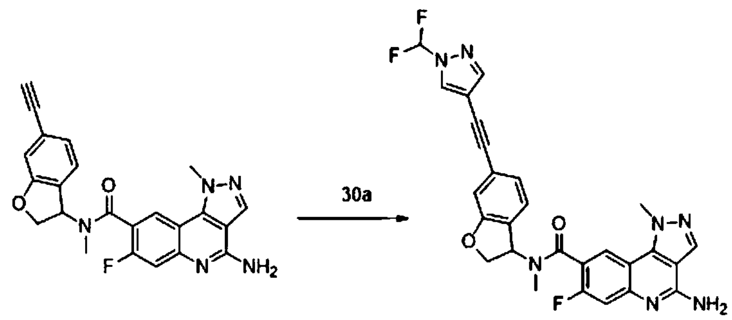


12b

Compound 41

Referring to the preparation process of compound **12,** compound 1-cyclopropyl-4-iodo-1*H*-pyrazole was replaced with compound **30a** (24.4 mg, 0.10 mmol), and the mixture was reacted in a microwave reactor at 80 °C for 3 h to give compound **41** (35 mg). ESI-MS (m/z): 531.98 $[M+H]^+$. $^1H$ NMR (400 MHz, DMSO-d6) δ 8.66 (s, 1H), 8.40 - 8.22 (m, 2H), 8.07 (s, 1H), 7.86 (t, *J* = 58.8 Hz, 1H), 7.56 - 7.27 (m, 4H), 7.21 - 7.10 (m, 1H), 7.08 - 7.00 (m, 1H), 6.50 - 6.42 and 5.63 - 5.56 (m, 1H), 4.91 - 4.58 (m, 2H), 4.43 - 4.41 (m, 3H), 2.71 - 2.60 (m, 3H).

**Example 42: 4-amino-*N*-(6-((4-(difluoromethyl)pyridin-3-yl)ethynyl)-2,3-dihydrobenzofuran-3 -yl)-7-fluoro-*N*,1-dimethyl-1*H*-pyrazolo[4,3-*c*]quinoline-8-carboxamide (Compound 42)**

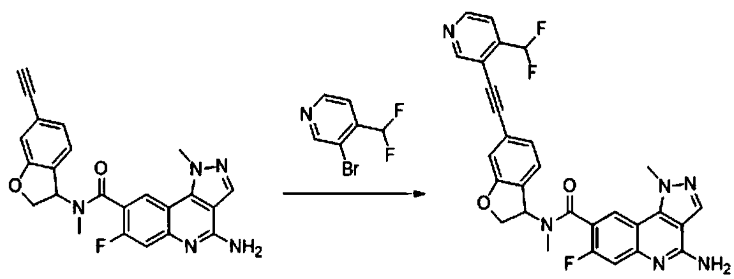

12b

Compound 42

Referring to the preparation process of compound **12,** compound 1-cyclopropyl-4-iodo-1*H*-pyrazole was replaced with compound 3-bromo-4-(difluoromethyl)pyridine (50.1 mg, 0.24 mmol), and the mixture was reacted in a microwave reactor at 100 °C for 4 h to give compound **42** (8 mg). ESI-MS (m/z): 543.2 $[M+H]^+$. $^1H$ NMR (400 MHz, DMSO) δ 8.90 (s, 1H), 8.76 (d, *J* = 5.2 Hz, 1H), 8.41 - 8.21 (m, 2H), 7.69 (d, *J* = 5.2 Hz, 1H), 7.48 - 7.12 (m,7H), 6.53 - 6.41 and 5.68 - 5.55 (m, 1H), 4.90 - 4.60 (m, 2H), 4.42 - 4.39 (m, 3H), 2.71 - 2.59 m, 3H).

**Example 43: 4-amino-7-chloro-*N*-(6-((1-cyclopropyl-1*H*-pyrazol-4-yl)ethynyl)-2,3-dihydroben zofuran-3-yl)-*N*-methyl-1,3-dihydrofuro[3,4-*c*]quinoline-8-carboxamide (Compound 43)**

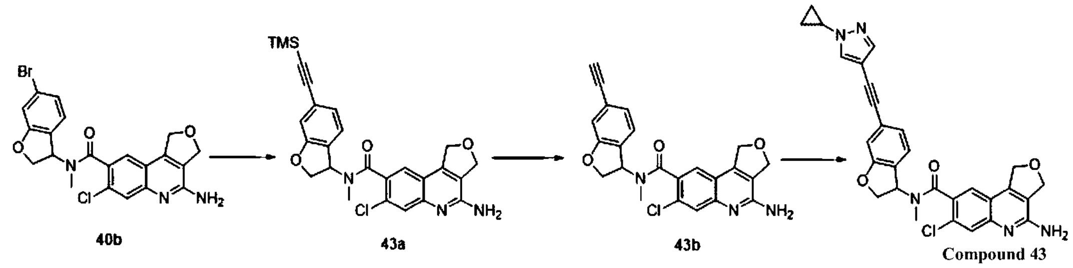

40b

43a

43b

Compound 43

### Preparation of compound 43a

To a solution of **40b** (298 mg, 0.63 mmol) in *N*-methylpyrrolidone (3 mL) were added cuprous iodide (24.1 mg, 0.13 mmol), tetrakis(triphenylphosphine)palladium (73.0 mg, 0.06 mmol), *N,N*-diisopropylethylamine (314.04 μL, 1.90 mmol), and trimethylsilylacetylene (446.48 μL, 3.16 mmol). After completion of the addition, the mixture was reacted in a microwave reactor at 80 °C for 4 h under nitrogen atmosphere. The reaction solution was filtered through celite. Water (50 mL) was added, and the reaction solution was extracted with ethyl acetate (25 mL × 2). The organic phases were combined, washed with saturated brine, dried over anhydrous sodium sulfate, and filtered. The filtrate was concentrated, and the residue was subjected to normal phase column chromatography (dichloromethane/methanol = 10:1) to give compound **43a** (300 mg). ESI-MS (m/z): 492.0 $[M+H]^+$.

### Preparation of compound 43b

Tetrabutylammonium fluoride (1.5 mL, 1.52 mmol) was added to a solution of compound **43a** (300 mg, 0.61 mmol) in tetrahydrofuran (3 mL), and the mixture was allowed to react at 25 °C for 1 h. Water (15 mL) was added, and the reaction solution was extracted with ethyl acetate (25 mL × 2). The organic phase was dried over anhydrous sodium sulfate and filtered. The filtrate was concentrated, and the residue was separated and purified by a preparative plate (dichloromethane/methanol = 10:1) to give a crude product. The crude product was then subjected to reversed phase column chromatography (acetonitrile/0.05% ammonium bicarbonate solution = 0:100% - 40%:60%) to give compound **43b** (210 mg). ESI-MS (m/z): 419.94 $[M+H]^+$.

### Preparation of compound 43

To a solution of compound **43b** (100 mg, 0.24 mmol) in *N*-methylpyrrolidone (2 mL) were added cuprous iodide (9.1 mg, 0.05 mmol), tetrakis(triphenylphosphine)palladium (27.5 mg, 0.02 mmol), triethylamine (99.05 μL, 0.71 mmol), and 1-cyclopropyl-4-iodopyrazole (112 mg, 0.48 mmol). After completion of the addition, the mixture was allowed to react at 25 °C for 16 h under nitrogen atmosphere. The reaction solution was filtered through celite. Water (50 mL) was added, and the reaction solution was extracted with ethyl acetate (25 mL × 2). The organic phases were combined, washed with saturated brine, dried over anhydrous sodium sulfate, and filtered. The filtrate was concentrated, and the residue was subjected to normal phase column chromatography (dichloromethane/methanol = 20:1) to give a crude compound. The crude compound was then subjected to reversed phase column chromatography (acetonitrile/0.05% ammonium bicarbonate solution = 0:100% - 40%:60%) to give compound **43** (36 mg). ESI-MS (m/z): 526.1 $[M+H]^+$. $^1H$ NMR (400 MHz, DMSO) δ 8.18 - 8.13 (m, 1H), 7.86 - 7.56 (m, 3H), 7.44 - 7.27 (m, 1H), 7.12 - 7.01 (m, 1H), 6.99 - 6.91 (m, 1H), 6.83 (s, 2H), 6.51 - 5.33 (m, 3H), 5.03 - 4.96 (m, 2H), 4.84 - 4.45 (m, 2H), 3.79 - 3.71 (m, 1H), 2.67 and 2.45 (s, 3H), 1.10 - 0.93 (m, 4H).

### Example 43A: (*S*)-4-amino-7-chloro-*N*-(6-((1-cyclopropyl-1*H*-pyrazol-4-yl)ethynyl)-2,3-dihydro benzofuran-3-yl)-*N*-methyl-1,3-dihydrofuro[3,4-*c*]quinoline-8-carboxamide (Compound 43A)

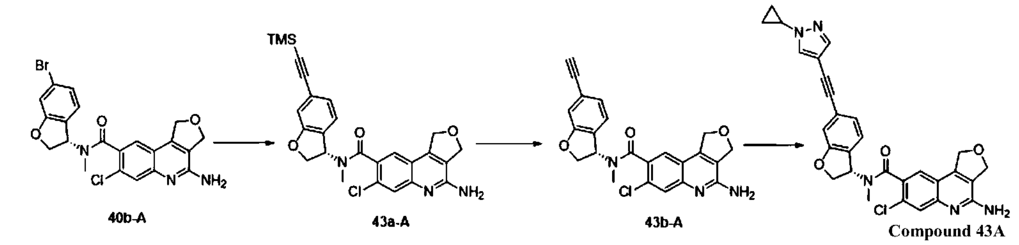
40b-A
43a-A
43b-A
Compound 43A

For the synthesis process of preparing compound **43A** from compound **40b-A,** reference was made to the synthesis process of preparing compound **43** from compound **40b.**

Compound **43A:** ESI-MS (m/z): 526.1 $[M+H]^+$. $^1H$ NMR (400 MHz, DMSO) δ 8.16 - 8.13 (m, 1H), 7.84 - 7.58 (m, 3H), 7.42 - 7.29 (m, 1H), 7.10 - 7.01 (m, 1H), 6.99 - 6.91 (m, 1H), 6.83 (s, 2H), 6.49 - 5.32 (m, 3H), 5.01 - 4.98 (m, 2H), 4.84 - 4.45 (m, 2H), 3.79 - 3.71 (m, 1H), 2.67 and 2.45 (s, 3H), 1.09 - 0.96 (m, 4H). $^1H$ NMR (400 MHz, DMSO-$d_6$, 90 °C) δ 8.05 (s, 1H), 7.62 - 7.53 (m, 3H), 7.34 (d, *J* = 2.8 Hz, 1H), 7.05 (d, *J* = 2.8 Hz, 1H), 6.90 (s, 1H), 6.53 (s, 2H), 6.44 - 5.32 (m, 3H), 5.01 (s, 2H), 4.77 - 4.58 (m, 2H), 3.75 - 3.70 (m, 1H), 2.68 - 2.49 (m, 3H), 1.06 - 0.94 (m, 4H). $[\alpha]^D_{25}$ 179.6 (c 1 mg/mL, DMF).

**Example 44: 4-amino-7-fluoro-*N*,1-dimethyl-*N*-(6-((4-methylpyridin-3-yl)ethynyl)-2,3-dihydro benzofuran-3-yl)-1*H*-pyrazolo[4,3-*c*]quinoline-8-carboxamide (Compound 44)**

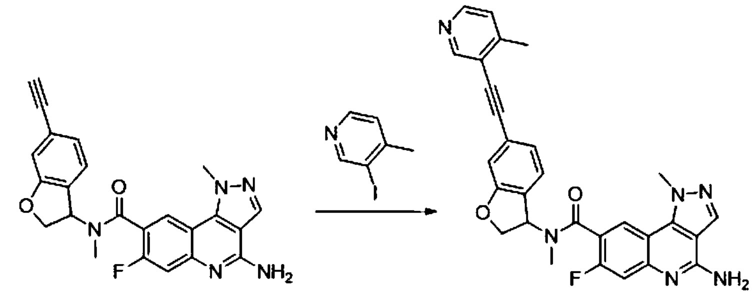
12b
Compound 44

Referring to the preparation process of compound **12,** compound 1-cyclopropyl-4-iodo-1*H*-pyrazole was replaced with compound 3-iodo-4-methylpyridine (22.0 mg, 0.1 mmol), and the mixture was reacted under an oil bath at 40 °C for 3 h to give compound **44** (15 mg). ESI-MS (m/z): 506.95 $[M+H]^+$. $^1H$ NMR (400 MHz, DMSO) δ 8.66 (s, 1H), 8.49 - 8.41 (m, 1H), 8.36 - 8.24 (m, 2H), 7.56 - 7.32 (m, 5H), 7.29 - 7.09 (m, 2H), 6.52 - 5.56 (m, 1H), 4.92 - 4.65 (m, 2H), 4.48 - 4.33 (m, 3H), 2.77 - 2.56 (m, 3H), 2.48 (s, 3H).

**Example 45: 4-amino-7-fluoro-*N*,1-dimethyl-*N*-(6-((2-methylpyridin-3-yl)ethynyl)-2,3-dihydro benzofuran-3-yl)-1*H*-pyrazolo[4,3-*c*]quinoline-8-carboxamide (Compound 45)**

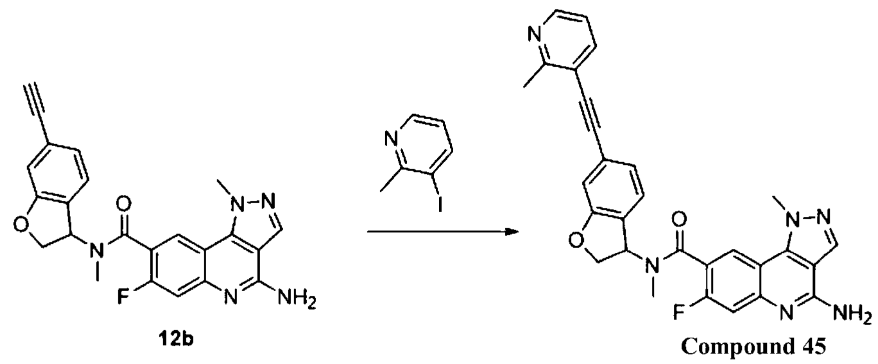
12b
Compound 45

Referring to the preparation process of compound **12,** compound 1-cyclopropyl-4-iodo-1*H*-pyrazole was

replaced with compound 3-iodo-2-methylpyridine (43.8 mg, 0.2 mmol), and the mixture was reacted at 40 C for 3 h to give compound **45** (20 mg). ESI-MS (m/z): 507.02 $[M+H]^+$. $^1H$ NMR (400 MHz, DMSO) δ 8.51 - 8.42 (m, 1H), 8.35 - 8.22 (m, 2H), 7.96 - 7.85 (m, 1H), 7.54 - 7.05 (m, 7H), 6.53 - 5.55 (m, 1H), 4.91 - 4.62 (m, 2H), 4.48 - 4.34 (m, 3H), 2.74 - 2.62 (m, 6H).

**Example 45A: (*S*)-4-amino-7-fluoro-*N*,1-dimethyl-*N*-(6-((2-methylpyridin-3-yl)ethynyl)-2,3-dihy drobenzofuran-3-yl)-1*H*-pyrazolo[4,3-*c*]quinoline-8-carboxamide (Compound 45A)**

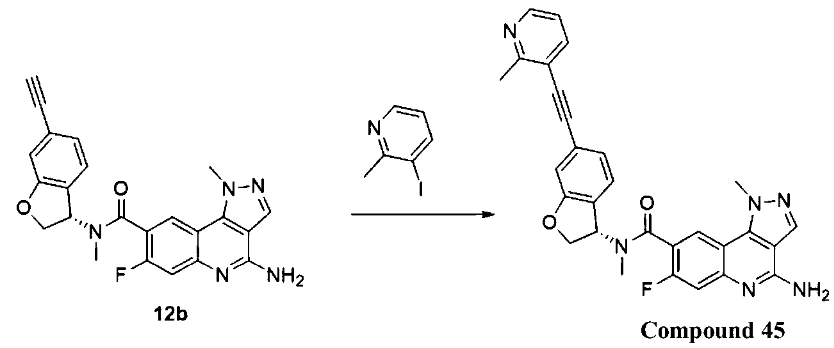

12b

Compound 45

For the synthesis process of preparing compound **45A** from compound **12b-A,** reference was made to the synthesis process of preparing compound **45** from compound **12b.**

Compound **45A:** ESI-MS (m/z): 506.99 $[M+H]^+$. $^1H$ NMR (400 MHz, DMSO) δ 8.51 - 8.45 (m, 1H), 8.39 - 8.20 (m, 2H), 7.94 - 7.86 (m, 1H), 7.54 - 7.07 (m, 7H), 6.54 - 6.44 and 5.67 - 5.57 (m, 1H), 4.92 - 4.79 and 4.74 - 4.61 (m, 2H), 4.43 (s, 3H), 2.75 - 2.60 (m, 6H).

**Example 46: 4-amino-7-fluoro-*N*,1-dimethyl-*N*-(6-((7-methyl-5,6,7,8-tetrahydroimidazo[1,2-*a*] pyrazin-3-yl)ethynyl)-2,3-dihydrobenzofuran-3-yl)-1*H*-pyrazolo[4,3-*c*]quinoline-8-carboxamide (Compound 46)**

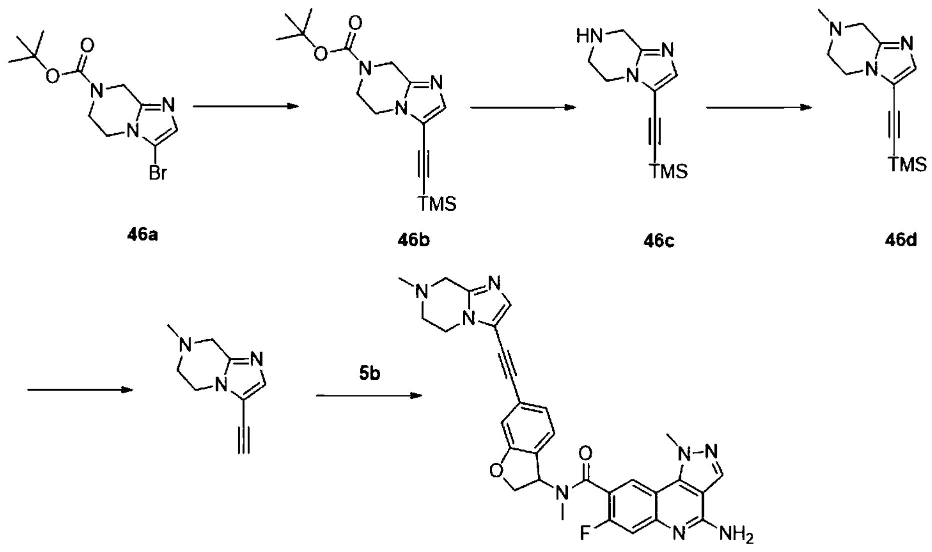


46a 46b 46c 46d

46e

Compound 46

**Preparation of compound 46b**

To a solution of **46a** (3000 mg, 9.93 mmol) in *N,N*-dimethylacetamide (5 mL) were added methanesulfonato(2-dicyclohexylphosphino-2',4',6'-tri-isopropyl-1,1'-biphenyl)(2'-ami no-1,1'-biphenyl-2-yl)palladium(II) (565.2 mg, 0.66 mmol), triethylamine (4.13 mL, 29.78 mmol), and trimethylsilylyne (4.21 mL, 29.78 mmol). The reaction solution was purged three times with nitrogen and reacted in a microwave reactor at 70 C for 3 h. The reaction

solution was filtered through celite. Water (100 mL) was added, and the reaction solution was extracted with ethyl acetate (100 mL × 2). The organic phases were combined, washed with saturated brine, dried over anhydrous sodium sulfate, and filtered. The filtrate was concentrated, and the residue was purified by normal phase column chromatography (dichloromethane/methanol = 20:1) to give compound **46b** (2700 mg). ESI-MS (m/z): 320.11 $[M+H]^+$.

**Preparation of compound 46c**

To a solution of compound **46b** (2700 mg, 8.45 mmol) in dichloromethane (20 mL) was added trifluoroacetic acid (6.47 mL, 84.50 mmol). After completion of the addition, the mixture was allowed to react at 25 °C for 16 h. After the reaction was completed, the reaction solution was concentrated to give a crude product **46c** (1800 mg), which was directly used in the next step. ESI-MS (m/z): 220.11 $[M+H]^+$.

**Preparation of compound 46d**

To a solution of **46c** (1800 mg, 8.20 mmol) in formic acid (5 mL) was added a formaldehyde solution (2400.0 mg, 29.60 mmol). After completion of the addition, the mixture was allowed to react at 100 °C for 1 h. Ammonium bicarbonate was added to quench the reaction. Water (50 mL) was added, and the reaction solution was extracted with ethyl acetate (50 mL × 2). The organic phases were combined, washed with saturated brine, dried over anhydrous sodium sulfate, and filtered. The filtrate was concentrated, and the residue was purified by normal phase column chromatography (dichloromethane/methanol = 20:1) to give compound **46d** (1000 mg). ESI-MS (m/z): 234.13 $[M+H]^+$.

**Preparation of compound 46e**

Cesium fluoride (2603.3 mg, 17.14 mmol) was added to a solution of **46d** (1000 mg, 4.28 mmol) in *N,N*-dimethylformamide (5 mL). After completion of the addition, the mixture was reacted at 25 °C for 30 min. Water (50 mL) was added, and the reaction solution was extracted with ethyl acetate (50 mL × 2). The organic phases were combined, washed with saturated brine, dried over anhydrous sodium sulfate, and filtered. The filtrate was concentrated, and the residue was purified by normal phase column chromatography (dichloromethane/methanol = 20:1) to give compound **46e** (450 mg). ESI-MS (m/z): 162.12 $[M+H]^+$.

**Preparation of compound 46**

Referring to the preparation process of compound **5,** compound 4-ethynyl-1-methylpyrazole was replaced with compound **46e** (102.8 mg, 0.64 mmol), and the mixture was reacted in a microwave reactor at 100 °C for 3 h to give compound **46** (7.3 mg). ESI-MS (m/z): 550.8 $[M+H]^+$. $^1H$ NMR (400 MHz, DMSO) δ 8.37 - 8.20 (m, 2H), 7.47 - 7.04 (m, 7H), 6.51 - 5.53 (m, 1H), 4.87 - 4.56 (m, 2H), 4.45 - 4.33 (m, 3H), 3.99 (s, 2H), 3.56 (s, 2H), 2.86 - 2.79 (m, 2H), 2.69 - 2.58 m, 3H), 2.39 (s, 3H).

**Example 46A: (*S*)-4-amino-7-fluoro-*N*,1-dimethyl-*N*-(6-((7-methyl-5,6,7,8-tetrahydroimidazo[1, 2-*a*]pyrazin-3-yl) ethynyl)-2,3-dihydrobenzofuran-3-yl)-1*H*-pyrazolo[4,3-*c*]quinoli ne-8-carboxamide (Compound 46A)**

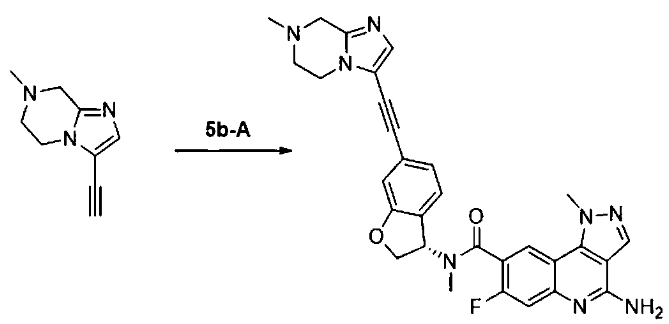


46e

Compound 46A

For the synthesis process of preparing compound **46A** from compound 46e and compound **5b-A,** reference was made to the synthesis process of preparing compound **46** from compound 46e and compound **5b.**

Compound **46A:** ESI-MS (m/z): 551.0 $[M+H]^+$. $^1H$ NMR (400 MHz, DMSO) δ 8.36 - 8.19 (m, 2H), 7.46 - 7.28 (m,

5H), 7.19 - 6.99 (m, 2H), 6.49 - 5.54 (m, 1H), 4.85 - 4.61 (m, 2H), 4.45 - 4.37 (m, 3H), 4.02 - 3.97 (m, 2H), 3.54 (s, 2H), 2.86 - 2.81 (m, 2H), 2.70 and 2.58 (s, 3H), 2.40 (s, 3H).

**Example 47A: (*S*)-4-amino-7-fluoro-*N*,1-dimethyl-*N*-(6-((5-methylpyridin-3-yl)ethynyl)-2,3-dihy drobenzofuran-3-yl)-1*H*-pyrazolo[4,3-*c*]quinoline-8-carboxamide (Compound 47A)**

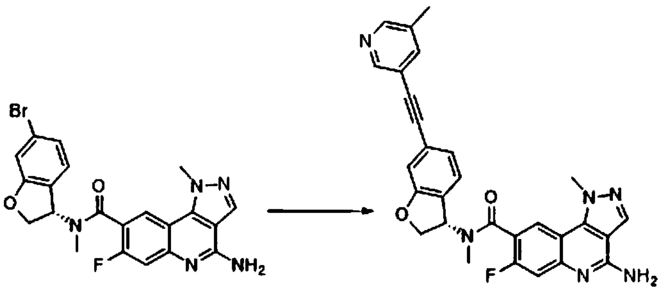

5b-A

Compound 47A

Referring to the preparation process of compound **5A,** compound 4-ethynyl-1-methylpyrazole was replaced with compound 3-ethynyl-5-methylpyridine (23.4 mg, 0.2 mmol), and the mixture was reacted in a microwave reactor at 100 °C for 4 h to give compound **47A** (25 mg). ESI-MS (m/z): 507.27 [M+H]$^+$. $^1$H NMR (400 MHz, DMSO) δ 8.73 - 8.23 (m, 4H), 7.83 (s, 1H), 7.60 - 7.03 (m, 6H), 6.54 - 5.57 (m, 1H), 4.91 - 4.60 (m, 2H), 4.51 - 4.35 (m, 3H), 2.77 - 2.58 (m, 3H), 2.34 (s, 3H).

**Example 48A: (*S*)-4-amino-7-fluoro-*N*,1-dimethyl-*N*-(6-(pyridin-3-ylethynyl)-2,3-dihydrobenzof uran-3-yl)-1*H*-pyrazolo[4,3-*c*]quinoline-8-carboxamide (Compound 48A)**

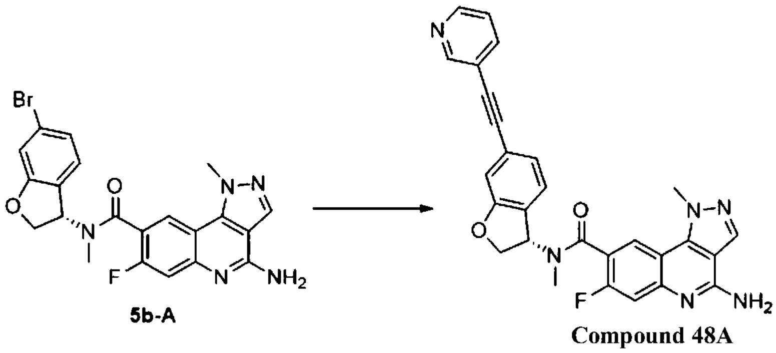

5b-A

Compound 48A

Referring to the preparation process of compound **5A,** compound 4-ethynyl-1-methylpyrazole was replaced with compound 3-ethynylpyridine (52.6 mg, 0.51 mmol), and the mixture was reacted in a microwave reactor at 100 C for 4 h to give compound **48A** (40 mg). ESI-MS (m/z): 493.1 [M+H]$^+$. $^1$H NMR (400 MHz, DMSO) δ 8.75 (s, 1H), 8.60 - 8.59 (m, 1H), 8.39 - 8.22 (m, 2H), 8.00 - 7.94 (m, 1H), 7.52 - 7.16 (m, 6H), 7.15 - 7.05 (m, 1H), 6.54 - 5.53 (m, 1H), 4.88 - 4.61 (m, 2H), 4.41 - 4.39 (m, 3H), 2.70 - 2.59 (m, 3H).

**Example 49A: (*S*)-4-amino-*N*-(6-((1,3-dimethyl-1*H*-pyrazol-4-yl)ethynyl)-2,3-dihydrobenzofura n-3-yl)-N,1-dimethyl-1*H*-pyrazolo[4,3-*c*]quinoline-8-carboxamide (Compound 49A)**

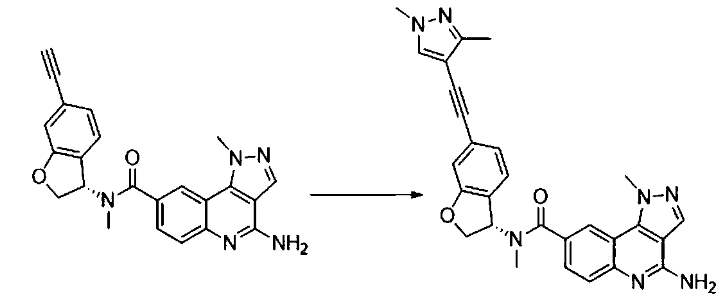

6b-A Compound 49A

Referring to the preparation process of compound **19,** compound 4-iodo-1-isopropyl-1*H*-pyrazole was replaced with compound 4-iodo-1,3-dimethylpyrazole (22.2 mg, 0.1 mmol), and compound **6b** was replaced with compound **6b-A** (39.7 mg, 0.12 mmol). The mixture was reacted at 25 °C for 3 h to give compound **49A** (23 mg). ESI-MS (m/z): 492.19 [M+H]+. 1H NMR (400 MHz, DMSO-d6) δ 8.34 - 8.33 (m, 1H), 8.28 (s, 1H), 7.95 (s, 1H), 7.67 - 7.62 (m, 2H), 7.45 (d, *J* = 7.6 Hz, 1H), 7.19 (s, 2H), 7.10 (d, *J* = 7.6 Hz, 1H), 6.99 (s, 1H), 6.40 - 5.56 (m, 1H), 4.88 - 4.60 (m, 2H), 4.44 (s, 3H), 3.78 (s, 3H), 2.70 (s, 3H), 2.24 (s, 3H).

**Example 50A: (*S*)-4-amino-7-chloro-*N*-(6-((1,3-dimethyl-1*H*-pyrazol-4-yl)ethynyl)-2,3-dihydrob enzofuran-3-yl)-*N*-methyl-1,3-dihydrofuro[3,4-*c*]quinoline-8-carboxamide (Compound 50A)**

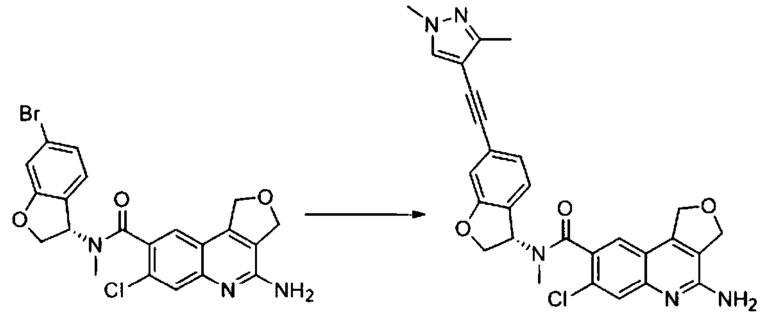

40b-A Compound 50A

Referring to the preparation process of compound **40A,** compound 4-ethynyl-1-methylpyrazole was replaced with compound 4-ethynyl-1,3-dimethylpyrazole (75.7 mg, 0.63 mmol), and the mixture was reacted in a microwave reactor at 100 °C for 3 h under nitrogen atmosphere to give compound **50A** (50 mg). ESI-MS (m/z): 513.96 $[M+H]^+$. $^1$H NMR (400 MHz, DMSO) δ 7.95 - 7.88 (m, 1H), 7.86 - 7.55 (m, 2H), 7.44 - 7.26 (m, 1H), 7.13 - 7.02 (m, 1H), 7.01 - 6.91 (m, 1H), 6.83 (s, 2H), 6.52 - 5.28 (m, 3H), 5.06 - 4.92 (m, 2H), 4.84 - 4.44 (m, 2H), 3.76 - 3.75 (m, 3H), 2.67 and 2.45 (s, 3H), 2.22 - 2.21 (s, 3H).

**Example 51A: (*S*)-4-amino-7-chloro-*N*,1-dimethyl-*N*-(6-((7-methyl-5,6,7,8-tetrahydroimidazo[1, 2-*a*]pyrazin-3-yl) ethynyl)-2,3-dihydrobenzofuran-3-yl)-1*H*-pyrazolo[4,3-*c*]quinoli ne-8-carboxamide (Compound 51A)**

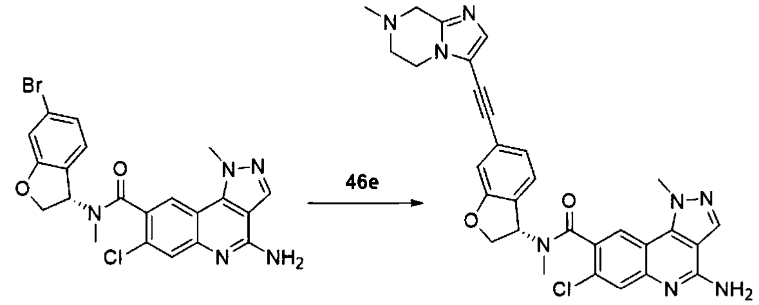


8b-A

Compound 51A

Referring to the preparation process of compound **46A,** compound **5b-A** was replaced with compound **8b-A** (100 mg, 0.21 mmol), and the mixture was reacted in a microwave reactor at 100 °C for 3 h under nitrogen atmosphere to give compound **51A** (41 mg). ESI-MS (m/z): 566.97 $[M+H]^+$.$^1$H NMR (400 MHz, DMSO) δ 8.43 - 8.19 (m, 2H), 7.72 - 7.62 (m, 1H), 7.58 - 7.29 (m, 4H), 7.22 - 7.01 (m, 2H), 6.60 - 5.40 (m, 1H), 4.90 - 4.56 (m, 2H), 4.46 - 4.41 (m, 3H), 4.05 - 3.97 (m, 2H), 3.58 - 3.57 (m, 2H), 2.88 - 2.81 (m, 2H), 2.73 - 2.51 (m, 3H), 2.42 - 2.41 (m, 3H).

**Example 52A: (*S*)-4-amino-7-chloro-*N*-(6-((1,5-dimethyl-1*H*-pyrazol-4-yl)ethynyl)-2,3-dihydrob enzofuran-3-yl)-*N*-methyl-1,3-dihydrofuro[3,4-*c*]quinoline-8-carboxamide (Compound 52A)**

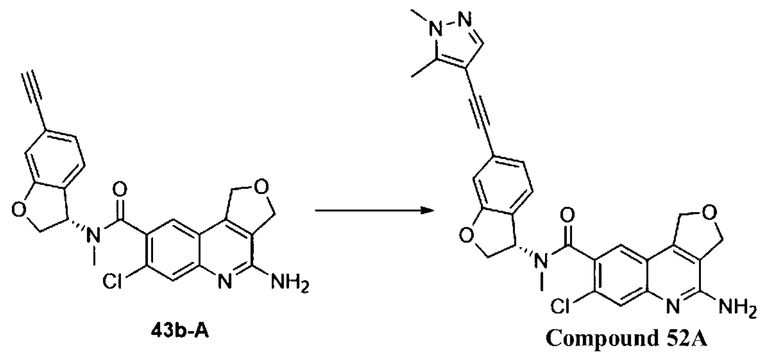

43b-A

Compound 52A

Referring to the preparation process of compound **43A,** compound 1-cyclopropyl-4-iodopyrazole was replaced with compound 4-iodo-1,5-dimethylpyrazole (66.2 mg, 0.30 mmol), and the mixture was reacted at room temperature for 16 h under nitrogen atmosphere to give compound **52A** (51 mg). ESI-MS (m/z): 513.96 $[M+H]^+$. $^1$H NMR (400 MHz, DMSO) δ 7.85 - 7.55 (m, 3H), 7.45 - 7.25 (m, 1H), 7.14 - 7.03 (m, 1H), 7.02 - 6.93 (m, 1H), 6.83 (s, 2H), 6.50 - 5.27 (m, 3H), 5.08 - 4.95 (m, 2H), 4.88 - 4.40 (m, 2H), 3.75 (s, 3H), 2.67 and 2.45 (s, 2H), 2.35 - 2.34 (s, 3H).

**Example 53A: (*S*)-4-amino-7-chloro-*N*,1-dimethyl-*N*-(6-((1-methyl-1*H*-pyrazol-5-yl)ethynyl)-2,3 -dihydrobenzofuran-3-yl)-1H-pyrazolo[4,3-c]quinoline-8-carboxamide (Compound 53A)**

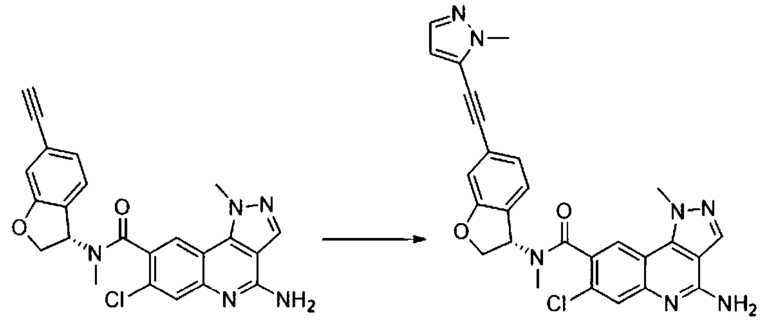

8d-A

Compound 53A

Referring to the preparation process of compound **8A,** compound 1-cyclopropyl-4-iodopyrazole was replaced with compound 5-iodo-1-methylpyrazole (105.7 mg, 0.51 mmol), and the mixture was reacted at room temperature for 16 h under nitrogen atmosphere to give compound **53A** (55 mg). ESI-MS (m/z): 511.99 $[M+H]^+$. $^1H$ NMR (400 MHz, DMSO) δ 8.44 - 8.17 (m, 2H), 7.70 - 7.59 (m, 1H), 7.58 - 7.33 (m, 2H), 7.27 - 7.08 (m, 4H), 6.65 - 6.58 (m, 1H), 6.55 - 5.41 (m, 1H), 4.88 - 4.55 (m, 2H), 4.45 - 4.39 (s, 3H), 3.94 - 3.92 (m, 3H), 2.72 - 2.47 (m, 3H).

**Example 54A: (*S*)-4-amino-7-fluoro-*N*-(6-((2-fluoropyridin-3-yl)ethynyl)-2,3-dihydrobenzofuran -3-yl)-N,1-dimethyl-1H-pyrazolo[4,3-c]quinoline-8-carboxamide (Compound 54A)**

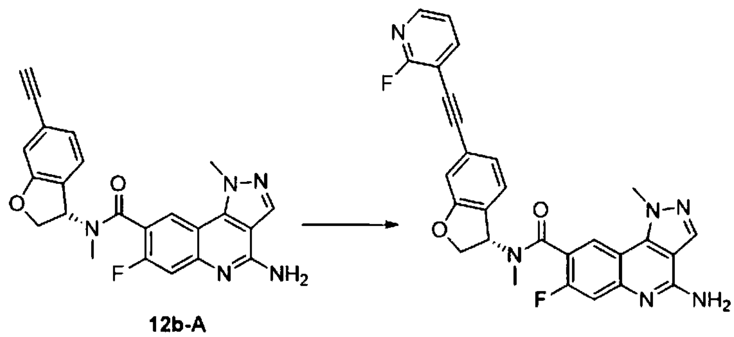

12b-A

Compound 54A

Referring to the preparation process of compound **12A,** compound 1-cyclopropyl-4-iodo-1*H*-pyrazole was replaced with compound 2-fluoro-3-iodopyridine (22.3 mg, 0.1 mmol), and the mixture was reacted at 40 °C for 3 h to give compound **54A** (15 mg). ESI-MS (m/z): 511.07 $[M+H]^+$. $^1H$ NMR (400 MHz, DMSO) δ 8.44 - 8.17 (m, 4H), 7.57 - 7.43 (m, 2H), 7.43 - 7.19 (m, 4H), 7.18 - 7.07 (m, 1H), 6.56 - 5.57 (m, 1H), 4.92 - 4.63 (m, 2H), 4.49 - 4.33 (m, 3H), 2.78 - 2.57 (m, 3H).

**Example 55A: (*S*)-4-amino-7-chloro-*N*,1-dimethyl-*N*-(6-((6-methylpyridin-3-yl)ethynyl)-2,3-dihy drobenzofuran-3-yl)-1*H*-pyrazolo[4,3-*c*]quinoline-8-carboxamide (Compound 55A)**

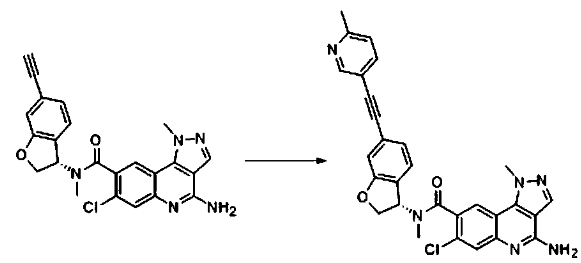

8d-A

Compound 55A

Referring to the preparation process of compound **8A,** compound 1-cyclopropyl-4-iodopyrazole was replaced with compound 5-iodo-2-methylpyridine (21.9 mg, 0.10 mmol), and the mixture was reacted at 25 °C for 3 h to give compound **55A** (15 mg). ESI-MS (m/z): 522.91 $[M+H]^+$. $^1H$ NMR (400 MHz, DMSO-d6) δ 8.69 - 8.59 (m, 1H), 8.44 - 8.20 (m, 2H), 7.90 - 7.84 (m, 1H), 7.72 - 7.63 (m, 1H), 7.59 - 7.05 (m, 6H), 6.57 - 5.44 (m, 1H), 4.91 - 4.57 (m, 2H), 4.48 - 4.42 (s, 3H), 3.34 (s, 3H), 2.76 - 2.54 (m, 3H).

**Example 56A: (*S*)-4-amino-7-chloro-*N*-(6-((2-fluoropyridin-3-yl)ethynyl)-2,3-dihydrobenzofura n-3-yl)-N,1-dimethyl-1H-pyrazolo[4,3-c]quinoline-8-carboxamide (Compound 56A)**

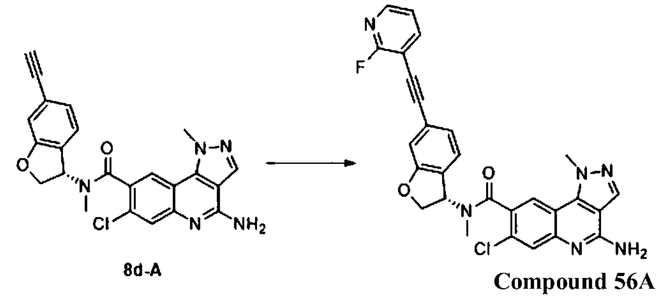

8d-A

Compound 56A

Referring to the preparation process of compound **8A,** compound 1-cyclopropyl-4-iodopyrazole was replaced with compound 2-fluoro-3-iodopyridine (44.6 mg, 0.20 mmol), and the mixture was reacted at 25 °C for 3 h to give compound **56A** (10 mg). ESI-MS (m/z): 526.73 $[M+H]^+$. $^1H$ NMR (400 MHz, DMSO-d6) δ 8.45 - 8.17 (m, 4H), 7.72 - 7.64 (m, 1H), 7.62 - 7.35 (m, 2H), 7.34 - 7.07 (m, 1H), 6.57 - 5.45 (m, 1H), 4.94 - 4.57 (m, 2H), 4.47 - 4.41 (s, 3H), 2.76 - 2.53 (m, 3H).

**Example 57A: (*S*)-4-amino-7-chloro-N-methyl-N-(6-((2-methylpyridin-3-yl)ethynyl)-2,3-dihydro benzofuran-3-yl)-1,3-dihydrofuro[3,4-c]quinoline-8-carboxamide (Compound 57A)**

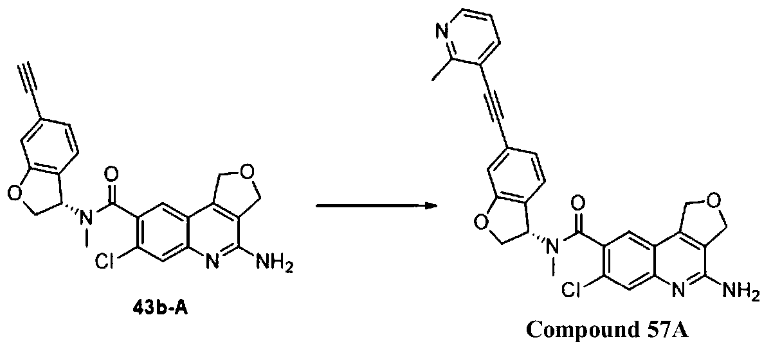

43b-A

Compound 57A

Referring to the preparation process of compound 43A, compound 1-cyclopropyl-4-iodopyrazole was replaced with compound 3-iodo-2-methylpyridine (31.3 mg, 0.14 mmol), and the mixture was reacted at room temperature for 3 h under nitrogen atmosphere to give compound 57A (13 mg). ESI-MS (m/z): 510.92 $[M+H]^+$. $^1H$ NMR (400 MHz, DMSO-d6) δ 8.50 - 8.46 (m, 1H), 7.93 - 7.87 (m, 1H), 7.70 - 7.57 (m, 2H), 7.52 - 7.17 (m, 3H), 7.17 - 7.07 (m, 1H), 6.86 (s, 2H), 6.55 - 5.32 (m, 3H), 5.05 - 4.98 (m, 2H), 4.89 - 4.49 (m, 2H), 2.71 - 2.68 (m, 3H), 2.66 - 2.67 (m, 3H).

**Example 58A: (S)-4-amino-7-chloro-N-methyl-N-(6-((6-methylpyridin-3-yl)ethynyl)-2,3-dihydro benzofuran-3-yl)-1,3-dihydrofuro[3,4-c]quinoline-8-carboxamide (Compound 58A)**

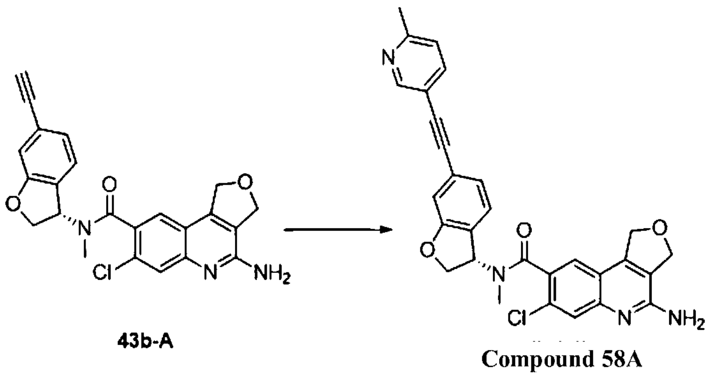

43b-A

Compound 58A

Referring to the preparation process of compound **43A,** compound 1-cyclopropyl-4-iodopyrazole was replaced

with compound 5-iodo-2-methylpyridine (30.7 mg, 0.14 mmol), and the mixture was reacted at room temperature for 3 h under nitrogen atmosphere to give compound **58A** (15 mg). ESI-MS (m/z): 510.93 [M+H]$^+$. $^1$H NMR (400 MHz, DMSO-d6) δ 8.66 - 8.61 (m, 1H), 7.89 - 7.58 (m, 3H), 7.52 - 7.32 (m, 2H), 7.25 - 7.15 (m, 1H), 7.13 - 7.06 (m, 1H), 6.86 (s, 2H), 6.53 - 5.30 (m, 3H), 5.05 - 4.98 (m, 2H), 4.89 - 4.48 (m, 2H), 2.72 - 2.47 (m, 6H).

**Example 59A: (*S*)-4-amino-7-fluoro-N,1-dimethyl-*N*-(6-((6-methylpyridin-3-yl)ethynyl)-2,3-dihy drobenzofuran-3-yl)-1*H*-pyrazolo[4,3-*c*]quinoline-8-carboxamide (Compound 59A)**

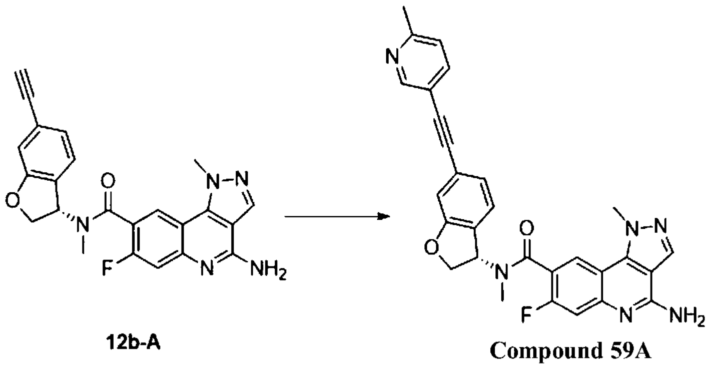

12b-A

Compound 59A

Referring to the preparation process of compound **12A,** compound 1-cyclopropyl-4-iodo-1*H*-pyrazole was replaced with compound 5-iodo-2-methylpyridine (36.9 mg, 0.17 mmol), and the mixture was reacted at 25 °C for 3 h to give compound **59A** (15 mg). ESI-MS (m/z): 507.21 [M+H]$^+$. $^1$H NMR (400 MHz, DMSO-d6) δ 8.64 (s, 1H), 8.29 - 8.24 (m, 2H), 7.89 - 7.84 (m, 1H), 7.69 - 7.07 (m, 6H), 6.52 - 5.57 (m, 1H), 4.92 - 4.64 (m, 2H), 4.43 - 4.41 (s, 3H), 3.33 - 3.24 (m, 3H), 2.72 - 2.61 (m, 3H).

**Example 60A: (5)-4-amino-7-fluoro-N,1-dimethyl-*N*-(6-((1-methyl-1*H*-indazol-6-yl)ethynyl)-2,3-dihydrobenzofuran-3-yl)-1H-pyrazolo[4,3-c]quinoline-8-carboxamide (Compound 60A)**

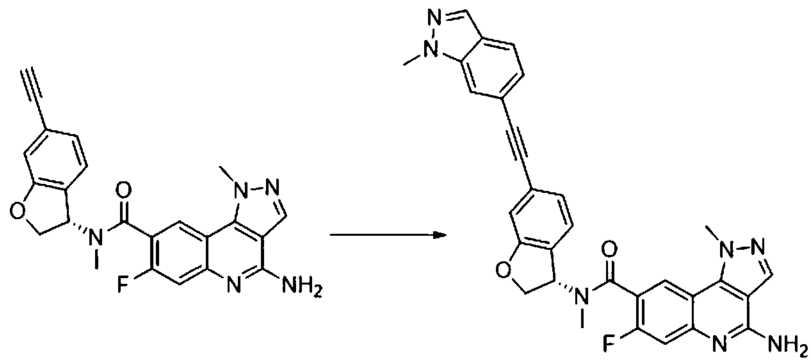

12b-A

Compound 60A

Referring to the preparation process of compound **12A,** compound 1-cyclopropyl-4-iodo-1H-pyrazole was replaced with compound 6-iodo-1-methylindazole (41.3 mg, 0.16 mmol), and the mixture was reacted at 25 °C for 3 h to give compound **60A** (8 mg). ESI-MS (m/z): 546.08 [M+H]$^+$. $^1$H NMR (400 MHz, DMSO-d6) δ 8.39 - 8.24 (m, 2H), 8.12 (s, 1H), 8.02 (s, 1H), 7.75 - 7.70 (m, 1H), 7.58 - 7.52 (m, 1H), 7.47 - 7.31 (m, 4H), 7.26 - 7.14 (m, 1H), 7.13 - 7.05 (m, 1H), 6.52 - 5.56 (m, 1H), 4.92 - 4.60 (m, 2H), 4.44 - 4.41 (m, 3H), 4.08 (s, 3H), 2.73 - 2.61 (m, 3H).

**Example 61A: (*S*)-4-amino-7-chloro-*N*-(6-((2-fluoropyridin-3-yl)ethynyl)-2,3-dihydrobenzofura n-3-yl)-*N*-methyl-1,3-dihydrofuro[3,4-*c*]quinoline-8-carboxamide (Compound 61A)**

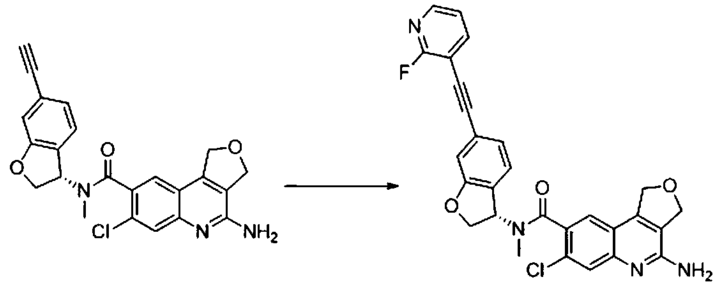

43b-A

Compound 61A

Referring to the preparation process of compound **43A,** compound 1-cyclopropyl-4-iodopyrazole was replaced with compound 2-fluoro-3-iodopyridine (31.2 mg, 0.14 mmol), and the mixture was reacted at room temperature for 3 h to give compound **61A** (20 mg). ESI-MS (m/z): 515.13 [M+H]$^+$. $^1$H NMR (400 MHz, DMSO-d6) δ 8.32 - 8.28 (m, 1H), 8.26 - 8.18 (m, 1H), 7.70 - 7.56 (m, 2H), 7.52 - 7.36 (m, 2H), 7.28 - 7.17 (m, 1H), 7.15 - 7.07 (m, 1H), 6.86 (s, 2H), 6.56 - 5.28 (m, 3H), 5.07 - 4.97 (m, 2H), 4.92 - 4.44 (m, 2H), 2.70 and 2.48 (s, 3H).

**Example 62A: (*S*)-4-amino-7-chloro-*N*-methyl-*N*-(6-((1-methyl-1*H*-indazol-6-yl)ethynyl)-2,3-dih ydrobenzofuran-3-yl)-1,3-dihydrofuro[3,4-*c*]quinoline-8-carboxamide (Compound 62A)**

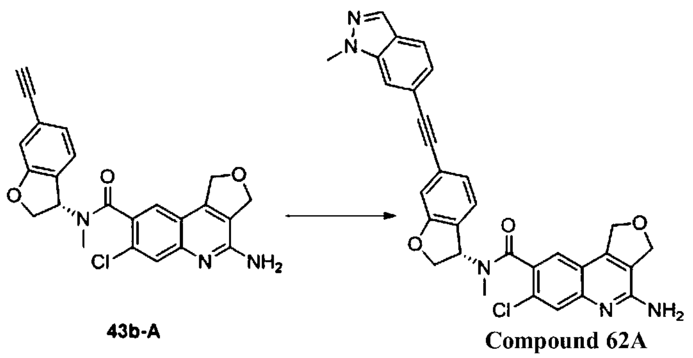

43b-A

Compound 62A

Referring to the preparation process of compound **43A,** compound 1-cyclopropyl-4-iodopyrazole was replaced with compound 6-iodo-1-methylindazole (36.1 mg, 0.14 mmol), and the mixture was reacted at room temperature for 3 h to give compound **62A** (13 mg). ESI-MS (m/z): 549.92 [M+H]$^+$. $^1$H NMR (400 MHz, DMSO-d6) δ 8.12 - 8.08 (m, 1H), 7.96 - 7.92 (m, 1H), 7.88 - 7.59 (m, 3H), 7.52 - 7.34 (m, 1H), 7.30 - 7.17 (m, 2H), 7.14 - 7.07 (m, 1H), 6.86 (s, 2H), 6.54 - 6.46 and 5.43 - 5.40 (m, 1H), 5.39 - 5.29 (m, 2H), 5.06 - 4.98 (m, 2H), 4.89 - 4.48 (m, 2H), 4.09 - 4.08 (m, 3H), 2.71 and 2.49 (s, 3H).

**Example 63A: (*S*)-4-amino-7-chloro-*N*,1-dimethyl-*N*-(6-((4-methylpyridin-3-yl)ethynyl)-2,3-dihy drobenzofuran-3-yl)-1*H*-pyrazolo[4,3-*c*]quinoline-8-carboxamide (Compound 63A)**

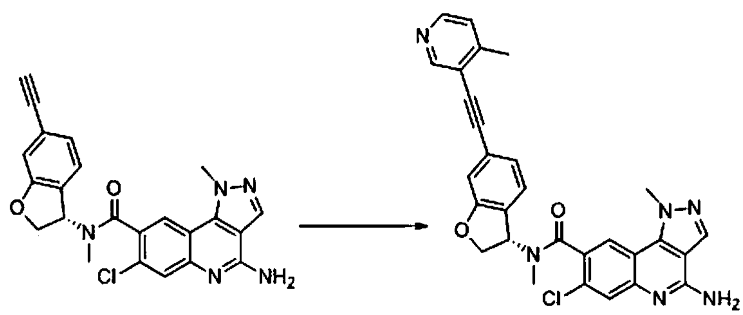

8d-A

Compound 63A

Referring to the preparation process of compound **8A,** compound 1-cyclopropyl-4-iodopyrazole was replaced with compound 3-iodo-4-methylpyridine (43.8 mg, 0.2 mmol), and the mixture was reacted at 40 °C for 3 h to give compound **63A** (15 mg). ESI-MS (m/z): 522.89 $[M+H]^+$. $^1H$ NMR (400 MHz, DMSO) δ 8.69 - 8.64 (m, 1H), 8.48 - 8.21 (m, 3H), 7.71 - 7.63 (m, 1H), 7.41 - 7.24 (m, 6H), 6.59 - 5.42 (m, 1H), 4.89 - 4.62 (m, 2H), 4.47 - 4.40 (m, 3H), 2.77 - 2.47 (m, 6H).

**Example 64A: (*S*)-4-amino-7-chloro-*N*,1-dimethyl-*N*-(6-((2-methylpyridin-3-yl)ethynyl)-2,3-dihy drobenzofuran-3-yl)-1*H*-pyrazolo[4,3-*c*]quinoline-8-carboxamide (Compound' 64A)**

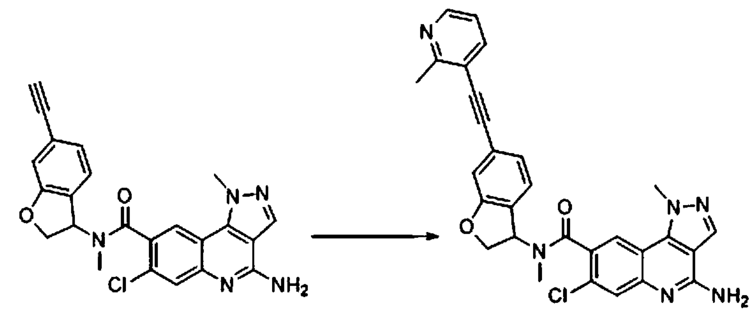

8d-A

Compound 64A

Referring to the preparation process of compound **8A,** compound 1-cyclopropyl-4-iodopyrazole was replaced with compound 3-iodo-2-methylpyridine (43.8 mg, 0.2 mmol), and the mixture was reacted at 40 °C for 3 h to give compound **64A** (15 mg). ESI-MS (m/z): 522.92 $[M+H]^+$. $^1H$ NMR (400 MHz, DMSO) δ 8.51 - 8.45 (m, 1H), 8.42 - 8.19 (m, 2H), 7.93 - 7.86 (m, 1H), 7.70 - 7.61 (m, 1H), 7.58 - 7.07 (m, 6H), 6.57 - 5.43 (m, 1H), 4.88 - 4.64 (m, 2H), 4.47 - 4.40 (m, 3H), 2.74 - 2.66 **(m, 6H).**

**Example 65A: (S)-4-amino-7-chloro-*N*,1-dimethyl-*N*-(6-((5-methylpyridin-3-yl)ethynyl)-2,3-dihy drobenzofuran-3-yl)-1*H*-pyrazolo[4,3-*c*]quinoline-8-carboxamide (Compound 65A)**

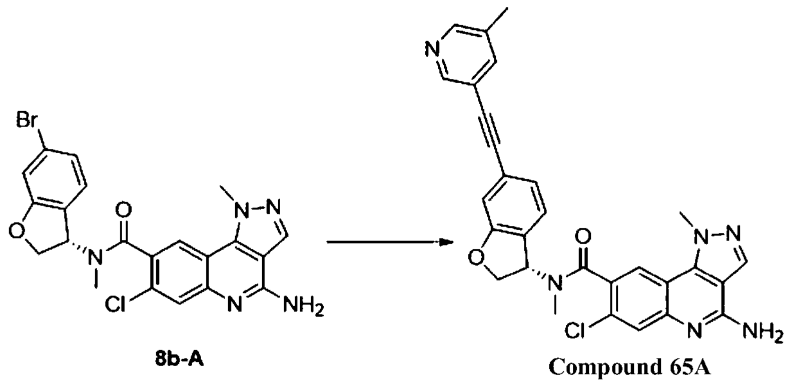

8b-A

Compound 65A

Referring to the preparation process of compound **51A,** compound **46e** was replaced with compound 3-ethynyl-5-methylpyridine (23.4 mg, 0.2 mmol), and the mixture was reacted in a microwave reactor at 100 °C for 4 h under nitrogen atmosphere to give compound 65A (12 mg). ESI-MS (m/z): 522.96 $[M+H]^+$. $^1H$ NMR (400 MHz, DMSO) δ 8.59 - 8.55 (m, 1H), 8.48 - 8.21 (m, 3H), 7.84 - 7.81 (m, 1H), 7.71 - 7.64 (m, 1H), 7.60 - 7.08 (m, 5H), 6.58 - 5.42 (m, 1H), 4.88 - 4.54 (m, 2H), 4.47 - 4.41 (m, 3H), 2.75 - 2.33 (m, 6H).

Example **66A: (*S*)-4-amino-7-fluoro-*N*,1-dimethyl-*N*-(6-((5,6,7,8-tetrahydroimidazo[1,2-*a*]pyridi n-3-yl)ethynyl)-2,3-dihydrobenzofuran-3-yl)-1H-pyrazolo[4,3-c]quinoline-8-carb oxamide (Compound 66A)**

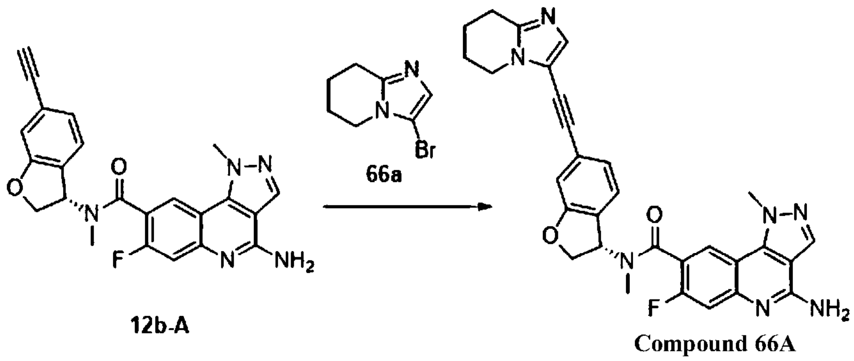


12b-A

Compound 66A

Referring to the preparation process of compound **12A,** compound 1-cyclopropyl-4-iodo-1*H*-pyrazole was replaced with compound **66a** (50.5 mg, 0.20 mmol), and the mixture was reacted in a microwave reactor at 100 °C for 3 h to give compound **66A** (18 mg). ESI-MS (m/z): 536.39 $[M+H]^+$. $^1H$ NMR (400 MHz, DMSO-d6) δ 8.41 - 8.22 (m, 2H), 7.47 - 7.26 (m, 5H), 7.22 - 7.13 (m, 1H), 7.10 - 7.03 (m, 1H), 6.51 - 5.56 (m, 1H), 4.90 - 4.67 (m, 2H), 4.43 - 4.41 (s, 3H), 4.01 - 3.95 (m, 2H), 2.82 - 2.75 (m, 2H), 2.72 - 2.60 (m, 3H), 2.01 - 1.94 (m, 2H), 1.91 - 1.83 (m, 2H).

**Example 67A: (*S*)-4-amino-7-chloro-*N*-(6-((1-(difluoromethyl)-1*H*-pyrazol-4-yl)ethynyl)-2,3-dih ydrobenzofuran-3-yl)-*N*,1-dimethyl-1*H*-pyrazolo[4,3-*c*]quinoline-8-carboxamide (Compound 67A)**

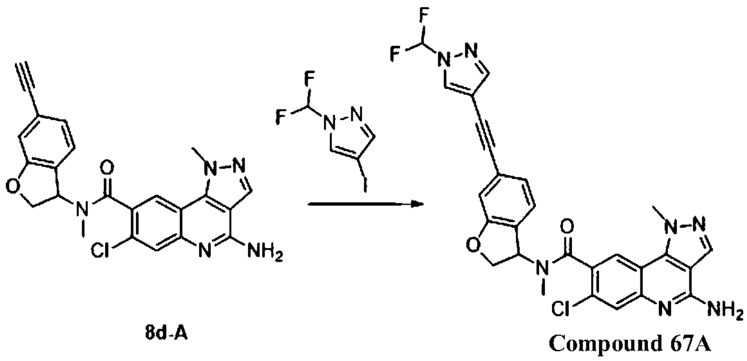

8d-A

Compound 67A

Referring to the preparation process of compound **8A,** compound 1-cyclopropyl-4-iodopyrazole was replaced with compound 1-(difluoromethyl)-4-iodopyrazole (46.4 mg, 0.19 mmol), and the mixture was reacted at room temperature of 25 °C for 3 h to give compound **67A** (8 mg). ESI-MS (m/z): 547.9 $[M+H]^+$. $^1H$ NMR (400 MHz, DMSO-d6) δ 8.69 - 8.63 (m, 1H), 8.42 - 8.18 (m, 2H), 8.10 - 8.04 (m, 1H), 7.99 - 7.62 (m, 2H), 7.58 - 7.29 (m, 3H), 7.20 - 6.96 (m, 2H), 6.55 - 5.41 (m, 1H), 4.86 - 4.55 (m, 2H), 4.46 - 4.40 (m, 3H), 2.74 - 2.47 (m, 3H).

**Example 68A: (*S*)-4-amino-7-chloro-*N*-(6-((3-chloro-1-methyl-1*H*-pyrazol-4-yl)ethynyl)-2,3-dihy drobenzofuran-3-yl)-*N*-methyl-1,3-dihydrofuro[3,4-*c*]quinoline-8-carboxamide (Compound 68A)**

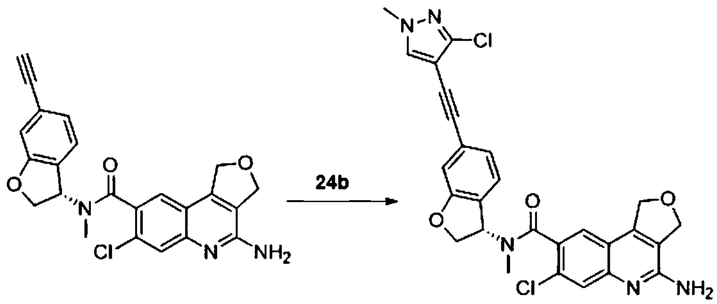

43b-A

Compound 68A

Referring to the preparation process of compound **43A,** compound 1-cyclopropyl-4-iodopyrazole was replaced with compound **24b** (38.8 mg, 0.16 mmol), and the mixture was reacted at room temperature for 3 h to give compound 68A (10 mg). ESI-MS (m/z): 533.89 $[M+H]^+$. $^1$H NMR (400 MHz, DMSO-d6) δ 8.20 - 8.17 (m, 1H), 7.89 - 7.61 (m, 2H), 7.54 - 7.32 (m, 1H), 7.18 - 7.06 (m, 1H), 7.04 - 6.97 (m, 1H), 6.86 (s, 2H), 6.53 - 5.31 (m, 3H), 5.05 - 4.98 (m, 2H), 4.86 - 4.48 (m, 2H), 4.19 - 4.11 (m, 2H), 3.85 - 3.84 (s, 3H), 2.69 and 2.47 (s, 3H).

**Example 69A: (*S*)-4-amino-7-chloro-*N*-(6-((1-ethyl-1*H*-pyrazol-4-yl)ethynyl)-2,3-dihydrobenzof uran-3-yl)-*N*-methyl-1,3-dihydrofuro[3,4-*c*]quinoline-8-carboxamide (Compound 69A)**

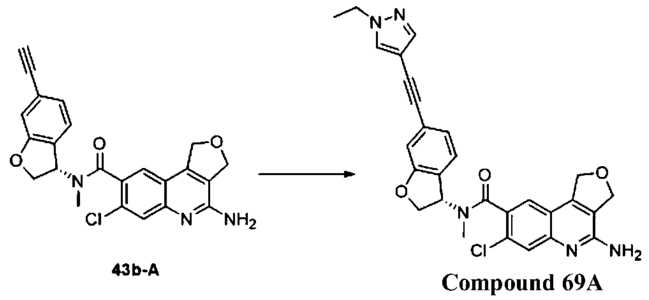

43b-A

Compound 69A

Referring to the preparation process of compound **43A,** compound 1-cyclopropyl-4-iodopyrazole was replaced with compound 1-ethyl-4-iodo-1*H*-pyrazole (52.9 mg, 0.24 mmol), and the mixture was reacted at room temperature for 3 h to give compound **69A** (10 mg). (13 mg) ESI-MS (m/z): 514.0 $[M+H]^+$. $^1$H NMR (400 MHz, DMSO-d6) δ 8.15 - 8.11 (m, 1H), 7.89 - 7.61 (m, 3H), 7.47 - 7.26 (m, 1H), 7.14 - 7.03 (m, 1H), 7.02 - 6.92 (m, 1H), 6.85 (s, 2H), 6.51 - 5.30 (m, 3H), 5.05 - 4.97 (m, 2H), 4.84 - 4.44 (m, 2H), 4.19 - 4.11 (m, 2H), 2.69 and 2.46 (s, 3H), 1.42 - 1.34 (m, 3H).

**Example 70A: (*S*)-4-amino-7-chloro-*N*-methyl-*N*-(6-(pyridin-3-ylethynyl)-2,3-dihydrobenzofura n-3-yl)-1,3-dihydrofuro[3,4-c]quinoline-8-carboxamide (Compound 70A)**

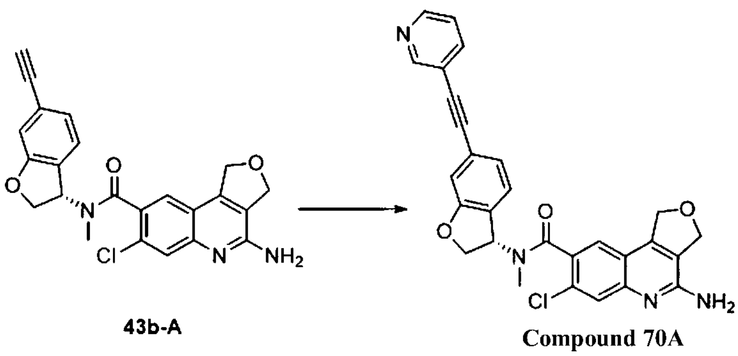

43b-A

Compound 70A

Referring to the preparation process of compound **43A,** compound 1-cyclopropyl-4-iodopyrazole was replaced

with compound 3-iodopyridine (48.8 mg, 0.24 mmol), and the mixture was reacted at room temperature for 3 h to give compound **70A** (24 mg). ESI-MS (m/z): 497.0 [M+H]$^+$. $^1$H NMR (400 MHz, DMSO-d6) δ 8.79 - 8.74 (m, 1H), 8.63 - 8.58 (m, 1H), 8.02 - 7.96 (m, 1H), 7.90 - 7.57 (m, 2H), 7.54 - 7.34 (m, 2H), 7.28 - 7.07 (m, 2H), 6.86 (s, 2H), 6.54 - 5.33 (m, 3H), 5.08 - 4.97 (m, 2H), 4.89 - 4.48 (m, 2H), 2.70 and 2.48 (s, 3H).

**Example 71A: (*S*)-4-amino-7-chloro-*N*-(6-((1-cyclopropyl-5-methyl-1*H*-pyrazol-4-yl)ethynyl)-2,3 -dihydrobenzofuran-3-yl)-*N*-methyl-1,3-dihydrofuro[3,4-*c*]quinoline-8-carboxam ide (Compound 71A)**

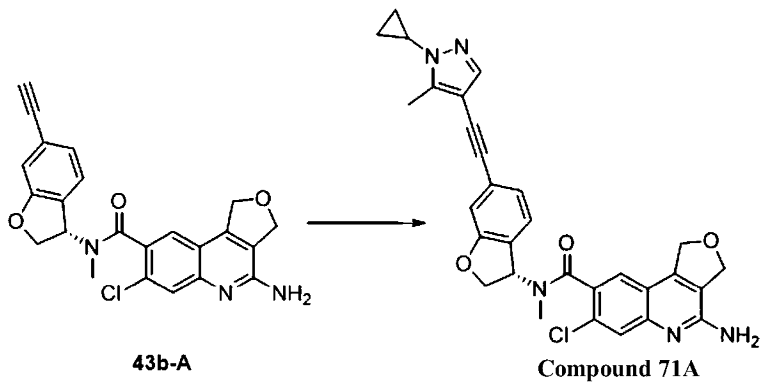

43b-A

Compound 71A

Referring to the preparation process of compound **43A,** compound 1-cyclopropyl-4-iodopyrazole was replaced with compound 1-cyclopropyl-4-iodo-5-methyl-1H-pyrazole (59.52 mg, 0.24 mmol, see WO2023044171 A1 for the preparation method), and the mixture was reacted at room temperature for 3 h to give compound **71A** (8 mg). ESI-MS (m/z): 540.2 [M+H]$^+$. $^1$H NMR (400 MHz, DMSO-d6) δ 7.90 - 7.60 (m, 2H), 7.57 - 7.52 (m, 1H), 7.48 - 7.28 (m, 1H), 7.16 - 7.05 (m, 1H), 7.04 - 6.95 (m, 1H), 6.86 (s, 2H), 6.52 - 5.28 (m, 3H), 5.07 - 4.97 (m, 2H), 4.86 - 4.46 (m, 2H), 3.60 - 3.53 (m, 1H), 2.69 and 2.47 (s, 3H), 2.45 - 2.44(m, 3H), 1.08 - 1.01 (m, 4H).

**Example 72: 4-amino-7-fluoro-*N*,1-dimethyl-*N*-(7-((1-methyl-1*H*-pyrazol-4-yl)ethynyl)isochro man-4-yl)-1*H*-pyrazolo[4,3-c]quinoline-8-carboxamide (Compound 72)**

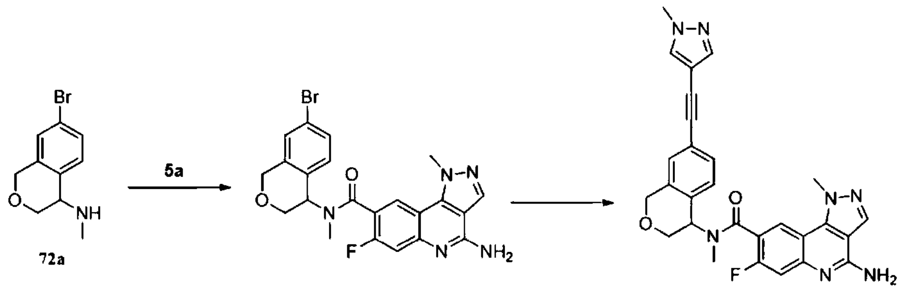


72a

72b

Compound 72

Referring to the preparation method for compound 9c in WO2024131901 A1, compound **72a** was condensed with compound **5c** to give compound **72b.**

To a solution of compound **72a** (48.4 mg, 0.1 mmol) in *N,N*-dimethylacetamide (2 mL) were sequentially added 4-ethynyl-1-methyl-1*H*-pyrazole (21.2 mg, 0.2 mmol), Xphos Pd G3 (8.5 mg, 0.01 mmol), and triethylamine (40.5 mg, 0.4 mmol) under nitrogen atmosphere. After completion of the addition, the mixture was reacted in a microwave reactor at 90 °C for 4 h. Water (50 mL) was added to the reaction solution, and the reaction solution was extracted with ethyl acetate (20 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give compound **72** (25 mg). ESI-MS (m/z): 510.01 [M+H]$^+$. $^1$H NMR (400 MHz, DMSO) δ 8.40 - 8.26 (m, 2H), 8.10 - 8.05 (m, 1H), 7.72 - 7.67 (m, 1H), 7.49 - 7.23 (m, 6H), 5.84 - 4.52 (m, 3H), 4.44 - 4.39 (m, 3H), 4.23 - 3.97 (m, 2H), 3.89 - 3.86 (m, 3H), 2.85 - 2.65 (m, 3H).

**Example** 73A: **(*S*)-4-amino-7-chloro-*N*-(6-((1-(difluoromethyl)-1*H*-pyrazol-4-yl)ethynyl)-2,3-dih ydrobenzofuran-3-yl)-*N*-methyl-1,3-dihydrofuro[3,4-*c*]quinoline-8-carboxamide (Compound 73A)**

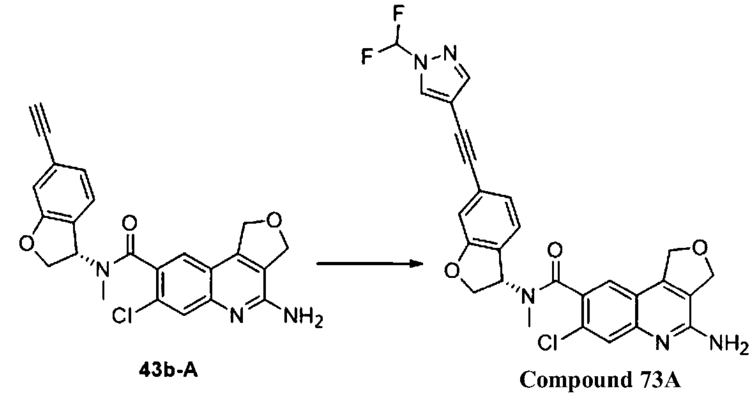

43b-A

Compound 73A

Referring to the preparation process of compound **43A,** compound 1-cyclopropyl-4-iodopyrazole was replaced with compound 1-difluoromethyl-4-iodo-1H-pyrazole (48.8 mg, 0.2 mmol), and the mixture was reacted at 40 °C for 3 h to give compound **73A** (20 mg). ESI-MS (m/z): 536.12 $[M+H]^+$. $^1H$ NMR (400 MHz, DMSO) δ 8.71 - 8.64 (m, 1H), 8.12 - 8.04 (m, 1H), 8.02 - 7.82 (m, 1H), 7.73 - 7.58 (m, 2H), 7.51 - 7.31 (m, 1H), 7.20 - 6.99 (m, 2H), 6.86 (s, 2H), 6.55 - 5.29 (m, 3H), 5.08 - 4.99 (m, 2H), 4.88 - 4.45 (m, 2H), 2.71 and 2.46 (s, 1H).

**Example 74A: (*S*)-4-amino-7-chloro-*N*-methyl-*N*-(6-((5,6,7,8-tetrahydroimidazo[1,2-*a*]pyridin-3 -yl)ethynyl)-2,3-dihydrobenzofuran-3-yl)-1,3-dihydrofuro [3,4-c] quinoline-8-carb oxamide (Compound 74A)**

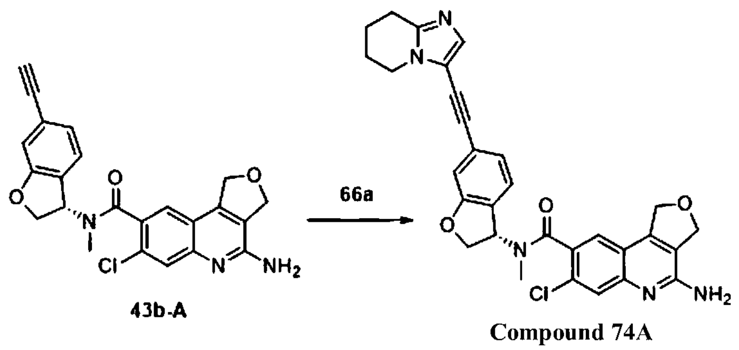


43b-A

Compound 74A

Referring to the preparation process of compound **43A,** compound 1-cyclopropyl-4-iodopyrazole was replaced with compound **66b** (40.2 mg, 0.2 mmol), and the mixture was reacted at 100 °C for 3 h to give compound **74A** (25 mg). ESI-MS (m/z): 540.25 $[M+H]^+$. $^1H$ NMR (400 MHz, DMSO) δ 7.90 - 7.58 (m, 2H), 7.47 - 7.32 (m, 1H), 7.29 - 7.23 (m, 1H), 7.21 - 7.00 (m, 2H), 6.86 (s, 2H), 6.54 - 6.42 (m, 1H), 6.51 - 5.28 (m, 3H), 5.07 - 4.97 (m, 2H), 4.86 - 4.48 (m, 2H), 4.03 - 3.93 (m, 2H), 2.82 - 2.73 (m, 2H), 2.71 and 2.46 (s, 3H), 2.01 - 1.93 (m, 2H), 1.90 - 1.82 (m, 2H).

**Example 75A: (*S*)-4-amino-7-chloro-*N*-(6-((6,7-dihydro-5*H*-pyrrolo[1,2-*a*]imidazol-3-yl)ethynyl) -2,3-dihydrobenzofuran-3-yl)-N-methyl-1,3-dihydrofuro[3,4-c]quinoline-8-carbo xamide (Compound 75A)**

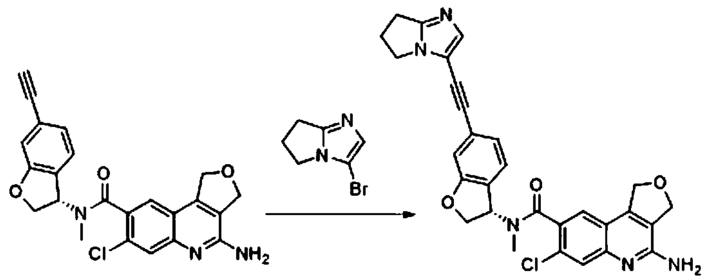

43b·A

Compound 75A

Referring to the preparation process of compound **43A,** compound 1-cyclopropyl-4-iodopyrazole was replaced **with** compound 3-bromo-6,7-dihydro-5*H*-pyrrolo[1,2-*a*]imidazole (37.4 mg, 0.2 mmol), and the mixture was reacted at 100 °C for 4 h to give compound **75A** (25 mg). ESI-MS (m/z): 526.08 $[M+H]^+$. $^1H$ NMR (400 MHz, DMSO) δ 7.87 - 7.58 (m, 2H), 7.49 - 7.26 (m, 2H), 7.18 - 6.97 (m, H), 6.85 (s, 2H), 6.53 - 5.29 (m, 3H), 5.06 - 4.97 (m, 2H), 4.86 - 4.46 (m, 2H), 4.09 - 3.96 (m, 2H), 2.86 - 2.75 (m, 2H), 2.72 - 2.45 (m, 5H).

**Example 76A: (S)-4-amino-7-chloro-*N*-methyl-N-(6-((1-methyl-1*H*-pyrazolo[4,3-b]pyridin-6-yl)e thynyl)-2,3-dihydrobenzofuran-3-yl)-1,3-dihydrofuro[3,4-*c*]quinoline-8-carboxa mide (Compound 76A)**

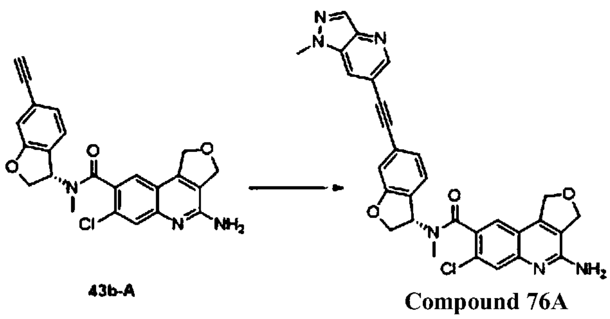

43b-A

Compound 76A

Referring to the preparation process of compound **43A,** compound 1-cyclopropyl-4-iodopyrazole was replaced with compound 6-bromo-1-methylpyrazolo[4,3-b]pyridine (50.5 mg, 0.24 mmol), and the mixture was reacted at 100 C for 4 h to give compound **76A** (18 mg). ESI-MS (m/z): 551.0 $[M+H]^+$. $^1H$ NMR (400 MHz, DMSO-d6) δ 8.69 - 8.64 (m, 1H), 8.51 - 8.43 (m, 1H), 8.36 - 8.31 (m, 1H), 7.90 - 7.36 (m, 3H), 7.30 - 7.09 (m, 2H), 6.85 (s, 2H), 6.56 - 5.30 (m, 3H), 5.07 - 4.97 (m, 2H), 4.89 - 4.50 (m, 2H), 4.12 - 4.11 (m, 3H), 2.71 and 2.50 (s, 3H).

**Example 77A: (*S*)-4-amino-*N*-(6-((6-cyclopropylpyridin-3-yl)ethynyl)-2,3-dihyrobenzofuran-3-yl )-7-fluoro-N,1-dimethyl-1H-pyrazolo[4,3-c]quinoline-8-carboxamide (Compound 77A)**

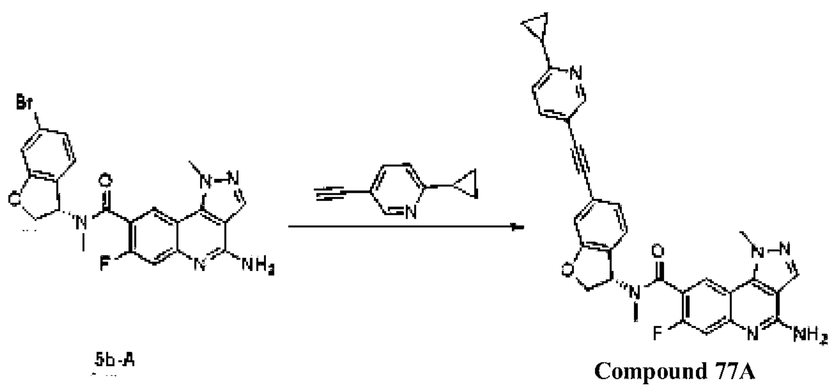

5b-A

Compound 77A

Referring to the preparation process of compound **5A,** compound 4-ethynyl-1-methylpyrazole was replaced with compound 2-cyclopropyl-5-ethynylpyridine (70 mg, 0.49 mmol), and the mixture was reacted in a microwave reactor at 80 C for 4 h to give compound **77A** (33 mg). ESI-MS (m/z): 533.3 $[M+H]^+$. $^1H$ NMR (400 MHz, DMSO-$d_6$) δ 8.58 (s, 1H), 8.31 - 8.23 (m, 2H), 7.82 (d, $J$ = 8.0 Hz, 1H), 7.49 - 7.05 (m, 7H), 6.53 - 5.55 (m, 1H), 4.89 - 4.61 (m, 2H), 4.50 - 4.30 (m, 3H), 2.72 and 2.60 (s, 3H), 2.21 - 2.12 (m, 1H), 1.07 - 0.93 (m, 4H).

**Example 78A: (*S*)-4-amino-7-chloro-*N*-methyl-*N*-(6-((1-methyl-1*H*-pyrazol-5-yl)ethynyl)-2,3-dih ydrobenzofuran-3-yl)-1,3-dihydrofuro[3,4-*c*]quinoline-8-carboxamide (Compound 78A)**

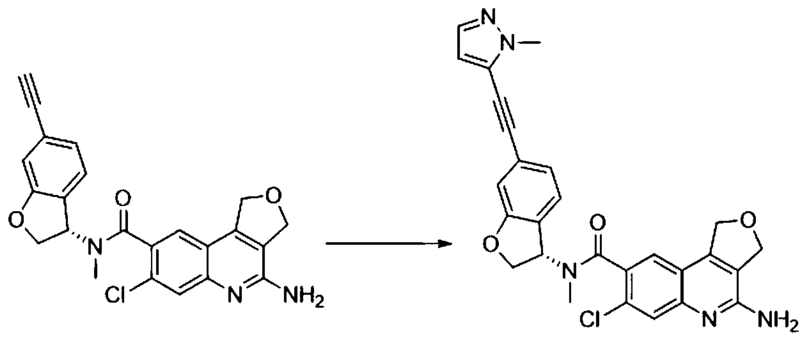

43b-A

Compound 78A

Referring to the preparation process of compound **43A,** compound 1-cyclopropyl-4-iodopyrazole was replaced with compound 5-iodo-1-methylpyrazole (59.4 mg, 0.29 mmol), and the mixture was reacted at room temperature for 3 h under nitrogen atmosphere to give compound **78A** (23 mg). ESI-MS (m/z): 499.91 $[M+H]^+$. $^1H$ NMR (400 MHz, DMSO) δ 7.88 - 7.62 (m, 1H), 7.62 - 7.59 (m, 1H), 7.55 - 7.50 (m, 1H), 7.49 - 7.33 (m, 1H), 7.27 - 7.07 (m, 2H), 6.84 (s, 2H), 6.65 - 6.59 (m, 1H), 6.57 - 5.27 (m, 3H), 5.05 - 4.96 (m, 2H), 4.85 - 4.47 (m, 2H), 3.94 and 3.93 (s, 3H), 2.68 and 2.46 (s, 3H).

**Example 79A: (S)-4-amino-7-fluoro-1-methyl-N-(methyl-D3)-N-(6-((1-methyl-1H-pyrazol-4-yl)e thynyl)-2,3-dihydrobenzofuran-3-yl)-1*H*-pyrazolo[4,3-*c*]quinoline-8-carboxamide (Compound 79A)**

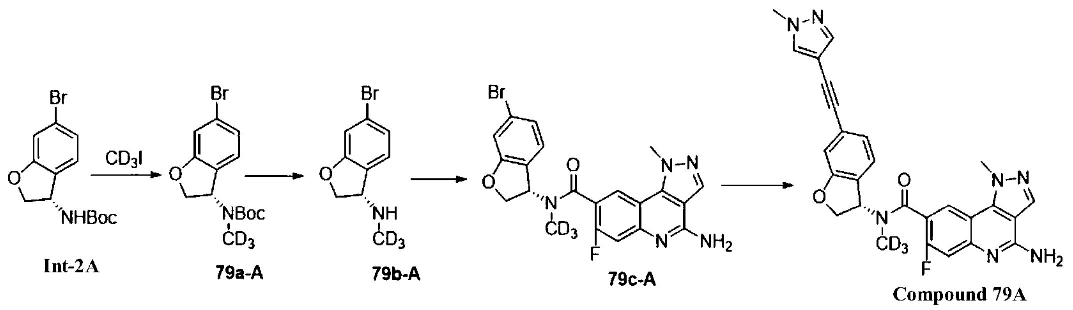


Int-2A 79a-A 79b-A 79c-A Compound 79A

For the synthesis process of preparing compound **79A** from compound **Int-2A,** reference was made to the synthesis process of preparing compound **5A** from compound **Int-2A.** Compound **79A** (25 mg). ESI-MS (m/z): 499.00 $[M+H]^+$. $^1H$ NMR (400 MHz, DMSO) δ 8.38 - 8.20 (m, 2H), 8.08 (s, 1H), 7.69 (s, 1H), 7.45 - 7.25 (m, 4H), 7.17 - 6.93 (m, 2H), 6.50 - 5.54 (m, 1H), 4.87 - 4.61 (m, 2H), 4.46 - 4.37 (m, 3H), 3.87 (s, 3H).

**Example 80A: (*S*)-4-amino-7-fluoro-*N*-methyl-*N*-(6-((5,6,7,8-tetrahydroimidazo[1,2-*a*]pyridin-3-yl)ethynyl)-2,3-dihydrobenzofuran-3-yl)-1,3-dihydrofuro [3,4-c] quinoline-8-carbo xamide (Compound 80A)**

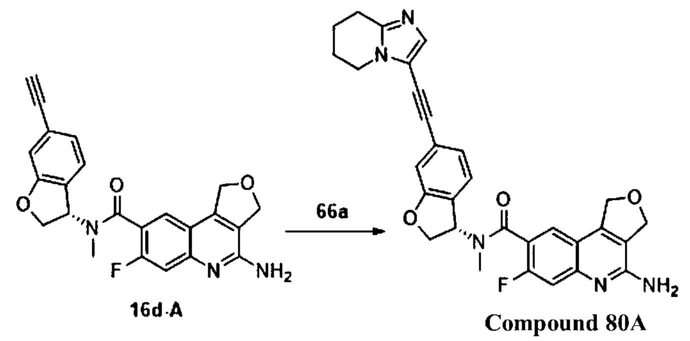


16d·A

Compound 80A

Referring to the preparation process of compound **16A,** compound 1-cyclopropyl-4-iodopyrazole was replaced with compound **66a** (40.2 mg, 0.2 mmol), and the mixture was reacted in a microwave reactor at 100 °C for 4 h to give compound **80A** (15 mg). ESI-MS (m/z): 524.32 $[M+H]^+$. $^1H$ NMR (400 MHz, DMSO) δ 7.68 - 7.62 (m, 1H), 7.42 - 7.29 (m, 2H), 7.28 - 7.24 (m, 1H), 7.21 - 7.11 (m, 1H), 7.09 - 7.02 (m, 1H), 6.85 (s, 2H), 6.49 - 5.50 (m, 1H), 5.43 - 5.30 (m, 2H), 5.06 - 4.98 (m, 2H), 4.85 - 4.57 (m, 2H), 4.02 - 3.93 (m, 2H), 2.81 - 2.74 (m, 2H), 2.70 and - 2.55 (s, 3H), 2.01 - 1.94 (m, 2H), 1.90 - 1.82 (m, 2H).

**Example 81A: (*S*)-4-amino-7-chloro-*N*-(6-((1,4-dimethyl-1*H*-pyrazol-3-yl)ethynyl)-2,3-dihydrob enzofuran-3-yl)-*N*,1-dimethyl-1*H*-pyrazolo[4,3-*c*]quinoline-8-carboxamide (Compound 81A)**

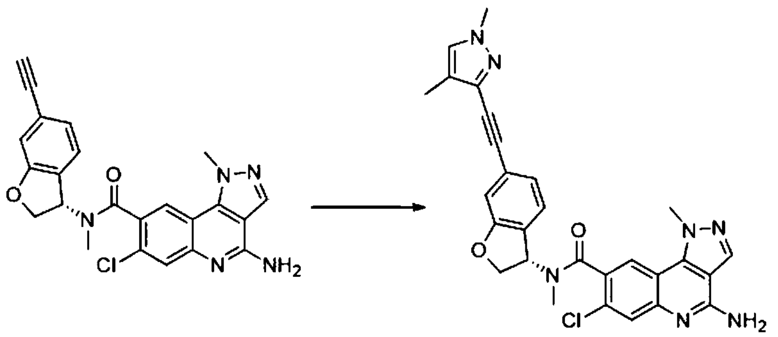

8d-A

Compound 81A

Referring to the preparation process of compound **8A,** compound 1-cyclopropyl-4-iodopyrazole was replaced with compound 3-iodo-1,4-dimethylpyrazole (35.5 mg, 0.16 mmol), and the mixture was reacted at room temperature for 16 h under nitrogen atmosphere to give compound **81A** (15 mg). ESI-MS (m/z): 526.2 $[M+H]^+$. $^1H$ NMR (400 MHz, DMSO-d6) δ 8.42 - 8.21 (m, 2H), 7.72 - 7.26 (m, 5H), 7.21 - 7.10 (m, 1H), 7.10 - 6.99 (m, 1H), 6.55 - 5.43 (m, 1H), 4.89 - 4.57 (m, 2H), 4.47 and 4.41 (s, 3H), 3.82 - 3.81 (m, 3H), 2.75 - 2.51 (m, 3H), 2.10 - 2.08 (m, 3H).

**Example 82A: (*S*)-4-amino-7-fluoro-*N*,1-dimethyl-*N*-(6-((1-(methyl-*D*3)-1*H*-pyrazol-4-yl)ethynyl )-2,3-dihydrobenzofuran-3-yl)-1*H*-pyrazolo[4,3-c]quinoline-8-carboxamide (Compound 82A)**

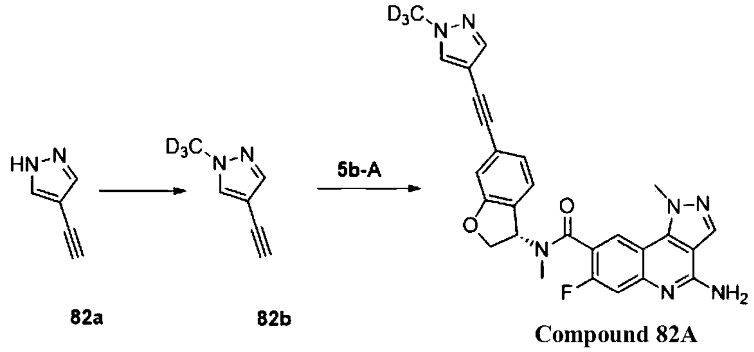


82a 82b Compound 82A

### Preparation of compound 82b

Compound **82a** (236.7 mg, 2.57 mmol) was dissolved in N,N-dimethylformamide (10 mL). The reaction solution was cooled to 0 °C, and sodium hydride (205.6 mg, 5.14 mmol, 60% purity) was added. After the reaction solution was reacted at 0 °C for 1 h, deuterated iodomethane (745.1 mg, 5.14 mmol) was added. Then the reaction solution was stirred at 25 °C for 12 h, water (5 mL) was added to the reaction solution to quench the reaction, and the reaction solution was extracted with ethyl acetate (25 mL × 3). The organic phases were washed three times with saturated brine, combined, dried over anhydrous sodium sulfate, and filtered. The filtrate was concentrated, and the residue was subjected to column chromatography (ethyl acetate:petroleum ether = 1:10) to give compound **82b** (250 mg). ESI-MS (m/z): 110.02[M+H]$^+$.

### Preparation of compound 82A

Referring to the preparation process of compound **5A,** compound 4-ethynyl-1-methylpyrazole was replaced with compound **82b** (21.8 mg, 0.2 mmol), and the mixture was reacted in a microwave reactor at 100 °C for 4 h to give compound **82A** (25 mg). ESI-MS (m/z): 498.98 [M+H]$^+$. $^1$H NMR (400 MHz, DMSO) δ 8.40 - 8.22 (m, 2H), 8.07 (s, 1H), 7.69 (s, 1H), 7.46 - 7.24 (m, 4H), 7.17 - 7.06 (m, 1H), 7.05 - 6.93 (m, 1H), 6.50 - 5.52 (m, 1H), 4.88 - 4.64 (m, 2H), 4.48 - 4.35 (m, 3H), 2.74 - 2.57 (m, 3H).

### Example 83A: (S)-4-amino-N-(6-((1-(difluoromethyl)-1H-pyrazol-4-yl)ethynyl)-2,3-dihydrobenz ofuran-3-yl)-7-fluoro-*N*,1-dimethyl-1*H*-pyrazolo[4,3-*c*]quinoline-8-carboxamide (Compound 83A)

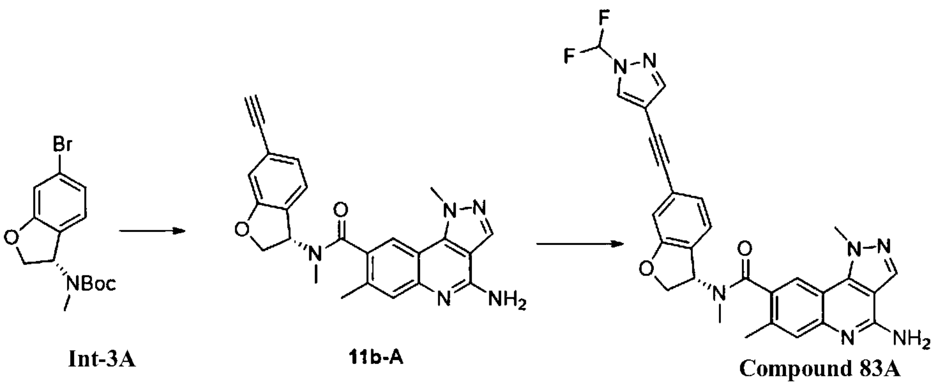

Int-3A 11b-A Compound 83A

Reference was made to WO2024131901 A1 for the preparation process of compound **11b-A.**

### Preparation of compound 83A

To a solution of compound 11b-A (60 mg, 0.15 mmol) in N-methylpyrrolidone (2 mL) were added cuprous iodide (5.6 mg, 0.03 mmol), tetrakis(triphenylphosphine)palladium (16.8 mg, 0.01 mmol), triethylamine (60.64 μL, 0.44 mmol), and 1-(difluoromethyl)-4-iodopyrazole (106.7 mg, 0.44 mmol). After completion of the addition, the mixture was reacted at 25 °C for 3 h under nitrogen atmosphere. The reaction solution was filtered through celite. Water (50 mL) was added, and the reaction solution was extracted with ethyl acetate (25 mL × 2). The organic phases were combined, washed with

saturated brine, dried over anhydrous sodium sulfate, and filtered. The filtrate was concentrated, and the residue was subjected to normal phase column chromatography (dichloromethane/methanol = 20:1) to give a crude compound. The crude compound was then subjected to reversed phase column chromatography (acetonitrile/0.05% ammonium bicarbonate solution = 0:100% - 45%:55%) to give compound **83A** (18 mg). ESI-MS (m/z): 528.17 $[M+H]^+$. $^1H$ NMR (400 MHz, DMSO) δ 8.67 (s, 1H), 8.24 (s, 1H), 8.11 - 8.04 (m, 2H), 7.85 (t, *J= 58* Hz, 1H), 7.59 - 7.41 (m, 2H), 7.25 - 7.13 (m, 1H), 7.09 - 6.95 (m, 3H), 6.58 - 5.48 (m, 1H), 4.87 - 4.55 (m, 2H), 4.39 (s, 3H), 2.72 and 2.37 (s, 3H).

**Example 84A: (*S*)-4-amino-*N*-(6-((6,7-dihydro-5*H*-pyrrolo[1,2-*a*]imidazol-3-yl)ethynyl)-2,3-dihy drobenzofuran-3-yl)-7-fluoro-*N*,1-dimethyl-1*H*-pyrazolo[4,3-*c*]quinoline-8-carbo xamide (Compound 84A)**

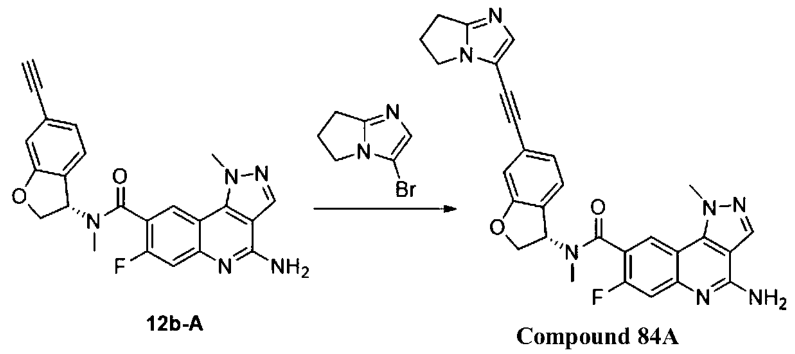

12b-A

Compound 84A

Referring to the preparation process of compound **12A,** compound 1-cyclopropyl-4-iodo-1*H*-pyrazole was replaced **with** compound 3-bromo-6,7-dihydro-5*H*-pyrrolo[1,2-*a*]imidazole (45.0 mg, 0.24 mmol), and the mixture was reacted at 100 C for 3 h to give compound **84A** (19 mg). ESI-MS (m/z): 522.0 $[M+H]^+$. $^1H$ NMR (400 MHz, DMSO) δ 8.38 - 8.19 (m, 2H), 7.43 - 7.27 (s, 5H), 7.19 - 7.07 (m, 1H), 7.06 - 6.95 (m, 1H), 6.50 - 5.52 (m, 1H), 4.86 - 4.59 (m, 2H), 4.47 - 4.32 (m, 3H), 4.07 - 3.93 (m, 2H), 2.84 - 2.75 (m, 2H), 2.69 (s, 2H), 2.60 - 2.53 (m, 3H).

**Example 85: 4-amino-*N*-(7-((1-cyclopropyl-1*H*-pyrazol-4-yl)ethynyl)isochroman-4-yl)-7-fluoro -*N*,1-dimethyl-1*H*-pyrazolo[4,3-c]quinoline-8-carboxamide (Compound 85)**

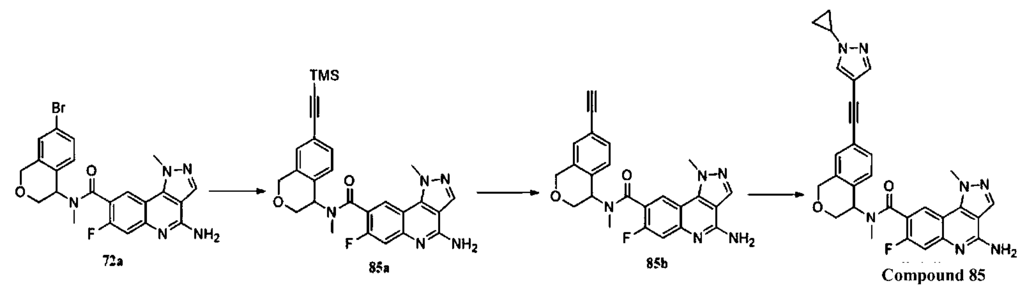

72a 85a 85b Compound 85

**Preparation of compound 85a**

To a solution of compound **72a** (214 mg, 0.44 mmol) in N,N-dimethylacetamide (5 mL) were sequentially added trimethylsilylacetylene (86.9 mg, 0.88 mmol), Xphos Pd G3 (37.4 mg, 0.044 mmol), and triethylamine (179 mg, 1.77 mmol) under nitrogen atmosphere. After completion of the addition, the mixture was reacted in a microwave reactor at 90 C for 4 h. Water (50 mL) was added to the reaction solution, and the reaction solution was extracted with ethyl acetate (20 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give compound **85a** (224 mg). ESI-MS (m/z): 501.7 $[M+H]^+$.

**Preparation of compound 85b**

To a solution of compound **85a** (224 mg, 0.45 mmol) in tetrahydrofuran (5 mL) was added a solution of tetrabutylammonium fluoride (0.9 mL, 1 M). After completion of the addition, the mixture was allowed to react at room temperature for 1 h. Water was added, and the reaction solution was extracted with ethyl acetate (30 mL × 3). The organic phases were combined, washed with saturated brine, dried over anhydrous sodium sulfate, and filtered. The filtrate was concentrated, and the residue was subjected to column chromatography (methanol:dichloromethane = 1:20) to give compound **85b** (140 mg). ESI-MS (m/z): 430.2 $[M+H]^+$.

**Preparation of compound 85**

To a solution of compound **85b** (43 mg, 0.1 mmol) in *N*-methylpyrrolidone (2 mL) were sequentially added 1-cyclopropyl-4-iodo-1*H*-pyrazole (46.8 mg, 0.2 mmol), cuprous iodide (3.8 mg, 0.02 mmol), *N,N*-diisopropylethylamine (51.7 mg, 0.4 mmol), and tetrakis(triphenylphosphine)palladium (11.6 mg, 0.01 mmol) under nitrogen atmosphere. After completion of the addition, the mixture was allowed to react under an oil bath at 40 °C for 3 h. Water (100 mL) was added to the reaction solution, and the reaction solution was extracted with ethyl acetate (20 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give compound **85** (20 mg). ESI-MS (m/z): 535.84 $[M+H]^+$. $^1H$ NMR (400 MHz, DMSO) δ 8.40 - 8.25 (m, 2H), 8.23 - 8.16 (m, 1H), 7.71 - 7.67 (m, 1H), 7.47 - 7.26 (m, 6H), 5.82 - 4.51 (m, 3H), 4.42 (s, 3H), 4.23 - 3.96 (m, 2H), 3.83 - 3.72 (m, 1H), 2.82 and 2.69 (s, 3H), 1.13 - 0.97 (m, 4H).

**Example 86A: (*S*)-4-amino-7-chloro-*N*-(6-((1-cyclopropyl-1*H*-pyrazol-4-yl)ethynyl)-2,3-dihydro benzofuran-3-yl)-N-(methyl-D3)-1,3-dihydrofuro[3,4-c]quinoline-8-carboxamide (Compound 86A)**

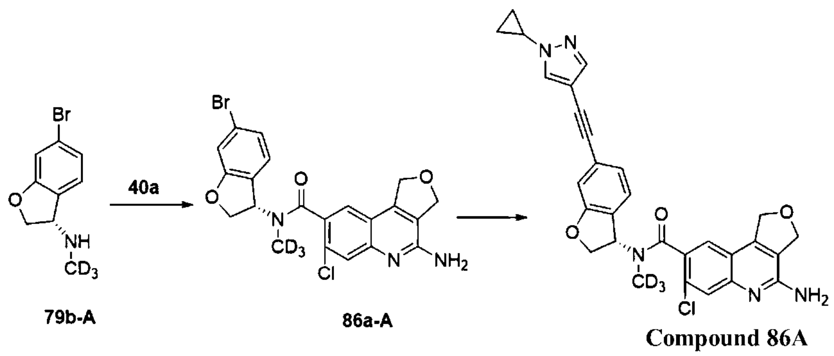


79b-A 86a-A Compound 86A

Referring to the preparation process of compound **40b-A,** compound **1a-A** was replaced with compound **79b-A** to give compound **86a-A.**

Referring to the preparation process of compound **43A,** compound **40b-A** was replaced with compound **86a-A** to give compound **86A** (30 mg). ESI-MS (m/z): 528.96 $[M+H]^+$. $^1H$ NMR (400 MHz, DMSO) δ 8.20 - 8.15 (m, 1H), 7.87 - 7.57 (m, 3H), 7.47 - 7.29 (m, 1H), 7.15 - 7.03 (m, 1H), 7.02 - 6.92 (m, 1H), 6.85 (s, 2H), 6.54 - 5.28 (m, 3H), 5.08 - 4.96 (m, 2H), 4.85 - 4.43 (m, 2H), 3.81 - 3.71 (m, 1H), 1.10 - 0.97 (m, 4H).

**Example 87: 4-amino-7-chloro-N-(7-((1-cyclopropyl-1H-pyrazol-4-yl)ethynyl)isochroman-4-yl) -N-methyl-1,3-dihydrofuro[3,4-c]quinoline-8-carboxamide (Compound 87)**

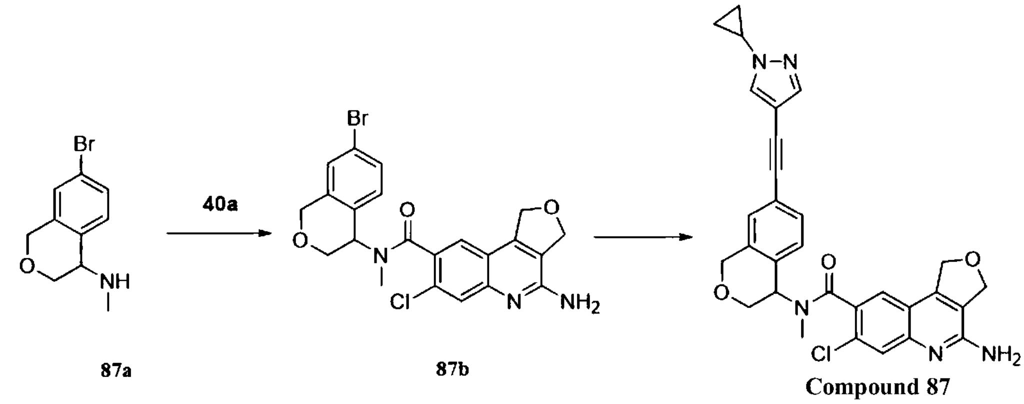


87a 87b Compound 87

### Preparation of compound 87b

Compound **40a** (193 mg, 0.73 mmol) was dissolved in N,N-dimethylformamide (10 mL), and N,N-diisopropylethylamine (0.63 mL, 3.64 mmol) was added. The mixture was stirred for 5 min under an ice bath, and *N,N,N',N'*-tetramethyl-*O*-(7-azabenzotriazol-1-yl)uronium hexafluorophosphate (415 mg, 1.09 mmol) was then added, followed by addition of **87a** (176.2 mg, 0.73 mmol, see WO2024131901 A1 for the preparation method). The mixture was allowed to react for another hour. Water (100 mL) was added to the reaction solution, and the reaction solution was extracted with ethyl acetate (20 mL × 3). The organic phases were washed three times with saturated brine, combined, dried over anhydrous sodium sulfate, and filtered. The filtrate was concentrated, and the residue was subjected to column chromatography (methanol:dichloromethane = 1:20) to give compound **87b** (212 mg). ESI-MS (m/z): 488.0[M+H]$^+$.

### Preparation of compound 87

To a solution of compound **87b** (48.8 mg, 0.1 mmol) in *N,N*-dimethylacetamide (2 mL) were sequentially added 1-cyclopropyl-4-ethynyl-1*H*-pyrazole (26.4 mg, 0.2 mmol), cuprous iodide (3.8 mg, 0.02 mmol), N,N-diisopropylethylamine (0.07 mL, 0.4 mmol), and tetrakis(triphenylphosphine)palladium (11.6 mg, 0.01 mmol) under nitrogen atmosphere. After completion of the addition, the mixture was reacted in a microwave reactor at 100 °C for 4 h. Water (50 mL) was added to the reaction solution, and the reaction solution was extracted with ethyl acetate (20 mL × 3). The organic phases were combined, washed with saturated brine, dried over anhydrous sodium sulfate, and filtered. The solvent was removed by rotary evaporation, and the residue was subjected to column chromatography (methanol:dichloromethane = 1:20) to give compound **87** (30 mg). ESI-MS (m/z): 539.96 [M+H]$^+$. $^1$H NMR (400 MHz, DMSO) δ 8.21 - 8.17 (m, 1H), 7.76 - 7.63 (m, 3H), 7.51 - 7.38 (m, 2H), 7.34 - 7.24 (m, 1H), 6.85 (s, 2H), 5.84 - 5.76 (m, 1H), 5.40 - 5.31 (m, 2H), 5.07 - 4.98 (m, 2H), 4.89 - 4.51 (m, 2H), 4.22 - 3.91 (m, 2H), 3.84 - 3.73 (m, 1H), 2.79 - 2.57 (m, 3H), 1.12 - 0.98 (m, 4H).

### Example 88A: (*S*)-4-amino-7-chloro-*N*-(6-((5,6-dihydro-4*H*-pyrrolo[1,2-*b*]pyrazol-3-yl)ethynyl)-2,3-dihydrobenzofuran-3-yl)-*N*-methyl-1,3-dihydrofuro[3,4-*c*]quinoline-8-carbox amide (Compound 88A)

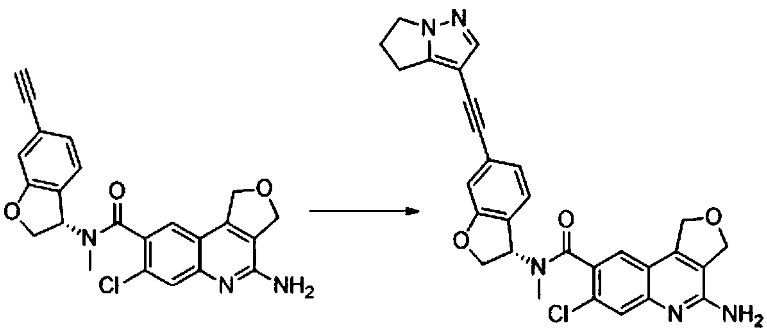

43b-A Compound 88A

Referring to the preparation process of compound 43A, compound 1-cyclopropyl-4-iodopyrazole was replaced with compound 3-bromo-5,6-dihydro-4H-pyrrolo[2,1-e]pyrazole (44.5 mg, 0.24 mmol), and the mixture was reacted in a

microwave reactor at 100 °C for 4 h to give compound 88A (10 mg). ESI-MS (m/z): 525.9 $[M+H]^+$. $^1H$ NMR (400 MHz, DMSO-$d_6$) δ 7.90 - 7.58 (m, 3H), 7.45 - 7.27 (m, 1H), 7.15 - 7.02 (m, 1H), 6.99 - 6.93 (m, 1H), 6.85 (s, 2H), 6.50 - 5.28 (m, 3H), 5.06 - 4.99 (m, 2H), 4.87 - 4.45 (m, 2H), 4.16 - 4.08 (m, 2H), 2.98 - 2.90 (m, 2H), 2.69 - 2.46 (m, 5H).

**Example 89A: (*S*)-4-amino-*N*-(6-((1-cyclobutyl-1*H*-pyrazol-4-yl)ethynyl)-2,3-dihydrobenzofura n-3-yl)-7-fluoro-N,1-dimethyl-1H-pyrazolo [4,3-c] quinoline-8-carboxamide (Compound 89A)**

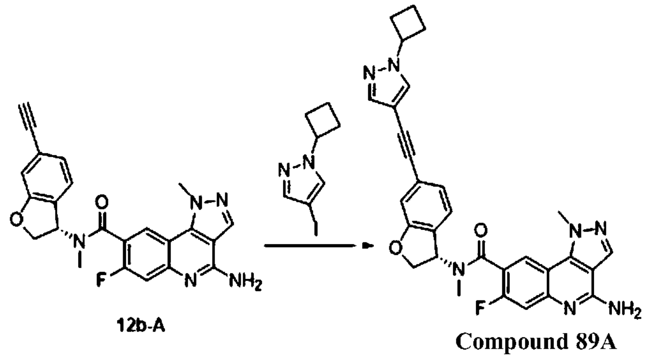

12b-A

Compound 89A

Referring to the preparation process of compound **12A,** compound 1-cyclopropyl-4-iodo-1H-pyrazole was replaced with compound 1-cyclobutyl-4-iodopyrazole (25 mg, 0.1 mmol), and the mixture was reacted at 25 °C for 2 h under nitrogen atmosphere to give compound **89A** (32 mg). ESI-MS (m/z): 536.02 $[M+H]^+$. $^1H$ NMR (400 MHz, DMSO-$d_6$) δ 8.42 - 8.20 (m, 3H), 7.73 (s, 1H), 7.47 - 7.31 (m, 4H), 7.15 - 7.05 (m, 1H), 7.03 - 6.94 (m, 1H), 6.50 - 5.54 (m, 1H), 4.92 - 4.56 (m, 3H), 4.53 - 4.29 (m, 3H), 2.75 - 2.56 (m, 3H), 2.50 - 2.29 (m, 4H), 1.86 - 1.74 (m, 2H).

**Example ’90A: (*S*)-4-amino-7-chloro-*N*-(6-((1-cyclobutyl-1*H*-pyrazol-4-yl)ethynyl)-2,3-dihydrobe nzofuran-3-yl)-*N*-methyl-1,3-dihydrofuro[3,4-*c*]quinoline-8-carboxamide (Compound 90A)**

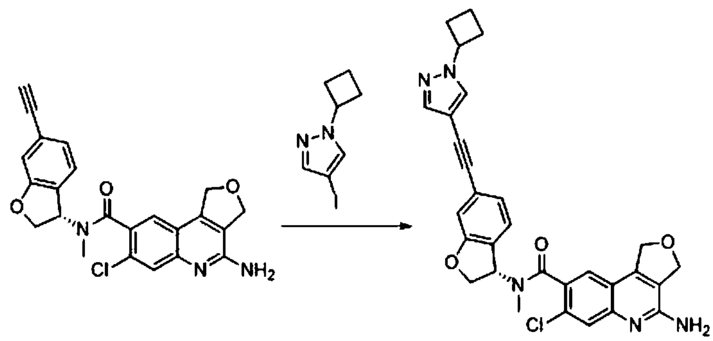

43b-A

Compound 90A

Referring to the preparation process of compound **43A,** compound 1-cyclopropyl-4-iodopyrazole was replaced with compound 1-cyclobutyl-4-iodopyrazole (50 mg, 0.20 mmol), and the mixture was reacted at room temperature for 2 h under nitrogen atmosphere to give compound **90A** (24 mg). ESI-MS (m/z): 540.00 $[M+H]^+$. $^1H$ NMR (400 MHz, DMSO-d6) δ 8.24 - 8.20 (m, 1H), 7.88 - 7.58 (m, 3H), 7.47 - 7.29 (m, 1H), 7.15 - 7.03 (m, 1H), 7.02 - 6.93 (m, 1H), 6.86 (s, 2H), 6.52 - 5.29 (m, 3H), 5.07 - 4.98 (m, 2H), 4.93 - 4.46 (m, 3H), 2.69 - 2.35 (m, 7H), 1.87 - 1.74 (m, 2H).

**Example 91A: (*S*)-4-amino-7-chloro-*N*-methyl-*N*-(6-((4,5,6,7-tetrahydropyrazolo[1,5-*a*]pyridin-3-yl)ethynyl)-2,3-dihydrobenzofuran-3-yl)-1,3-dihydrofuro[3,4-*c*]quinoline-8-car boxamide (Compound 91A)**

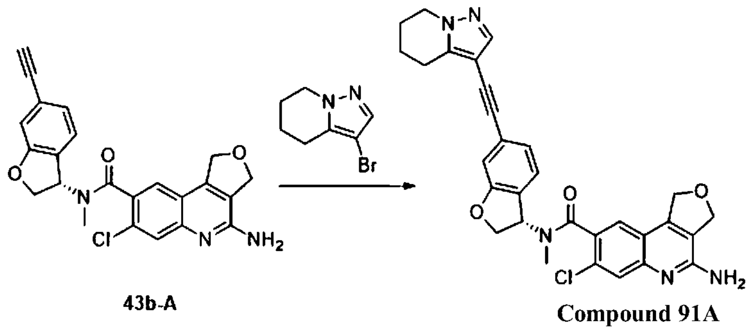

43b-A

Compound 91A

Referring to the preparation process of compound **43A,** compound 1-cyclopropyl-4-iodopyrazole was replaced with compound 3-bromo-4,5,6,7-tetrahydropyrazolo[1,5-a]pyridine (47.8 mg, 0.24 mmol), and the mixture was reacted in a microwave reactor at 100 °C for 4 h to give compound **91A** (8 mg). ESI-MS (m/z): 539.87 $[M+H]^+$. $^1H$ NMR (400 MHz, DMSO-$d_6$) δ 7.93 - 7.54 (m, 3H), 7.47 - 7.31 (m, 1H), 7.14 - 6.88 (m, 4H), 6.52 - 5.29 (m, 3H), 5.06 - 4.98 (m, 2H), 4.85 - 4.45 (m, 2H), 4.12 - 4.04 (m, 2H), 2.85 - 2.81 (m, 2H), 2.69 and 2.47 (s, 3H), 2.04 - 1.94 (m, 2H), 1.88 - 1.80 (m, 2H).

Reference Example 1:

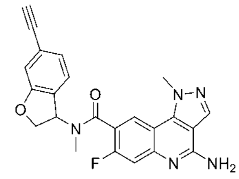,

which was prepared with reference to the method in WO2024131901A1.

Reference Example 2:

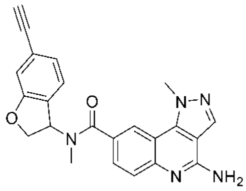,

which was prepared with reference to the method in WO2024131901A1.

Reference Example 3:

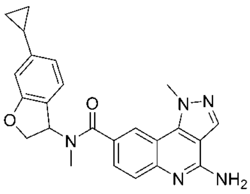,

which was prepared with reference to the method in WO2022169948 A1.

Reference Example 4:

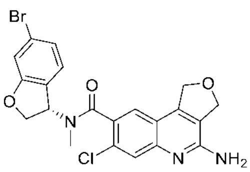.

Reference Example 5:

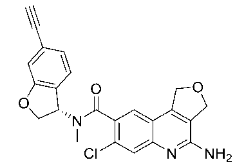.

Reference Example 6:

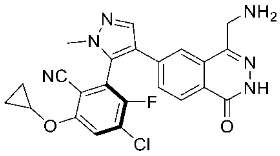

MRTX1719,

which was prepared with reference to the method in J. Med. Chem. 2022, 65(3), 1749-1766.

Reference Example 7:

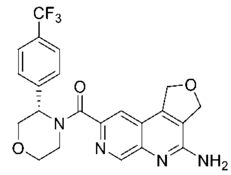

AMG-193,

which was prepared with reference to the method in WO2022132914 A1.

## Experimental Example 1: Inhibitory Activity of Compounds of the Present Disclosure Against Proliferation of Human Colon Cancer Cells

### 1. Construction of $MTAP^{-/-}$HCT-116 cell strain

The colon cancer cell strain HCT-116 (purchased from Nanjing Cobioer) was co-transfected with the gene editing tool CRISPR/Cas9 and sgRNA, and the $MTAP^{-/-}$ HCT-116 cell strain with biallelic inactivation of MTAP was obtained by assays such as Western Blotting and Sanger sequencing. The $MTAP^{-/-}$ HCT-116 cell strain was used for compound selectivity screening and evaluation based on the synergistic mechanism of MTAP deletion and PRMT5, including a symmetric dimethylarginine (SDMA) level test, a cell proliferation test, and the like.

### 2. Cell proliferation

$MTAP^{-/-}$ HCT-116 and wild-type HCT-116 cell strains were cultured in complete medium McCoy's 5A (Gibco, 16600082)/10% FBS (Gibco, 10099141C)/1% p/s (Gibco, 15140122) to evaluate the enhanced inhibitory effect of the compounds against the proliferation selectivity of $MTAP^{-/-}$ HCT-116 cells. On day 0, $MTAP^{-/-}$ HCT-116 cells or wild-type HCT-116 cells were added to a 96-well cell culture plate (Corning, 3599) at 100 cells/well, and the plate was incubated in an incubator at 37 °C with 5% $CO_2$. The compound was diluted in a 3-fold gradient with DMSO (Sigma, D5879) (initial concentration: 20 μM, 3-fold dilution, 8 concentration points in total). On day 1, the compound was diluted to multiple concentration points, and the cells were treated separately and incubated in the incubator at 37 °C with 5% $CO_2$ for another 10 days. On Day 11, 20 μL of MTS (CellTiter 96® $A_{Queous}$ One Solution Cell Proliferation Assay) (Promega, G3581) was added to each well. After the plate was incubated in the incubator at 37 °C with 5% $CO_2$ for another 2 h, readings (OD = 490 nM) were taken on Tecan Spark, and the data were then analyzed using the data analysis software GraphPad Prism 8 and using the equation "log(inhibitor) vs. normalized response -- variable slope" (formula Y = Bottom + (Top - Bottom)/(1 + 10^ ((Log$IC_{50}$ - X) × HillSlope))) to give $IC_{50}$ values of the compound. In the formula, Y represents the inhibition rate, X represents the logarithm of the compound concentration, Top represents the maximum response (the inhibition rate at the maximum compound concentration), Bottom represents the baseline response (the inhibition rate where the compound concentration was 0), Hill Slope represents the slope of the $IC_{50}$ curve, and $IC_{50}$ represents the half maximal inhibitory

concentration. The experimental results are shown in Table 1.

Table 1. $IC_{50}$ (μM)

| Compound | HCT116 WT | HCT116 MTAP-/- | Selectivity | Compound | HCT116 WT | HCT116 MTAP-/- | Selectivity |
|---|---|---|---|---|---|---|---|
| 1 | 1.802 | 0.0129 | 140 | 2 | 1.026 | 0.0139 | 74 |
| 3 | 4.312 | 0.027 | 160 | 4 | 1.322 | 0.008 | 165 |
| 4A | 0.872 | 0.0045 | 194 | 5 | 0.584 | 0.006 | 97 |
| 5A | 0.170 | 0.0017 | 100 | 6 | 2.777 | 0.0108 | 257 |
| 7 | 0.593 | 0.0054 | 110 | 8 | 0.141 | 0.003 | 47 |
| 9 | 0.0977 | 0.0009 | 109 | 9A | 0.06 | 0.0012 | 50 |
| 10 | 0.162 | 0.002 | 81 | 10A | 0.028 | 0.0005 | 56 |
| 11 | 0.185 | 0.003 | 62 | 12 | 0.650 | 0.008 | 81 |
| 12A | 0.426 | 0.0037 | 115 | 13 | 0.607 | 0.011 | 55 |
| 14 | - | 0.009 | - | 15 | - | 0.0048 | - |
| / | / | / | / | 16A | 1.165 | 0.0139 | 84 |
| 17 | 0.405 | 0.007 | 58 | 18 | 0.159 | 0.003 | 53 |
| 19 | 0.986 | 0.0097 | 102 | 19A | 1.295 | 0.0087 | 149 |
| 20 | 0.948 | 0.0098 | 97 | 20A | 1.039 | 0.0061 | 170 |
| 21 | 0.365 | 0.007 | 52 | 22 | 0.095 | 0.0019 | 50 |
| 21A | 0.233 | 0.0027 | 86.3 | 22A | 0.055 | 0.001 | 55 |
| 23 | 0.307 | 0.004 | 77 | 24 | 0.115 | 0.0019 | 61 |
| 23A | 0.507 | 0.0031 | 164 | 24A | 0.214 | 0.0027 | 79 |
| 25 | 0.471 | 0.0047 | 100 | 26 | - | 0.0028 | - |
| 25A | 0.249 | 0.0018 | 138 | 26A | - | 0.0009 | - |
| 27 | 0.185 | 0.0032 | 58 | 28 | - | 0.0023 | - |
| 29 | 1.498 | 0.0098 | 153 | 30 | 1.753 | 0.0087 | 201 |
| 29A | 0.480 | 0.0036 | 133 | 30A | 0.240 | 0.0029 | 83 |
| 31 | 1.455 | 0.0142 | 102 | 32 | 2.271 | 0.022 | 103 |
| 31A | 0.192 | 0.0033 | 58 | 33 | 0.567 | 0.0044 | 129 |
| 34 | 1.14 | 0.0076 | 150 | 34A | 0.266 | 0.002 | 133 |
| 36 | 1.73 | 0.0137 | 126 | 37 | 2.04 | 0.022 | 93 |
| 39 | 2.965 | 0.020 | 148 | 40 | 1.461 | 0.0058 | 252 |
| 39A | - | 0.011 | - | 40A | 0.483 | 0.0046 | 105 |
| 41 | 1.782 | 0.0138 | 129 | 42 | 0.785 | 0.0185 | 42 |
| 43A | 0.646 | 0.0038 | 170 | 44 | 0.736 | 0.011 | 67 |
| 45 | 0.654 | 0.0059 | 111 | 45A | 0.213 | 0.004 | 53 |
| 46 | 0.467 | 0.003 | 156 | 46A | 0.158 | 0.0011 | 144 |
| 47 | 0.56 | 0.0074 | 76 | 47A | 0.598 | 0.0052 | 115 |
| 48A | 0.141 | 0.002 | 71 | 49A | 0.378 | 0.0018 | 210 |
| 50A | 0.431 | 0.0024 | 180 | 51A | - | 0.001 | - |
| 52A | 0.487 | 0.0032 | 152 | 53A | 0.045 | 0.001 | 45 |

(continued)

| Compound | HCT116 WT | HCT116 MTAP-/- | Selectivity | Compound | HCT116 WT | HCT116 MTAP-/- | Selectivity |
|---|---|---|---|---|---|---|---|
| 54A | - | 0.0055 | - | 55A | 0.217 | 0.005 | 43 |
| 56A | 0.16 | 0.003 | 53 | 57A | 1.002 | 0.008 | 125 |
| 58A | 2.222 | 0.0095 | 234 | 59A | 0.305 | 0.0046 | 66 |
| 60A | 0.486 | 0.008 | 61 | 61A | 0.64 | 0.007 | 91 |
| 62A | 1.264 | 0.0159 | 79 | 63A | 0.085 | 0.002 | 43 |
| 64A | - | 0.003 | - | 65A | 0.141 | 0.002 | 71 |
| 66A | 0.299 | 0.002 | 150 | 67A | 0.085 | 0.002 | 43 |
| 68A | 0.312 | 0.0066 | 47 | 69A | 0.864 | 0.005 | 173 |
| 70A | 0.475 | 0.0036 | 132 | 71A | 0.674 | 0.014 | 48 |
| 72 | - | 0.006 | - | 73A | - | 0.011 | - |
| 74A | 0.472 | 0.0027 | 175 | 75A | 0.424 | 0.004 | 106 |
| 76A | 0.167 | 0.003 | 56 | 77A | - | 0.011 | - |
| 78A | 0.512 | 0.0039 | 131 | 79A | 0.304 | 0.002 | 152 |
| 80A | 1.506 | 0.006 | 251 | 81A | 0.122 | 0.0027 | 45 |
| 82A | 0.196 | 0.0026 | 75 | 83A | 0.062 | 0.001 | 62 |
| 84A | 0.143 | 0.0026 | 55 | 85 | 0.165 | 0.003 | 55 |
| 86A | 1.077 | 0.0087 | 124 | 87 | 0.504 | 0.009 | 56 |
| 88A | 0.777 | 0.0045 | 173 | 89A | 0.263 | 0.003 | 88 |
| 90A | 1.181 | 0.012 | 98 | 91A | 1.388 | 0.0071 | 195 |
| Reference Example 7 | 2.009 | 0.0974 | 21 | | | | |

The results show that the specific compounds and isomers thereof exemplified herein have strong inhibitory activity against the proliferation of the human MTAP-deleted HCT-116 cell strain, and most of the exemplified compounds have $IC_{50}$ values less than 0.01 μM for the human MTAP-deleted HCT-116 cell strain. In terms of selectivity, each of the specific compounds and isomers thereof exemplified herein has relatively good selective inhibition on the human MTAP-deleted HCT-116 cell strain relative to the HCT-116 cell strain with wild-type (WT) MTAP, and the selective inhibition of most compounds is more than 90 times.

**Experimental Example 2: Human Liver Microsome Stability Experiment**

**Experimental materials:** human liver microsomes (purchased from BIOIVT);

**Preparation of reagents:**

PBS: 0.1 M $KH_2PO_4$ and $K_2HPO_4$ buffers, pH 7.4.
$MgCl_2$: a certain amount of $MgCl_2$ was weighed out and prepared into a 16 mM $MgCl_2$ solution with PBS.
NADPH (Chinese name: reduced nicotinamide adenine dinucleotide phosphate, purchased from Sigma, Cat. No.: 481973-500 mg): a certain amount of NADPH was weighed out and a 4 mM solution of NADPH was prepared with a 16 mM $MgCl_2$ solution, and the final incubation concentration was 1 mM.
Compounds: 4 μM solutions of the test compounds and positive compounds were prepared with PBS, and the final incubation concentration was 1 μM.
Liver microsomes (purchased from BIOIVT, Cat. No.: X008070): the liver microsomes were diluted to 1 mg/mL with PBS, and the final incubation concentration was 0.5 mg/mL.

**Experimental procedures:**

The prepared test compound or positive compound was added to a test tube, and then the prepared NADPH was added. The mixture was mixed well. The mixture was pre-incubated in a thermostat incubator at 220 rpm at 37 °C for 5 min, and the prepared liver microsomes were added to start the reaction. All steps were performed in replicate wells. At 0 min, 15 min, 30 min, 45 min, and 60 min, a certain volume of glacial acetonitrile solution containing an internal standard was added to the corresponding tube to precipitate the protein. The mixture was vortexed for 5 min with shaking, and then centrifuged at 4000 rpm for 10 min. The supernatant was collected and added to a 96-well plate. The samples were analyzed by LC-MS/MS.

The concentrations (peak area ratios) of the example compounds were determined by LC-MS/MS. Rate constants were obtained by plotting "Ln (residual amount of compound %)" versus "incubation time" in Excel, thereby calculating the half-lives and intrinsic clearances of the drugs and providing a basis for the prediction of the in-vivo clearance. The experimental results are shown in Table 2.

**Data analysis:**

$CL_{int}$ = (0.693/$t_{1/2}$, microsome) × [incubation solution volume (mL)/microsome protein mass (mg)] × [microsome protein mass (mg)/liver mass (g)] × [liver mass (g)/body weight (kg)][1]

$$CL_H = CL_{int} \times f_u \times Q_h/(CL_{int} \times f_u + Q_h)$$

In the formula,

$CL_{int}$--Intrinsic clearance (mL/min/kg)
$CL_H$--Hepatic clearance (mL/min/kg)
$f_u$--Plasma protein unbound fraction of 1
$Q_h$--Hepatic blood flow

Table 2. Stability in human liver microsomes

| Compound | T1/2 (min) | Compound | T1/2 (min) |
|---|---|---|---|
| Reference Example 1 | 16.6 | Example 4 | 169 |
| Reference Example 2 | 33.6 | Compound 5A | 66.5 |
| Reference Example 5 | 25.6 | Compound 25A | 68.9 |
| Reference Example 7 | 28.9 | Compound 40A | 62.9 |
| Compound 50A | 49.9 | Compound 43A | 122 |
| | | Compound 91A | 47.2 |

Table 2 shows that the compounds of the present disclosure have excellent stability in human liver microsomes.

**Experimental Example** 3: hERG Test

**Experimental reagents:**

Predictor™ hERG Fluorescence Polarization Assay Kit (ThermoFisher Scientific, PV5365).

**Experimental procedures:**

The procedures in the kit instructions were followed: 4× Predictor™ hERG Tracer Red, 4× E-4031, and 4× test substance solutions (final assay concentration: 10 μM) were prepared in advance. 5 μL Predictor™ hERG FP Assay Buffer

and 5 μL Predictor™ hERG Membrane were added sequentially to the Assay Blank group in a 384-well plate. 2.5 μL Predictor™ hERG FP Assay Buffer, 2.5 μL of 4× E-4031, and 2.5 μL of 4× test substance were added to the negative control group, the positive control group, and the test substance group, respectively, and then 5 μL Predictor™ hERG Membrane and 2.5 μL of 4× Predictor™ hERG Tracer Red were sequentially added. The total volume of the experimental system was 10 μL, and three duplicate wells were set for each group. After loading, the plate was gently tapped to mix well and incubated at room temperature (25 °C) in the dark for 3.5 h. After the incubation was completed, the fluorescence polarization value mP was measured on a microplate reader, and G Factor was 2.14.

**Data analysis:**

The inhibition rate of the test substance was calculated using the following formula:

Inhibition rate % = (mP negative control - mP test substance)/(mP negative control - mP positive control) × 100%.

Using the experimental method described above, the experimental results of hERG inhibition of some compounds of the present disclosure are shown in Table 3 below.

Table 3. hERG test

| Compound | Inhibition (10 μM) | $IC_{50}$ (μM) | Compound | Inhibition (10 μM) | $IC_{50}$ (μM) |
|---|---|---|---|---|---|
| Reference Example 6 | 77.39%±23.77% | < 10 | Compound 43A | 30.33±29.9% | > 10 |
| Compound 5A | 27.67%±11.38% | > 10 | Compound 45 | 20.97%±14.19% | > 10 |
| Compound 9A | 35.70%±3.15% | > 10 | Compound 46A | 43.93 ±18.76% | > 10 |
| Compound 12A | 22.42+1.73% | > 10 | Compound 50A | 22.42+25.47% | > 10 |
| Compound 23A | 39.48%±13.86% | > 10 | Compound 52A | 10.4±49.06% | > 10 |
| Compound 25A | 32.97%±23.29% | > 10 | Compound 57A | 14.15±18% | > 10 |
| Compound 39 | 23.78%±11.14% | > 10 | Compound 66A | 14.22±13.96% | > 10 |
| Compound 40 | 22.04%±1.45% | > 10 | | | |

Table 3 shows that the compounds of the present disclosure have lower hERG inhibition toxicity than the prior art (e.g., Reference Example 6).

**Experimental Example 4: CYP Inhibition Test**

**Experimental materials:** human liver microsomes (purchased from BIOIVT);

Preparation of reagents:

PBS: 0.1 M $KH_2PO_4$ and $K_2HPO_4$ buffers, pH 7.4.
$MgCl_2$: a certain amount of $MgCl_2$ was weighed out and prepared into a 20 mM $MgCl_2$ solution with PBS.
NADPH (Chinese name: reduced nicotinamide adenine dinucleotide phosphate, purchased from Sigma, Cat. No.: 481973-500 mg): a certain amount of NADPH was weighed out and a 5 mM solution of NADPH was prepared with a 20 mM $MgCl_2$ solution, and the final incubation concentration was 1 mM.
Compounds: 3 mM, 1 mM, 0.3 mM, 0.1 mM, 0.03 mM, 0.01 mM, and 0 mM solutions of the test compounds were prepared with a DMSO-methanol (v:v = 1:9) solution, and the final incubation concentrations were 30 μM, 10 μM, 3 μM, 1 μM, 0.3 μM, 0.1 μM, and 0 μM. 30 μM, 10 μM, 3 μM, 1 μM, 0.3 μM, and 0 μM solutions of the positive compound (ketoconazole) were prepared with a DMSO-methanol (v:v = 1:9) solution, and the final incubation concentrations were 0.3 μM, 0.1 μM, 0.03 μM, 0.01 μM, 0.003 μM, and 0 μM.
Substrate working solutions: 200 μM and 15 μM solutions of substrate working solutions (testosterone and midazolam) were prepared with PBS, separately, and the final incubation concentrations were 40 μM and 3 μM.
Liver microsomes (purchased from BIOIVT, Cat. No.: X008070): the liver microsomes were diluted to 0.17 mg/mL with PBS, and the final incubation concentration was 0.1 mg/mL.

Experimental procedures:

The prepared test compounds or positive compounds were added to a 96-well plate, and then the prepared human liver microsome working solution and substrate working solution were added. The mixture was mixed well. The mixture was pre-incubated in a thermostat incubator at 37 °C for 5 min, and then the prepared NADPH was added to start the reaction. The mixture was incubated for 5 min or 10 min. After the incubation was completed, a pre-cooled acetonitrile solution containing an internal standard was added to terminate the reaction. After protein precipitation, the mixture was vortexed for 5 min with shaking and then centrifuged at 4000 rpm for 10 min. The supernatant was collected and added to the 96-well plate. The content (peak concentration ratio) of the metabolites of the substrate was determined by an LC-MS/MS method. The residual activity and the half maximal inhibitory concentration ($IC_{50}$) of the enzyme were calculated based on the detected content.

Data analysis:

Percentage of remaining enzyme activity:
The activity of each subtype of CYP450 enzyme was expressed as the amount of metabolite produced, and the percentage of remaining enzyme activity was calculated as follows:

Remaining activity (%) = (activity of the CYP450 enzyme subtype in the presence of the test substance ÷ activity of the solvent control group) × 100%, and the activity of the solvent control group (0 μM) was set as 100%.

$IC_{50}$:

The concentrations of the test substances and the corresponding residual activity percentages were fitted using a nonlinear model of Excel software, and the concentration of the test substances at which 50% inhibition of metabolite production was achieved was the $IC_{50}$ value. The experimental results are shown in Table 4.

Table 4

| Compound | $IC_{50}$ (μM) | |
|---|---|---|
| | 3A4-Midazolam | 3A4-Testosterone |
| Reference Example 6 | 2.13 | 1.95 |
| Compound 5A | >30 | >30 |
| Compound 23A | >30 | >30 |
| Compound 25A | >30 | >30 |
| Compound 40A | >30 | >30 |
| Compound 43A | >30 | >30 |
| Compound 50A | >30 | >30 |

Table 4 shows that the compounds of the present disclosure have lower CYP inhibitory toxicity than the prior art (e.g., Reference Example 6).

**Experimental Example 5:** PK Experiment in Mice

**Experimental materials:** the experimental animals were healthy adult female BALB/c mice (provided by Sichuan Vital River Laboratory Animal Technology Co., Ltd.).

**Procedures:**

Administration mode and sample collection in mice: BALB/c female mice received oral intragastrical administration (10 mg/kg, vehicle: 0.5% methylcellulose/0.2% Tween 80), and 60 μL of whole blood from the venous plexus of the fundus of the mice at different time points was collected at 0.25 h, 0.5 h, 1 h, 2 h, 4 h, 6 h, 8 h, and 24 h after administration and centrifuged at 4000 rpm for 6 min to collect plasma.

**Sample analysis:**

10 μL of mouse plasma sample was added to 190 μL of acetonitrile solution containing an internal standard to precipitate protein. The mixture was vortexed for 10 min and centrifuged at 4000 rpm for 10 min. The supernatant was collected and added to a 96-well plate. The samples were analyzed by LC-MS/MS. The drug concentrations in the plasma of mice at different time points after intragastric administration of the example compounds were measured by an LC-MS/MS method, and the related pharmacokinetic parameters were calculated, such that the pharmacokinetic behavior of the compounds in the mice was studied, and the pharmacokinetic characteristics were evaluated. The experimental results are shown in Table 5.

Table 5. Pharmacokinetic parameters of the compounds of the present disclosure for mice

| **Compound**[1] | $C_{max}$ **(ng/mL)** | **AUC$_{last}$ (h*ng/mL)** | **Compound** [1] | $C_{max}$ **(ng/mL)** | **AUC$_{last}$ (h*ng/mL)** |
|---|---|---|---|---|---|
| Reference Example 3 | 741±601 | 5131±2925 | Compound 39 | 2667±180 | 20888±8399 |
| Reference Example 4 | 649±45 | 3268±588 | Compound 40A | 793 ±28 | 10070±3504 |
| Reference Example 5 | 1400±165 | 9083±2788 | Compound 43A | 3420±913 | 32339±3341 |
| Reference Example 6[2] | 428±220 | 1970±316 | Compound 52A | 1503±313 | 14200±2432 |
| Reference Example 7 | 1923±76 | 12626±1915 | Compound 57A | 1810±195 | 10534±2400 |
| Compound 4 | 634±71 | 9154±2244 | Compound 61A | 2077±299 | 22184±2713 |
| Compound 5A | 2223±230 | 15699±1601 | Compound 66A | 3827±533 | 23153±3387 |
| Compound 9A | 4500±147 | 24341±3342 | Compound 74A | 2807±780 | 14527±1756 |
| Compound 12A | 2673±421 | 26904±7453 | Compound 75A | 5013±846 | 26976±4260 |
| Compound 16A | 1600±352 | 23324±5524 | Compound 82A | 1433±195 | 14072±4741 |
| Compound 23A | 1967±508 | 19401±7262 | Compound 86A | 1480±187 | 15339±2935 |
| Compound 30 | 984±123 | 12607±1363 | | | |
| Note: 1. The vehicle was 0.5% MC/0.2% Tween 80, with PO administration.<br>2. Reference was made to J. Med. Chem. 2022, 65(3), 1749-1766 for the test method of PK in MRTX1719 mice. | | | | | |

Table 5 shows that the compounds provided herein have excellent pharmacokinetic properties in mice.

**Experimental Example 6:** PK Experiment in Rats

**Experimental materials:** the experimental animals were healthy adult SD male rats (provided by Sichuan Vital River Laboratory Animal Technology Co., Ltd.).

**Procedures:**

Administration mode and sample collection in rats: SD male rats received oral intragastrical administration (10 mg/kg, vehicle: 0.5% methylcellulose/0.2% Tween 80), and 200 μL of whole blood from the venous plexus of the fundus of the rats at different time points was collected at 0.25 h, 0.5 h, 1 h, 2 h, 4 h, 6 h, 8 h, and 24 h after administration and centrifuged at 4000 rpm for 6 min to collect plasma.

**Sample analysis:**

10 μL of rat plasma sample was added to 190 μL of acetonitrile solution containing an internal standard to precipitate protein. The mixture was vortexed for 10 min and centrifuged at 4000 rpm for 10 min. The supernatant was collected and added to a 96-well plate. The samples were analyzed by LC-MS/MS. The drug concentrations in the plasma of rats at different time points after intragastric administration of the example compounds were measured by an LC-MS/MS method, and the related pharmacokinetic parameters were calculated, such that the pharmacokinetic behavior of the compounds in the rats was studied, and the pharmacokinetic characteristics were evaluated. The experimental results are shown in Table 6.

Table 6. Pharmacokinetic parameters for rats

| **Compound**[1] | $C_{max}$ | $AUC_{last}$ | **Compound**[1] | $C_{max}$ | $AUC_{last}$ |
|---|---|---|---|---|---|
| | **(ng/mL)** | **(h*ng/mL)** | | **(ng/mL)** | **(h*ng/mL)** |
| Reference Example 2 | 131±17 | 784±595 | Compound 5A | 2177±777 | 15583±4638 |
| Reference Example 5 | 549±139 | 2940±438 | Compound 16A | 1280±270 | 13731±2775 |
| Reference Example 6 | 107±149 | 100±103 | Compound 23A | 1163±181 | 6330±2495 |
| Reference Example 7 | 477±110 | 853±79 | Compound 43A | 1221±417 | 9653±1169 |
| Note: 1. The vehicle was 0.5% MC/0.2% Tween 80, with PO administration. | | | | | |

Table 6 shows that the compounds provided herein have excellent pharmacokinetic properties in rats.

**Experimental Example 7: Efficacy Evaluation in Gene-Editing MTAP-/- Human Colon Cancer Cell Strain HCT116 Subcutaneous Xenograft Mouse Model**

**Experimental materials:**

The gene-editing MTAP-/- human colon cancer cell strain HCT116 (purchased from Nanjing Cobioer) was cultured with a McCoys 5A medium (Invitrogen) containing 10% fetal bovine serum (Gibco);
the administration was performed after the test substances were prepared into suspensions at concentrations of 0.3 mg/mL and 1 mg/mL using a 0.5% MC + 0.2% Tween 80 aqueous solution. The administration was performed after Reference Example 6 (MRTX1719) was prepared into a 5 mg/mL clear solution using 40% PEG400 normal saline containing 2% DMSO. The blank group contained 40% PEG400 normal saline with 2% DMSO.

**Experimental method:**

HCT116 MTAP-/- cells in the logarithmic growth phase were digested, collected, and counted, and the cell density was adjusted to 1 × $10^8$ cells/mL using a serum-free medium. 20-22 g of Balb/c nude mice (Beijing Huafukang Biotechnology Co., Ltd.) were inoculated subcutaneously with 100 μL of cell suspension per mouse. On day 10 after inoculation, animals with tumor volumes of 100-150 $mm^3$ were randomly grouped, with 8 animals in each group, according to the body weight and the tumor volume. The test substance was intragastrically administered at 10 mg/kg once daily, and MRTX1719 was intragastrically administered at 50 mg/kg once daily. Model control: The vehicle (2% DMSO + 40% PEG400 + 58% normal saline) was intragastrically administered at a dose of 10 mL/kg once daily. Administration was performed for 28 days in total.
After the animals were inoculated with HCT116 MTAP-/- cells, the body weight and tumor volume of the mice were measured 2-3 times a week, and the tumor volume was calculated based on length × $width^2$ × 0.5. The mice were euthanized on the day when the last administration was completed.
The tumor growth inhibition TGI of each administration group was calculated based on the following formula: TGI (tumor volume) was calculated as follows: $TGI_{TV} = 1 - ((V_{Tt} - V_{T0})/(V_{Ct} - V_{C0})) \times 100\%$. In the formula, $V_{Tt}$ represents the tumor volume of the treatment group at each measurement, and $V_{T0}$ represents the tumor volume measured when the treatment group is grouped; $V_{Ct}$ represents the tumor volume of the vehicle group at each measurement (same as $V_{Tt}$), and $V_{C0}$ represents the tumor volume measured when the vehicle group is grouped.
Data were analyzed using GraphPad Prism 9.0 software and expressed as Mean ± SEM (standard error). Pairwise comparisons between each administration group and the control group were performed using one-way analysis of variance (ANOVA). Variance homogeneity was determined using the Brown-Forsythe method, and variance heterogeneity was determined using the Dunnett's T3 method.

Table 7. Efficacy evaluation in HCT116 subcutaneous xenograft mouse model

| Test substance | Dose of administration | Tumor volume ($mm^3$) | TGI |
|---|---|---|---|
| Blank | - | 1580.00 | - |
| MRTX1719 | 50 mg/kg, PO, qd | 525.74 | 72.21% |
| Compound 5A | 10 mg/kg, PO, qd | 378.72 | 82.25% |
| Compound 23A | 10 mg/kg, PO, qd | 423.62 | 79.22% |

(continued)

| Test substance | Dose of administration | Tumor volume ($mm^3$) | TGI |
|---|---|---|---|
| Compound 25A | 10 mg/kg, PO, qd | 534.36 | 71.59% |

As shown in Table 7, the in-vivo anti-tumor effect of the compound of the present disclosure in the HCT116 subcutaneous xenograft mouse model is significantly better than that of the clinical molecule MRTX1719.

**Experimental Example 8:** Efficacy Evaluation in Xenograft Mouse Model of Human Large Cell Lung Cancer LU99

**Experimental materials:**

The human large cell lung cancer cell line LU99 was purchased from Nanjing Cobioer Biosciences Co., Ltd. (CBP61512) and cultured in RPMI-1640 medium (Gibco) containing 10% fetal bovine serum (Gibco);
the test substances were prepared into suspensions at concentrations of 0.3 mg/mL, 1 mg/mL, and 5 mg/mL using a 0.5% aqueous MC solution containing 0.2% Tween 80. Reference Example 6 (MRTX1719) was prepared into a 5 mg/mL clear solution using 40% PEG400 normal saline containing 2% DMSO.

**Experimental method:**

LU99 cells in the logarithmic growth phase were digested, collected, and counted, and the cell density was adjusted to $2 \times 10^7$ cells/mL using a 1:1 serum-free medium and matrigel (Corning). 18-20 g of Balb/c nude mice (Beijing Huafukang Biotechnology Co., Ltd.) were inoculated subcutaneously with 100 μL of cell suspension per mouse. On day 12 after inoculation, animals with a mean tumor volume of 116 $mm^3$ were randomly grouped, with 7 animals in each group. The test substances were intragastrically administered at 3 mg/kg, 10 mg/kg, and 50 mg/kg once daily, and MRTX1719 was intragastrically administered at 50 mg/kg once daily. A vehicle (2% DMSO + 40% PEG400 + 58% normal saline) was intragastrically administered to the blank group at a dose of 10 mL/kg once daily. Administration was performed for 28 days in total.

After the animals were inoculated with LU99 cells, the tumor volume was measured 2-3 times a week, and the tumor volume was calculated based on length $\times$ width$^2$ $\times$ 0.5. On the day when the last administration was completed, except for the mice with tumor regression, which were subjected to discontinuation observation, the remaining mice were euthanized.

The tumor growth inhibition TGI of each administration group was calculated based on the following formula: TGI (tumor volume) was calculated as follows: $TGI_{TV} = 1 - ((V_{Tt} - V_{T0})/(V_{Ct} - V_{C0})) \times 100\%$. In the formula, $V_{Tt}$ represents the tumor volume of the treatment group at each measurement, and $V_{T0}$ represents the tumor volume measured when the treatment group is grouped; $V_{Ct}$ represents the tumor volume of the model control group at each measurement, and $V_{C0}$ represents the tumor volume measured when the model control group is grouped.

Data were analyzed using GraphPad Prism 9.0 software and expressed as Mean ± SEM (standard error). Pairwise comparisons between each administration group and the model control group were performed using one-way analysis of variance (ANOVA). Variance homogeneity was determined using the Brown-Forsythe method, and variance heterogeneity was determined using the Dunnett's T3 method.

Table 8. Efficacy evaluation in LU99 xenograft mouse model

| Test substance | Dose of administration | Tumor volume ($mm^3$) | TGI |
|---|---|---|---|
| Blank | - | 1911.67 | - |
| MRTX1719 | 50 mg/kg, PO, qd | 279.68 | 90.96% |
| Compound 5A | 3 mg/kg, PO, qd | 438.73 | 81.96% |
| | 10 mg/kg, PO, qd | 202.05 | 95.20% |
| | 50 mg/kg, PO, qd | 60.72 | 103.12% |
| Compound 43A | 3 mg/kg, PO, qd | 415.58 | 83.27% |
| | 10 mg/kg, PO, qd | 210.31 | 94.77% |
| | 50 mg/kg, PO, qd | 28.13 | 104.92% |

Results and analysis:

The experimental results are shown in Table 8. The compounds of the present disclosure can significantly reduce the tumor volume at 3 mg/kg, 10 mg/kg, and 50 mg/kg ($P < 0.0001$). The anti-tumor effect of the test substances at 10 mg/kg and 50 mg/kg is significantly better than that of MRTX1719 at 50 mg/kg, and the test substances at 50 mg/kg can cause tumor regression.

In conclusion, the experimental results show that, in the human large cell lung cancer LU99 CDX model, the test substances of the present disclosure have a relatively good anti-tumor effect, and the anti-tumor efficacy is significantly superior to that of the positive control at a relatively low dose.

**Industrial applicability**

The compounds of the present disclosure exhibit excellent inhibitory activity against PRMT5. The compounds of the present disclosure demonstrate excellent selective inhibition against the human MTAP-deficient HCT-116 cell line over the MTAP wild-type (WT) HCT-116 cell line. The compounds can be used in the development of small-molecule drugs targeting PRMT5 MTA and preferentially act on MTAP-deficient tumor cells.

Furthermore, through tests such as human liver microsome stability, mouse pK, rat pK, and in-vivo anti-tumor efficacy evaluation in mice, it can be found that the compounds of the present disclosure have excellent anti-tumor application prospects. Further, compared with existing compounds, the compounds of the present disclosure possess a higher safety window, and are significantly superior to the prior art in terms of safety (e.g., toxicity from inhibition of hERG, CYP, etc.).

In conclusion, compared with the prior art, the compounds of the present disclosure have achieved unexpected technical effects in the aspects of druggability and safety, and have great development potential.

The above description merely represents preferred embodiments of the present disclosure. It should be noted that those of ordinary skill in the art can make several improvements and modifications without departing from the principles of the present disclosure, and these improvements and modifications shall fall within the protection scope of the present disclosure.

Reference

[1] Davies B, Morris T. Physiological parameters in laboratory animals and humans. Pharm Res. 1993; 10: 1093-5

**Claims**

1. A compound represented by formula I or a tautomer, a stereoisomer, a pharmaceutically acceptable salt, or a deuterated compound thereof:

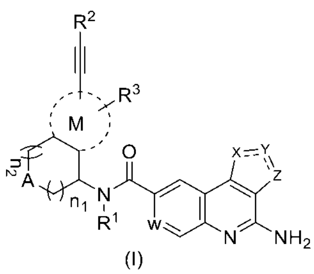

(I)

wherein

$\overline{- - -}$ is selected from a single bond and a double bond, or $\overline{- - -}$ is absent;
when $\overline{- - -}$ is a single bond or a double bond, X, Y, and Z are independently selected from $CR^4R^5$, $NR^6$, O, N, S, and $CR^4$;
when $\overline{- - -}$ is absent, Y is absent, X is hydrogen, and Z is $CR^4R^5$;
W is selected from $CR^7$ and N;
A is selected from $CR^8R^9$, $NR^8$, and O;

ring M is selected from 5-10 membered aryl and 5-10 membered heteroaryl, wherein the 5-10 membered heteroaryl contains 1-4 heteroatoms selected from N, O, and S;

$R^3$, $R^4$, $R^5$, $R^6$, $R^7$, $R^8$, and $R^9$ are independently selected from hydrogen, deuterium, halogen, cyano, $C_{1-6}$ alkyl, $C_{1-6}$ alkoxy, $C_{3-6}$ cycloalkyl, and $C_{3-6}$ cycloalkoxy, wherein the $C_{1-6}$ alkyl and $C_{1-6}$ alkoxy are unsubstituted or substituted with one or more $R_i$, and the $C_{3-6}$ cycloalkyl and $C_{3-6}$ cycloalkoxy are unsubstituted or substituted with one or more $R_j$; $R^1$ is selected from $C_{1-6}$ alkyl, $C_{3-10}$ cycloalkyl-($C_{1-6}$ alkyl)$_m$-, 4-10 membered heterocyclyl-($C_{1-6}$ alkyl)$_n$-, 5-10 membered heteroaryl-($C_{1-6}$ alkyl)$_p$-, and 5-10 membered aryl-($C_{1-6}$ alkyl)$_q$-, wherein n = 0 or 1, m = 0 or 1, p = 0 or 1, and q = 0 or 1; the heterocyclyl and heteroaryl each independently contain 1-4 ring heteroatoms selected from O, N, and S, wherein the $C_{1-6}$ alkyl is unsubstituted or substituted with one or more $R_i$, the $C_{3-10}$ cycloalkyl is unsubstituted or substituted with one or more $R_j$, the 4-10 membered heterocyclyl is unsubstituted or substituted with one or more $R_k$, and the 5-10 membered aryl and 5-10 membered heteroaryl are unsubstituted or are each independently substituted with one or more $R_l$;

$R^2$ is selected from 5-10 membered heteroaryl and 4-10 membered heterocyclyl, wherein the 5-10 membered heteroaryl and 4-10 membered heterocyclyl each independently contain 1-4 heteroatoms selected from N, O, and S, and the 5-10 membered heteroaryl and 4-10 membered heterocyclyl are unsubstituted or substituted with one or more identical or different $R^b$;

$n_1$ and $n_2$ are each independently 0, 1, or 2;

each $R_i$, $R_j$, $R_k$, and $R_l$ is independently selected from hydroxy, deuterium, halogen, cyano, $C_{1-6}$ alkyl optionally substituted with one or more groups selected from halogen, cyano, and hydroxy, $C_{1-6}$ alkoxy optionally substituted with one or more groups selected from halogen, cyano, hydroxy, -NR'R", and $C_{1-6}$ alkoxy, $C_{3-10}$ cycloalkyl, and 5-10 membered heteroaryl optionally substituted with one or more $C_{1-6}$ alkyl, the 5-10 membered heteroaryl contains 1-4 heteroatoms selected from N, O, and S, wherein R' and R" are each independently selected from H, $C_{1-6}$ alkyl, and halogenated $C_{1-6}$ alkyl, or R' and R", together with the nitrogen atom attached thereto, form a 4-8 membered heterocyclyl, wherein the 4-8 membered heterocyclyl contains1-4 heteroatoms selected from N, O, and S;

each $R^b$ is independently selected from hydroxy, deuterium, cyano, halogen, -NR'R", $C_1$-$C_6$ alkyl, $C_{1-6}$ alkoxy, and $C_3$-$C_6$ cycloalkyl, wherein the $C_1$-$C_6$ alkyl, $C_1$-$C_6$ alkoxy, and $C_3$-$C_6$ cycloalkyl are unsubstituted or are each independently substituted with one or more substituents selected from halogen, hydroxy, cyano, and $C_1$-$C_6$ alkoxy; in -NR'R", R' and R" are each independently selected from hydrogen, $C_{1-6}$ alkyl, and halogenated $C_{1-6}$ alkyl, or R' and R", together with the nitrogen atom attached thereto, form a 4-8 membered heterocyclyl, wherein the 4-8 membered heterocyclyl contains 1-4 heteroatoms selected from N, O, and S.

**2.** The compound or the tautomer, the stereoisomer, the pharmaceutically acceptable salt, or the deuterated compound thereof according to claim 1, wherein

ring M is selected from 5-6 membered aryl and 5-6 membered heteroaryl;

preferably, ring M is selected from phenyl, pyridyl, pyrazinyl, pyrimidinyl, pyridazinyl, pyrazolyl, imidazolyl, triazolyl, pyrrolyl, furanyl, oxazolyl, isoxazolyl, and thiazolyl;

further preferably, ring M is selected from phenyl and pyridyl;

preferably, ring A is selected from O;

preferably, $n_1$ and $n_2$ are each independently 0 or 1;

preferably, the group

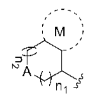

is selected from

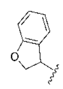 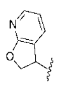 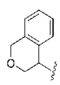 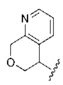 ;

preferably, the group

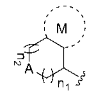

is selected from

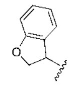;

preferably, the group

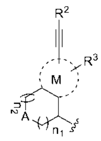

is selected from

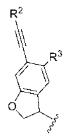 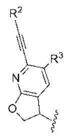 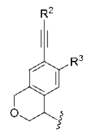 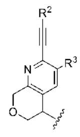;

preferably, the group

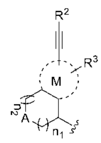

is selected from

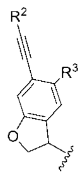.

**3.** The compound or the tautomer, the stereoisomer, the pharmaceutically acceptable salt, or the deuterated compound thereof according to claim 1, wherein

$R^2$ is selected from 5-6 membered heteroaryl and 4-6 membered heterocyclyl, wherein the 5-6 membered heteroaryl and 4-6 membered heterocyclyl each independently contain 1-3 heteroatoms selected from N, O, and S, and the 5-6 membered heteroaryl and 4-6 membered heterocyclyl are unsubstituted or are each independently substituted with one or more identical or different $R^b$; or
$R^2$ is selected from 5-6 membered heteroaryl, 4-6 membered heterocyclyl, and 5-6 membered heteroaryl-fused 5-6 membered heterocyclyl, wherein the 5-6 membered heteroaryl, 4-6 membered heterocyclyl, and 5-6 membered heterocyclyl each independently contain 1, 2, or 3 heteroatoms selected from N, O, and S, the 5-6 membered heteroaryl, 4-6 membered heterocyclyl, and 5-6 membered heteroaryl-fused 5-6 membered heterocyclyl are unsubstituted or are each independently substituted with one or more identical or different $R^b$; preferably, $R^2$ is selected from 5-6 membered heteroaryl, wherein the 5-6 membered heteroaryl contains 1-3 heteroatoms selected from N, O, and S, and the 5-6 membered heteroaryl is unsubstituted or substituted with one or more identical or different $R^b$; preferably, $R^2$ is selected from pyrazolyl, imidazolyl, thiazolyl, isothiazolyl, oxazolyl, isoxazolyl, pyridyl, pyrazinyl, pyrimidinyl, and pyridazinyl, which are unsubstituted or are each independently substituted with 1, 2, or 3 $R^b$; or

preferably, $R^2$ is selected from 5-6 membered heteroaryl, wherein the 5-6 membered heteroaryl contains 1, 2, or 3 N heteroatoms, and the 5-6 membered heteroaryl is unsubstituted or substituted with one or more identical or different $R^b$;

preferably, $R^2$ is selected from pyrazolyl, imidazolyl, and pyridyl, which are unsubstituted or are each independently substituted with 1, 2, or 3 $R^b$;

preferably, $R^2$ is selected from

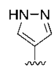 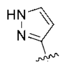 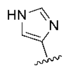 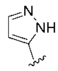 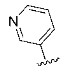,

which are unsubstituted or independently substituted with 1, 2, or 3 $R^b$; or

preferably, $R^2$ is selected from 5-membered heteroaryl, wherein the 5-membered heteroaryl contains 1-3 heteroatoms selected from N, O, and S, and the 5-membered heteroaryl is unsubstituted or substituted with 1, 2, or 3 $R^b$;

preferably, $R^2$ is selected from pyrazolyl, imidazolyl, thiazolyl, isothiazolyl, oxazolyl, and isoxazolyl, which are unsubstituted or substituted with 1, 2, or 3 $R^b$; or

preferably, $R^2$ is selected from 5-membered heteroaryl, wherein the 5-membered heteroaryl contains 1-2 heteroatoms selected from N and S, and the 5-membered heteroaryl is unsubstituted or substituted with 1, 2, or 3 $R^b$;

preferably, $R^2$ is selected from pyrazolyl, imidazolyl, thiazolyl, and isothiazolyl, which are unsubstituted or are each independently substituted with 1, 2, or 3 $R^b$; or

preferably, $R^2$ is selected from 5-membered heteroaryl, wherein the 5-membered heteroaryl contains 1, 2, or 3 N heteroatoms, and the 5-membered heteroaryl is unsubstituted or substituted with 1, 2, or 3 identical or different $R^b$;

preferably, $R^2$ is selected from pyrazolyl and imidazolyl, which are unsubstituted or are each independently substituted with 1, 2, or 3 $R^b$;

preferably, $R^2$ is selected from pyrazolyl, wherein the pyrazolyl is independently unsubstituted or substituted with 1, 2, or 3 $R^b$;

preferably, $R^2$ is selected from

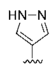,

wherein the

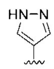

is unsubstituted or independently substituted with 1, 2, or 3 $R^b$;

preferably, $R^2$ is selected from

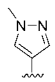 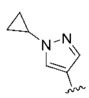 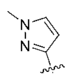 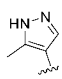 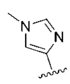 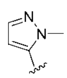 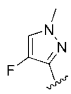 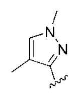

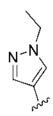 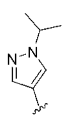 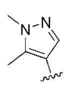 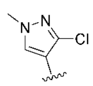 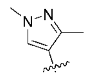 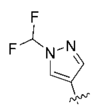 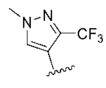 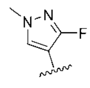;

or

preferably, $R^2$ is selected from 6-membered heteroaryl, wherein the 6-membered heteroaryl contains 1, 2, or 3 heteroatoms selected from N, O, and S, and the 6-membered heteroaryl is unsubstituted or substituted with one or more identical or different $R^b$;

preferably, $R^2$ is selected from 6-membered heteroaryl, wherein the 6-membered heteroaryl contains 1, 2, or 3 N heteroatoms, and the 6-membered heteroaryl is unsubstituted or substituted with 1, 2, or 3 identical or different $R^b$;
preferably, $R^2$ is selected from pyridyl, wherein the pyridyl is unsubstituted or substituted with 1, 2, or 3 identical or different $R^b$;
preferably, $R^2$ is selected from

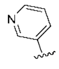,

wherein the

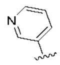

is unsubstituted or substituted with 1, 2, or 3 identical or different $R^b$;
preferably, $R^2$ is selected from

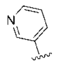 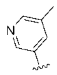 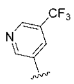 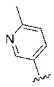 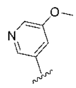 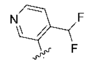 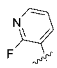 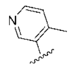 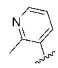;

or
preferably, $R^2$ is selected from 5-6 membered heteroaryl-fused 5-6 membered heterocyclyl, wherein the 5-6 membered heteroaryl and 5-6 membered heterocyclyl each independently contain 1, 2, or 3 heteroatoms selected from N, O, and S, and the 5-6 membered heteroaryl-fused 5-6 membered heterocyclyl is unsubstituted or substituted with one or more identical or different $R^b$;
preferably, $R^2$ is selected from 5-6 membered heteroaryl-fused 5-6 membered heterocyclyl, wherein the 5-6 membered heteroaryl and 5-6 membered heterocyclyl each independently contain 1, 2, or 3 N heteroatoms, and the 5-6 membered heteroaryl-fused 5-6 membered heterocyclyl is unsubstituted or substituted with one or more identical or different $R^b$;
preferably, $R^2$ is selected from 5-membered heteroaryl-fused 6-membered heterocyclyl, wherein the 5-membered heteroaryl and 6-membered heterocyclyl each independently contain 1, 2, or 3 N heteroatoms, and the 5-membered heteroaryl-fused 6-membered heterocyclyl is unsubstituted or substituted with one or more identical or different $R^b$;
preferably, $R^2$ is selected from imidazolyl-fused piperazinyl, wherein the imidazolyl-fused piperazinyl is unsubstituted or substituted with 1, 2, or 3 identical or different $R^b$;
preferably, $R^2$ is selected from

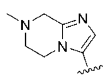;

or
preferably, $R^2$ is selected from 5-6 membered heteroaryl-fused 5-6 membered heterocyclyl, 5-6 membered heteroaryl-fused 5-6 membered heteroaryl, and 5-6 membered heteroaryl-fused phenyl, wherein the 5-6 membered heteroaryl and 5-6 membered heterocyclyl each independently contain 1, 2, or 3 heteroatoms selected from N, O, and S, and the 5-6 membered heteroaryl-fused 5-6 membered heterocyclyl, 5-6 membered heteroaryl-fused 5-6 membered heteroaryl, and 5-6 membered heteroaryl-fused phenyl are unsubstituted or substituted with one or more identical or different $R^b$;
preferably, $R^2$ is selected from 5-6 membered heteroaryl-fused 5-6 membered heterocyclyl, 5-6 membered heteroaryl-fused 5-6 membered heteroaryl, and 5-6 membered heteroaryl-fused phenyl, wherein the 5-6 membered heteroaryl is selected from imidazolyl, pyrazolyl, and pyridyl, the 5-6 membered heterocyclyl is selected from pyrrolidinyl, piperidinyl, and piperazinyl, and the 5-6 membered heteroaryl-fused 5-6 membered heterocyclyl, 5-6 membered heteroaryl-fused 5-6 membered heteroaryl, and 5-6 membered heteroaryl-fused phenyl are unsubstituted or substituted with one or more identical or different $R^b$;
preferably, $R^2$ is selected from imidazopyrrolidinyl, imidazopiperidinyl, imidazopiperazinyl, pyrazolophenyl, and

pyrazolopyridyl, wherein the imidazopyrrolidinyl, imidazopiperidinyl, imidazopiperazinyl, pyrazolophenyl, and pyrazolopyridyl are unsubstituted or substituted with one or more identical or different $R^b$;
preferably, $R^2$ is selected from

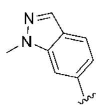 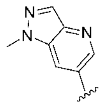 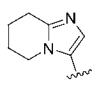 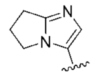 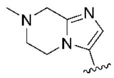;

or
preferably, $R^2$ is selected from 4-6 membered heterocyclyl, wherein the 4-6 membered heterocyclyl contains 1-3 heteroatoms selected from N, O, and S, and the 4-6 membered heterocyclyl is unsubstituted or substituted with 1, 2, or 3 identical or different $R^b$;
preferably, $R^2$ is selected from 4-6 membered heterocycloalkyl and 4-6 membered heterocycloalkenyl, wherein the 4-6 membered heterocycloalkyl and 4-6 membered heterocycloalkenyl each independently contain 1-3 heteroatoms selected from N, O, and S, and the 4-6 membered heterocycloalkyl and 4-6 membered heterocycloalkenyl are unsubstituted or are each independently substituted with 1, 2, or 3 identical or different $R^b$;
preferably, $R^2$ is selected from 4-6 membered heterocycloalkyl and 4-6 membered heterocycloalkenyl, wherein the 4-6 membered heterocycloalkyl and 4-6 membered heterocycloalkenyl each independently contain 1-2 heteroatoms selected from N and O, and the 4-6 membered heterocycloalkyl and 4-6 membered heterocycloalkenyl are unsubstituted or are each independently substituted with 1, 2, or 3 identical or different $R^b$;
preferably, $R^2$ is selected from tetrahydro-2H-pyranyl, oxetanyl, 1,2,3,6-tetrahydropyridyl, and 3,6-dihydro-2H-pyranyl, wherein the tetrahydro-2H-pyranyl, oxetanyl, 1,2,3,6-tetrahydropyridyl, and 3,6-dihydro-2H-pyranyl are unsubstituted or are each independently substituted with 1, 2, or 3 identical or different $R^b$;
preferably, $R^2$ is selected from

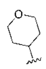 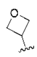 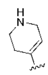 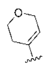,

wherein the

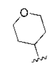 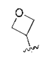 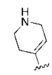 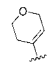

is unsubstituted or independently substituted with 1, 2, or 3 identical or different $R^b$; or
preferably, $R^2$ is selected from

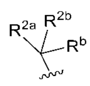,

wherein $R^{2a}$ and $R^{2b}$, together with the carbon atom attached thereto, form a 4-6 membered heterocyclyl, wherein the 4-6 membered heterocyclyl contains 1-3 heteroatoms selected from N, O, and S, and the 4-6 membered heterocyclyl is unsubstituted or independently substituted with 1, 2, or 3 identical or different $R^b$;
preferably, $R^2$ is selected from

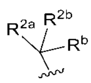,

wherein $R^{2a}$ and $R^{2b}$, together with the carbon atom attached thereto, form a 4-6 membered heterocycloalkyl or 4-6 membered heterocycloalkenyl, wherein the 4-6 membered heterocycloalkyl and 4-6 membered heterocycloalkenyl each independently contain 1-3 heteroatoms selected from N, O, and S, and the 4-6 membered heterocycloalkyl and 4-6 membered heterocycloalkenyl are unsubstituted or are each independently substituted with 1, 2, or 3 identical or different $R^b$;
preferably, $R^2$ is selected from

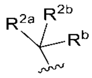,

wherein $R^{2a}$ and $R^{2b}$, together with the carbon atom attached thereto, form a 4-6 membered heterocycloalkyl or 4-6 membered heterocycloalkenyl, wherein the 4-6 membered heterocycloalkyl and 4-6 membered heterocycloalkenyl each independently contain 1-2 heteroatoms selected from N and O, and the 4-6 membered heterocycloalkyl and 4-6 membered heterocycloalkenyl are unsubstituted or are each independently substituted with 1, 2, or 3 identical or different $R^b$;

preferably, $R^2$ is selected from

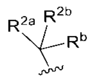,

wherein $R^{2a}$ and $R^{2b}$, together with the carbon atom attached thereto, form tetrahydro-2H-pyranyl, oxetanyl, 1,2,3,6-tetrahydropyridyl, and 3,6-dihydro-2H-pyranyl, wherein the tetrahydro-2H-pyranyl, oxetanyl, 1,2,3,6-tetrahydropyridyl, and 3,6-dihydro-2H-pyranyl are unsubstituted or are each independently substituted with 1, 2, or 3 identical or different $R^b$;

preferably, $R^2$ is selected from

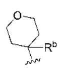 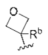 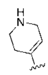 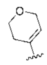,

wherein the group

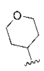 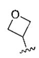 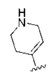 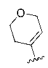

is unsubstituted or independently substituted with 1, 2, or 3 identical or different $R^b$;

preferably, each $R^b$ is independently selected from H, $C_1$-$C_6$ alkyl, $C_1$-$C_6$ alkoxy, and $C_3$-$C_6$ cycloalkyl, wherein the $C_1$-$C_6$ alkyl, $C_1$-$C_6$ alkoxy, and $C_3$-$C_6$ cycloalkyl are unsubstituted or are each independently substituted with 1, 2, 3, or 4 halogens;

preferably, each $R^b$ is independently selected from H, $C_1$-$C_3$ alkyl, $C_1$-$C_3$ alkoxy, and $C_3$-$C_5$ cycloalkyl, wherein the $C_1$-$C_3$ alkyl, $C_1$-$C_3$ alkoxy, and $C_3$-$C_5$ cycloalkyl are unsubstituted or are each independently substituted with 1, 2, 3, or 4 substituents selected from F, Cl, and Br;

preferably, each $R^b$ is independently selected from H, methyl, ethyl, propyl, isopropyl, methoxy, ethoxy, propoxy, isopropoxy, cyclopropyl, cyclobutyl, cyclopentyl, and cyclohexyl;

preferably, each $R^b$ is independently selected from methyl, methoxy, and cyclopropyl;

preferably, each $R^b$ is independently selected from cyclopropyl;

preferably, each $R^b$ is independently selected from methoxy;

preferably, each $R^b$ is independently selected from methyl;

preferably, $R^2$ is selected from

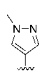 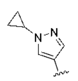 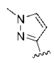 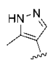 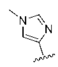 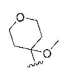 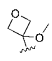 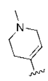 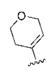;

preferably, $R^2$ is selected from

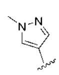;

or

preferably, each $R^b$ is independently selected from H, deuterium, halogen, $C_1$-$C_6$ alkyl, $C_1$-$C_6$ alkoxy, and $C_3$-$C_6$ cycloalkyl, wherein the $C_1$-$C_6$ alkyl, $C_1$-$C_6$ alkoxy, and $C_3$-$C_6$ cycloalkyl are unsubstituted or are each independently substituted with 1, 2, 3, or 4 halogens;

further preferably, each $R^b$ is independently selected from H, deuterium, halogen, $C_1$-$C_3$ alkyl, $C_1$-$C_3$ alkoxy, and $C_3$-$C_5$ cycloalkyl, wherein the $C_1$-$C_3$ alkyl, $C_1$-$C_3$ alkoxy, and $C_3$-$C_5$ cycloalkyl are unsubstituted or are each independently substituted with 1, 2, 3, or 4 halogens;

further preferably, each $R^b$ is independently selected from H, deuterium, F, Cl, Br, methyl, ethyl, propyl, isopropyl, methoxy, ethoxy, propoxy, isopropoxy, cyclopropyl, cyclobutyl, cyclopentyl, and cyclohexyl, wherein the methyl, ethyl, propyl, isopropyl, methoxy, ethoxy, propoxy, isopropoxy, cyclopropyl, cyclobutyl, cyclopentyl, and cyclohexyl are unsubstituted or are each independently substituted with 1, 2, 3, or 4 substituents selected from F, Cl, and Br;

further preferably, each $R^b$ is independently selected from H, deuterium, F, Cl, Br, methyl, ethyl, propyl, isopropyl, methoxy, ethoxy, propoxy, isopropoxy, cyclopropyl, cyclobutyl, cyclopentyl, cyclohexyl, $-CHF_2$, and $-CF_3$;

further preferably, each $R^b$ is independently selected from H, deuterium, F, Cl, methyl, ethyl, isopropyl, methoxy, cyclopropyl, $-CHF_2$, and $-CF_3$;

further preferably, $R^b$ may be further deuterated;

preferably, each $R^b$ is independently selected from H, deuterium, F, Cl, methyl, ethyl, isopropyl, methoxy, cyclopropyl, $-CHF_2$, $-CF_3$, and $-CD_3$;

further preferably, $R^2$ is selected from

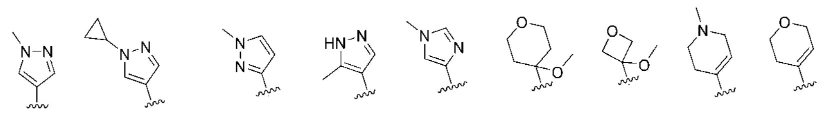

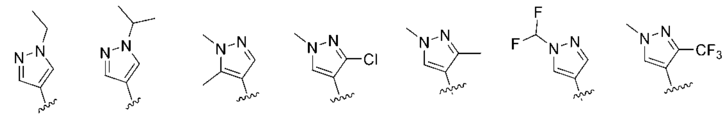

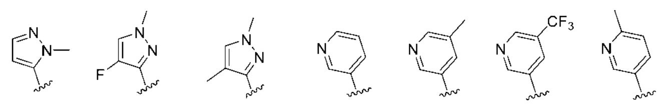

;

or

preferably, $R^2$ is selected from

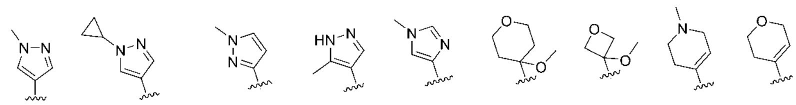

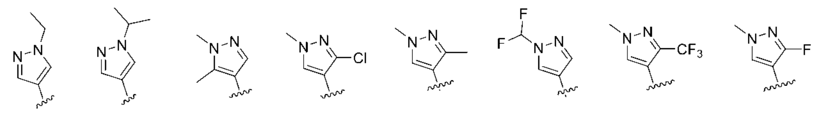

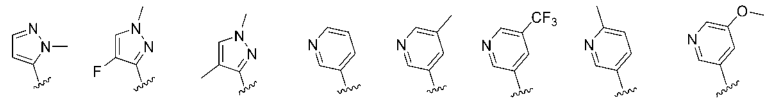

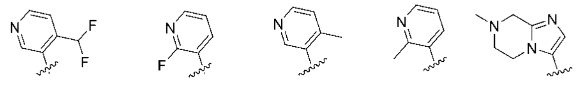

;

or

preferably, $R^2$ is selected from

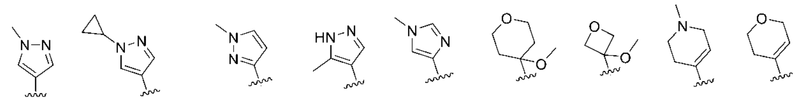

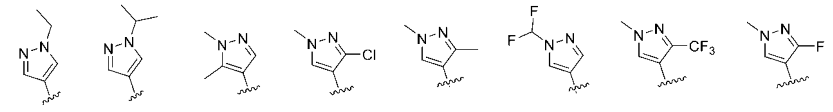

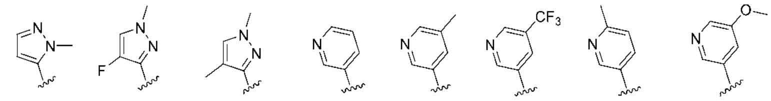

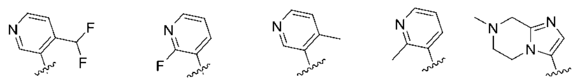

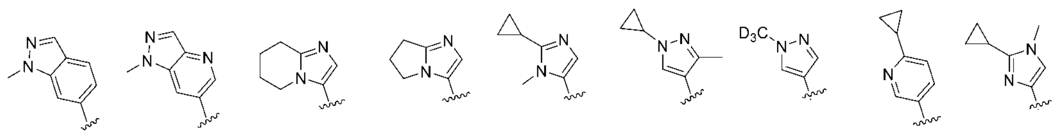

.

**4.** The compound or the tautomer, the stereoisomer, the pharmaceutically acceptable salt, or the deuterated compound thereof according to any one of claims 1 to 3, wherein

$R^3$ is selected from hydrogen, halogen, $C_{1-6}$ alkyl, and $C_{3-6}$ cycloalkyl;
preferably, $R^3$ is selected from hydrogen, F, Cl, Br, $C_{1-4}$ alkyl, $C_{5-6}$ alkyl, and $C_{3-5}$ cycloalkyl;
preferably, $R^3$ is selected from hydrogen, F, Cl, Br, $C_{1-4}$ alkyl, and $C_{3-5}$ cycloalkyl;
preferably, $R^3$ is selected from hydrogen, F, Cl, Br, methyl, ethyl, propyl, isopropyl, butyl, cyclopropyl, cyclobutyl, and cyclopentyl;
preferably, $R^3$ is selected from hydrogen, F, Cl, methyl, ethyl, and cyclopropyl; or
preferably, $R^3$ is selected from hydrogen, F, Cl, Br, and $C_{1-4}$ alkyl;
preferably, $R^3$ is selected from hydrogen, F, Cl, methyl, and ethyl;
preferably, $R^3$ is selected from hydrogen, F, and methyl;
preferably, the group

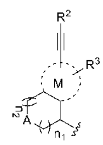

is selected from

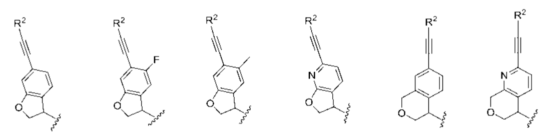

;

preferably, the group

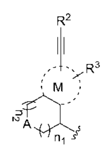

is selected from

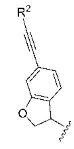;

preferably, the group

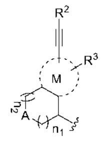

is selected from

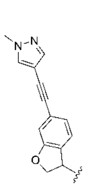 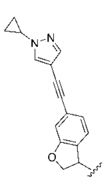 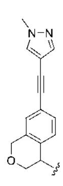 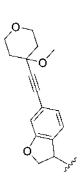 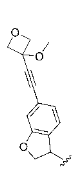 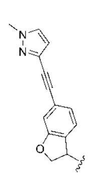 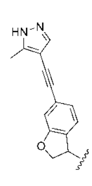 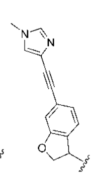 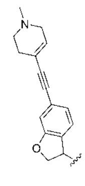 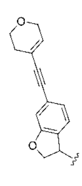;

preferably, the group

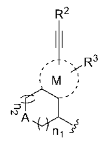

is selected from

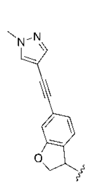 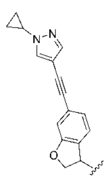 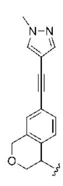 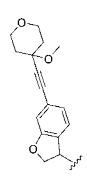 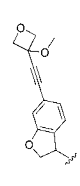 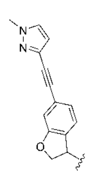 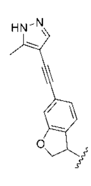 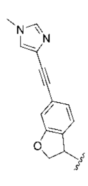 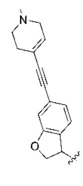 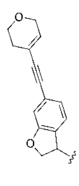

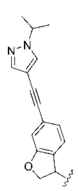 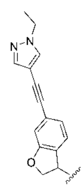 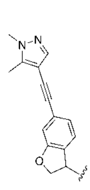 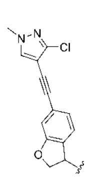 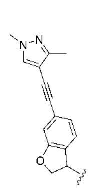 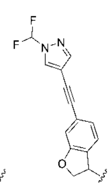 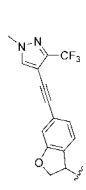 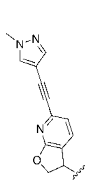

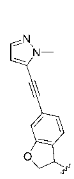 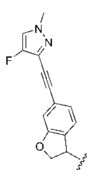 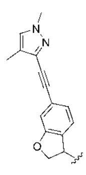 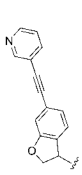 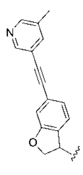 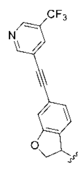 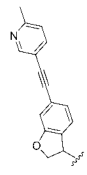;

or

preferably, the group

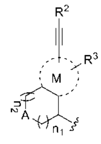

is selected from

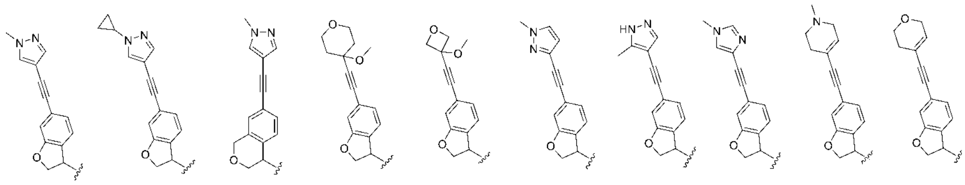

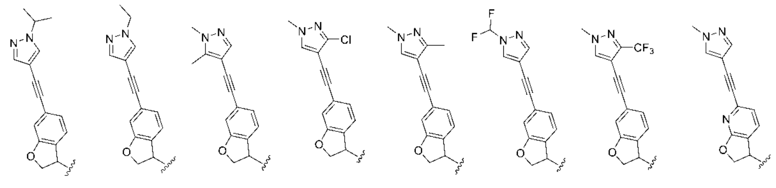

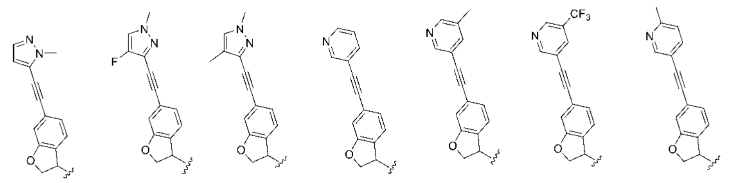

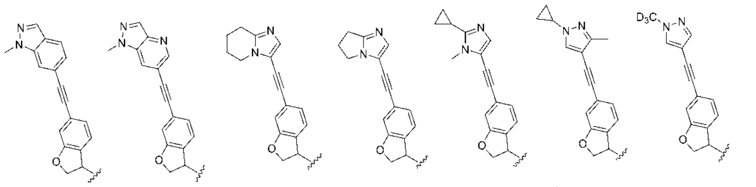

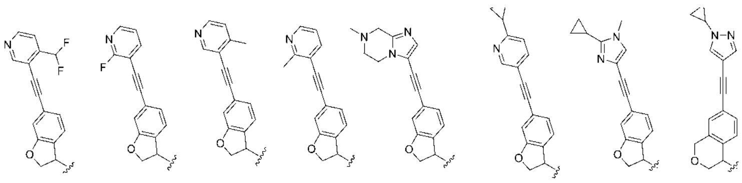
;

preferably, the group

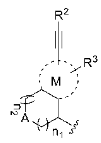

is selected from

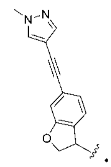.

**5.** The compound or the tautomer, the stereoisomer, the pharmaceutically acceptable salt, or the deuterated compound thereof according to claim 1, wherein

the group

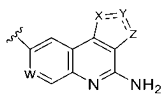

is selected from

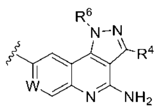 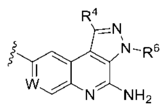 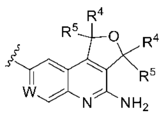 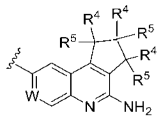 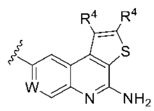,

wherein W is selected from N and $CR^7$;
preferably, $R^4$, $R^5$, $R^6$, and $R^7$ are each independently selected from hydrogen, halogen, and $C_{1-6}$ alkyl;
preferably, $R^4$, $R^5$, $R^6$, and $R^7$ are each independently selected from hydrogen, halogen, $C_{1-4}$ alkyl, and $C_{5-6}$ alkyl;
preferably, $R^4$, $R^5$, $R^6$, and $R^7$ are each independently selected from hydrogen, halogen, and $C_{1-4}$ alkyl;
preferably, $R^4$, $R^5$, $R^6$, and $R^7$ are each independently selected from hydrogen, F, Cl, Br, methyl, ethyl, propyl, and isopropyl;
preferably, $R^4$, $R^5$, $R^6$, and $R^7$ are each independently selected from hydrogen, F, Cl, and methyl;
further preferably, $R^4$, $R^5$, $R^6$, and $R^7$ described above may be further deuterated;
preferably, $R^4$, $R^5$, $R^6$, and $R^7$ are each independently selected from hydrogen, F, Cl, methyl, and $-CD_3$;
preferably, the group

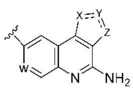

is selected from

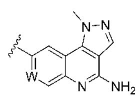 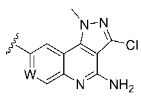 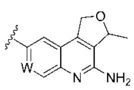 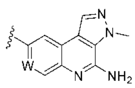

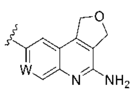 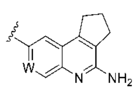 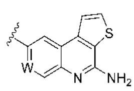,

wherein W is selected from N and $CR^7$; and $R^7$ is selected from H, F, Cl, and methyl;
preferably, the group

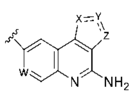

is selected from:

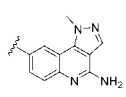 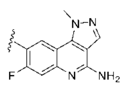 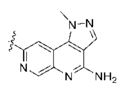 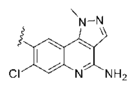 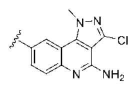 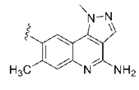

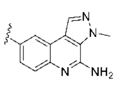 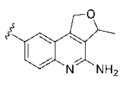 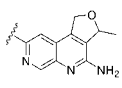 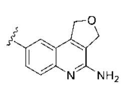 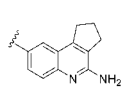 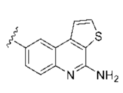 ;

preferably, the group

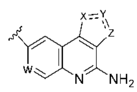

is selected from

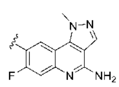 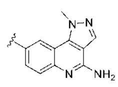 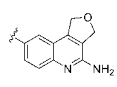 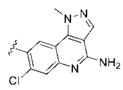 ;

or

preferably, the group

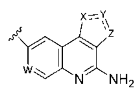

is selected from

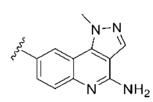 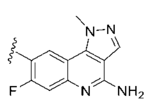 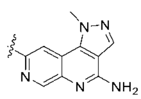 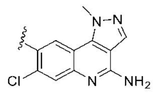 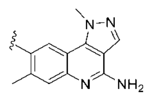

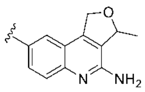 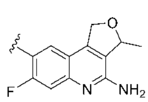 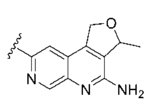 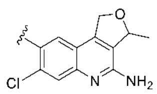 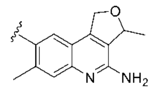

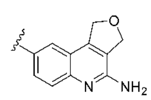 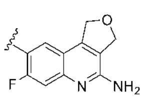 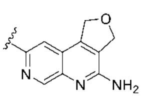 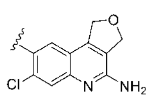 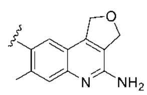

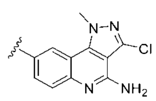 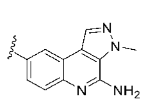 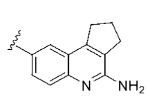 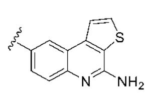 ;

preferably, the group

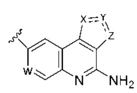

is selected from

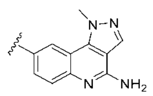 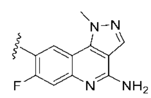 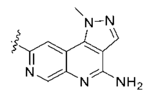 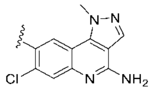 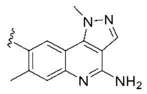

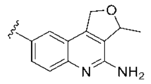 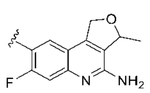 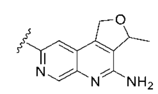 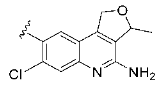 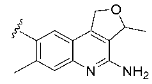

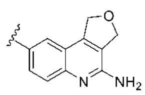 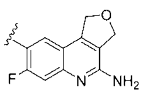 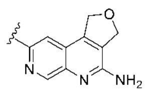 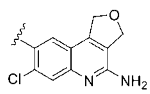 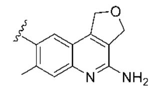 ;

preferably, the group

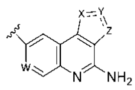

is selected from

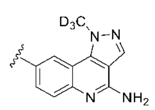 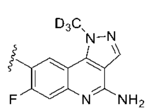 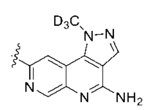 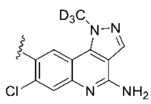 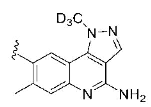

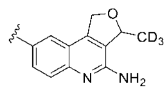 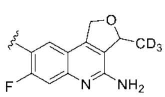 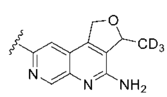 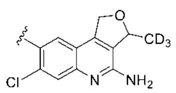 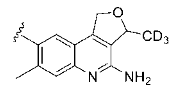 .

**6.** The compound or the tautomer, the stereoisomer, the pharmaceutically acceptable salt, or the deuterated compound thereof according to claim 1, wherein

$R^1$ is selected from $C_{1-6}$ alkyl, $C_{3-10}$ cycloalkyl, $C_{3-10}$ cycloalkyl-$C_{1-6}$ alkyl-, and 5-10 membered heteroaryl-, wherein the $C_1$-$C_6$ alkyl is unsubstituted or substituted with one or more $R_i$, the $C_3$-$C_{10}$ cycloalkyl is unsubstituted or substituted with one or more $R_j$, and the 5-10 membered heteroaryl is unsubstituted or substituted with one or more $R_l$;

preferably, $R^1$ is selected from $C_{1-4}$ alkyl, $C_{3-6}$ monocycloalkyl, $C_{5-8}$ bridged cycloalkyl, $C_{5-8}$ spirocycloalkyl, $C_{3-6}$ monocycloalkyl-$C_{1-4}$ alkyl-, $C_{5-8}$ bridged cycloalkyl-$C_{1-4}$ alkyl-, $C_{5-8}$ spirocycloalkyl-$C_{1-4}$ alkyl-, and 5-6 membered heteroaryl, wherein the $C_{1-4}$ alkyl is unsubstituted or substituted with one or more $R_i$, the $C_{3-6}$ monocycloalkyl, $C_{5-8}$ bridged cycloalkyl, and $C_{5-8}$ spirocycloalkyl are unsubstituted or substituted with one or more $R_j$, and the 5-10 membered heteroaryl is unsubstituted or substituted with one or more $R_l$;

preferably, $R^1$ is selected from methyl, ethyl, propyl, butyl, cyclopropyl, cyclobutyl, cyclopentyl, cyclohexyl,

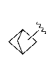 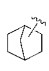 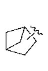 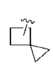 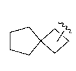 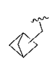 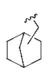 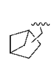 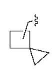 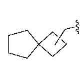 ,

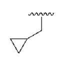 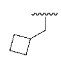 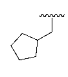 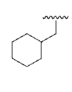 ,

pyridyl, pyrazinyl, pyrimidinyl, pyridazinyl, pyrazolyl, imidazolyl, triazolyl, pyrrolyl, furanyl, oxazolyl, isoxazolyl, and thiazolyl, wherein the methyl, ethyl, propyl, and butyl are unsubstituted or substituted with one or more $R_i$, the cyclopropyl, cyclobutyl, cyclopentyl, cyclohexyl,

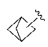 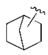 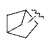 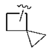 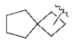 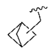 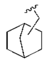 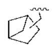 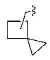 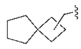, and 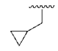 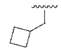 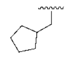 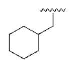

are unsubstituted or substituted with one or more $R_j$, and the pyridyl, pyrazinyl, pyrimidinyl, pyridazinyl, pyrazolyl, imidazolyl, triazolyl, pyrrolyl, furanyl, oxazolyl, isoxazolyl, and thiazolyl are unsubstituted or substituted with one or more $R_l$;

preferably, $R^1$ is selected from methyl, ethyl, cyclopropyl, cyclobutyl, cyclopentyl,

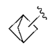 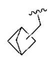, 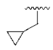 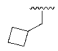 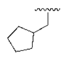,

pyrazolyl, imidazolyl, triazolyl, and pyrrolyl, wherein the methyl and ethyl are unsubstituted or substituted with 1, 2, or 3 $R_i$, the cyclopropyl, cyclobutyl, cyclopentyl,

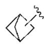 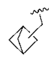, and 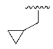 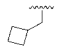 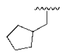

are unsubstituted or substituted with 1, 2, or 3 $R_j$, and the pyrazolyl, imidazolyl, triazolyl, and pyrrolyl are unsubstituted or substituted with 1, 2, or 3 $R_l$;

preferably, $R^1$ is selected from methyl, ethyl, cyclopropyl,

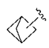 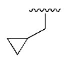,

and pyrazolyl, wherein the methyl and ethyl are unsubstituted or substituted with 1, 2, or 3 $R_i$, the cyclopropyl and

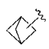 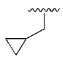

are unsubstituted or substituted with 1, 2, or 3 $R_j$, and the pyrazolyl is unsubstituted or substituted with 1, 2, or 3 $R_l$;

preferably, $R^1$ is selected from methyl, ethyl, cyclopropyl,

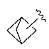 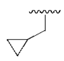,

and pyrazolyl, wherein the methyl, ethyl, cyclopropyl, and

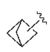 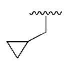

are unsubstituted, and the pyrazolyl is unsubstituted or substituted with 1, 2, or 3 $R_l$;

preferably, each $R_l$ is independently selected from halogen, halogenated $C_{1-6}$ alkyl, $C_{1-6}$ alkyl, and $C_{3-6}$ cycloalkyl;

preferably, each $R_l$ is independently selected from halogen, halogenated $C_{1-4}$ alkyl, $C_{1-4}$ alkyl, and $C_{3-6}$ cycloalkyl;

preferably, each $R_l$ is independently selected from halogen, $C_{1-4}$ alkyl, and $C_{3-6}$ cycloalkyl;

preferably, each $R_l$ is independently selected from F, Cl, Br, methyl, ethyl, propyl, isopropyl, cyclopropyl, and cyclobutyl;

preferably, each $R_l$ is independently selected from F, Cl, methyl, and ethyl;

preferably, each $R_l$ is independently selected from methyl;

preferably, each $R_l$ is independently selected from D, F, Cl, methyl, and ethyl;

preferably, $R^1$ is selected from methyl, ethyl, cyclopropyl, and

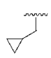 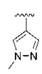 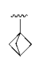;

preferably, $R^1$ is selected from methyl, ethyl, and cyclopropyl;

preferably, $R^1$ is selected from methyl;

preferably, $R^1$ is selected from $-CD_3$.

7. The compound or the tautomer, the stereoisomer, the pharmaceutically acceptable salt, or the deuterated compound thereof according to any one of claims 1 to 6, wherein formula (I) has a structure shown as formula IA:

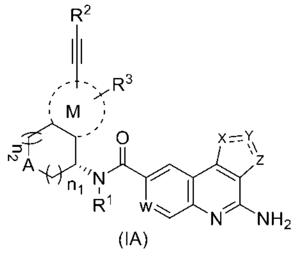

(IA) ,

wherein $R^1$, $R^2$, $R^3$, A, $n_1$, $n_2$, ring M, W, X, Y, Z, and $\overline{---}$ are each as defined in formula I; or formula (I) has a structure shown as formula IB:

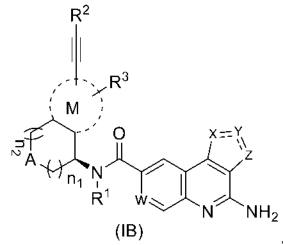

(IB) ,

wherein $R^1$, $R^2$, $R^3$, A, $n_1$, $n_2$, ring M, W, X, Y, Z and $\overline{---}$ are each as defined in formula I.

8. The compound or the tautomer, the stereoisomer, the pharmaceutically acceptable salt, or the deuterated compound thereof according to any one of claims 1 to 7, wherein the compound represented by formula (I) is selected from: compound 1, compound 2, compound 3, compound 4, compound 5, compound 6, compound 7, compound 8, compound 9, compound 10, compound 11, compound 12, compound 13, compound 14, compound 15, compound 16, compound 17, compound 18, compound 19, compound 20, compound 21, compound 22, compound 23, compound 24, compound 25, compound 26, compound 27, compound 28, compound 29, compound 30, compound 31, compound 32, compound 33, compound 34, compound 35, compound 36, compound 37, compound 38, compound 39, compound 40, compound 41, compound 42, compound 43, compound 44, compound 45, compound 46, compound 47, compound 48, compound 49, compound 50, compound 51, compound 52, compound 53, compound 54, compound 55, compound 56, compound 57, compound 58, compound 59, compound 60, compound 61, compound 62, compound 63, compound 64, compound 65, compound 66, compound 67, compound 68, compound 69, compound 70, compound 71, compound 72, compound 73, compound 74, compound 75, compound 76, compound 77, compound 78, compound 79, compound 80, compound 81, compound 82, compound 83, compound 84, compound 85, compound 86, compound 87, compound 88, compound 89, compound 90, compound 91, compound 93, compound 94, compound 95, compound 98, compound 100, compound 101, compound 103, compound 104, compound 105, compound 106, compound 107, compound 108, compound 113, compound 114, compound 115, compound 116, compound 117, compound 118, compound 119, compound 120, compound 121, compound 122, compound 123, compound 124, compound 125, compound 126, compound 127, compound 128, compound 129, compound 130, compound 131, compound 132, compound 133, compound 134, compound 135, compound 136, compound 137, compound 138, compound 139, compound 140, compound 141, compound 142, compound 143, compound 144, compound 145, compound 146, compound 147, compound 148, compound 149, compound 150, compound 151, compound 152, compound 153, compound 154, compound 155, compound 156, compound 157, and an S-configuration of any of the compounds described above.

9. A pharmaceutical composition for treating and/or preventing a disease associated with abnormal PRMT5 expression, comprising a therapeutically and/or prophylactically effective amount of the compound or the tautomer, the stereoisomer, the pharmaceutically acceptable salt, or the deuterated compound thereof according to any one of claims 1 to 8, and an optional pharmaceutically acceptable excipient;

preferably, the disease associated with abnormal PRMT5 expression is a tumor or cancer;
preferably, the disease associated with abnormal PRMT5 expression is a disease associated with abnormal PRMT5 expression and **characterized by** MTAP deletion;
preferably, the disease associated with abnormal PRMT5 expression is a tumor or cancer **characterized by** MTAP deletion.

**10.** Use of the compound or the tautomer, the stereoisomer, the pharmaceutically acceptable salt, or the deuterated compound thereof according to any one of claims 1 to 8, or the pharmaceutical composition according to claim 9 in manufacture of a medicament for treating and/or preventing a disease associated with abnormal PRMT5 expression and/or in treating and/or preventing a disease associated with abnormal PRMT5 expression;

preferably, the disease associated with abnormal PRMT5 expression is a tumor or cancer;
preferably, the disease associated with abnormal PRMT5 expression is a disease associated with abnormal PRMT5 expression and **characterized by** MTAP deletion;
preferably, the disease associated with abnormal PRMT5 expression is a tumor or cancer **characterized by** MTAP deletion.

**INTERNATIONAL SEARCH REPORT**

International application No.
**PCT/CN2024/109119**

**A. CLASSIFICATION OF SUBJECT MATTER**

C07D 471/04(2006.01)i; C07D 519/00(2006.01)i; C07D 405/12(2006.01)i; A61K 31/4709(2006.01)i; A61K 31/437(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNABS, CNTXT, WPABS, REGISTRY(STN), CAPLUS(STN): 微芯药业有限公司, 酰胺, 三杂环, 炔, 蛋白质精氨酸甲基转移酶, 癌症, 肿瘤, PRMT5, amide, alkynyl, cancer, tumor, tomour, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2024037459 A1 (MEDSHINE DISCOVERY INC.) 22 February 2024 (2024-02-22) claims 1-15 | 1-7, 9, 10 |
| A | WO 2022169948 A1 (AMGEN INC.) 11 August 2022 (2022-08-11) claims 1-27, and description, paragraphs [0005]-[0031] and [0252]-[0276] | 1-10 |
| A | WO 2021163344 A1 (AMGEN INC.) 19 August 2021 (2021-08-19) abstract, claim 1, and description, embodiments | 1-10 |
| A | WO 2022132914 A1 (AMGEN INC.) 23 June 2022 (2022-06-23) abstract, claim 1, and description, embodiments | 1-10 |
| A | WO 2022115377 A1 (AMGEN INC.) 02 June 2022 (2022-06-02) claim 1, and description, page 297, table, compound 878 | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 October 2024** | **12 October 2024** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/109119**

| Patent document cited in search report | Publication date (day/month/year) | Patent family member(s) | Publication date (day/month/year) |
|---|---|---|---|
| WO 2024037459 A1 | 22 February 2024 | None | |
| WO 2022169948 A1 | 11 August 2022 | CA 3210332 A1 | 11 August 2022 |
| | | AU 2022217791 A1 | 17 August 2023 |
| | | AU 2022217791 A9 | 11 July 2024 |
| | | US 2024124442 A1 | 18 April 2024 |
| | | JP 2024508235 A | 26 February 2024 |
| | | EP 4288435 A1 | 13 December 2023 |
| | | MX 2023009222 A | 11 September 2023 |
| WO 2021163344 A1 | 19 August 2021 | AU 2021219730 A1 | 25 August 2022 |
| | | JP 2023513580 A | 31 March 2023 |
| | | EP 4103558 A1 | 21 December 2022 |
| | | MX 2022010011 A | 18 October 2022 |
| | | US 2023159510 A1 | 25 May 2023 |
| | | CA 3170321 A1 | 19 August 2021 |
| WO 2022132914 A1 | 23 June 2022 | CL 2023001738 A1 | 17 November 2023 |
| | | AR 124369 A1 | 22 March 2023 |
| | | PE 20231295 A1 | 22 August 2023 |
| | | KR 20230121820 A | 21 August 2023 |
| | | MX 2023007192 A | 03 July 2023 |
| | | US 2022194955 A1 | 23 June 2022 |
| | | US 11845760 B2 | 19 December 2023 |
| | | CO 2023008167 A2 | 21 July 2023 |
| | | CR 20230310 A | 01 September 2023 |
| | | CA 3204823 A1 | 23 June 2022 |
| | | TW 202233183 A | 01 September 2022 |
| | | US 2024092794 A1 | 21 March 2024 |
| | | JP 2023550530 A | 01 December 2023 |
| | | JP 7487421 B2 | 20 May 2024 |
| | | UY 39565 A | 30 June 2022 |
| | | EP 4263545 A1 | 25 October 2023 |
| | | JP 2024102236 A | 30 July 2024 |
| | | IL 303451 A | 01 August 2023 |
| | | AU 2021400942 A1 | 06 July 2023 |
| WO 2022115377 A1 | 02 June 2022 | EP 4251624 A1 | 04 October 2023 |
| | | CO 2023007504 A2 | 08 September 2023 |
| | | KR 20230113339 A | 28 July 2023 |
| | | CL 2023001461 A1 | 01 December 2023 |
| | | AU 2021386149 A1 | 22 June 2023 |
| | | AU 2021386149 A9 | 08 August 2024 |
| | | JP 2023550466 A | 01 December 2023 |
| | | PE 20231312 A1 | 24 August 2023 |
| | | US 2024101570 A1 | 28 March 2024 |
| | | IL 302820 A | 01 July 2023 |
| | | CA 3202141 A1 | 02 June 2022 |
| | | MX 2023006145 A | 06 June 2023 |
| | | CR 20230286 A | 24 August 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022169948 A1
- WO 2024131901 A1
- WO 2023044171 A1
- WO 2022132914 A1


### Non-patent literature cited in the description


- *Cell Mol. Life Sci.*, 2015, vol. 72 (11), 2041-2059
- *Cell Reports*, 2016, vol. 15, 574-587
- *Science*, 2016, vol. 351, 1214-1217
- *Journal of Clinical Investigation*, 2015, vol. 125 (9), 3532-44
- REMINGTON'S PHARMACEUTICAL SCIENCES. Mack Publishing Company, 1995
- *J. Med. Chem.*, 2022, vol. 65 (3), 1749-1766
- **DAVIES B** ; **MORRIS T**. Physiological parameters in laboratory animals and humans. *Pharm Res*, 1993, vol. 10, 1093-5